# EUROPEAN PATENT APPLICATION

(11) **EP 1 291 440 A1**
(43) Date of publication of application: **12.03.2003**
(21) Application number: 01934502.4
(22) Date of filing: 01.06.2001
(51) Int. Cl.: C12Q 1/68, C12M 1/00, C12N 15/09, G01N 33/53

(54) **KIT AND METHOD FOR DETERMINING HLA TYPE**

(30) Priority: 01.06.2000 JP 2000164798
(71) Applicant: Nisshinbo Industries, Inc., Chuo-ku, Tokyo 103-0013 (JP)
(72) Inventor: INOKO, Hidetoshi, Yokohama-shi, Kanagawa 245-0002 (JP); KAGIYA, Taeko, Naka-gun, Kanagawa 255-0003 (JP); ICHIHARA, Tatsuo, c/o NISSHINBO INDUSTRIES, INC., Chiba-shi, Chiba 267-0056 (JP); MATSUMURA, Yoshiyuki, c/o NISSHINBO INDUSTRIES INC, Chiba-shi, Chiba 267-0056 (JP); MORIYA, Shogo, c/o NISSHINBO INDUSTRIES, INC., Chiba-shi, Chiba 267-0056 (JP); NISHIDA, Michio, Chigasaki-shi, Kanagawa 253-0012 (JP)
(74) Representative: Tombling, Adrian George
(86) International application number: JP0104662
(87) International publication number: WO01092572

(57) **Abstract**

HLA genotype of a test specimen is determined by hybridization of a nucleic acid sequence derived from the test specimen by using a substrate on which oligonucleotides of 10-24 nucleotide length derived from sequences of a group of genes belonging to HLA class I or class II antigen on a human genome and including polymorphism of gene as alloantigens in the sequences are immobilized through covalent bonds. There are provided a typing kit and typing method that are suitable for processing of a large number of specimens and enable high accuracy typing by one test.

SEQUENCE LISTING

<110> NISSHINBO INDUSTRIES. INC.
      SYSTEM RESEARCH INC.

<120> KIT AND METHOD FOR DETERMINING HLA TYPES

<130> 13140P1174

<140>
<141> 2001-06-01

<150> JP 2000-164798
<151> 2000-06-01

<160> 1298

<170> PatentIn Ver. 2.1

<210> 1
<211> 18
<212> DNA
<213> Artificial Sequence

<220>
<223> Description of Artificial Sequence:capture

<400> 1
ctgaccacgt tgcctctt                                                18

<210> 2
<211> 18
<212> DNA
<213> Artificial Sequence

<220>
<223> Description of Artificial Sequencc:capture

<400> 2
ctgaccatgt tgcctctt                                                18

<210> 3
<211> 18
<212> DNA
<213> Artificial Sequence

<220>
<223> Description of Artificial Sequence:capture

<400> 3
ctgaccacgt cgcctctt                                                18

<210> 4
<211> 18

<212> DNA
<213> Artificial Sequence

<220>
<223> Description of Artificial Sequence:capture

<400> 4
gcctcttgtg gtgtaaac                                                    18

<210> 5
<211> 18
<212> DNA
<213> Artificial Sequence

<220>
<223> Description of Artificial Sequence:capture

<400> 5
gcctcttacg gtgtaaac                                                    18

<210> 6
<211> 18
<212> DNA
<213> Artificial Sequence

<220>
<223> Description of Artificial Sequence:capture

<400> 6
gcctcttatg gtgtaaac                                                    18

<210> 7
<211> 16
<212> DNA
<213> Artificial Sequence

<220>
<223> Description of Artificial Sequence:capture

<400> 7
ccagttttac ggtccc                                                      16

<210> 8
<211> 16
<212> DNA
<213> Artificial Sequence

<220>
<223> Description of Artificial Sequence:capture

<400> 8
ccagtcttac ggtccc                                                      16

<210> 9
<211> 16
<212> DNA
<213> Artificial Sequence

<220>
<223> Description of Artificial Sequence:capture

<400> 9
ccagtcttat ggtccc                                                    16

<210> 10
<211> 18
<212> DNA
<213> Artificial Sequence

<220>
<223> Description of Artificial Sequence:capture

<400> 10
ttacggtccc tctggcca                                                  18

<210> 11
<211> 18
<212> DNA
<213> Artificial Sequence

<220>
<223> Description of Artificial Sequence:capture

<400> 11
ttacggtctc tctggcca                                                  18

<210> 12
<211> 17
<212> DNA
<213> Artificial Sequence

<220>
<223> Description of Artificial Sequence:capture

<400> 12
cctctggcca gtacacc                                                   17

<210> 13
<211> 17
<212> DNA
<213> Artificial Sequence

<220>
<223> Description of Artificial Sequence:capture

<400> 13
cctctgggca gtacacc                                                   17

<210> 14
<211> 16
<212> DNA
<213> Artificial Sequence

<220>
<223> Description of Artificial Sequence:capture

<400> 14
ggccagtaca cccatg                                                        16

<210> 15
<211> 16
<212> DNA
<213> Artificial Sequence

<220>
<223> Description of Artificial Sequence:capture

<400> 15
ggccagttca cccatg                                                        16

<210> 16
<211> 18
<212> DNA
<213> Artificial Sequence

<220>
<223> Description of Artificial Sequence:capture

<400> 16
cagtacaccc atgaattt                                                      18

<210> 17
<211> 18
<212> DNA
<213> Artificial Sequence

<220>
<223> Description of Artificial Sequence:capture

<400> 17
cagtacagcc atgaattt                                                      18

<210> 18
<211> 18
<212> DNA
<213> Artificial Sequence

<220>
<223> Description of Artificial Sequence:capture

<400> 18
atggagatga ggagttct                                                      18

<210> 19
<211> 18
<212> DNA
<213> Artificial Sequence

<220>
<223> Description of Artificial Sequence:capture

<400> 19
atggagacga ggagttct                                                      18

<210> 20
<211> 18
<212> DNA
<213> Artificial Sequence

<220>
<223> Description of Artificial Sequence:capture

<400> 20
atggagatga gcagttct                                                    18

<210> 21
<211> 18
<212> DNA
<213> Artificial Sequence

<220>
<223> Description of Artificial Sequence:capture

<400> 21
atggagacga gcagttct                                                    18

<210> 22
<211> 18
<212> DNA
<213> Artificial Sequence

<220>
<223> Description of Artificial Sequence:capture

<400> 22
agttctacgt ggacctgg                                                    18

<210> 23
<211> 18
<212> DNA
<213> Artificial Sequence

<220>
<223> Description of Artificial Sequence:capture

<400> 23
agttctatgt ggacctgg                                                    18

<210> 24
<211> 15
<212> DNA
<213> Artificial Sequence

<220>
<223> Description of Artificial Sequence:capture

<400> 24
ctggagagga aggag                                                       15

<210> 25
<211> 15
<212> DNA

<213> Artificial Sequence

<220>
<223> Description of Artificial Sequence:capture

<400> 25
ctggggagga aggag                                                    15

<210> 26
<211> 15
<212> DNA
<213> Artificial Sequence

<220>
<223> Description of Artificial Sequence:capture

<400> 26
ctggagaaga aggag                                                    15

<210> 27
<211> 17
<212> DNA
<213> Artificial Sequence

<220>
<223> Description of Artificial Sequence:capture

<400> 27
agactgcctg gcggtgg                                                  17

<210> 28
<211> 17
<212> DNA
<213> Artificial Sequence

<220>
<223> Description of Artificial Sequence:capture

<400> 28
agactgtctg gaagttg                                                  17

<210> 29
<211> 17
<212> DNA
<213> Artificial Sequence

<220>
<223> Description of Artificial Sequence:capture

<400> 29
agactgtctg gcagttg                                                  17

<210> 30
<211> 18
<212> DNA
<213> Artificial Sequence

<220>

<223> Description of Artificial Sequence:capture

<400> 30
actgtctggt gtttgcct                                                    18

<210> 31
<211> 18
<212> DNA
<213> Artificial Sequence

<220>
<223> Description of Artificial Sequence:capture

<400> 31
gagttcagca aatttgga                                                    18

<210> 32
<211> 18
<212> DNA
<213> Artificial Sequence

<220>
<223> Description of Artificial Sequence:capture

<400> 32
ctgttccaca gacttaga                                                    18

<210> 33
<211> 18
<212> DNA
<213> Artificial Sequence

<220>
<223> Description of Artificial Sequence:capture

<400> 33
ctgttccgca gatttaga                                                    18

<210> 34
<211> 18
<212> DNA
<213> Artificial Sequence

<220>
<223> Description of Artificial Sequence:capture

<400> 34
gttctcagac aatttaga                                                    18

<210> 35
<211> 18
<212> DNA
<213> Artificial Sequence

<220>
<223> Description of Artificial Sequence:capture

<400> 35

aaatttggag gttttgac                                                    18

<210> 36
<211> 18
<212> DNA
<213> Artificial Sequence

<220>
<223> Description of Artificial Sequence:capture

<400> 36
agatttagaa gatttgac                                                    18

<210> 37
<211> 18
<212> DNA
<213> Artificial Sequence

<220>
<223> Description of Artificial Sequence:capture

<400> 37
cacagactta gatttgac                                                    18

<210> 38
<211> 18
<212> DNA
<213> Artificial Sequence

<220>
<223> Description of Artificial Sequence:capture

<400> 38
agacaattta gatttgac                                                    18

<210> 39
<211> 18
<212> DNA
<213> Artificial Sequence

<220>
<223> Description of Artificial Sequence:capture

<400> 39
tttgacccgc agggtgca                                                    18

<210> 40
<211> 18
<212> DNA
<213> Artificial Sequence

<220>
<223> Description of Artificial Sequence:capture

<400> 40
tttgaccggc aatttgca                                                    18

<210> 41
<211> 18
<212> DNA
<213> Artificial Sequence

<220>
<223> Description of Artificial Sequence:capture

<400> 41
tttgacccgc aatttgca                                                    18

<210> 42
<211> 18
<212> DNA
<213> Artificial Sequence

<220>
<223> Description of Artificial Sequence:capture

<400> 42
catggctgtg gcaaaaca                                                    18

<210> 43
<211> 18
<212> DNA
<213> Artificial Sequence

<220>
<223> Description of Artificial Sequence:capture

<400> 43
catcgctgtg ctaaaaca                                                    18

<210> 44
<211> 18
<212> DNA
<213> Artificial Sequence

<220>
<223> Description of Artificial Sequence:capture

<400> 44
catcgctgtg acaaaaca                                                    18

<210> 45
<211> 18
<212> DNA
<213> Artificial Sequence

<220>
<223> Description of Artificial Sequence:capture

<400> 45
catcgctgtc ctaaaaca                                                    18

<210> 46
<211> 18
<212> DNA

<213> Artificial Sequence

<220>
<223> Description of Artificial Sequence:capture

<400> 46
catcgccgtg acaaaaca                                                    18

<210> 47
<211> 18
<212> DNA
<213> Artificial Sequence

<220>
<223> Description of Artificial Sequence:capture

<400> 47
acttgaacat catgatta                                                    18

<210> 48
<211> 18
<212> DNA
<213> Artificial Sequence

<220>
<223> Description of Artificial Sequence:capture

<400> 48
acttgaacat cctgatta                                                    18

<210> 49
<211> 18
<212> DNA
<213> Artificial Sequence

<220>
<223> Description of Artificial Sequence:capture

<400> 49
acttgaacat cgtgatta                                                    18

<210> 50
<211> 18
<212> DNA
<213> Artificial Sequence

<220>
<223> Description of Artificial Sequence:capture

<400> 50
acttgaacag tctgatta                                                    18

<210> 51
<211> 18
<212> DNA
<213> Artificial Sequence

<220>

<223> Description of Artificial Sequence:capture

<400> 51
ttaaacgcta caactcta                                                    18

<210> 52
<211> 18
<212> DNA
<213> Artificial Sequence

<220>
<223> Description of Artificial Sequence:capture

<400> 52
ttaaacgctc caactcta                                                    18

<210> 53
<211> 18
<212> DNA
<213> Artificial Sequence

<220>
<223> Description of Artificial Sequence:capture

<400> 53
aactctaccg ctgctacc                                                    18

<210> 54
<211> 18
<212> DNA
<213> Artificial Sequence

<220>
<223> Description of Artificial Sequence:capture

<400> 54
aactctactg ctgctacc                                                    18

<210> 55
<211> 18
<212> DNA
<213> Artificial Sequence

<220>
<223> Description of Artificial Sequence:capture

<400> 55
gcgggatctt gcagagga                                                    18

<210> 56
<211> 18
<212> DNA
<213> Artificial Sequence

<220>
<223> Description of Artificial Sequence:capture

<400> 56

gcgggatcct gcagagga                                                    18

<210> 57
<211> 18
<212> DNA
<213> Artificial Sequence

<220>
<223> Description of Artificial Sequence:capture

<400> 57
gcacgacctt gcagcggc                                                    18

<210> 58
<211> 18
<212> DNA
<213> Artificial Sequence

<220>
<223> Description of Artificial Sequence:capture

<400> 58
ggagaggaaa cgggcggc                                                    18

<210> 59
<211> 18
<212> DNA
<213> Artificial Sequence

<220>
<223> Description of Artificial Sequence:capture

<400> 59
tcggaatggc caggagga                                                    18

<210> 60
<211> 18
<212> DNA
<213> Artificial Sequence

<220>
<223> Description of Artificial Sequence:capture

<400> 60
ggagacagct ggcgttgt                                                    18

<210> 61
<211> 18
<212> DNA
<213> Artificial Sequence

<220>
<223> Description of Artificial Sequence:capture

<400> 61
attaggaatg gtgactgg                                                    18

<210> 62

<211> 18
<212> DNA
<213> Artificial Sequence

<220>
<223> Description of Artificial Sequence:capture

<400> 62
atttcgtgta ccagttta                                                    18

<210> 63
<211> 18
<212> DNA
<213> Artificial Sequence

<220>
<223> Description of Artificial Sequence:capture

<400> 63
atttcgtgct ccagttta                                                    18

<210> 64
<211> 18
<212> DNA
<213> Artificial Sequence

<220>
<223> Description of Artificial Sequence:capture

<400> 64
atttcgtgtt ccagttta                                                    18

<210> 65
<211> 18
<212> DNA
<213> Artificial Sequence

<220>
<223> Description of Artificial Sequence:capture

<400> 65
tttaagggcc tgtgctac                                                    18

<210> 66
<211> 18
<212> DNA
<213> Artificial Sequence

<220>
<223> Description of Artificial Sequence:capture

<400> 66
tttaaggcca tgtgctac                                                    18

<210> 67
<211> 18
<212> DNA
<213> Artificial Sequence

<220>
<223> Description of Artificial Sequence:capture

<400> 67
tttaagggca tgtgctac                                                    18

<210> 68
<211> 18
<212> DNA
<213> Artificial Sequence

<220>
<223> Description of Artificial Sequence:capture

<400> 68
ttcaccaacg ggacggag                                                    18

<210> 69
<211> 18
<212> DNA
<213> Artificial Sequence

<220>
<223> Description of Artificial Sequence:capture

<400> 69
ttcaccaatg ggacggag                                                    18

<210> 70
<211> 18
<212> DNA
<213> Artificial Sequence

<220>
<223> Description of Artificial Sequence:capture

<400> 70
cgggacggag cgcgtgcg                                                    18

<210> 71
<211> 18
<212> DNA
<213> Artificial Sequence

<220>
<223> Description of Artificial Sequence:capture

<400> 71
cgggacagag cgcgtgcg                                                    18

<210> 72
<211> 18
<212> DNA
<213> Artificial Sequence

<220>
<223> Description of Artificial Sequence:capture

<400> 72
cgggaccgag cgcgtgcg                                                    18

<210> 73
<211> 18
<212> DNA
<213> Artificial Sequence

<220>
<223> Description of Artificial Sequence:capture

<400> 73
cgggaccgag ctcgtgcg                                                    18

<210> 74
<211> 18
<212> DNA
<213> Artificial Sequence

<220>
<223> Description of Artificial Sequence:capture

<400> 74
gtgcggggtg tgaccaga                                                    18

<210> 75
<211> 18
<212> DNA
<213> Artificial Sequence

<220>
<223> Description of Artificial Sequence:capture

<400> 75
gtgcgttatg tgaccaga                                                    18

<210> 76
<211> 18
<212> DNA
<213> Artificial Sequence

<220>
<223> Description of Artificial Sequence:capture

<400> 76
gtgcgtcttg tgaccaga                                                    18

<210> 77
<211> 18
<212> DNA
<213> Artificial Sequence

<220>
<223> Description of Artificial Sequence:capture

<400> 77
gtgcgtcttg taaccaga                                                    18

<210> 78
<211> 18
<212> DNA
<213> Artificial Sequence

<220>
<223> Description of Artificial Sequence:capture

<400> 78
gtgcgtcttg tgagcaga                                                    18

<210> 79
<211> 18
<212> DNA
<213> Artificial Sequence

<220>
<223> Description of Artificial Sequence:capture

<400> 79
accagacaca tctataac                                                    18

<210> 80
<211> 18
<212> DNA
<213> Artificial Sequence

<220>
<223> Description of Artificial Sequence:capture

<400> 80
accagataca tctataac                                                    18

<210> 81
<211> 18
<212> DNA
<213> Artificial Sequence

<220>
<223> Description of Artificial Sequence:capture

<400> 81
agcagaagca tctataac                                                    18

<210> 82
<211> 18
<212> DNA
<213> Artificial Sequence

<220>
<223> Description of Artificial Sequence:capture

<400> 82
ccgagaggag tacgtgcg                                                    18

<210> 83
<211> 18

<212> DNA
<213> Artificial Sequence

<220>
<223> Description of Artificial Sequence:capture

<400> 83
ccgagaagag tacgtgcg                                                    18

<210> 84
<211> 18
<212> DNA
<213> Artificial Sequence

<220>
<223> Description of Artificial Sequence:capture

<400> 84
ccgagaggag gacgtgcg                                                    18

<210> 85
<211> 18
<212> DNA
<213> Artificial Sequence

<220>
<223> Description of Artificial Sequence:capture

<400> 85
ccgagaagag atcgtgcg                                                    18

<210> 86
<211> 18
<212> DNA
<213> Artificial Sequence

<220>
<223> Description of Artificial Sequence:capture

<400> 86
ggagtacgtg cgcttcga                                                    18

<210> 87
<211> 18
<212> DNA
<213> Artificial Sequence

<220>
<223> Description of Artificial Sequence:capture

<400> 87
ggagtacgcg cgcttcga                                                    18

<210> 88
<211> 18
<212> DNA
<213> Artificial Sequence

<220>
<223> Description of Artificial Sequence:capture

<400> 88
ggagtacgca cgcttcga                                                    18

<210> 89
<211> 18
<212> DNA
<213> Artificial Sequence

<220>
<223> Description of Artificial Sequence:capture

<400> 89
gacgtggggg tgtaccgg                                                    18

<210> 90
<211> 18
<212> DNA
<213> Artificial Sequence

<220>
<223> Description of Artificial Sequence:capture

<400> 90
gacgtggggg agttccgg                                                    18

<210> 91
<211> 18
<212> DNA
<213> Artificial Sequence

<220>
<223> Description of Artificial Sequencc:capture

<400> 91
gacgtggagg tgtaccgg                                                    18

<210> 92
<211> 18
<212> DNA
<213> Artificial Sequence

<220>
<223> Description of Artificial Sequence:capture

<400> 92
ggtgtaccgg gcagtgac                                                    18

<210> 93
<211> 18
<212> DNA
<213> Artificial Sequence

<220>
<223> Description of Artificial Sequence:capture

<400> 93
ggtgtaccgg gcggtgac                                                    18

<210> 94
<211> 18
<212> DNA
<213> Artificial Sequence

<220>
<223> Description of Artificial Sequence:capture

<400> 94
ggtgtatcgg gcggtgac                                                    18

<210> 95
<211> 18
<212> DNA
<213> Artificial Sequence

<220>
<223> Description of Artificial Sequence:capture

<400> 95
ggtgtaccgc gcggtgac                                                    18

<210> 96
<211> 18
<212> DNA
<213> Artificial Sequence

<220>
<223> Description of Artificial Sequence:capture

<400> 96
ggtgtatcgg gtggtgac                                                    18

<210> 97
<211> 18
<212> DNA
<213> Artificial Sequence

<220>
<223> Description of Artificial Sequence:capture

<400> 97
gcggtgacgc cgcagggg                                                    18

<210> 98
<211> 18
<212> DNA
<213> Artificial Sequence

<220>
<223> Description of Artificial Sequence:capture

<400> 98
gcggtgaccc cgcagggg                                                    18

<210> 99
<211> 18
<212> DNA
<213> Artificial Sequence

<220>
<223> Description of Artificial Sequence:capture

<400> 99
cgccgcaggg gcggcctg                                                    18

<210> 100
<211> 18
<212> DNA
<213> Artificial Sequence

<220>
<223> Description of Artificial Sequence:capture

<400> 100
cgctgctggg gctgcctg                                                    18

<210> 101
<211> 18
<212> DNA
<213> Artificial Sequence

<220>
<223> Description of Artificial Sequence:capture

<400> 101
cgccgctggg gccgcctg                                                    18

<210> 102
<211> 18
<212> DNA
<213> Artificial Sequence

<220>
<223> Description of Artificial Sequence:capture

<400> 102
cgccgctggg gcggcttg                                                    18

<210> 103
<211> 18
<212> DNA
<213> Artificial Sequence

<220>
<223> Description of Artificial Sequence:capture

<400> 103
gcggcctgtt gccgagta                                                    18

<210> 104
<211> 18
<212> DNA

<213> Artificial Sequence

<220>
<223> Description of Artificial Sequence:capture

<400> 104
gcggcctagc gccgagta                                                    18

<210> 105
<211> 18
<212> DNA
<213> Artificial Sequence

<220>
<223> Description of Artificial Sequence:capture

<400> 105
gcggcctgat gccgagta                                                    18

<210> 106
<211> 18
<212> DNA
<213> Artificial Sequence

<220>
<223> Description of Artificial Sequence:capture

<400> 106
gcggcctgac gccgagta                                                    18

<210> 107
<211> 18
<212> DNA
<213> Artificial Sequence

<220>
<223> Description of Artificial Sequence:capture

<400> 107
gcggcctgtc gccgagta                                                    18

<210> 108
<211> 18
<212> DNA
<213> Artificial Sequence

<220>
<223> Description of Artificial Sequence:capture

<400> 108
gctgcctgcc gccgagta                                                    18

<210> 109
<211> 18
<212> DNA
<213> Artificial Sequence

<220>

<223> Description of Artificial Sequence:capture

<400> 109
gctgcctgac gccgagta                                                    18

<210> 110
<211> 18
<212> DNA
<213> Artificial Sequence

<220>
<223> Description of Artificial Sequence:capture

<400> 110
gccgcctgac gccgagta                                                    18

<210> 111
<211> 18
<212> DNA
<213> Artificial Sequence

<220>
<223> Description of Artificial Sequence:capture

<400> 111
gccgcctgcc gccgagta                                                    18

<210> 112
<211> 18
<212> DNA
<213> Artificial Sequence

<220>
<223> Description of Artificial Sequence:capture

<400> 112
gcggcttgac gccgagta                                                    18

<210> 113
<211> 18
<212> DNA
<213> Artificial Sequence

<220>
<223> Description of Artificial Sequence:capture

<400> 113
gcggcttgac gccgagta                                                    18

<210> 114
<211> 18
<212> DNA
<213> Artificial Sequence

<220>
<223> Description of Artificial Sequence:capture

<400> 114

tactggaaca gccagaag 18

<210> 115
<211> 18
<212> DNA
<213> Artificial Sequence

<220>
<223> Description of Artificial Sequence:capture

<400> 115
tactggaata gccagaag 18

<210> 116
<211> 18
<212> DNA
<213> Artificial Sequence

<220>
<223> Description of Artificial Sequence:capture

<400> 116
cagaaggaag tcctggag 18

<210> 117
<211> 18
<212> DNA
<213> Artificial Sequence

<220>
<223> Description of Artificial Sequence:capture

<400> 117
cagaaggaca tcctggag 18

<210> 118
<211> 18
<212> DNA
<213> Artificial Sequence

<220>
<223> Description of Artificial Sequence:capture

<400> 118
ctggaggggg cccgggcg 18

<210> 119
<211> 18
<212> DNA
<213> Artificial Sequence

<220>
<223> Description of Artificial Sequence:capture

<400> 119
ctggaggagg accgggcg 18

<210> 120

<211> 18
<212> DNA
<213> Artificial Sequence

<220>
<223> Description of Artificial Sequence:capture

<400> 120
ctggagagga cccgagcg                                                    18

<210> 121
<211> 18
<212> DNA
<213> Artificial Sequence

<220>
<223> Description of Artificial Sequence:capture

<400> 121
ctggagggga cccgggcg                                                    18

<210> 122
<211> 18
<212> DNA
<213> Artificial Sequence

<220>
<223> Description of Artificial Sequence:capture

<400> 122
ctggagagga cccgggcg                                                    18

<210> 123
<211> 18
<212> DNA
<213> Artificial Sequence

<220>
<223> Description of Artificial Sequence:capture

<400> 123
ctggagagga aacgggcg                                                    18

<210> 124
<211> 18
<212> DNA
<213> Artificial Sequence

<220>
<223> Description of Artificial Sequence:capture

<400> 124
aggacccgag cggagttg                                                    18

<210> 125
<211> 18
<212> DNA
<213> Artificial Sequence

<220>
<223> Description of Artificial Sequence:capture

<400> 125
aggacccggg cggagttg                                                    18

<210> 126
<211> 18
<212> DNA
<213> Artificial Sequence

<220>
<223> Description of Artificial Sequence:capture

<400> 126
cccgggcgtc ggtggaca                                                    18

<210> 127
<211> 18
<212> DNA
<213> Artificial Sequence

<220>
<223> Description of Artificial Sequence:capture

<400> 127
cccgggcgga gttggaca                                                    18

<210> 128
<211> 18
<212> DNA
<213> Artificial Sequence

<220>
<223> Description of Artificial Sequence:capture

<400> 128
cccgggcggc ggtggaca                                                    18

<210> 129
<211> 18
<212> DNA
<213> Artificial Sequence

<220>
<223> Description of Artificial Sequence:capture

<400> 129
aacgggcggc ggtggaca                                                    18

<210> 130
<211> 18
<212> DNA
<213> Artificial Sequence

<220>
<223> Description of Artificial Sequence:capture

<400> 130
tggacagagt gtgcagac                                                    18

<210> 131
<211> 18
<212> DNA
<213> Artificial Sequence

<220>
<223> Description of Artificial Sequence:capture

<400> 131
tggacagggt gtgcagac                                                    18

<210> 132
<211> 18
<212> DNA
<213> Artificial Sequence

<220>
<223> Description of Artificial Sequence:capture

<400> 132
tggacacggt gtgcagac                                                    18

<210> 133
<211> 18
<212> DNA
<213> Artificial Sequence

<220>
<223> Description of Artificial Sequence:capture

<400> 133
tggacaccgt atgcagac                                                    18

<210> 134
<211> 18
<212> DNA
<213> Artificial Sequence

<220>
<223> Description of Artificial Sequence:capture

<400> 134
gaggtggggt accgcggg                                                    18

<210> 135
<211> 18
<212> DNA
<213> Artificial Sequence

<220>
<223> Description of Artificial Sequence:capture

<400> 135
gaggtggcgt tccgcggg                                                    18

<210> 136
<211> 18
<212> DNA
<213> Artificial Sequence

<220>
<223> Description of Artificial Sequence:capture

<400> 136
cagttggagc tccgcacg                                                    18

<210> 137
<211> 18
<212> DNA
<213> Artificial Sequence

<220>
<223> Description of Artificial Sequence:capture

<400> 137
gaggtggcgt accgcggg                                                    18

<210> 138
<211> 18
<212> DNA
<213> Artificial Sequence

<220>
<223> Description of Artificial Sequence:capture

<400> 138
cgcgggatcc tgcagagg                                                    18

<210> 139
<211> 18
<212> DNA
<213> Artificial Sequence

<220>
<223> Description of Artificial Sequence:capture

<400> 139
cgcgggatct tgcagagg                                                    18

<210> 140
<211> 18
<212> DNA
<213> Artificial Sequence

<220>
<223> Description of Artificial Sequence:capture

<400> 140
cgcacgacct tgcagcgg                                                    18

<210> 141
<211> 18

<212> DNA
<213> Artificial Sequence

<220>
<223> Description of Artificial Sequence:capture

<400> 141
gtgtgtgccc tgggcctg                                                    18

<210> 142
<211> 18
<212> DNA
<213> Artificial Sequence

<220>
<223> Description of Artificial Sequence:capture

<400> 142
gtgtgtgcct tgggcctg                                                    18

<210> 143
<211> 18
<212> DNA
<213> Artificial Sequence

<220>
<223> Description of Artificial Sequence:capture

<400> 143
atcaagggag tgcgcaaa                                                    18

<210> 144
<211> 18
<212> DNA
<213> Artificial Sequence

<220>
<223> Description of Artificial Sequence:capture

<400> 144
atcaagggat tgcgcaaa                                                    18

<210> 145
<211> 18
<212> DNA
<213> Artificial Sequence

<220>
<223> Description of Artificial Sequence:capture

<400> 145
tcttgtggca gcttaagt                                                    18

<210> 146
<211> 18
<212> DNA
<213> Artificial Sequence

<220>
<223> Description of Artificial Sequence:capture

<400> 146
ttcttggagt actctacg                                                    18

<210> 147
<211> 18
<212> DNA
<213> Artificial Sequence

<220>
<223> Description of Artificial Sequence:capture

<400> 147
tcttggagca ggttaaac                                                    18

<210> 148
<211> 18
<212> DNA
<213> Artificial Sequence

<220>
<223> Description of Artificial Sequence:capture

<400> 148
tttcctgtgg cagggtaa                                                    18

<210> 149
<211> 18
<212> DNA
<213> Artificial Sequence

<220>
<223> Description of Artificial Sequence:capture

<400> 149
cagcctaaga gggagtgt                                                    18

<210> 150
<211> 18
<212> DNA
<213> Artificial Sequence

<220>
<223> Description of Artificial Sequence:capture

<400> 150
tcttgaagca ggataagt                                                    18

<210> 151
<211> 18
<212> DNA
<213> Artificial Sequence

<220>
<223> Description of Artificial Sequence:capture

<400> 151
tcttggagga gcgtaagt                                                    18

<210> 152
<211> 18
<212> DNA
<213> Artificial Sequence

<220>
<223> Description of Artificial Sequence:capture

<400> 152
ttggagctgc gtaagtct                                                    18

<210> 153
<211> 18
<212> DNA
<213> Artificial Sequence

<220>
<223> Description of Artificial Sequence:capture

<400> 153
ttggagctgt gtaagtct                                                    18

<210> 154
<211> 18
<212> DNA
<213> Artificial Sequence

<220>
<223> Description of Artificial Sequence:capture

<400> 154
ttggagctgc ttaagtct                                                    18

<210> 155
<211> 18
<212> DNA
<213> Artificial Sequence

<220>
<223> Description of Artificial Sequence:capture

<400> 155
tcttggagca ggctaagt                                                    18

<210> 156
<211> 18
<212> DNA
<213> Artificial Sequence

<220>
<223> Description of Artificial Sequence:capture

<400> 156
tcttgcagca ggataagt                                                    18

<210> 157
<211> 18
<212> DNA
<213> Artificial Sequence

<220>
<223> Description of Artificial Sequence:capture

<400> 157
ctgtggcagc ctaagagg                                                    18

<210> 158
<211> 18
<212> DNA
<213> Artificial Sequence

<220>
<223> Description of Artificial Sequence:capture

<400> 158
tctacgggtg agtgttat                                                    18

<210> 159
<211> 18
<212> DNA
<213> Artificial Sequence

<220>
<223> Description of Artificial Sequence:capture

<400> 159
gagtgtcaat tcttcaat                                                    18

<210> 160
<211> 18
<212> DNA
<213> Artificial Sequence

<220>
<223> Description of Artificial Sequence:capture

<400> 160
agcaggttac acatgagt                                                    18

<210> 161
<211> 18
<212> DNA
<213> Artificial Sequence

<220>
<223> Description of Artificial Sequence:capture

<400> 161
agcaggttaa acatgagt                                                    18

<210> 162
<211> 18
<212> DNA

<213> Artificial Sequence

<220>
<223> Description of Artificial Sequence:capture

<400> 162
ggagcaggct acacatga                                                    18

<210> 163
<211> 18
<212> DNA
<213> Artificial Sequence

<220>
<223> Description of Artificial Sequence:capture

<400> 163
tgaagcagga taagtttg                                                    18

<210> 164
<211> 18
<212> DNA
<213> Artificial Sequence

<220>
<223> Description of Artificial Sequence:capture

<400> 164
cagggtaagt ataagtgt                                                    18

<210> 165
<211> 18
<212> DNA
<213> Artificial Sequence

<220>
<223> Description of Artificial Sequence:capture

<400> 165
tggagctgct taagtctg                                                    18

<210> 166
<211> 18
<212> DNA
<213> Artificial Sequence

<220>
<223> Description of Artificial Sequence:capture

<400> 166
gcttaagttt gaatgtca                                                    18

<210> 167
<211> 18
<212> DNA
<213> Artificial Sequence

<220>

<223> Description of Artificial Sequence:capture

<400> 167
agtactctag gggtgagt                                                    18

<210> 168
<211> 18
<212> DNA
<213> Artificial Sequence

<220>
<223> Description of Artificial Sequence:capture

<400> 168
agtactctat gggtgagt                                                    18

<210> 169
<211> 18
<212> DNA
<213> Artificial Sequence

<220>
<223> Description of Artificial Sequence:capture

<400> 169
tttcttcaac gggacgga                                                    18

<210> 170
<211> 18
<212> DNA
<213> Artificial Sequence

<220>
<223> Description of Artificial Sequence:capture

<400> 170
agcgggtgca gttcctgg                                                    18

<210> 171
<211> 18
<212> DNA
<213> Artificial Sequence

<220>
<223> Description of Artificial Sequence:capture

<400> 171
gggtgcggta cctggaca                                                    18

<210> 172
<211> 18
<212> DNA
<213> Artificial Sequence

<220>
<223> Description of Artificial Sequence:capture

<400> 172

ttactggaga gacacttc                                                    18

<210> 173
<211> 18
<212> DNA
<213> Artificial Sequence

<220>
<223> Description of Artificial Sequence:capture

<400> 173
gctggaaaga tgcatcta                                                    18

<210> 174
<211> 18
<212> DNA
<213> Artificial Sequence

<220>
<223> Description of Artificial Sequence:capture

<400> 174
ttcctggaga gatacttc                                                    18

<210> 175
<211> 18
<212> DNA
<213> Artificial Sequence

<220>
<223> Description of Artificial Sequence:capture

<400> 175
gcggtatctg cacagagg                                                    18

<210> 176
<211> 18
<212> DNA
<213> Artificial Sequence

<220>
<223> Description of Artificial Sequence:capture

<400> 176
tggaaagact cttctata                                                    18

<210> 177
<211> 18
<212> DNA
<213> Artificial Sequence

<220>
<223> Description of Artificial Sequence:capture

<400> 177
tggaaagtct cttctata                                                    18

<210> 178

<211> 18
<212> DNA
<213> Artificial Sequence

<220>
<223> Description of Artificial Sequence:capture

<400> 178
tggacagaca cttctata                                                    18

<210> 179
<211> 18
<212> DNA
<213> Artificial Sequence

<220>
<223> Description of Artificial Sequence:capture

<400> 179
cagatacttc cataacca                                                    18

<210> 180
<211> 18
<212> DNA
<213> Artificial Sequence

<220>
<223> Description of Artificial Sequence:capture

<400> 180
acttctatca ccaagagg                                                    18

<210> 181
<211> 18
<212> DNA
<213> Artificial Sequence

<220>
<223> Description of Artificial Sequence:capture

<400> 181
cttctataat caggagga                                                    18

<210> 182
<211> 18
<212> DNA
<213> Artificial Sequence

<220>
<223> Description of Artificial Sequence:capture

<400> 182
ccataaccgg gaggagaa                                                    18

<210> 183
<211> 18
<212> DNA
<213> Artificial Sequence

<220>
<223> Description of Artificial Sequence:capture

<400> 183
tcaccaagaa gagtacgt                                                    18

<210> 184
<211> 18
<212> DNA
<213> Artificial Sequence

<220>
<223> Description of Artificial Sequence:capture

<400> 184
gaggagaacg tgcgcttc                                                    18

<210> 185
<211> 18
<212> DNA
<213> Artificial Sequence

<220>
<223> Description of Artificial Sequence:capture

<400> 185
aagaggagga cgtgcgct                                                    18

<210> 186
<211> 18
<212> DNA
<213> Artificial Sequence

<220>
<223> Description of Artificial Sequence:capture

<400> 186
agaggaggac ttgcgctt                                                    18

<210> 187
<211> 18
<212> DNA
<213> Artificial Sequence

<220>
<223> Description of Artificial Sequence:capture

<400> 187
aagaggagtc cgtgcgct                                                    18

<210> 188
<211> 18
<212> DNA
<213> Artificial Sequence

<220>
<223> Description of Artificial Sequence:capture

<400> 188
gaggagttcg tgcgcttc                                                    18

<210> 189
<211> 18
<212> DNA
<213> Artificial Sequence

<220>
<223> Description of Artificial Sequence:capture

<400> 189
gaggagaacc tgcgcttc                                                    18

<210> 190
<211> 18
<212> DNA
<213> Artificial Sequence

<220>
<223> Description of Artificial Sequence:capture

<400> 190
gaggagttcc tgcgcttc                                                    18

<210> 191
<211> 18
<212> DNA
<213> Artificial Sequence

<220>
<223> Description of Artificial Sequence:capture

<400> 191
gaggagctcc tgcgcttc                                                    18

<210> 192
<211> 18
<212> DNA
<213> Artificial Sequence

<220>
<223> Description of Artificial Sequence:capture

<400> 192
gcgacgtgag ggagtacc                                                    18

<210> 193
<211> 18
<212> DNA
<213> Artificial Sequence

<220>
<223> Description of Artificial Sequence:capture

<400> 193
gtacgtgcgg ttcgacag                                                    18

<210> 194
<211> 18
<212> DNA
<213> Artificial Sequence

<220>
<223> Description of Artificial Sequence:capture

<400> 194
tgggggagtt ccgggcgg                                                    18

<210> 195
<211> 18
<212> DNA
<213> Artificial Sequence

<220>
<223> Description of Artificial Sequence:capture

<400> 195
gtaccgggtg gtgacgga                                                    18

<210> 196
<211> 17
<212> DNA
<213> Artificial Sequence

<220>
<223> Description of Artificial Sequence:capture

<400> 196
cgggcggcga cggagct                                                     17

<210> 197
<211> 18
<212> DNA
<213> Artificial Sequence

<220>
<223> Description of Artificial Sequence:capture

<400> 197
gggcggtgat ggagctgg                                                    18

<210> 198
<211> 18
<212> DNA
<213> Artificial Sequence

<220>
<223> Description of Artificial Sequence:capture

<400> 198
gacggagcta gggcggcc                                                    18

<210> 199
<211> 18

<212> DNA
<213> Artificial Sequence

<220>
<223> Description of Artificial Sequence:capture

<400> 199
gcctagcgcc gagtactg                                                    18

<210> 200
<211> 18
<212> DNA
<213> Artificial Sequence

<220>
<223> Description of Artificial Sequence:capture

<400> 200
ggggcggcct gctgcgga                                                    18

<210> 201
<211> 18
<212> DNA
<213> Artificial Sequence

<220>
<223> Description of Artificial Sequence:capture

<400> 201
ggcctgacgc tgagtact                                                    18

<210> 202
<211> 18
<212> DNA
<213> Artificial Sequence

<220>
<223> Description of Artificial Sequence:capture

<400> 202
cctgtcgccg agtcctgg                                                    18

<210> 203
<211> 18
<212> DNA
<213> Artificial Sequence

<220>
<223> Description of Artificial Sequence:capture

<400> 203
tgatgcggag cactggaa                                                    18

<210> 204
<211> 18
<212> DNA
<213> Artificial Sequence

<220>
<223> Description of Artificial Sequence:capture

<400> 204
gcctgatgag gagtactg                                                    18

<210> 205
<211> 18
<212> DNA
<213> Artificial Sequence

<220>
<223> Description of Artificial Sequence:capture

<400> 205
gcctgatgct gagtactg                                                    18

<210> 206
<211> 18
<212> DNA
<213> Artificial Sequence

<220>
<223> Description of Artificial Sequence:capture

<400> 206
gcctgatgag gagtactg                                                    18

<210> 207
<211> 18
<212> DNA
<213> Artificial Sequence

<220>
<223> Description of Artificial Sequence:capture

<400> 207
cctgatgccc agtactgg                                                    18

<210> 208
<211> 18
<212> DNA
<213> Artificial Sequence

<220>
<223> Description of Artificial Sequence:capture

<400> 208
cctgatgagg agcactgg                                                    18

<210> 209
<211> 18
<212> DNA
<213> Artificial Sequence

<220>
<223> Description of Artificial Sequence:capture

<400> 209
ggcctgatac cgagtact                                                    18

<210> 210
<211> 18
<212> DNA
<213> Artificial Sequence

<220>
<223> Description of Artificial Sequence:capture

<400> 210
ccagaaggac ttcctgga                                                    18

<210> 211
<211> 18
<212> DNA
<213> Artificial Sequence

<220>
<223> Description of Artificial Sequence:capture

<400> 211
ccagaaggac atcctgga                                                    18

<210> 212
<211> 17
<212> DNA
<213> Artificial Sequence

<220>
<223> Description of Artificial Sequence:capture

<400> 212
ctggagcagg cgcgggc                                                     17

<210> 213
<211> 17
<212> DNA
<213> Artificial Sequence

<220>
<223> Description of Artificial Sequence:capture

<400> 213
ctggagcaga ggcgggc                                                     17

<210> 214
<211> 17
<212> DNA
<213> Artificial Sequence

<220>
<223> Description of Artificial Sequence:capture

<400> 214
ctggagcgga ggcgggc                                                     17

<210> 215
<211> 18
<212> DNA
<213> Artificial Sequence

<220>
<223> Description of Artificial Sequence:capture

<400> 215
ggaagacgag cgggccgc                                                    18

<210> 216
<211> 18
<212> DNA
<213> Artificial Sequence

<220>
<223> Description of Artificial Sequence:capture

<400> 216
tcctggagca gaagcggg                                                    18

<210> 217
<211> 18
<212> DNA
<213> Artificial Sequence

<220>
<223> Description of Artificial Sequence:capture

<400> 217
ctggaggaca ggcggggc                                                    18

<210> 218
<211> 18
<212> DNA
<213> Artificial Sequence

<220>
<223> Description of Artificial Sequence:capture

<400> 218
tggaagacaa gcgggccg                                                    18

<210> 219
<211> 18
<212> DNA
<213> Artificial Sequence

<220>
<223> Description of Artificial Sequence:capture

<400> 219
agcagaggca ggccgcgg                                                    18

<210> 220
<211> 18
<212> DNA

<213> Artificial Sequence

<220>
<223> Description of Artificial Sequence:capture

<400> 220
tggaagacag gcgcgccg                                                    18

<210> 221
<211> 18
<212> DNA
<213> Artificial Sequence

<220>
<223> Description of Artificial Sequence:capture

<400> 221
aagacagggc cgccgcgg                                                    18

<210> 222
<211> 18
<212> DNA
<213> Artificial Sequence

<220>
<223> Description of Artificial Sequence:capture

<400> 222
ggcgggccct ggtggaca                                                    18

<210> 223
<211> 18
<212> DNA
<213> Artificial Sequence

<220>
<223> Description of Artificial Sequence:capture

<400> 223
ggcgggccga ggtggaca                                                    18

<210> 224
<211> 18
<212> DNA
<213> Artificial Sequence

<220>
<223> Description of Artificial Sequence:capture

<400> 224
ggcgggccgt ggtggaca                                                    18

<210> 225
<211> 18
<212> DNA
<213> Artificial Sequence

<220>

<223> Description of Artificial Sequence:capture

<400> 225
ggccgcgctg gacaacta 18

<210> 226
<211> 18
<212> DNA
<213> Artificial Sequence

<220>
<223> Description of Artificial Sequence:capture

<400> 226
agcggggccg ggtggaca 18

<210> 227
<211> 17
<212> DNA
<213> Artificial Sequence

<220>
<223> Description of Artificial Sequence:capture

<400> 227
gcggggccag gtggaca 17

<210> 228
<211> 18
<212> DNA
<213> Artificial Sequence

<220>
<223> Description of Artificial Sequence:capture

<400> 228
gggccgccgt ggacacct 18

<210> 229
<211> 18
<212> DNA
<213> Artificial Sequence

<220>
<223> Description of Artificial Sequence:capture

<400> 229
tggacaacta ctgcagac 18

<210> 230
<211> 18
<212> DNA
<213> Artificial Sequence

<220>
<223> Description of Artificial Sequence:capture

<400> 230

ggtggacaat tactgcag                                                    18

<210> 231
<211> 18
<212> DNA
<213> Artificial Sequence

<220>
<223> Description of Artificial Sequence:capture

<400> 231
tggacaccgt gtgcagac                                                    18

<210> 232
<211> 18
<212> DNA
<213> Artificial Sequence

<220>
<223> Description of Artificial Sequence:capture

<400> 232
ggacacctat tgcagaca                                                    18

<210> 233
<211> 18
<212> DNA
<213> Artificial Sequence

<220>
<223> Description of Artificial Sequence:capture

<400> 233
ctacggagtt gtggagag                                                    18

<210> 234
<211> 18
<212> DNA
<213> Artificial Sequence

<220>
<223> Description of Artificial Sequence:capture

<400> 234
cggggttgtg gagagctt                                                    18

<210> 235
<211> 18
<212> DNA
<213> Artificial Sequence

<220>
<223> Description of Artificial Sequence:capture

<400> 235
acggggttga tgagagct                                                    18

<210> 236

<211> 18
<212> DNA
<213> Artificial Sequence

<220>
<223> Description of Artificial Sequence:capture

<400> 236
tacggggctg tggagagc                                                    18

<210> 237
<211> 18
<212> DNA
<213> Artificial Sequence

<220>
<223> Description of Artificial Sequence:capture

<400> 237
aactaccggg ttgtggag                                                    18

<210> 238
<211> 18
<212> DNA
<213> Artificial Sequence

<220>
<223> Description of Artificial Sequence:capture

<400> 238
acggggttgt tgagagct                                                    18

<210> 239
<211> 18
<212> DNA
<213> Artificial Sequence

<220>
<223> Description of Artificial Sequence:capture

<400> 239
agcttcacgg tgcagcgg                                                    18

<210> 240
<211> 18
<212> DNA
<213> Artificial Sequence

<220>
<223> Description of Artificial Sequence:capture

<400> 240
tcacagtgca ccggcgac                                                    18

<210> 241
<211> 18
<212> DNA
<213> Artificial Sequence

<220>
<223> Description of Artificial Sequence:capture

<400> 241
ggttcccgga cagatact                                                    18

<210> 242
<211> 18
<212> DNA
<213> Artificial Sequence

<220>
<223> Description of Artificial Sequence:capture

<400> 242
cctgatgagg actactgg                                                    18

<210> 243
<211> 18
<212> DNA
<213> Artificial Sequence

<220>
<223> Description of Artificial Sequence:capture

<400> 243
acagaggcat ctataacc                                                    18

<210> 244
<211> 18
<212> DNA
<213> Artificial Sequence

<220>
<223> Description of Artificial Sequence:capture

<400> 244
tgcggagcac tggaacag                                                    18

<210> 245
<211> 18
<212> DNA
<213> Artificial Sequence

<220>
<223> Description of Artificial Sequence:capture

<400> 245
gtggagagat tcacagtg                                                    18

<210> 246
<211> 18
<212> DNA
<213> Artificial Sequence

<220>
<223> Description of Artificial Sequence:capture

<400> 246
acggggttgg tgagagct                                                    18

<210> 247
<211> 18
<212> DNA
<213> Artificial Sequence

<220>
<223> Description of Artificial Sequence:capture

<400> 247
ttctataacc aggaggag                                                    18

<210> 248
<211> 16
<212> DNA
<213> Artificial Sequence

<220>
<223> Description of Artificial Sequence:capture

<400> 248
ccataaccag gaggag                                                      16

<210> 249
<211> 18
<212> DNA
<213> Artificial Sequence

<220>
<223> Description of Artificial Sequence:capture

<400> 249
gaggagtacg tgcgcttc                                                    18

<210> 250
<211> 18
<212> DNA
<213> Artificial Sequence

<220>
<223> Description of Artificial Sequence:capture

<400> 250
ggcctgatgc cgagtact                                                    18

<210> 251
<211> 17
<212> DNA
<213> Artificial Sequence

<220>
<223> Description of Artificial Sequence:capture

<400> 251
catggcacct ctgacag                                                     17

<210> 252
<211> 18
<212> DNA
<213> Artificial Sequence

<220>
<223> Description of Artificial Sequence:capture

<400> 252
tggagctgcg taagtctg                                                    18

<210> 253
<211> 18
<212> DNA
<213> Artificial Sequence

<220>
<223> Description of Artificial Sequence:capture

<400> 253
tggagctgtg taagtctg                                                    18

<210> 254
<211> 18
<212> DNA
<213> Artificial Sequence

<220>
<223> Description of Artificial Sequence:capture

<400> 254
tggagctgct taagtctg                                                    18

<210> 255
<211> 18
<212> DNA
<213> Artificial Sequence

<220>
<223> Description of Artificial Sequence:capture

<400> 255
tgcggtacct ggacagat                                                    18

<210> 256
<211> 18
<212> DNA
<213> Artificial Sequence

<220>
<223> Description of Artificial Sequence:capture

<400> 256
tgcggtacct gaacagat                                                    18

<210> 257
<211> 18

<212> DNA
<213> Artificial Sequence

<220>
<223> Description of Artificial Sequence:capture

<400> 257
tgcggttcct ggagagac                                                    18

<210> 258
<211> 18
<212> DNA
<213> Artificial Sequence

<220>
<223> Description of Artificial Sequence:capture

<400> 258
tggacagata cttccata                                                    18

<210> 259
<211> 18
<212> DNA
<213> Artificial Sequence

<220>
<223> Description of Artificial Sequence:capture

<400> 259
tggagagaca cttccata                                                    18

<210> 260
<211> 17
<212> DNA
<213> Artificial Sequence

<220>
<223> Description of Artificial Sequence:capture

<400> 260
gaggagttcc tgcgctt                                                     17

<210> 261
<211> 17
<212> DNA
<213> Artificial Sequence

<220>
<223> Description of Artificial Sequence:capture

<400> 261
gaggagttcg tgcgctt                                                     17

<210> 262
<211> 17
<212> DNA
<213> Artificial Sequence

<220>
<223> Description of Artificial Sequence:capture

<400> 262
gaggagtacg cgcgctt                                                    17

<210> 263
<211> 17
<212> DNA
<213> Artificial Sequence

<220>
<223> Description of Artificial Sequence:capture

<400> 263
gaggagtccg tgcgctt                                                    17

<210> 264
<211> 17
<212> DNA
<213> Artificial Sequence

<220>
<223> Description of Artificial Sequence:capture

<400> 264
gaggagaacg cgcgctt                                                    17

<210> 265
<211> 18
<212> DNA
<213> Artificial Sequence

<220>
<223> Description of Artificial Sequence:capture

<400> 265
ggcggtgacg gagctggg                                                   18

<210> 266
<211> 18
<212> DNA
<213> Artificial Sequence

<220>
<223> Description of Artificial Sequence:capture

<400> 266
ggcggtgagg gagctggg                                                   18

<210> 267
<211> 16
<212> DNA
<213> Artificial Sequence

<220>
<223> Description of Artificial Sequence:capture

<400> 267
cggcctgtcg ccgagt                                                        16

<210> 268
<211> 16
<212> DNA
<213> Artificial Sequence

<220>
<223> Description of Artificial Sequence:capture

<400> 268
cggcctgttg ccgagt                                                        16

<210> 269
<211> 16
<212> DNA
<213> Artificial Sequence

<220>
<223> Description of Artificial Sequence:capture

<400> 269
cggcctgatg ccgagt                                                        16

<210> 270
<211> 16
<212> DNA
<213> Artificial Sequence

<220>
<223> Description of Artificial Sequence:capture

<400> 270
cggcctagcg ccgagt                                                        16

<210> 271
<211> 18
<212> DNA
<213> Artificial Sequence

<220>
<223> Description of Artificial Sequence:capture

<400> 271
agcggggccg ggtggaca                                                      18

<210> 272
<211> 18
<212> DNA
<213> Artificial Sequence

<220>
<223> Description of Artificial Sequence:capture

<400> 272
agcggggcca ggtggaca                                                      18

<210> 273
<211> 18
<212> DNA
<213> Artificial Sequence

<220>
<223> Description of Artificial Sequence:capture

<400> 273
ggtggacaat tactgcag                                                    18

<210> 274
<211> 18
<212> DNA
<213> Artificial Sequence

<220>
<223> Description of Artificial Sequence:capture

<400> 274
ggtggacaac tactgcag                                                    18

<210> 275
<211> 18
<212> DNA
<213> Artificial Sequence

<220>
<223> Description of Artificial Sequence:capture

<400> 275
tactgcagac acaactac                                                    18

<210> 276
<211> 18
<212> DNA
<213> Artificial Sequence

<220>
<223> Description of Artificial Sequence:capture

<400> 276
tactgcaggc acaactac                                                    18

<210> 277
<211> 17
<212> DNA
<213> Artificial Sequence

<220>
<223> Description of Artificial Sequence:capture

<400> 277
aactacgggg ttggtga                                                     17

<210> 278
<211> 17
<212> DNA

<213> Artificial Sequence

<220>
<223> Description of Artificial Sequence:capture

<400> 278
aactacggag ttggtga                                                    17

<210> 279
<211> 18
<212> DNA
<213> Artificial Sequence

<220>
<223> Description of Artificial Sequence:capture

<400> 279
cggggttggt gagagctt                                                   18

<210> 280
<211> 18
<212> DNA
<213> Artificial Sequence

<220>
<223> Description of Artificial Sequence:capture

<400> 280
cggggttgtg gagagctt                                                   18

<210> 281
<211> 18
<212> DNA
<213> Artificial Sequence

<220>
<223> Description of Artificial Sequence:capture

<400> 281
ggtggacacc tactgcag                                                   18

<210> 282
<211> 18
<212> DNA
<213> Artificial Sequence

<220>
<223> Description of Artificial Sequence:capture

<400> 282
ggtgggcacc tactgcag                                                   18

<210> 283
<211> 18
<212> DNA
<213> Artificial Sequence

<220>

<223> Description of Artificial Sequence:capture

<400> 283
ggtggacaac tactgcag                                                    18

<210> 284
<211> 18
<212> DNA
<213> Artificial Sequence

<220>
<223> Description of Artificial Sequence:capture

<400> 284
actgcagata caactacg                                                    18

<210> 285
<211> 18
<212> DNA
<213> Artificial Sequence

<220>
<223> Description of Artificial Sequence:capture

<400> 285
actgcagaca caactacg                                                    18

<210> 286
<211> 17
<212> DNA
<213> Artificial Sequence

<220>
<223> Description of Artificial Sequence:capture

<400> 286
cggaacagcc aggaaga                                                     17

<210> 287
<211> 17
<212> DNA
<213> Artificial Sequence

<220>
<223> Description of Artificial Sequence:capture

<400> 287
cggaacggcc aggaaga                                                     17

<210> 288
<211> 18
<212> DNA
<213> Artificial Sequence

<220>
<223> Description of Artificial Sequence:capture

<400> 288

ccctctcacg gtgcaatg                                                    18

<210> 289
<211> 18
<212> DNA
<213> Artificial Sequence

<220>
<223> Description of Artificial Sequence:capture

<400> 289
ccctctcaca gtgcaatg                                                    18

<210> 290
<211> 17
<212> DNA
<213> Artificial Sequence

<220>
<223> Description of Artificial Sequence:capture

<400> 290
gcacagagac atctata                                                     17

<210> 291
<211> 17
<212> DNA
<213> Artificial Sequence

<220>
<223> Description of Artificial Sequence:capture

<400> 291
gcacagaggc atctata                                                     17

<210> 292
<211> 18
<212> DNA
<213> Artificial Sequence

<220>
<223> Description of Artificial Sequence:capture

<400> 292
agaggaggac ttgcgctt                                                    18

<210> 293
<211> 18
<212> DNA
<213> Artificial Sequence

<220>
<223> Description of Artificial Sequence:capture

<400> 293
agaggagaac gtgcgctt                                                    18

<210> 294

<211> 18
<212> DNA
<213> Artificial Sequence

<220>
<223> Description of Artificial Sequence:capture

<400> 294
agaggaggac gtgcgctt                                                    18

<210> 295
<211> 18
<212> DNA
<213> Artificial Sequence

<220>
<223> Description of Artificial Sequence:capture

<400> 295
ccagaaggac ttcctgga                                                    18

<210> 296
<211> 18
<212> DNA
<213> Artificial Sequence

<220>
<223> Description of Artificial Sequence:capture

<400> 296
ccagaaggac atcctgga                                                    18

<210> 297
<211> 16
<212> DNA
<213> Artificial Sequence

<220>
<223> Description of Artificial Sequence:capture

<400> 297
tcctggaaga caggcg                                                      16

<210> 298
<211> 17
<212> DNA
<213> Artificial Sequence

<220>
<223> Description of Artificial Sequence:capture

<400> 298
tggaagacac gcgcgcc                                                     17

<210> 299
<211> 17
<212> DNA
<213> Artificial Sequence

<220>
<223> Description of Artificial Sequence:capture

<400> 299
tcctggagca ggcgcgg                                                    17

<210> 300
<211> 18
<212> DNA
<213> Artificial Sequence

<220>
<223> Description of Artificial Sequence:capture

<400> 300
acaggcgggc cgcggtgg                                                   18

<210> 301
<211> 18
<212> DNA
<213> Artificial Sequence

<220>
<223> Description of Artificial Sequence:capture

<400> 301
aggcgcgggc cgcggtgg                                                   18

<210> 302
<211> 18
<212> DNA
<213> Artificial Sequence

<220>
<223> Description of Artificial Sequence:capture

<400> 302
acaggcgcgc cgcggtgg                                                   18

<210> 303
<211> 18
<212> DNA
<213> Artificial Sequence

<220>
<223> Description of Artificial Sequence:capture

<400> 303
aggcgggccc tggtggac                                                   18

<210> 304
<211> 18
<212> DNA
<213> Artificial Sequence

<220>
<223> Description of Artificial Sequence:capture

<400> 304
aggcgcgccg cggtggac                                                    18

<210> 305
<211> 18
<212> DNA
<213> Artificial Sequence

<220>
<223> Description of Artificial Sequence:capture

<400> 305
gcgggccgcg gtggacac                                                    18

<210> 306
<211> 17
<212> DNA
<213> Artificial Sequence

<220>
<223> Description of Artificial Sequence:capture

<400> 306
acggggttgg tgagagc                                                     17

<210> 307
<211> 17
<212> DNA
<213> Artificial Sequence

<220>
<223> Description of Artificial Sequence:capture

<400> 307
acggggctgt ggagagc                                                     17

<210> 308
<211> 17
<212> DNA
<213> Artificial Sequence

<220>
<223> Description of Artificial Sequence:capture

<400> 308
gaacctgccc ttcgaca                                                     17

<210> 309
<211> 17
<212> DNA
<213> Artificial Sequence

<220>
<223> Description of Artificial Sequence:capture

<400> 309
gaacctgcgc ttcgaca                                                     17

<210> 310
<211> 17
<212> DNA
<213> Artificial Sequence

<220>
<223> Description of Artificial Sequence:capture

<400> 310
cgacagcgac gtggagg                                                    17

<210> 311
<211> 17
<212> DNA
<213> Artificial Sequence

<220>
<223> Description of Artificial Sequence:capture

<400> 311
cgacagcaac gtggagg                                                    17

<210> 312
<211> 18
<212> DNA
<213> Artificial Sequence

<220>
<223> Description of Artificial Sequence:capture

<400> 312
gaagggaatc ctggagga                                                   18

<210> 313
<211> 18
<212> DNA
<213> Artificial Sequence

<220>
<223> Description of Artificial Sequence:capture

<400> 313
gaagggcatc ccggagga                                                   18

<210> 314
<211> 17
<212> DNA
<213> Artificial Sequence

<220>
<223> Description of Artificial Sequence:capture

<400> 314
gaggagaatc gggacaa                                                    17

<210> 315
<211> 17

<212> DNA
<213> Artificial Sequence

<220>
<223> Description of Artificial Sequence:capture

<400> 315
gaggagaagc gggacaa                                                    17

<210> 316
<211> 18
<212> DNA
<213> Artificial Sequence

<220>
<223> Description of Artificial Sequence:capture

<400> 316
gtggacacct actgcaga                                                   18

<210> 317
<211> 18
<212> DNA
<213> Artificial Sequence

<220>
<223> Description of Artificial Sequence:capture

<400> 317
atggacgact actgcaga                                                   18

<210> 318
<211> 18
<212> DNA
<213> Artificial Sequence

<220>
<223> Description of Artificial Sequence:capture

<400> 318
gatacagtta cggggttt                                                   18

<210> 319
<211> 18
<212> DNA
<213> Artificial Sequence

<220>
<223> Description of Artificial Sequence:capture

<400> 319
gatacaatta cggggttt                                                   18

<210> 320
<211> 18
<212> DNA
<213> Artificial Sequence

<220>
<223> Description of Artificial Sequence:capture

<400> 320
gatacaatta cagggttt                                                    18

<210> 321
<211> 18
<212> DNA
<213> Artificial Sequence

<220>
<223> Description of Artificial Sequence:capture

<400> 321
taaagatact tctataac                                                    18

<210> 322
<211> 18
<212> DNA
<213> Artificial Sequence

<220>
<223> Description of Artificial Sequence:capture

<400> 322
taaagatatt tctataac                                                    18

<210> 323
<211> 18
<212> DNA
<213> Artificial Sequence

<220>
<223> Description of Artificial Sequence:capture

<400> 323
tccagacact gatgatgc                                                    18

<210> 324
<211> 18
<212> DNA
<213> Artificial Sequence

<220>
<223> Description of Artificial Sequence:capture

<400> 324
cttatgccgc gtttgtac                                                    18

<210> 325
<211> 18
<212> DNA
<213> Artificial Sequence

<220>
<223> Description of Artificial Sequence:capture

<400> 325
cttatgccat gtttgtac                                                    18

<210> 326
<211> 18
<212> DNA
<213> Artificial Sequence

<220>
<223> Description of Artificial Sequence:capture

<400> 326
tgtacagacg catagacc                                                    18

<210> 327
<211> 18
<212> DNA
<213> Artificial Sequence

<220>
<223> Description of Artificial Sequence:capture

<400> 327
tgtacagacg catagacc                                                    18

<210> 328
<211> 18
<212> DNA
<213> Artificial Sequence

<220>
<223> Description of Artificial Sequence:capture

<400> 328
catagaacaa caggagag                                                    18

<210> 329
<211> 18
<212> DNA
<213> Artificial Sequence

<220>
<223> Description of Artificial Sequence:capture

<400> 329
caacaggaga gtttatgt                                                    18

<210> 330
<211> 18
<212> DNA
<213> Artificial Sequence

<220>
<223> Description of Artificial Sequence:capture

<400> 330
caacagggga gtttatgt                                                    18

<210> 331
<211> 18
<212> DNA
<213> Artificial Sequence

<220>
<223> Description of Artificial Sequence:capture

<400> 331
tatgtttgaa tttgatga                                                    18

<210> 332
<211> 18
<212> DNA
<213> Artificial Sequence

<220>
<223> Description of Artificial Sequence:capture

<400> 332
tatgtttgag tttgatga                                                    18

<210> 333
<211> 18
<212> DNA
<213> Artificial Sequence

<220>
<223> Description of Artificial Sequence:capture

<400> 333
gaatttgatg aagatgag                                                    18

<210> 334
<211> 18
<212> DNA
<213> Artificial Sequence

<220>
<223> Description of Artificial Sequence:capture

<400> 334
gaatttgatg acgatgag                                                    18

<210> 335
<211> 18
<212> DNA
<213> Artificial Sequence

<220>
<223> Description of Artificial Sequence:capture

<400> 335
gagtttgatg atgatgag                                                    18

<210> 336
<211> 18
<212> DNA

<213> Artificial Sequence

<220>
<223> Description of Artificial Sequence:capture

<400> 336
gatgagatgt tctatgtg                                                    18

<210> 337
<211> 18
<212> DNA
<213> Artificial Sequence

<220>
<223> Description of Artificial Sequence:capture

<400> 337
gatgagcagt tctatgtg                                                    18

<210> 338
<211> 18
<212> DNA
<213> Artificial Sequence

<220>
<223> Description of Artificial Sequence:capture

<400> 338
atctggacaa gaaggagg                                                    18

<210> 339
<211> 18
<212> DNA
<213> Artificial Sequence

<220>
<223> Description of Artificial Sequence:capture

<400> 339
atctggataa aaaggagg                                                    18

<210> 340
<211> 18
<212> DNA
<213> Artificial Sequence

<220>
<223> Description of Artificial Sequence:capture

<400> 340
atctggataa gaaggagg                                                    18

<210> 341
<211> 18
<212> DNA
<213> Artificial Sequence

<220>

<223> Description of Artificial Sequence:capture

<400> 341
gtttggccaa gcctttc                                                    18

<210> 342
<211> 18
<212> DNA
<213> Artificial Sequence

<220>
<223> Description of Artificial Sequence:capture

<400> 342
gtttggccga gcctttc                                                    18

<210> 343
<211> 18
<212> DNA
<213> Artificial Sequence

<220>
<223> Description of Artificial Sequence:capture

<400> 343
ttgctatatt gaacaaca                                                   18

<210> 344
<211> 18
<212> DNA
<213> Artificial Sequence

<220>
<223> Description of Artificial Sequence:capture

<400> 344
ttgctatatc gaacaaca                                                   18

<210> 345
<211> 18
<212> DNA
<213> Artificial Sequence

<220>
<223> Description of Artificial Sequence:capture

<400> 345
ttgaatacct tgatccag                                                   18

<210> 346
<211> 18
<212> DNA
<213> Artificial Sequence

<220>
<223> Description of Artificial Sequence:capture

<400> 346

ttgaatatcg ctatccag                                                    18

<210> 347
<211> 18
<212> DNA
<213> Artificial Sequence

<220>
<223> Description of Artificial Sequence:capture

<400> 347
tcaggccacc aacggtac                                                    18

<210> 348
<211> 18
<212> DNA
<213> Artificial Sequence

<220>
<223> Description of Artificial Sequence:capture

<400> 348
tcaggccgcc aatggtac                                                    18

<210> 349
<211> 18
<212> DNA
<213> Artificial Sequence

<220>
<223> Description of Artificial Sequence:capture

<400> 349
gagaattacg tgtaccag                                                    18

<210> 350
<211> 18
<212> DNA
<213> Artificial Sequence

<220>
<223> Description of Artificial Sequence:capture

<400> 350
gagaattacc ttttccag                                                    18

<210> 351
<211> 18
<212> DNA
<213> Artificial Sequence

<220>
<223> Description of Artificial Sequence:capture

<400> 351
gagaattacg tgcaccag                                                    18

<210> 352

<211> 18
<212> DNA
<213> Artificial Sequence

<220>
<223> Description of Artificial Sequence:capture

<400> 352
gagaattaag tgtaccag 18

<210> 353
<211> 18
<212> DNA
<213> Artificial Sequence

<220>
<223> Description of Artificial Sequence:capture

<400> 353
gagaattacg tggaccag 18

<210> 354
<211> 18
<212> DNA
<213> Artificial Sequence

<220>
<223> Description of Artificial Sequence:capture

<400> 354
accagggacg gcaggaat 18

<210> 355
<211> 18
<212> DNA
<213> Artificial Sequence

<220>
<223> Description of Artificial Sequence:capture

<400> 355
tccagggacg acaggaat 18

<210> 356
<211> 18
<212> DNA
<213> Artificial Sequence

<220>
<223> Description of Artificial Sequence:capture

<400> 356
accagttacg gcaggaat 18

<210> 357
<211> 18
<212> DNA
<213> Artificial Sequence

<220>
<223> Description of Artificial Sequence:capture

<400> 357
tccagggact gcaggaat                                                    18

<210> 358
<211> 18
<212> DNA
<213> Artificial Sequence

<220>
<223> Description of Artificial Sequence:capture

<400> 358
tctacaaccg ggaggagt                                                    18

<210> 359
<211> 18
<212> DNA
<213> Artificial Sequence

<220>
<223> Description of Artificial Sequence:capture

<400> 359
tctacaacag gcaggagt                                                    18

<210> 360
<211> 18
<212> DNA
<213> Artificial Sequence

<220>
<223> Description of Artificial Sequence:capture

<400> 360
tctacaaccg gcaggagt                                                    18

<210> 361
<211> 18
<212> DNA
<213> Artificial Sequence

<220>
<223> Description of Artificial Sequence:capture

<400> 361
aggagtacgc gcgcttcg                                                    18

<210> 362
<211> 18
<212> DNA
<213> Artificial Sequence

<220>
<223> Description of Artificial Sequence:capture

<400> 362
aggagctcgt gcgcttcg                                                    18

<210> 363
<211> 18
<212> DNA
<213> Artificial Sequence

<220>
<223> Description of Artificial Sequence:capture

<400> 363
aggagttcgc gcgcttcg                                                    18

<210> 364
<211> 18
<212> DNA
<213> Artificial Sequence

<220>
<223> Description of Artificial Sequence:capture

<400> 364
aggagttcgt gcgcttcg                                                    18

<210> 365
<211> 18
<212> DNA
<213> Artificial Sequence

<220>
<223> Description of Artificial Sequence:capture

<400> 365
gacgtggggg agttccgg                                                    18

<210> 366
<211> 18
<212> DNA
<213> Artificial Sequence

<220>
<223> Description of Artificial Sequence:capture

<400> 366
gacgtgggag agttccgg                                                    18

<210> 367
<211> 18
<212> DNA
<213> Artificial Sequence

<220>
<223> Description of Artificial Sequence:capture

<400> 367
cggcctgctg cggagtac                                                    18

<210> 368
<211> 18
<212> DNA
<213> Artificial Sequence

<220>
<223> Description of Artificial Sequence:capture

<400> 368
cggcctgatg aggagtac                                                    18

<210> 369
<211> 18
<212> DNA
<213> Artificial Sequence

<220>
<223> Description of Artificial Sequence:capture

<400> 369
cggcctgacg aggagtac                                                    18

<210> 370
<211> 18
<212> DNA
<213> Artificial Sequence

<220>
<223> Description of Artificial Sequence:capture

<400> 370
cggcctgatg aggactac                                                    18

<210> 371
<211> 18
<212> DNA
<213> Artificial Sequence

<220>
<223> Description of Artificial Sequence:capture

<400> 371
cggcctgagg cggagtac                                                    18

<210> 372
<211> 18
<212> DNA
<213> Artificial Sequence

<220>
<223> Description of Artificial Sequence:capture

<400> 372
cggcctgatg aggtgtac                                                    18

<210> 373
<211> 18

<212> DNA
<213> Artificial Sequence

<220>
<223> Description of Artificial Sequence:capture

<400> 373
tgatgaggag tactggaa                                                    18

<210> 374
<211> 18
<212> DNA
<213> Artificial Sequence

<220>
<223> Description of Artificial Sequence:capture

<400> 374
tgatgaggac tactggaa                                                    18

<210> 375
<211> 18
<212> DNA
<213> Artificial Sequence

<220>
<223> Description of Artificial Sequence:capture

<400> 375
tgatgaggtg tactggaa                                                    18

<210> 376
<211> 18
<212> DNA
<213> Artificial Sequence

<220>
<223> Description of Artificial Sequence:capture

<400> 376
cagaaggaca tcctggag                                                    18

<210> 377
<211> 18
<212> DNA
<213> Artificial Sequence

<220>
<223> Description of Artificial Sequence:capture

<400> 377
cagaaggacc tcctggag                                                    18

<210> 378
<211> 18
<212> DNA
<213> Artificial Sequence

<220>
<223> Description of Artificial Sequence:capture

<400> 378
cagaaggact tcctggag                                                    18

<210> 379
<211> 18
<212> DNA
<213> Artificial Sequence

<220>
<223> Description of Artificial Sequence:capture

<400> 379
cagaaggaca acctggag                                                    18

<210> 380
<211> 18
<212> DNA
<213> Artificial Sequence

<220>
<223> Description of Artificial Sequence:capture

<400> 380
cagaaggacc tcctgtag                                                    18

<210> 381
<211> 18
<212> DNA
<213> Artificial Sequence

<220>
<223> Description of Artificial Sequence:capture

<400> 381
gacctcctgt aggagaag                                                    18

<210> 382
<211> 18
<212> DNA
<213> Artificial Sequence

<220>
<223> Description of Artificial Sequence:capture

<400> 382
ctggaggaga agcgggca                                                    18

<210> 383
<211> 18
<212> DNA
<213> Artificial Sequence

<220>
<223> Description of Artificial Sequence:capture

<400> 383
ctggaggagg agcgggca                                                    18

<210> 384
<211> 18
<212> DNA
<213> Artificial Sequence

<220>
<223> Description of Artificial Sequence:capture

<400> 384
ctggaggaga ggcgggca                                                    18

<210> 385
<211> 18
<212> DNA
<213> Artificial Sequence

<220>
<223> Description of Artificial Sequence:capture

<400> 385
ctgtaggaga agcgggca                                                    18

<210> 386
<211> 18
<212> DNA
<213> Artificial Sequence

<220>
<223> Description of Artificial Sequence:capture

<400> 386
agcgggcagt gccggaca                                                    18

<210> 387
<211> 18
<212> DNA
<213> Artificial Sequence

<220>
<223> Description of Artificial Sequence:capture

<400> 387
agcgggcatt gccggaca                                                    18

<210> 388
<211> 18
<212> DNA
<213> Artificial Sequence

<220>
<223> Description of Artificial Sequence:capture

<400> 388
gcgggcagtg ctggacag                                                    18

<210> 389
<211> 18
<212> DNA
<213> Artificial Sequence

<220>
<223> Description of Artificial Sequence:capture

<400> 389
gcgggcagtg ctggacag                                                    18

<210> 390
<211> 18
<212> DNA
<213> Artificial Sequence

<220>
<223> Description of Artificial Sequence:capture

<400> 390
ggacagggta tgcagaca                                                    18

<210> 391
<211> 18
<212> DNA
<213> Artificial Sequence

<220>
<223> Description of Artificial Sequence:capture

<400> 391
ggacaggatg tgcagaca                                                    18

<210> 392
<211> 18
<212> DNA
<213> Artificial Sequence

<220>
<223> Description of Artificial Sequence:capture

<400> 392
ggacaggata tgcagaca                                                    18

<210> 393
<211> 18
<212> DNA
<213> Artificial Sequence

<220>
<223> Description of Artificial Sequence:capture

<400> 393
acgagctgga cgaggccg                                                    18

<210> 394
<211> 18
<212> DNA

<213> Artificial Sequence

<220>
<223> Description of Artificial Sequence:capture

<400> 394
acgagctggg cgggccca                                                    18

<210> 395
<211> 18
<212> DNA
<213> Artificial Sequence

<220>
<223> Description of Artificial Sequence:capture

<400> 395
acgagctggt cgggccca                                                    18

<210> 396
<211> 18
<212> DNA
<213> Artificial Sequence

<220>
<223> Description of Artificial Sequence:capture

<400> 396
gaatgctacg cgtttaat                                                    18

<210> 397
<211> 18
<212> DNA
<213> Artificial Sequence

<220>
<223> Description of Artificial Sequence:capture

<400> 397
gaatgctacc cgtttaat                                                    18

<210> 398
<211> 23
<212> DNA
<213> Artificial Sequence

<220>
<223> Description of Artificial Sequence:primer

<400> 398
gagtactgga atagccagaa gga                                              23

<210> 399
<211> 23
<212> DNA
<213> Artificial Sequence

<220>

<223> Description of Artificial Sequence:primer

<400> 399
gagtactgga acagccagaa gga                                              23

<210> 400
<211> 18
<212> DNA
<213> Artificial Sequence

<220>
<223> Description of Artificial Sequence:primer

<400> 400
ggcgacgacg ctcacctc                                                    18

<210> 401
<211> 18
<212> DNA
<213> Artificial Sequence

<220>
<223> Description of Artificial Sequence:primer

<400> 401
ttcgtgtccc cacagcac                                                    18

<210> 402
<211> 18
<212> DNA
<213> Artificial Sequence

<220>
<223> Description of Artificial Sequence:primer

<400> 402
ttcgtgtccc cccagcac                                                    18

<210> 403
<211> 16
<212> DNA
<213> Artificial Sequence

<220>
<223> Description of Artificial Sequence:primer

<400> 403
gcacccgctc cgtccc                                                      16

<210> 404
<211> 21
<212> DNA
<213> Artificial Sequence

<220>
<223> Description of Artificial Sequence:primer

<400> 404

gattccccgc agaggatttc g                                                21

<210> 405
<211> 21
<212> DNA
<213> Artificial Sequence

<220>
<223> Description of Artificial Sequence:primer

<400> 405
gctcacctct cctctgcaag a                                                21

<210> 406
<211> 20
<212> DNA
<213> Artificial Sequence

<220>
<223> Description of Artificial Sequence:primer

<400> 406
ctcacctctc ctctgcagga                                                  20

<210> 407
<211> 20
<212> DNA
<213> Artificial Sequence

<220>
<223> Description of Artificial Sequence:primer

<400> 407
gctacttcac caacgggacc                                                  20

<210> 408
<211> 20
<212> DNA
<213> Artificial Sequence

<220>
<223> Description of Artificial Sequence:primer

<400> 408
gctacttcac caacgggacg                                                  20

<210> 409
<211> 19
<212> DNA
<213> Artificial Sequence

<220>
<223> Description of Artificial Sequence:primer

<400> 409
tcgtgcggag ctccaactg                                                   19

<210> 410

<211> 23
<212> DNA
<213> Artificial Sequence

<220>
<223> Description of Artificial Sequence:primer

<400> 410
tcggtgacag atttctatcc agc                                                23

<210> 411
<211> 24
<212> DNA
<213> Artificial Sequence

<220>
<223> Description of Artificial Sequence:primer

<400> 411
ctcagtgaca gatttctatc cagc                                               24

<210> 412
<211> 21
<212> DNA
<213> Artificial Sequence

<220>
<223> Description of Artificial Sequence:primer

<400> 412
agcatcacca ggatctggaa g                                                  21

<210> 413
<211> 21
<212> DNA
<213> Artificial Sequence

<220>
<223> Description of Artificial Sequence:primer

<400> 413
tccccacagc acgtttcttg t                                                  21

<210> 414
<211> 23
<212> DNA
<213> Artificial Sequence

<220>
<223> Description of Artificial Sequence:primer

<400> 414
gcacgtttct tggagtactc tac                                                23

<210> 415
<211> 26
<212> DNA
<213> Artificial Sequence

<220>
<223> Description of Artificial Sequence:primer

<400> 415
cacgtttctt ggagcaggtt aaacat                                          26

<210> 416
<211> 19
<212> DNA
<213> Artificial Sequence

<220>
<223> Description of Artificial Sequence:primer

<400> 416
gtccccacag cacgtttcc                                                  19

<210> 417
<211> 17
<212> DNA
<213> Artificial Sequence

<220>
<223> Description of Artificial Sequence:primer

<400> 417
tcacctcgcc gctgcac                                                    17

<210> 418
<211> 29
<212> DNA
<213> Artificial Sequence

<220>
<223> Description of Artificial Sequence:primer

<400> 418
gcttgtcatc ttcactcatc agctgacca                                       29

<210> 419
<211> 25
<212> DNA
<213> Artificial Sequence

<220>
<223> Description of Artificial Sequence:primer

<400> 419
gtcaccttca ctcgtcagct gacca                                           25

<210> 420
<211> 26
<212> DNA
<213> Artificial Sequence

<220>
<223> Description of Artificial Sequence:primer

<400> 420
ggtggacaca taccattggt agcagc                                          26

<210> 421
<211> 25
<212> DNA
<213> Artificial Sequence

<220>
<223> Description of Artificial Sequence:primer

<400> 421
ctgattcccc gcagaggatt tcgtg                                           25

<210> 422
<211> 20
<212> DNA
<213> Artificial Sequence

<220>
<223> Description of Artificial Sequence:primer

<400> 422
ggggcgacga cgctcacctc                                                 20

<210> 423
<211> 23
<212> DNA
<213> Artificial Sequence

<220>
<223> Description of Artificial Sequence:primer

<400> 423
gatgctccaa gccctctccc aga                                             23

<210> 424
<211> 23
<212> DNA
<213> Artificial Sequence

<220>
<223> Description of Artificial Sequence:primer

<400> 424
ttacagaggc cccctgcgtt ctg                                             23

<210> 425
<211> 25
<212> DNA
<213> Artificial Sequence

<220>
<223> Description of Artificial Sequence:primer

<400> 425
cgttcttgtc cccccagcac gtttc                                           25

<210> 426
<211> 25
<212> DNA
<213> Artificial Sequence

<220>
<223> Description of Artificial Sequence:primer

<400> 426
cgtgtcccca cagcacgttt cctgt                                          25

<210> 427
<211> 24
<212> DNA
<213> Artificial Sequence

<220>
<223> Description of Artificial Sequence:primer

<400> 427
ttcgtgtccc cacagcacgt ttct                                           24

<210> 428
<211> 20
<212> DNA
<213> Artificial Sequence

<220>
<223> Description of Artificial Sequence:primer

<400> 428
cgctcacctc gccgctgcac                                                20

<210> 429
<211> 24
<212> DNA
<213> Artificial Sequence

<220>
<223> Description of Artificial Sequence:primer

<400> 429
acccgcagca cgtttcttgg agct                                           24

<210> 430
<211> 23
<212> DNA
<213> Artificial Sequence

<220>
<223> Description of Artificial Sequence:primer

<400> 430
cccgcagcac gtttctcgga gct                                            23

<210> 431
<211> 23

<212> DNA
<213> Artificial Sequence

<220>
<223> Description of Artificial Sequence:primer

<400> 431
tcgccgctgc actgtgaagc tct                                                23

<210> 432
<211> 24
<212> DNA
<213> Artificial Sequence

<220>
<223> Description of Artificial Sequence:primer

<400> 432
agtacgcgcg ctacaacagt gacc                                               24

<210> 433
<211> 18
<212> DNA
<213> Artificial Sequence

<220>
<223> Description of Artificial Sequence:primer

<400> 433
ggacccaggc cccgccct                                                      18

<210> 434
<211> 34
<212> DNA
<213> Artificial Sequence

<220>
<223> Description of Artificial Sequence:primer

<400> 434
tcattcccac tacctttttt ttctcctagt ccaa                                    34

<210> 435
<211> 32
<212> DNA
<213> Artificial Sequence

<220>
<223> Description of Artificial Sequence:primer

<400> 435
tttaggaagt cagaaagctg ctaactccat tg                                      32

<210> 436
<211> 20
<212> DNA
<213> Artificial Sequence

<220>
<223> Description of Artificial Sequence:primer

<400> 436
ggagcgggtg cggttcctgc                                                    20

<210> 437
<211> 23
<212> DNA
<213> Artificial Sequence

<220>
<223> Description of Artificial Sequence:primer

<400> 437
tcgccgctgc actgtgaagc tct                                                23

<210> 438
<211> 33
<212> DNA
<213> Artificial Sequence

<220>
<223> Description of Artificial Sequence:primer

<400> 438
ctaagtgtga gtgtcatatc ttcaatggga tga                                     33

<210> 439
<211> 28
<212> DNA
<213> Artificial Sequence

<220>
<223> Description of Artificial Sequence:primer

<400> 439
gctgcactgt gaagctctca aaaaaccc                                           28

<210> 440
<211> 27
<212> DNA
<213> Artificial Sequence

<220>
<223> Description of Artificial Sequence:primer

<400> 440
gctgcactgt gaagctctca aaaaccc                                            27

<210> 441
<211> 25
<212> DNA
<213> Artificial Sequence

<220>
<223> Description of Artificial Sequence:primer

<400> 441
caatgggacg gagcggtacc tgtaa                                          25

<210> 442
<211> 23
<212> DNA
<213> Artificial Sequence

<220>
<223> Description of Artificial Sequence:primer

<400> 442
cggaactccc ccatgtcgct gtt                                            23

<210> 443
<211> 26
<212> DNA
<213> Artificial Sequence

<220>
<223> Description of Artificial Sequence:primer

<400> 443
cagcggacca tgtgtcaact tatgcc                                         26

<210> 444
<211> 27
<212> DNA
<213> Artificial Sequence

<220>
<223> Description of Artificial Sequence:primer

<400> 444
ggctacagag gaagaggcaa agatagg                                        27

<210> 445
<211> 21
<212> DNA
<213> Artificial Sequence

<220>
<223> Description of Artificial Sequence:primer

<400> 445
gccccctccc cgcagagaat t                                              21

<210> 446
<211> 20
<212> DNA
<213> Artificial Sequence

<220>
<223> Description of Artificial Sequence:primer

<400> 446
ctcggcgctg cagggtcacg                                                20

<210> 447
<211> 20
<212> DNA
<213> Artificial Sequence

<220>
<223> Description of Artificial Sequence:primer

<400> 447
ctcggcgctg cagggtcatg                                                    20

<210> 448
<211> 19
<212> DNA
<213> Artificial Sequence

<220>
<223> Description of Artificial Sequence:primer

<400> 448
ctccccgcag agaattacg                                                     19

<210> 449
<211> 19
<212> DNA
<213> Artificial Sequence

<220>
<223> Description of Artificial Sequence:primer

<400> 449
ctccccgcag agaattacc                                                     19

<210> 450
<211> 20
<212> DNA
<213> Artificial Sequence

<220>
<223> Description of Artificial Sequence:primer

<400> 450
caaagccctc actacctcgg                                                    20

<210> 451
<211> 19
<212> DNA
<213> Artificial Sequence

<220>
<223> Description of Artificial Sequence:primer

<400> 451
caaagcccta ctacctcgg                                                     19

<210> 452
<211> 20
<212> DNA

<213> Artificial Sequence

<220>
<223> Description of Artificial Sequence:primer

<400> 452
caaagccctc actacctcgt                                                    20

<210> 453
<211> 19
<212> DNA
<213> Artificial Sequence

<220>
<223> Description of Artificial Sequence:primer

<400> 453
caaagcccta ctacctcgt                                                     19

<210> 454
<211> 20
<212> DNA
<213> Artificial Sequence

<220>
<223> Description of Artificial Sequence:primer

<400> 454
caaagccctc actacctcga                                                    20

<210> 455
<211> 19
<212> DNA
<213> Artificial Sequence

<220>
<223> Description of Artificial Sequence:primer

<400> 455
caaagcccta ctacctcga                                                     19

<210> 456
<211> 17
<212> DNA
<213> Artificial Sequence

<220>
<223> Description of Artificial Sequence:capture

<400> 456
attacgtgca ccagtta                                                       17

<210> 457
<211> 17
<212> DNA
<213> Artificial Sequence

<220>

<223> Description of Artificial Sequence:capture

<400> 457
attacgtgga ccagtta                                                    17

<210> 458
<211> 16
<212> DNA
<213> Artificial Sequence

<220>
<223> Description of Artificial Sequence:capture

<400> 458
gtaccaggga cggcag                                                     16

<210> 459
<211> 16
<212> DNA
<213> Artificial Sequence

<220>
<223> Description of Artificial Sequence:capture

<400> 459
gtaccagtta cggcag                                                     16

<210> 460
<211> 16
<212> DNA
<213> Artificial Sequence

<220>
<223> Description of Artificial Sequence:capture

<400> 460
tttccaggga cggcag                                                     16

<210> 461
<211> 16
<212> DNA
<213> Artificial Sequence

<220>
<223> Description of Artificial Sequence:capture

<400> 461
gcaccagtta cggcag                                                     16

<210> 462
<211> 16
<212> DNA
<213> Artificial Sequence

<220>
<223> Description of Artificial Sequence:capture

<400> 462

tttccaggga ctgcag 16

<210> 463
<211> 16
<212> DNA
<213> Artificial Sequence

<220>
<223> Description of Artificial Sequence:capture

<400> 463
ggaccagtta cggcag 16

<210> 464
<211> 17
<212> DNA
<213> Artificial Sequence

<220>
<223> Description of Artificial Sequence:capture

<400> 464
ccagggactg caggaat 17

<210> 465
<211> 18
<212> DNA
<213> Artificial Sequence

<220>
<223> Description of Artificial Sequence:capture

<400> 465
aatgctaccc gtttaatg 18

<210> 466
<211> 18
<212> DNA
<213> Artificial Sequence

<220>
<223> Description of Artificial Sequence:capture

<400> 466
ctacaacagg caggagta 18

<210> 467
<211> 18
<212> DNA
<213> Artificial Sequence

<220>
<223> Description of Artificial Sequence:capture

<400> 467
tacaaccggc aggagtac 18

<210> 468

<211> 17
<212> DNA
<213> Artificial Sequence

<220>
<223> Description of Artificial Sequence:capture

<400> 468
ggaggagttc gcgcgct                                                    17

<210> 469
<211> 18
<212> DNA
<213> Artificial Sequence

<220>
<223> Description of Artificial Sequence:capture

<400> 469
gaggagtacg cgcgcttg                                                   18

<210> 470
<211> 18
<212> DNA
<213> Artificial Sequence

<220>
<223> Description of Artificial Sequence:capture

<400> 470
gaggagttcg tgcgcttg                                                   18

<210> 471
<211> 18
<212> DNA
<213> Artificial Sequence

<220>
<223> Description of Artificial Sequence:capture

<400> 471
ggaggagctc gtgcgctt                                                   18

<210> 472
<211> 18
<212> DNA
<213> Artificial Sequence

<220>
<223> Description of Artificial Sequence:capture

<400> 472
caggagtacg cgcgcttg                                                   18

<210> 473
<211> 17
<212> DNA
<213> Artificial Sequence

<220>
<223> Description of Artificial Sequence:capture

<400> 473
gacgtgggag agttccg                                                    17

<210> 474
<211> 18
<212> DNA
<213> Artificial Sequence

<220>
<223> Description of Artificial Sequence:capture

<400> 474
cggcctgctg cggagtac                                                   18

<210> 475
<211> 18
<212> DNA
<213> Artificial Sequence

<220>
<223> Description of Artificial Sequence:capture

<400> 475
cggcctgatg aggagtac                                                   18

<210> 476
<211> 18
<212> DNA
<213> Artificial Sequence

<220>
<223> Description of Artificial Sequence:capture

<400> 476
cggcctgacg aggagtac                                                   18

<210> 477
<211> 18
<212> DNA
<213> Artificial Sequence

<220>
<223> Description of Artificial Sequence:capture

<400> 477
gcggcctgag gcggagta                                                   18

<210> 478
<211> 18
<212> DNA
<213> Artificial Sequence

<220>
<223> Description of Artificial Sequence:capture

<400> 478
cggcctgagg aggagtac                                                    18

<210> 479
<211> 18
<212> DNA
<213> Artificial Sequence

<220>
<223> Description of Artificial Sequence:capture

<400> 479
cggcctgatg aggactac                                                    18

<210> 480
<211> 18
<212> DNA
<213> Artificial Sequence

<220>
<223> Description of Artificial Sequence:capture

<400> 480
cggcctgatg aggtgtac                                                    18

<210> 481
<211> 17
<212> DNA
<213> Artificial Sequence

<220>
<223> Description of Artificial Sequence:capture

<400> 481
tgatgaggtg tactgga                                                     17

<210> 482
<211> 17
<212> DNA
<213> Artificial Sequence

<220>
<223> Description of Artificial Sequence:capture

<400> 482
gatgaggact actggaa                                                     17

<210> 483
<211> 17
<212> DNA
<213> Artificial Sequence

<220>
<223> Description of Artificial Sequence:capture

<400> 483
agaaggacat cctggag                                                     17

<210> 484
<211> 17
<212> DNA
<213> Artificial Sequence

<220>
<223> Description of Artificial Sequence:capture

<400> 484
agaaggacct cctggag                                                    17

<210> 485
<211> 17
<212> DNA
<213> Artificial Sequence

<220>
<223> Description of Artificial Sequence:capture

<400> 485
agaaggactt cctggag                                                    17

<210> 486
<211> 17
<212> DNA
<213> Artificial Sequence

<220>
<223> Description of Artificial Sequence:capture

<400> 486
agaaggacct cctgtag                                                    17

<210> 487
<211> 17
<212> DNA
<213> Artificial Sequence

<220>
<223> Description of Artificial Sequence:capture

<400> 487
gaaggacaac ctggagg                                                    17

<210> 488
<211> 17
<212> DNA
<213> Artificial Sequence

<220>
<223> Description of Artificial Sequence:capture

<400> 488
acctcctgta ggagaag                                                    17

<210> 489
<211> 17

<212> DNA
<213> Artificial Sequence

<220>
<223> Description of Artificial Sequence:capture

<400> 489
tggaggagaa gcgggca                                                    17

<210> 490
<211> 17
<212> DNA
<213> Artificial Sequence

<220>
<223> Description of Artificial Sequence:capture

<400> 490
tggaggagga gcgggca                                                    17

<210> 491
<211> 17
<212> DNA
<213> Artificial Sequence

<220>
<223> Description of Artificial Sequence:capture

<400> 491
ggaggagagg cgggcag                                                    17

<210> 492
<211> 17
<212> DNA
<213> Artificial Sequence

<220>
<223> Description of Artificial Sequence:capture

<400> 492
tgtaggagaa gcgggca                                                    17

<210> 493
<211> 17
<212> DNA
<213> Artificial Sequence

<220>
<223> Description of Artificial Sequence:capture

<400> 493
agcgggcatt gccggac                                                    17

<210> 494
<211> 17
<212> DNA
<213> Artificial Sequence

<220>
<223> Description of Artificial Sequence:capture

<400> 494
gcttgtgctg gacaggg                                                    17

<210> 495
<211> 17
<212> DNA
<213> Artificial Sequence

<220>
<223> Description of Artificial Sequence:capture

<400> 495
ccggacagag tatgcag                                                    17

<210> 496
<211> 19
<212> DNA
<213> Artificial Sequence

<220>
<223> Description of Artificial Sequence:capture

<400> 496
cggacagggt atgcagaca                                                  19

<210> 497
<211> 19
<212> DNA
<213> Artificial Sequence

<220>
<223> Description of Artificial Sequence:capture

<400> 497
cggacaggat gtgcagaca                                                  19

<210> 498
<211> 19
<212> DNA
<213> Artificial Sequence

<220>
<223> Description of Artificial Sequence:capture

<400> 498
tggacagggt atgcagaca                                                  19

<210> 499
<211> 17
<212> DNA
<213> Artificial Sequence

<220>
<223> Description of Artificial Sequence:capture

<400> 499
cggacaggat atgcaga                                                    17

<210> 500
<211> 17
<212> DNA
<213> Artificial Sequence

<220>
<223> Description of Artificial Sequence:capture

<400> 500
cgagctggac gaggccg                                                    17

<210> 501
<211> 17
<212> DNA
<213> Artificial Sequence

<220>
<223> Description of Artificial Sequence:capture

<400> 501
cgagctgggc gggccca                                                    17

<210> 502
<211> 17
<212> DNA
<213> Artificial Sequence

<220>
<223> Description of Artificial Sequence:capture

<400> 502
cgagctggtc gggccca                                                    17

<210> 503
<211> 17
<212> DNA
<213> Artificial Sequence

<220>
<223> Description of Artificial Sequence:capture

<400> 503
gtgaccctac agcgccg                                                    17

<210> 504
<211> 21
<212> DNA
<213> Artificial Sequence

<220>
<223> Description of Artificial Sequence:primer

<400> 504
gattcctcgc agaggatttc g                                               21

<210> 505
<211> 21
<212> DNA
<213> Artificial Sequence

<220>
<223> Description of Artificial Sequence:primer

<400> 505
gattccccgc agaggatttc g                                                21

<210> 506
<211> 17
<212> DNA
<213> Artificial Sequence

<220>
<223> Description of Artificial Sequence:primer

<400> 506
gcgacgacgc tcacctc                                                     17

<210> 507
<211> 18
<212> DNA
<213> Artificial Sequence

<220>
<223> Description of Artificial Sequence:capture

<400> 507
atttcgtgta ccagttta                                                    18

<210> 508
<211> 18
<212> DNA
<213> Artificial Sequence

<220>
<223> Description of Artificial Sequence:capture

<400> 508
atttcgtgtt ccagttta                                                    18

<210> 509
<211> 18
<212> DNA
<213> Artificial Sequence

<220>
<223> Description of Artificial Sequence:capture

<400> 509
atttcgtgct ccagttta                                                    18

<210> 510
<211> 17
<212> DNA

<213> Artificial Sequence

<220>
<223> Description of Artificial Sequence:capture

<400> 510
tttaagggcc tgtgcta                                                    17

<210> 511
<211> 17
<212> DNA
<213> Artificial Sequence

<220>
<223> Description of Artificial Sequence:capture

<400> 511
ttaagggcat gtgctac                                                    17

<210> 512
<211> 17
<212> DNA
<213> Artificial Sequence

<220>
<223> Description of Artificial Sequence:capture

<400> 512
tttaaggcca tgtgcta                                                    17

<210> 513
<211> 17
<212> DNA
<213> Artificial Sequence

<220>
<223> Description of Artificial Sequence:capture

<400> 513
ttcaccaatg ggacgga                                                    17

<210> 514
<211> 17
<212> DNA
<213> Artificial Sequence

<220>
<223> Description of Artificial Sequence:capture

<400> 514
aacgggacag agcgcgt                                                    17

<210> 515
<211> 17
<212> DNA
<213> Artificial Sequence

<220>

<223> Description of Artificial Sequence:capture

<400> 515
aacgggaccg agcgcgt                                                    17

<210> 516
<211> 17
<212> DNA
<213> Artificial Sequence

<220>
<223> Description of Artificial Sequence:capture

<400> 516
gaccgagctc gtgcggg                                                    17

<210> 517
<211> 17
<212> DNA
<213> Artificial Sequence

<220>
<223> Description of Artificial Sequence:capture

<400> 517
gcgtgcgggg tgtgacc                                                    17

<210> 518
<211> 17
<212> DNA
<213> Artificial Sequence

<220>
<223> Description of Artificial Sequence:capture

<400> 518
gcgtgcgtct tgtgacc                                                    17

<210> 519
<211> 17
<212> DNA
<213> Artificial Sequence

<220>
<223> Description of Artificial Sequence:capture

<400> 519
gcgtgcgtta tgtgacc                                                    17

<210> 520
<211> 17
<212> DNA
<213> Artificial Sequence

<220>
<223> Description of Artificial Sequence:capture

<400> 520

gcgtgcgtct tgtgacc                                                    17

<210> 521
<211> 17
<212> DNA
<213> Artificial Sequence

<220>
<223> Description of Artificial Sequence:capture

<400> 521
gcgtgcgtct tgtgagc                                                    17

<210> 522
<211> 17
<212> DNA
<213> Artificial Sequence

<220>
<223> Description of Artificial Sequence:capture

<400> 522
tcgtgcgggg tgtgacc                                                    17

<210> 523
<211> 17
<212> DNA
<213> Artificial Sequence

<220>
<223> Description of Artificial Sequence:capture

<400> 523
gcgtgcgtct tgtaacc                                                    17

<210> 524
<211> 17
<212> DNA
<213> Artificial Sequence

<220>
<223> Description of Artificial Sequence:capture

<400> 524
cgtcttgtaa ccagata                                                    17

<210> 525
<211> 18
<212> DNA
<213> Artificial Sequence

<220>
<223> Description of Artificial Sequence:capture

<400> 525
tcttgtgagc agaagcat                                                   18

<210> 526

<211> 17
<212> DNA
<213> Artificial Sequence

<220>
<223> Description of Artificial Sequence:capture

<400> 526
taaccagata catctat                                                    17

<210> 527
<211> 18
<212> DNA
<213> Artificial Sequence

<220>
<223> Description of Artificial Sequence:capture

<400> 527
tgagcagaag catctata                                                   18

<210> 528
<211> 17
<212> DNA
<213> Artificial Sequence

<220>
<223> Description of Artificial Sequence:capture

<400> 528
aaccgagaag agtacgt                                                    17

<210> 529
<211> 18
<212> DNA
<213> Artificial Sequence

<220>
<223> Description of Artificial Sequence:capture

<400> 529
gagaagagat cgtgcgct                                                   18

<210> 530
<211> 16
<212> DNA
<213> Artificial Sequence

<220>
<223> Description of Artificial Sequence:capture

<400> 530
gagaggagac gtgcgc                                                     16

<210> 531
<211> 17
<212> DNA
<213> Artificial Sequence

<220>
<223> Description of Artificial Sequence:capture

<400> 531
tgaccagata catctat                                                    17

<210> 532
<211> 17
<212> DNA
<213> Artificial Sequence

<220>
<223> Description of Artificial Sequence:capture

<400> 532
aaccgagaag agatcgt                                                    17

<210> 533
<211> 16
<212> DNA
<213> Artificial Sequence

<220>
<223> Description of Artificial Sequence:capture

<400> 533
gagtacgtgc gcttcg                                                     16

<210> 534
<211> 16
<212> DNA
<213> Artificial Sequence

<220>
<223> Description of Artificial Sequence:capture

<400> 534
gagtacgcac gcttcg                                                     16

<210> 535
<211> 17
<212> DNA
<213> Artificial Sequence

<220>
<223> Description of Artificial Sequence:capture

<400> 535
ggagtacgcg cgcttcg                                                    17

<210> 536
<211> 16
<212> DNA
<213> Artificial Sequence

<220>
<223> Description of Artificial Sequence:capture

<400> 536
gagatcgtgc gcttcg                                                    16

<210> 537
<211> 16
<212> DNA
<213> Artificial Sequence

<220>
<223> Description of Artificial Sequence:capture

<400> 537
gaggacgtgc gcttcg                                                    16

<210> 538
<211> 16
<212> DNA
<213> Artificial Sequence

<220>
<223> Description of Artificial Sequence:capture

<400> 538
cgacgtggag gtgtac                                                    16

<210> 539
<211> 17
<212> DNA
<213> Artificial Sequence

<220>
<223> Description of Artificial Sequence:capture

<400> 539
cgtgggggag ttccggg                                                   17

<210> 540
<211> 16
<212> DNA
<213> Artificial Sequence

<220>
<223> Description of Artificial Sequence:capture

<400> 540
ggggtgtacc gggcag                                                    16

<210> 541
<211> 16
<212> DNA
<213> Artificial Sequence

<220>
<223> Description of Artificial Sequence:capture

<400> 541
ggggtgtatc gggcgg                                                    16

<210> 542
<211> 16
<212> DNA
<213> Artificial Sequence

<220>
<223> Description of Artificial Sequence:capture

<400> 542
ggggagttcc gggcgg                                                    16

<210> 543
<211> 16
<212> DNA
<213> Artificial Sequence

<220>
<223> Description of Artificial Sequence:capture

<400> 543
ggggtgtacc gggcgg                                                    16

<210> 544
<211> 16
<212> DNA
<213> Artificial Sequence

<220>
<223> Description of Artificial Sequence:capture

<400> 544
gaggtgtacc gggcgg                                                    16

<210> 545
<211> 16
<212> DNA
<213> Artificial Sequence

<220>
<223> Description of Artificial Sequence:capture

<400> 545
ggggtgtatc gggtgg                                                    16

<210> 546
<211> 16
<212> DNA
<213> Artificial Sequence

<220>
<223> Description of Artificial Sequence:capture

<400> 546
ggggtgtatc gggcgg                                                    16

<210> 547
<211> 16

<212> DNA
<213> Artificial Sequence

<220>
<223> Description of Artificial Sequence:capture

<400> 547
ggggtgtacc gcgcgg                                                    16

<210> 548
<211> 17
<212> DNA
<213> Artificial Sequence

<220>
<223> Description of Artificial Sequence:capture

<400> 548
gtgtaccgcg cggtgac                                                   17

<210> 549
<211> 17
<212> DNA
<213> Artificial Sequence

<220>
<223> Description of Artificial Sequence:capture

<400> 549
taccgggcag tgacgcc                                                   17

<210> 550
<211> 16
<212> DNA
<213> Artificial Sequence

<220>
<223> Description of Artificial Sequence:capture

<400> 550
tatcgggtgg tgacgc                                                    16

<210> 551
<211> 17
<212> DNA
<213> Artificial Sequence

<220>
<223> Description of Artificial Sequence:capture

<400> 551
gcggtgaccc cgcaggg                                                   17

<210> 552
<211> 17
<212> DNA
<213> Artificial Sequence

<220>
<223> Description of Artificial Sequence:capture

<400> 552
ggtgacgctg caggggc                                                17

<210> 553
<211> 17
<212> DNA
<213> Artificial Sequence

<220>
<223> Description of Artificial Sequence:capture

<400> 553
gacgctgctg gggctgc                                                17

<210> 554
<211> 17
<212> DNA
<213> Artificial Sequence

<220>
<223> Description of Artificial Sequence:capture

<400> 554
gctggggctg cctgccg                                                17

<210> 555
<211> 17
<212> DNA
<213> Artificial Sequence

<220>
<223> Description of Artificial Sequence:capture

<400> 555
gctggggccg cctgacg                                                17

<210> 556
<211> 17
<212> DNA
<213> Artificial Sequence

<220>
<223> Description of Artificial Sequence:capture

<400> 556
ggggcggctt gacgccg                                                17

<210> 557
<211> 17
<212> DNA
<213> Artificial Sequence

<220>
<223> Description of Artificial Sequence:capture

<400> 557
gctggggccg cctgccg                                                    17

<210> 558
<211> 17
<212> DNA
<213> Artificial Sequence

<220>
<223> Description of Artificial Sequence:capture

<400> 558
gctggggctg cctgacg                                                    17

<210> 559
<211> 17
<212> DNA
<213> Artificial Sequence

<220>
<223> Description of Artificial Sequence:capture

<400> 559
gcggcctgtt gccgagt                                                    17

<210> 560
<211> 17
<212> DNA
<213> Artificial Sequence

<220>
<223> Description of Artificial Sequence:capture

<400> 560
gcggcctagc gccgagt                                                    17

<210> 561
<211> 17
<212> DNA
<213> Artificial Sequence

<220>
<223> Description of Artificial Sequence:capture

<400> 561
gcggcctgac gccgagt                                                    17

<210> 562
<211> 17
<212> DNA
<213> Artificial Sequence

<220>
<223> Description of Artificial Sequence:capture

<400> 562
gctgcctgcc gccgagt                                                    17

<210> 563
<211> 17
<212> DNA
<213> Artificial Sequence

<220>
<223> Description of Artificial Sequence:capture

<400> 563
gcggcctgat gccgagt                                                    17

<210> 564
<211> 17
<212> DNA
<213> Artificial Sequence

<220>
<223> Description of Artificial Sequence:capture

<400> 564
gcggcctgtc gccgagt                                                    17

<210> 565
<211> 17
<212> DNA
<213> Artificial Sequence

<220>
<223> Description of Artificial Sequence:capture

<400> 565
gctgcctgac gccgagt                                                    17

<210> 566
<211> 17
<212> DNA
<213> Artificial Sequence

<220>
<223> Description of Artificial Sequence:capture

<400> 566
gccgcctgcc gccgagt                                                    17

<210> 567
<211> 17
<212> DNA
<213> Artificial Sequence

<220>
<223> Description of Artificial Sequence:capture

<400> 567
gcggcctgat gccgaga                                                    17

<210> 568
<211> 17
<212> DNA

<213> Artificial Sequence

<220>
<223> Description of Artificial Sequence:capture

<400> 568
gccgcctgac gccgagt                                                    17

<210> 569
<211> 17
<212> DNA
<213> Artificial Sequence

<220>
<223> Description of Artificial Sequence:capture

<400> 569
gcggcttgac gccgagt                                                    17

<210> 570
<211> 17
<212> DNA
<213> Artificial Sequence

<220>
<223> Description of Artificial Sequence:capture

<400> 570
atgccgagaa ctggaac                                                    17

<210> 571
<211> 17
<212> DNA
<213> Artificial Sequence

<220>
<223> Description of Artificial Sequence:capture

<400> 571
tactggaata gccagaa                                                    17

<210> 572
<211> 16
<212> DNA
<213> Artificial Sequence

<220>
<223> Description of Artificial Sequence:capture

<400> 572
agaaggaagt cctgga                                                     16

<210> 573
<211> 16
<212> DNA
<213> Artificial Sequence

<220>

<223> Description of Artificial Sequence:capture

<400> 573
agaaggacat cctgga                                                    16

<210> 574
<211> 18
<212> DNA
<213> Artificial Sequence

<220>
<223> Description of Artificial Sequence:capture

<400> 574
ctggaggggg cccgggcg                                                  18

<210> 575
<211> 18
<212> DNA
<213> Artificial Sequence

<220>
<223> Description of Artificial Sequence:capture

<400> 575
ctggaggagg accgggcg                                                  18

<210> 576
<211> 18
<212> DNA
<213> Artificial Sequence

<220>
<223> Description of Artificial Sequence:capture

<400> 576
ctggagagga aacgggcg                                                  18

<210> 577
<211> 18
<212> DNA
<213> Artificial Sequence

<220>
<223> Description of Artificial Sequence:capture

<400> 577
ctggagagga cccgggcg                                                  18

<210> 578
<211> 18
<212> DNA
<213> Artificial Sequence

<220>
<223> Description of Artificial Sequence:capture

<400> 578

ctggagggga cccgggcg                                                    18

<210> 579
<211> 18
<212> DNA
<213> Artificial Sequence

<220>
<223> Description of Artificial Sequence:capture

<400> 579
ctggagagga cccgagcg                                                    18

<210> 580
<211> 17
<212> DNA
<213> Artificial Sequence

<220>
<223> Description of Artificial Sequence:capture

<400> 580
aggacccgag cggagtt                                                     17

<210> 581
<211> 17
<212> DNA
<213> Artificial Sequence

<220>
<223> Description of Artificial Sequence:capture

<400> 581
aacgggcggc ggtggac                                                     17

<210> 582
<211> 17
<212> DNA
<213> Artificial Sequence

<220>
<223> Description of Artificial Sequence:capture

<400> 582
cgggcggagt tggacac                                                     17

<210> 583
<211> 17
<212> DNA
<213> Artificial Sequence

<220>
<223> Description of Artificial Sequence:capture

<400> 583
gttggacacg gtgtgca                                                     17

<210> 584

<211> 17
<212> DNA
<213> Artificial Sequence

<220>
<223> Description of Artificial Sequence:capture

<400> 584
gtggacagag tgtgcag                                                    17

<210> 585
<211> 17
<212> DNA
<213> Artificial Sequence

<220>
<223> Description of Artificial Sequence:capture

<400> 585
gacaccgtat gcagaca                                                    17

<210> 586
<211> 18
<212> DNA
<213> Artificial Sequence

<220>
<223> Description of Artificial Sequence:capture

<400> 586
gaggtggcgt accgcggg                                                   18

<210> 587
<211> 18
<212> DNA
<213> Artificial Sequence

<220>
<223> Description of Artificial Sequcnce:capture

<400> 587
cagttggagc tccgcacg                                                   18

<210> 588
<211> 17
<212> DNA
<213> Artificial Sequence

<220>
<223> Description of Artificial Sequence:capture

<400> 588
ggtggcgttc cgcggga                                                    17

<210> 589
<211> 17
<212> DNA
<213> Artificial Sequence

<220>
<223> Description of Artificial Sequence:capture

<400> 589
ccaggtgggg taccgcg                                                    17

<210> 590
<211> 19
<212> DNA
<213> Artificial Sequence

<220>
<223> Description of Artificial Sequence:primer

<400> 590
tgtccccaca gcacgtttc                                                  19

<210> 591
<211> 18
<212> DNA
<213> Artificial Sequence

<220>
<223> Description of Artificial Sequence:primer

<400> 591
gtccccccag cacgtttc                                                   18

<210> 592
<211> 18
<212> DNA
<213> Artificial Sequence

<220>
<223> Description of Artificial Sequence:primer

<400> 592
gcccccacag cacgtttc                                                   18

<210> 593
<211> 18
<212> DNA
<213> Artificial Sequence

<220>
<223> Description of Artificial Sequence:primer

<400> 593
cgccgctgca ctgtgaag                                                   18

<210> 594
<211> 18
<212> DNA
<213> Artificial Sequence

<220>
<223> Description of Artificial Sequence:capture

<400> 594
tggagctgct taagtctg                                                    18

<210> 595
<211> 17
<212> DNA
<213> Artificial Sequence

<220>
<223> Description of Artificial Sequence:capture

<400> 595
cagggtaaat ataagtg                                                     17

<210> 596
<211> 16
<212> DNA
<213> Artificial Sequence

<220>
<223> Description of Artificial Sequence:capture

<400> 596
gcaggttaaa catgag                                                      16

<210> 597
<211> 18
<212> DNA
<213> Artificial Sequence

<220>
<223> Description of Artificial Sequence:capture

<400> 597
actctacggg tgagtgtt                                                    18

<210> 598
<211> 18
<212> DNA
<213> Artificial Sequence

<220>
<223> Description of Artificial Sequence:capture

<400> 598
actctagggg tgagtgtt                                                    18

<210> 599
<211> 18
<212> DNA
<213> Artificial Sequence

<220>
<223> Description of Artificial Sequence:capture

<400> 599
actctatggg tgagtgtt                                                    18

<210> 600
<211> 17
<212> DNA
<213> Artificial Sequence

<220>
<223> Description of Artificial Sequence:capture

<400> 600
ttgaatgtca tttcttc                                                    17

<210> 601
<211> 17
<212> DNA
<213> Artificial Sequence

<220>
<223> Description of Artificial Sequence:capture

<400> 601
gtgagtgtta tttcttc                                                    17

<210> 602
<211> 17
<212> DNA
<213> Artificial Sequence

<220>
<223> Description of Artificial Sequence:capture

<400> 602
gagtgtcaat tcttcaa                                                    17

<210> 603
<211> 17
<212> DNA
<213> Artificial Sequence

<220>
<223> Description of Artificial Sequence:capture

<400> 603
ctgagtgtca tttcttc                                                    17

<210> 604
<211> 17
<212> DNA
<213> Artificial Sequence

<220>
<223> Description of Artificial Sequence:capture

<400> 604
atgagtgtca tttcttc                                                    17

<210> 605
<211> 17

<212> DNA
<213> Artificial Sequence

<220>
<223> Description of Artificial Sequence:capture

<400> 605
ataagtgtca tttcttc                                                    17

<210> 606
<211> 17
<212> DNA
<213> Artificial Sequence

<220>
<223> Description of Artificial Sequence:capture

<400> 606
ttgagtgtca tttcttc                                                    17

<210> 607
<211> 17
<212> DNA
<213> Artificial Sequence

<220>
<223> Description of Artificial Sequence:capture

<400> 607
gtgagtgtta tttcttc                                                    17

<210> 608
<211> 17
<212> DNA
<213> Artificial Sequence

<220>
<223> Description of Artificial Sequence:capture

<400> 608
gcgggtgcag ttcctgg                                                    17

<210> 609
<211> 19
<212> DNA
<213> Artificial Sequence

<220>
<223> Description of Artificial Sequence:capture

<400> 609
gggtgcggtt gctggaaag                                                  19

<210> 610
<211> 17
<212> DNA
<213> Artificial Sequence

<220>
<223> Description of Artificial Sequence:capture

<400> 610
gtgcggttcc tggagag                                                    17

<210> 611
<211> 17
<212> DNA
<213> Artificial Sequence

<220>
<223> Description of Artificial Sequence:capture

<400> 611
gtgcggtacc tggacag                                                    17

<210> 612
<211> 17
<212> DNA
<213> Artificial Sequence

<220>
<223> Description of Artificial Sequence:capture

<400> 612
gtgcggtatc tgcacag                                                    17

<210> 613
<211> 17
<212> DNA
<213> Artificial Sequence

<220>
<223> Description of Artificial Sequence:capture

<400> 613
gtgcggttac tggagag                                                    17

<210> 614
<211> 17
<212> DNA
<213> Artificial Sequence

<220>
<223> Description of Artificial Sequence:capture

<400> 614
gtgcggttcc tggacag                                                    17

<210> 615
<211> 17
<212> DNA
<213> Artificial Sequence

<220>
<223> Description of Artificial Sequence:capture

<400> 615
gtgcagttcc tggaaag                                                    17

<210> 616
<211> 17
<212> DNA
<213> Artificial Sequence

<220>
<223> Description of Artificial Sequence:capture

<400> 616
gtgcagttcc tggacag                                                    17

<210> 617
<211> 17
<212> DNA
<213> Artificial Sequence

<220>
<223> Description of Artificial Sequence:capture

<400> 617
gtgcggttcc cggacag                                                    17

<210> 618
<211> 17
<212> DNA
<213> Artificial Sequence

<220>
<223> Description of Artificial Sequence:capture

<400> 618
gcggttcccg gacagat                                                    17

<210> 619
<211> 17
<212> DNA
<213> Artificial Sequence

<220>
<223> Description of Artificial Sequence:capture

<400> 619
gttgctggaa agatgca                                                    17

<210> 620
<211> 17
<212> DNA
<213> Artificial Sequence

<220>
<223> Description of Artificial Sequence:capture

<400> 620
gtacctggac agatact                                                    17

<210> 621
<211> 17
<212> DNA
<213> Artificial Sequence

<220>
<223> Description of Artificial Sequence:capture

<400> 621
gttcctggag agatact                                                    17

<210> 622
<211> 17
<212> DNA
<213> Artificial Sequence

<220>
<223> Description of Artificial Sequence:capture

<400> 622
gtatctgcac agaggca                                                    17

<210> 623
<211> 17
<212> DNA
<213> Artificial Sequence

<220>
<223> Description of Artificial Sequence:capture

<400> 623
gttcctggaa agactct                                                    17

<210> 624
<211> 17
<212> DNA
<213> Artificial Sequence

<220>
<223> Description of Artificial Sequence:capture

<400> 624
gttcctggac agatact                                                    17

<210> 625
<211> 17
<212> DNA
<213> Artificial Sequence

<220>
<223> Description of Artificial Sequence:capture

<400> 625
gttactggag agacact                                                    17

<210> 626
<211> 17
<212> DNA

<213> Artificial Sequence

<220>
<223> Description of Artificial Sequence:capture

<400> 626
gttcctggaa agtctct                                                    17

<210> 627
<211> 17
<212> DNA
<213> Artificial Sequence

<220>
<223> Description of Artificial Sequence:capture

<400> 627
gtacctggac agatact                                                    17

<210> 628
<211> 17
<212> DNA
<213> Artificial Sequence

<220>
<223> Description of Artificial Sequence:capture

<400> 628
gttgctggaa agacgcg                                                    17

<210> 629
<211> 17
<212> DNA
<213> Artificial Sequence

<220>
<223> Description of Artificial Sequence:capture

<400> 629
gttcctggac agacact                                                    17

<210> 630
<211> 17
<212> DNA
<213> Artificial Sequence

<220>
<223> Description of Artificial Sequence:capture

<400> 630
gttcccggac agatact                                                    17

<210> 631
<211> 17
<212> DNA
<213> Artificial Sequence

<220>

<223> Description of Artificial Sequence:capture

<400> 631
ctggaaagtc tcttcta                                                    17

<210> 632
<211> 17
<212> DNA
<213> Artificial Sequence

<220>
<223> Description of Artificial Sequence:capture

<400> 632
ggacagacac ttctata                                                    17

<210> 633
<211> 16
<212> DNA
<213> Artificial Sequence

<220>
<223> Description of Artificial Sequence:capture

<400> 633
gatgcatcta taacca                                                     16

<210> 634
<211> 16
<212> DNA
<213> Artificial Sequence

<220>
<223> Description of Artificial Sequence:capture

<400> 634
ataaccaaga ggagtc                                                     16

<210> 635
<211> 16
<212> DNA
<213> Artificial Sequence

<220>
<223> Description of Artificial Sequence:capture

<400> 635
gatacttcca taacca                                                     16

<210> 636
<211> 16
<212> DNA
<213> Artificial Sequence

<220>
<223> Description of Artificial Sequence:capture

<400> 636

ataaccagga ggagaa                                                    16

<210> 637
<211> 16
<212> DNA
<213> Artificial Sequence

<220>
<223> Description of Artificial Sequence:capture

<400> 637
gactcttcta taacca                                                    16

<210> 638
<211> 16
<212> DNA
<213> Artificial Sequence

<220>
<223> Description of Artificial Sequencc:capture

<400> 638
ataaccagga ggagtt                                                    16

<210> 639
<211> 17
<212> DNA
<213> Artificial Sequence

<220>
<223> Description of Artificial Sequence:capture

<400> 639
acttctatca ccaagag                                                   17

<210> 640
<211> 16
<212> DNA
<213> Artificial Sequence

<220>
<223> Description of Artificial Sequence:capture

<400> 640
ataaccggga ggagaa                                                    16

<210> 641
<211> 16
<212> DNA
<213> Artificial Sequence

<220>
<223> Description of Artificial Sequence:capture

<400> 641
gacgcgtcca taacca                                                    16

<210> 642

<211> 16
<212> DNA
<213> Artificial Sequence

<220>
<223> Description of Artificial Sequence:capture

<400> 642
ataaccaaga ggagta                                                    16

<210> 643
<211> 16
<212> DNA
<213> Artificial Sequence

<220>
<223> Description of Artificial Sequence:capture

<400> 643
gaggcatcta taacca                                                    16

<210> 644
<211> 16
<212> DNA
<213> Artificial Sequence

<220>
<223> Description of Artificial Sequence:capture

<400> 644
ataaccaaga ggagta                                                    16

<210> 645
<211> 16
<212> DNA
<213> Artificial Sequence

<220>
<223> Description of Artificial Sequence:capture

<400> 645
gacacttcca taacca                                                    16

<210> 646
<211> 16
<212> DNA
<213> Artificial Sequence

<220>
<223> Description of Artificial Sequence:capture

<400> 646
ataaccagga ggagtc                                                    16

<210> 647
<211> 16
<212> DNA
<213> Artificial Sequence

<220>
<223> Description of Artificial Sequence:capture

<400> 647
gatacttcta taacca                                                    16

<210> 648
<211> 16
<212> DNA
<213> Artificial Sequence

<220>
<223> Description of Artificial Sequence:capture

<400> 648
ataaccagga ggagta                                                    16

<210> 649
<211> 17
<212> DNA
<213> Artificial Sequence

<220>
<223> Description of Artificial Sequence:capture

<400> 649
acttctatca ccaagaa                                                   17

<210> 650
<211> 16
<212> DNA
<213> Artificial Sequence

<220>
<223> Description of Artificial Sequence:capture

<400> 650
gatacttcta taacca                                                    16

<210> 651
<211> 16
<212> DNA
<213> Artificial Sequence

<220>
<223> Description of Artificial Sequence:capture

<400> 651
ataaccaaga ggagga                                                    16

<210> 652
<211> 16
<212> DNA
<213> Artificial Sequence

<220>
<223> Description of Artificial Sequence:capture

<400> 652
ataaccagga ggagtt                                                    16

<210> 653
<211> 16
<212> DNA
<213> Artificial Sequence

<220>
<223> Description of Artificial Sequence:capture

<400> 653
gatacttcta taacca                                                    16

<210> 654
<211> 16
<212> DNA
<213> Artificial Sequence

<220>
<223> Description of Artificial Sequence:capture

<400> 654
ataaccagga ggagtc                                                    16

<210> 655
<211> 16
<212> DNA
<213> Artificial Sequence

<220>
<223> Description of Artificial Sequence:capture

<400> 655
ataaccaaga ggagtt                                                    16

<210> 656
<211> 16
<212> DNA
<213> Artificial Sequence

<220>
<223> Description of Artificial Sequence:capture

<400> 656
ataaccagga ggagct                                                    16

<210> 657
<211> 16
<212> DNA
<213> Artificial Sequence

<220>
<223> Description of Artificial Sequence:capture

<400> 657
gacacttcta taacca                                                    16

<210> 658
<211> 16
<212> DNA
<213> Artificial Sequence

<220>
<223> Description of Artificial Sequence:capture

<400> 658
ataaccagga ggagtc                                                        16

<210> 659
<211> 16
<212> DNA
<213> Artificial Sequence

<220>
<223> Description of Artificial Sequence:capture

<400> 659
ataaccaaga ggagaa                                                        16

<210> 660
<211> 16
<212> DNA
<213> Artificial Sequence

<220>
<223> Description of Artificial Sequence:capture

<400> 660
ataaccagga ggagtt                                                        16

<210> 661
<211> 16
<212> DNA
<213> Artificial Sequence

<220>
<223> Description of Artificial Sequence:capture

<400> 661
gatacttcta taacca                                                        16

<210> 662
<211> 16
<212> DNA
<213> Artificial Sequence

<220>
<223> Description of Artificial Sequence:capture

<400> 662
ataaccagga ggagaa                                                        16

<210> 663
<211> 16

<212> DNA
<213> Artificial Sequence

<220>
<223> Description of Artificial Sequence:capture

<400> 663
ataaccaaga ggagtc                                                    16

<210> 664
<211> 17
<212> DNA
<213> Artificial Sequence

<220>
<223> Description of Artificial Sequence:capture

<400> 664
aggagaacct gcgcttc                                                   17

<210> 665
<211> 17
<212> DNA
<213> Artificial Sequence

<220>
<223> Description of Artificial Sequence:capture

<400> 665
caccaagaag agtacgt                                                   17

<210> 666
<211> 17
<212> DNA
<213> Artificial Sequence

<220>
<223> Description of Artificial Sequence:capture

<400> 666
aggaggactt gcgcttc                                                   17

<210> 667
<211> 16
<212> DNA
<213> Artificial Sequence

<220>
<223> Description of Artificial Sequence:capture

<400> 667
agaggagtcc gtgcgc                                                    16

<210> 668
<211> 16
<212> DNA
<213> Artificial Sequence

<220>
<223> Description of Artificial Sequence:capture

<400> 668
agaggagaac gtgcgc                                                    16

<210> 669
<211> 16
<212> DNA
<213> Artificial Sequence

<220>
<223> Description of Artificial Sequence:capture

<400> 669
agaggagtac gtgcgc                                                    16

<210> 670
<211> 16
<212> DNA
<213> Artificial Sequence

<220>
<223> Description of Artificial Sequence:capture

<400> 670
agaggagttc gtgcgc                                                    16

<210> 671
<211> 16
<212> DNA
<213> Artificial Sequence

<220>
<223> Description of Artificial Sequence:capture

<400> 671
agaggaggac gtgcgc                                                    16

<210> 672
<211> 16
<212> DNA
<213> Artificial Sequence

<220>
<223> Description of Artificial Sequence:capture

<400> 672
ggaggagctc ctgcgc                                                    16

<210> 673
<211> 16
<212> DNA
<213> Artificial Sequence

<220>
<223> Description of Artificial Sequence:capture

<400> 673
ggaggagtcc gtgcgc                                                        16

<210> 674
<211> 16
<212> DNA
<213> Artificial Sequence

<220>
<223> Description of Artificial Sequence:capture

<400> 674
ggaggagaac gtgcgc                                                        16

<210> 675
<211> 16
<212> DNA
<213> Artificial Sequence

<220>
<223> Description of Artificial Sequence:capture

<400> 675
agaagagtac gtgcgc                                                        16

<210> 676
<211> 16
<212> DNA
<213> Artificial Sequence

<220>
<223> Description of Artificial Sequence:capture

<400> 676
ggaggagttc gtgcgc                                                        16

<210> 677
<211> 16
<212> DNA
<213> Artificial Sequence

<220>
<223> Description of Artificial Sequence:capture

<400> 677
agaggaggac ttgcgc                                                        16

<210> 678
<211> 16
<212> DNA
<213> Artificial Sequence

<220>
<223> Description of Artificial Sequence:capture

<400> 678
ggaggagaac ctgcgc                                                        16

<210> 679
<211> 16
<212> DNA
<213> Artificial Sequence

<220>
<223> Description of Artificial Sequence:capture

<400> 679
agaggagtac gtgcgg                                                    16

<210> 680
<211> 16
<212> DNA
<213> Artificial Sequence

<220>
<223> Description of Artificial Sequence:capture

<400> 680
ggaggagttc ctgcgc                                                    16

<210> 681
<211> 16
<212> DNA
<213> Artificial Sequence

<220>
<223> Description of Artificial Sequcnce:capture

<400> 681
agaggagtac gtgcac                                                    16

<210> 682
<211> 16
<212> DNA
<213> Artificial Sequence

<220>
<223> Description of Artificial Sequence:capture

<400> 682
agaggagtac gcgcgc                                                    16

<210> 683
<211> 16
<212> DNA
<213> Artificial Sequence

<220>
<223> Description of Artificial Sequence:capture

<400> 683
ggaggagtac gtgcgc                                                    16

<210> 684
<211> 17
<212> DNA

<213> Artificial Sequence

<220>
<223> Description of Artificial Sequence:capture

<400> 684
gtccgtgcac ttcgaca                                                    17

<210> 685
<211> 17
<212> DNA
<213> Artificial Sequence

<220>
<223> Description of Artificial Sequence:capture

<400> 685
tccgtgcggt tcgacag                                                    17

<210> 686
<211> 17
<212> DNA
<213> Artificial Sequence

<220>
<223> Description of Artificial Sequence:capture

<400> 686
gcgacgtgcg ggagtac                                                    17

<210> 687
<211> 17
<212> DNA
<213> Artificial Sequence

<220>
<223> Description of Artificial Sequence:capture

<400> 687
ggagttcctg gcggtga                                                    17

<210> 688
<211> 17
<212> DNA
<213> Artificial Sequence

<220>
<223> Description of Artificial Sequence:capture

<400> 688
gcgacgtgag ggagtac                                                    17

<210> 689
<211> 17
<212> DNA
<213> Artificial Sequence

<220>

<223> Description of Artificial Sequence:capture

<400> 689
gggggagtac cgggcgg                                                    17

<210> 690
<211> 17
<212> DNA
<213> Artificial Sequence

<220>
<223> Description of Artificial Sequence:capture

<400> 690
gggggagttc cgggcgg                                                    17

<210> 691
<211> 17
<212> DNA
<213> Artificial Sequence

<220>
<223> Description of Artificial Sequence:capture

<400> 691
   gagggagtac cgggcgg                                                   17

<210> 692
<211> 17
<212> DNA
<213> Artificial Sequence

<220>
<223> Description of Artificial Sequence:capture

<400> 692
ggggagttct gggcggt                                                    17

<210> 693
<211> 17
<212> DNA
<213> Artificial Sequence

<220>
<223> Description of Artificial Sequence:capture

<400> 693
gggggagtac cgggtgg                                                    17

<210> 694
<211> 17
<212> DNA
<213> Artificial Sequence

<220>
<223> Description of Artificial Sequence:capture

<400> 694

gggggagttc caggcgg                                                    17

<210> 695
<211> 17
<212> DNA
<213> Artificial Sequence

<220>
<223> Description of Artificial Sequence:capture

<400> 695
gcgggagtac cgggcgg                                                    17

<210> 696
<211> 17
<212> DNA
<213> Artificial Sequence

<220>
<223> Description of Artificial Sequence:capture

<400> 696
ggagttccag gcggtga                                                    17

<210> 697
<211> 17
<212> DNA
<213> Artificial Sequence

<220>
<223> Description of Artificial Sequence:capture

<400> 697
gtaccgggtg gtgacgg                                                    17

<210> 698
<211> 17
<212> DNA
<213> Artificial Sequence

<220>
<223> Description of Artificial Sequence:capture

<400> 698
ccgggcggcg acggagc                                                    17

<210> 699
<211> 17
<212> DNA
<213> Artificial Sequence

<220>
<223> Description of Artificial Sequence:capture

<400> 699
ggcggtgagg gagctgg                                                    17

<210> 700

<211> 17
<212> DNA
<213> Artificial Sequence

<220>
<223> Description of Artificial Sequence:capture

<400> 700
ggcggtgatg gagctgg                                                    17

<210> 701
<211> 20
<212> DNA
<213> Artificial Sequence

<220>
<223> Description of Artificial Sequence:capture

<400> 701
gcctgatgcc gagtactgga                                                 20

<210> 702
<211> 17
<212> DNA
<213> Artificial Sequence

<220>
<223> Description of Artificial Sequence:capture

<400> 702
cggcctagcg ccgagta                                                    17

<210> 703
<211> 20
<212> DNA
<213> Artificial Sequence

<220>
<223> Description of Artificial Sequence:capture

<400> 703
gcctgacgct gagtactgga                                                 20

<210> 704
<211> 16
<212> DNA
<213> Artificial Sequence

<220>
<223> Description of Artificial Sequence:capture

<400> 704
cggcctgtcg ccgagt                                                     16

<210> 705
<211> 17
<212> DNA
<213> Artificial Sequence

<220>
<223> Description of Artificial Sequence:capture

<400> 705
gccgagtcct ggaacag                                                    17

<210> 706
<211> 20
<212> DNA
<213> Artificial Sequence

<220>
<223> Description of Artificial Sequence:capture

<400> 706
gcctgacgcg gagcactgga                                                 20

<210> 707
<211> 17
<212> DNA
<213> Artificial Sequence

<220>
<223> Description of Artificial Sequence:capture

<400> 707
cctgatgctg agtactg                                                    17

<210> 708
<211> 17
<212> DNA
<213> Artificial Sequence

<220>
<223> Description of Artificial Sequence:capture

<400> 708
gcggcctgtt gccgagt                                                    17

<210> 709
<211> 17
<212> DNA
<213> Artificial Sequence

<220>
<223> Description of Artificial Sequence:capture

<400> 709
tgccgagtct gccgagt                                                    17

<210> 710
<211> 17
<212> DNA
<213> Artificial Sequence

<220>
<223> Description of Artificial Sequence:capture

<400> 710
gcggcctatc gccgagt                                                    17

<210> 711
<211> 17
<212> DNA
<213> Artificial Sequence

<220>
<223> Description of Artificial Sequence:capture

<400> 711
ggcctgatac cgagtac                                                    17

<210> 712
<211> 19
<212> DNA
<213> Artificial Sequence

<220>
<223> Description of Artificial Sequence:capture

<400> 712
tgatgcggag cactggaac                                                  19

<210> 713
<211> 20
<212> DNA
<213> Artificial Sequence

<220>
<223> Description of Artificial Sequence:capture

<400> 713
gcctgatgag gagtactgga                                                 20

<210> 714
<211> 17
<212> DNA
<213> Artificial Sequence

<220>
<223> Description of Artificial Sequence:capture

<400> 714
ctgatgccca gtactgg                                                    17

<210> 715
<211> 17
<212> DNA
<213> Artificial Sequence

<220>
<223> Description of Artificial Sequence:capture

<400> 715
tgatgagggg tactgga                                                    17

<210> 716
<211> 17
<212> DNA
<213> Artificial Sequence

<220>
<223> Description of Artificial Sequence:capture

<400> 716
gatgaggact actggaa                                                    17

<210> 717
<211> 17
<212> DNA
<213> Artificial Sequence

<220>
<223> Description of Artificial Sequence:capture

<400> 717
tgatgcggag cactgga                                                    17

<210> 718
<211> 17
<212> DNA
<213> Artificial Sequence

<220>
<223> Description of Artificial Sequence:capture

<400> 718
atgaggagca ctggaac                                                    17

<210> 719
<211> 17
<212> DNA
<213> Artificial Sequence

<220>
<223> Description of Artificial Sequence:capture

<400> 719
cctgtcgccg agtcctg                                                    17

<210> 720
<211> 17
<212> DNA
<213> Artificial Sequence

<220>
<223> Description of Artificial Sequence:capture

<400> 720
tgatgcggag cactgga                                                    17

<210> 721
<211> 17

<212> DNA
<213> Artificial Sequence

<220>
<223> Description of Artificial Sequence:capture

<400> 721
gcctgatgag gagtact                                                    17

<210> 722
<211> 15
<212> DNA
<213> Artificial Sequence

<220>
<223> Description of Artificial Sequence:capture

<400> 722
cctgatgagg gtact                                                      15

<210> 723
<211> 16
<212> DNA
<213> Artificial Sequence

<220>
<223> Description of Artificial Sequence:capture

<400> 723
cctgatgagg actact                                                     16

<210> 724
<211> 16
<212> DNA
<213> Artificial Sequence

<220>
<223> Description of Artificial Sequence:capture

<400> 724
cctgatgagg agcact                                                     16

<210> 725
<211> 17
<212> DNA
<213> Artificial Sequence

<220>
<223> Description of Artificial Sequence:capture

<400> 725
gccagaagaa catcctg                                                    17

<210> 726
<211> 17
<212> DNA
<213> Artificial Sequence

<220>
<223> Description of Artificial Sequence:capture

<400> 726
agaaggacct cctggag                                                    17

<210> 727
<211> 17
<212> DNA
<213> Artificial Sequence

<220>
<223> Description of Artificial Sequence:capture

<400> 727
agaaggacat cctggag                                                    17

<210> 728
<211> 17
<212> DNA
<213> Artificial Sequence

<220>
<223> Description of Artificial Sequence:capture

<400> 728
agaaggactt cctggag                                                    17

<210> 729
<211> 17
<212> DNA
<213> Artificial Sequence

<220>
<223> Description of Artificial Sequence:capture

<400> 729
agaaggacct cctggaa                                                    17

<210> 730
<211> 17
<212> DNA
<213> Artificial Sequence

<220>
<223> Description of Artificial Sequence:capture

<400> 730
agaaggacat cctggaa                                                    17

<210> 731
<211> 17
<212> DNA
<213> Artificial Sequence

<220>
<223> Description of Artificial Sequence:capture

<400> 731
agaaggactt cctggaa                                                    17

<210> 732
<211> 16
<212> DNA
<213> Artificial Sequence

<220>
<223> Description of Artificial Sequence:capture

<400> 732
agaagaacat cctgga                                                     16

<210> 733
<211> 19
<212> DNA
<213> Artificial Sequence

<220>
<223> Description of Artificial Sequence:capture

<400> 733
cctggagcag aggcgggcc                                                  19

<210> 734
<211> 19
<212> DNA
<213> Artificial Sequence

<220>
<223> Description of Artificial Sequence:capture

<400> 734
cctggaagac gagcgggcc                                                  19

<210> 735
<211> 18
<212> DNA
<213> Artificial Sequence

<220>
<223> Description of Artificial Sequence:capture

<400> 735
cctggaagac aagcgggc                                                   18

<210> 736
<211> 17
<212> DNA
<213> Artificial Sequence

<220>
<223> Description of Artificial Sequence:capture

<400> 736
cctggaagac aggcggg                                                    17

<210> 737
<211> 16
<212> DNA
<213> Artificial Sequence

<220>
<223> Description of Artificial Sequence:capture

<400> 737
cctggagcga ggcggg                                                    16

<210> 738
<211> 16
<212> DNA
<213> Artificial Sequence

<220>
<223> Description of Artificial Sequence:capture

<400> 738
ggagcagaag cgggcc                                                    16

<210> 739
<211> 17
<212> DNA
<213> Artificial Sequence

<220>
<223> Description of Artificial Sequence:capture

<400> 739
tggagcaggc gcgggcc                                                   17

<210> 740
<211> 17
<212> DNA
<213> Artificial Sequence

<220>
<223> Description of Artificial Sequence:capture

<400> 740
cctggaggac aggcggg                                                   17

<210> 741
<211> 19
<212> DNA
<213> Artificial Sequence

<220>
<223> Description of Artificial Sequence:capture

<400> 741
cctggagcag aggcaggcc                                                 19

<210> 742
<211> 18
<212> DNA

<213> Artificial Sequence

<220>
<223> Description of Artificial Sequence:capture

<400> 742
cctggaagac gagcgcgc                                                    18

<210> 743
<211> 16
<212> DNA
<213> Artificial Sequence

<220>
<223> Description of Artificial Sequence:capture

<400> 743
cctggaagac agggcc                                                      16

<210> 744
<211> 16
<212> DNA
<213> Artificial Sequence

<220>
<223> Description of Artificial Sequence:capture

<400> 744
cctggaagac aggcgc                                                      16

<210> 745
<211> 19
<212> DNA
<213> Artificial Sequence

<220>
<223> Description of Artificial Sequence:capture

<400> 745
cctggagcgg aggcgtgcc                                                   19

<210> 746
<211> 17
<212> DNA
<213> Artificial Sequence

<220>
<223> Description of Artificial Sequence:capture

<400> 746
ggagcagaag cggggcc                                                     17

<210> 747
<211> 19
<212> DNA
<213> Artificial Sequence

<220>

<223> Description of Artificial Sequence:capture

<400> 747
cctggagcgg aggcgcgcc                                                    19

<210> 748
<211> 17
<212> DNA
<213> Artificial Sequence

<220>
<223> Description of Artificial Sequence:capture

<400> 748
cagaggcggg ccgcggt                                                      17

<210> 749
<211> 17
<212> DNA
<213> Artificial Sequence

<220>
<223> Description of Artificial Sequence:capture

<400> 749
cggaggcgcg ccgcggt                                                      17

<210> 750
<211> 17
<212> DNA
<213> Artificial Sequence

<220>
<223> Description of Artificial Sequence:capture

<400> 750
gacagggccg ccgcggt                                                      17

<210> 751
<211> 17
<212> DNA
<213> Artificial Sequence

<220>
<223> Description of Artificial Sequence:capture

<400> 751
gcagaggcag gccgcgg                                                      17

<210> 752
<211> 17
<212> DNA
<213> Artificial Sequence

<220>
<223> Description of Artificial Sequence:capture

<400> 752

cagaggcgtg ccgcggt                                                    17

<210> 753
<211> 17
<212> DNA
<213> Artificial Sequence

<220>
<223> Description of Artificial Sequence:capture

<400> 753
cagaggcgcg ccgcggt                                                    17

<210> 754
<211> 17
<212> DNA
<213> Artificial Sequence

<220>
<223> Description of Artificial Sequence:capture

<400> 754
cagaggcggg ccgccgt                                                    17

<210> 755
<211> 17
<212> DNA
<213> Artificial Sequence

<220>
<223> Description of Artificial Sequence:capture

<400> 755
gacaggcgcg ccgcggt                                                    17

<210> 756
<211> 17
<212> DNA
<213> Artificial Sequence

<220>
<223> Description of Artificial Sequence:capture

<400> 756
gacgagcggg ccgcggt                                                    17

<210> 757
<211> 17
<212> DNA
<213> Artificial Sequence

<220>
<223> Description of Artificial Sequence:capture

<400> 757
gacaggcgcg ccgcggt                                                    17

<210> 758

<211> 17
<212> DNA
<213> Artificial Sequence

<220>
<223> Description of Artificial Sequence:capture

<400> 758
caggcgcggg ccgcggt                                                    17

<210> 759
<211> 17
<212> DNA
<213> Artificial Sequence

<220>
<223> Description of Artificial Sequence:capture

<400> 759
cagaagcggg gccgggt                                                    17

<210> 760
<211> 17
<212> DNA
<213> Artificial Sequence

<220>
<223> Description of Artificial Sequence:capture

<400> 760
cagaagcggg ccgcggt                                                    17

<210> 761
<211> 17
<212> DNA
<213> Artificial Sequence

<220>
<223> Description of Artificial Sequence:capture

<400> 761
cagaggcggg ccgaggt                                                    17

<210> 762
<211> 17
<212> DNA
<213> Artificial Sequence

<220>
<223> Description of Artificial Sequence:capture

<400> 762
gacaggcggg ccctggt                                                    17

<210> 763
<211> 17
<212> DNA
<213> Artificial Sequence

<220>
<223> Description of Artificial Sequence:capture

<400> 763
gacaggcggg ccgcggt                                                    17

<210> 764
<211> 17
<212> DNA
<213> Artificial Sequence

<220>
<223> Description of Artificial Sequence:capture

<400> 764
cggaggcggg ccgcggt                                                    17

<210> 765
<211> 17
<212> DNA
<213> Artificial Sequence

<220>
<223> Description of Artificial Sequence:capture

<400> 765
cagaggcggg ccctggt                                                    17

<210> 766
<211> 17
<212> DNA
<213> Artificial Sequence

<220>
<223> Description of Artificial Sequence:capture

<400> 766
gacaggcggg gccaggt                                                    17

<210> 767
<211> 17
<212> DNA
<213> Artificial Sequence

<220>
<223> Description of Artificial Sequence:capture

<400> 767
cggaggcggg ccgaggt                                                    17

<210> 768
<211> 17
<212> DNA
<213> Artificial Sequence

<220>
<223> Description of Artificial Sequence:capture

<400> 768
gacaggcggg ccgtggt                                                    17

<210> 769
<211> 17
<212> DNA
<213> Artificial Sequence

<220>
<223> Description of Artificial Sequence:capture

<400> 769
gacgagcgcg ccgcggt                                                    17

<210> 770
<211> 17
<212> DNA
<213> Artificial Sequence

<220>
<223> Description of Artificial Sequence:capture

<400> 770
gacaagcggg ccgcggt                                                    17

<210> 771
<211> 15
<212> DNA
<213> Artificial Sequence

<220>
<223> Description of Artificial Sequence:capture

<400> 771
cgggccgcgg tggac                                                      15

<210> 772
<211> 14
<212> DNA
<213> Artificial Sequence

<220>
<223> Description of Artificial Sequence:capture

<400> 772
cgggccctgg tgga                                                       14

<210> 773
<211> 14
<212> DNA
<213> Artificial Sequence

<220>
<223> Description of Artificial Sequence:capture

<400> 773
cgggccgagg tgga                                                       14

<210> 774
<211> 15
<212> DNA
<213> Artificial Sequence

<220>
<223> Description of Artificial Sequence:capture

<400> 774
cggggccggg tggac                                                    15

<210> 775
<211> 15
<212> DNA
<213> Artificial Sequence

<220>
<223> Description of Artificial Sequence:capture

<400> 775
cggggccagg tggac                                                    15

<210> 776
<211> 15
<212> DNA
<213> Artificial Sequence

<220>
<223> Description of Artificial Sequence:capture

<400> 776
cgggccgtgg tggac                                                    15

<210> 777
<211> 17
<212> DNA
<213> Artificial Sequence

<220>
<223> Description of Artificial Sequence:capture

<400> 777
cgggccgccg tggacac                                                  17

<210> 778
<211> 15
<212> DNA
<213> Artificial Sequence

<220>
<223> Description of Artificial Sequence:capture

<400> 778
caggccgcgg tggac                                                    15

<210> 779
<211> 15

<212> DNA
<213> Artificial Sequence

<220>
<223> Description of Artificial Sequence:capture

<400> 779
cgtgccgcgg tggac                                                        15

<210> 780
<211> 15
<212> DNA
<213> Artificial Sequence

<220>
<223> Description of Artificial Sequence:capture

<400> 780
cgcgccgcgg tggac                                                        15

<210> 781
<211> 15
<212> DNA
<213> Artificial Sequence

<220>
<223> Description of Artificial Sequence:capture

<400> 781
gccgccgcgg tggac                                                        15

<210> 782
<211> 18
<212> DNA
<213> Artificial Sequence

<220>
<223> Description of Artificial Sequence:capture

<400> 782
ggtggacaac tactgcag                                                     18

<210> 783
<211> 19
<212> DNA
<213> Artificial Sequence

<220>
<223> Description of Artificial Sequence:capture

<400> 783
ggtggacaat tactgcaga                                                    19

<210> 784
<211> 17
<212> DNA
<213> Artificial Sequence

<220>
<223> Description of Artificial Sequence:capture

<400> 784
gacacctact gcagaca                                                    17

<210> 785
<211> 17
<212> DNA
<213> Artificial Sequence

<220>
<223> Description of Artificial Sequence:capture

<400> 785
gacacctatt gcagaca                                                    17

<210> 786
<211> 18
<212> DNA
<213> Artificial Sequence

<220>
<223> Description of Artificial Sequence:capture

<400> 786
ggacaccgtg tgcagaca                                                   18

<210> 787
<211> 17
<212> DNA
<213> Artificial Sequence

<220>
<223> Description of Artificial Sequence:capture

<400> 787
gacaattact gcagaca                                                    17

<210> 788
<211> 17
<212> DNA
<213> Artificial Sequence

<220>
<223> Description of Artificial Sequence:capture

<400> 788
gacaactact gcagaca                                                    17

<210> 789
<211> 17
<212> DNA
<213> Artificial Sequence

<220>
<223> Description of Artificial Sequence:capture

<400> 789
acaactaccg ggttgtg                                                    17

<210> 790
<211> 17
<212> DNA
<213> Artificial Sequence

<220>
<223> Description of Artificial Sequence:capture

<400> 790
aactacggag ttgtgga                                                    17

<210> 791
<211> 17
<212> DNA
<213> Artificial Sequence

<220>
<223> Description of Artificial Sequence:capture

<400> 791
ctacggggct gtggaga                                                    17

<210> 792
<211> 16
<212> DNA
<213> Artificial Sequence

<220>
<223> Description of Artificial Sequence:capture

<400> 792
ggggttggtg agagct                                                     16

<210> 793
<211> 16
<212> DNA
<213> Artificial Sequence

<220>
<223> Description of Artificial Sequence:capture

<400> 793
ggggttgtgg agagct                                                     16

<210> 794
<211> 16
<212> DNA
<213> Artificial Sequence

<220>
<223> Description of Artificial Sequence:capture

<400> 794
ggggttgatg agagct                                                     16

<210> 795
<211> 16
<212> DNA
<213> Artificial Sequence

<220>
<223> Description of Artificial Sequence:capture

<400> 795
ggggctgtgg agagct                                                    16

<210> 796
<211> 16
<212> DNA
<213> Artificial Sequence

<220>
<223> Description of Artificial Sequence:capture

<400> 796
ggggttgtgg agagat                                                    16

<210> 797
<211> 16
<212> DNA
<213> Artificial Sequence

<220>
<223> Description of Artificial Sequence:capture

<400> 797
cgggttgtgg agagct                                                    16

<210> 798
<211> 16
<212> DNA
<213> Artificial Sequence

<220>
<223> Description of Artificial Sequence:capture

<400> 798
ggagttgtgg agagct                                                    16

<210> 799
<211> 17
<212> DNA
<213> Artificial Sequence

<220>
<223> Description of Artificial Sequence:capture

<400> 799
gtggagagat tcacagt                                                   17

<210> 800
<211> 18
<212> DNA

<213> Artificial Sequence

<220>
<223> Description of Artificial Sequence:capture

<400> 800
ggtgcggctc ctggagag                                                    18

<210> 801
<211> 18
<212> DNA
<213> Artificial Sequence

<220>
<223> Description of Artificial Sequence:capture

<400> 801
agtgtggaac ctgatcag                                                    18

<210> 802
<211> 17
<212> DNA
<213> Artificial Sequence

<220>
<223> Description of Artificial Sequence:capture

<400> 802
gagcctgatc agataca                                                     17

<210> 803
<211> 18
<212> DNA
<213> Artificial Sequence

<220>
<223> Description of Artificial Sequence:capture

<400> 803
ggtgcggttc ctgcacag                                                    18

<210> 804
<211> 18
<212> DNA
<213> Artificial Sequence

<220>
<223> Description of Artificial Sequence:capture

<400> 804
ggtgcggtac ctggagag                                                    18

<210> 805
<211> 18
<212> DNA
<213> Artificial Sequence

<220>

<223> Description of Artificial Sequence:capture

<400> 805
ggtgcggtac ctgaacag                                                    18

<210> 806
<211> 18
<212> DNA
<213> Artificial Sequence

<220>
<223> Description of Artificial Sequence:capture

<400> 806
gtacctgaac agatactt                                                    18

<210> 807
<211> 18
<212> DNA
<213> Artificial Sequence

<220>
<223> Description of Artificial Sequence:capture

<400> 807
gaacctgatc agatacat                                                    18

<210> 808
<211> 19
<212> DNA
<213> Artificial Sequence

<220>
<223> Description of Artificial Sequence:capture

<400> 808
ggagcctgat cagatacat                                                   19

<210> 809
<211> 19
<212> DNA
<213> Artificial Sequence

<220>
<223> Description of Artificial Sequence:capture

<400> 809
ggttcctgca cagagacat                                                   19

<210> 810
<211> 18
<212> DNA
<213> Artificial Sequence

<220>
<223> Description of Artificial Sequence:capture

<400> 810

gtacctggag agatactt                                                    18

<210> 811
<211> 19
<212> DNA
<213> Artificial Sequence

<220>
<223> Description of Artificial Sequence:capture

<400> 811
ggttcctgca cagaggcat                                                   19

<210> 812
<211> 18
<212> DNA
<213> Artificial Sequence

<220>
<223> Description of Artificial Sequence:capture

<400> 812
gttcctggag agacactt                                                    18

<210> 813
<211> 18
<212> DNA
<213> Artificial Sequence

<220>
<223> Description of Artificial Sequence:capture

<400> 813
gctcctggag agacactt                                                    18

<210> 814
<211> 17
<212> DNA
<213> Artificial Sequence

<220>
<223> Description of Artificial Sequence:capture

<400> 814
ggcacgatgc atctata                                                     17

<210> 815
<211> 17
<212> DNA
<213> Artificial Sequence

<220>
<223> Description of Artificial Sequence:capture

<400> 815
gatcagatac atctata                                                     17

<210> 816

<211> 18
<212> DNA
<213> Artificial Sequence

<220>
<223> Description of Artificial Sequence:capture

<400> 816
ggcacgatga atctataa                                                    18

<210> 817
<211> 19
<212> DNA
<213> Artificial Sequence

<220>
<223> Description of Artificial Sequence:capture

<400> 817
ggagagacac ttccataac                                                   19

<210> 818
<211> 19
<212> DNA
<213> Artificial Sequence

<220>
<223> Description of Artificial Sequence:capture

<400> 818
ggacagatac ttccataac                                                   19

<210> 819
<211> 19
<212> DNA
<213> Artificial Sequence

<220>
<223> Description of Artificial Sequence:capture

<400> 819
ggagagatac ttccataac                                                   19

<210> 820
<211> 19
<212> DNA
<213> Artificial Sequence

<220>
<223> Description of Artificial Sequence:capture

<400> 820
gaacagatac ttccataac                                                   19

<210> 821
<211> 16
<212> DNA
<213> Artificial Sequence

<220>
<223> Description of Artificial Sequence:capture

<400> 821
gatacatcta taacca                                                    16

<210> 822
<211> 16
<212> DNA
<213> Artificial Sequence

<220>
<223> Description of Artificial Sequence:capture

<400> 822
gagacatcta taacca                                                    16

<210> 823
<211> 16
<212> DNA
<213> Artificial Sequence

<220>
<223> Description of Artificial Sequence:capture

<400> 823
gatacttcca taacca                                                    16

<210> 824
<211> 16
<212> DNA
<213> Artificial Sequence

<220>
<223> Description of Artificial Sequence:capture

<400> 824
gacacttcca taacca                                                    16

<210> 825
<211> 16
<212> DNA
<213> Artificial Sequence

<220>
<223> Description of Artificial Sequence:capture

<400> 825
ataaccaaga ggagta                                                    16

<210> 826
<211> 16
<212> DNA
<213> Artificial Sequence

<220>
<223> Description of Artificial Sequence:capture

<400> 826
ataaccaaga ggagga                                                    16

<210> 827
<211> 16
<212> DNA
<213> Artificial Sequence

<220>
<223> Description of Artificial Sequence:capture

<400> 827
ataaccagga ggagtc                                                    16

<210> 828
<211> 16
<212> DNA
<213> Artificial Sequence

<220>
<223> Description of Artificial Sequence:capture

<400> 828
ataaccagga ggagaa                                                    16

<210> 829
<211> 16
<212> DNA
<213> Artificial Sequence

<220>
<223> Description of Artificial Sequence:capture

<400> 829
ataaccagga ggagta                                                    16

<210> 830
<211> 16
<212> DNA
<213> Artificial Sequence

<220>
<223> Description of Artificial Sequence:capture

<400> 830
agaggaggac ttgcgc                                                    16

<210> 831
<211> 19
<212> DNA
<213> Artificial Sequence

<220>
<223> Description of Artificial Sequence:capture

<400> 831
aggaggagta cgcgcgctt                                                 19

<210> 832
<211> 16
<212> DNA
<213> Artificial Sequence

<220>
<223> Description of Artificial Sequence:capture

<400> 832
ggagttcctg cgcttc                                                    16

<210> 833
<211> 17
<212> DNA
<213> Artificial Sequence

<220>
<223> Description of Artificial Sequence:capture

<400> 833
aggaggactt gcgcttc                                                   17

<210> 834
<211> 17
<212> DNA
<213> Artificial Sequence

<220>
<223> Description of Artificial Sequence:capture

<400> 834
ggagtacgcg cgcttcg                                                   17

<210> 835
<211> 18
<212> DNA
<213> Artificial Sequence

<220>
<223> Description of Artificial Sequence:capture

<400> 835
aggagaacgc gcgcttcg                                                  18

<210> 836
<211> 17
<212> DNA
<213> Artificial Sequence

<220>
<223> Description of Artificial Sequence:capture

<400> 836
ggcggtgagg gagctgg                                                   17

<210> 837
<211> 17

<212> DNA
<213> Artificial Sequence

<220>
<223> Description of Artificial Sequence:capture

<400> 837
gcggcctgtt gccgagt                                                    17

<210> 838
<211> 15
<212> DNA
<213> Artificial Sequence

<220>
<223> Description of Artificial Sequence:capture

<400> 838
gccgagtcct ggaac                                                      15

<210> 839
<211> 19
<212> DNA
<213> Artificial Sequence

<220>
<223> Description of Artificial Sequence:capture

<400> 839
tcctggaaga cacgcgcgc                                                  19

<210> 840
<211> 17
<212> DNA
<213> Artificial Sequence

<220>
<223> Description of Artificial Sequence:capture

<400> 840
gacacgcgcg ccgcggt                                                    17

<210> 841
<211> 17
<212> DNA
<213> Artificial Sequence

<220>
<223> Description of Artificial Sequence:capture

<400> 841
cagaagcggg gccaggt                                                    17

<210> 842
<211> 14
<212> DNA
<213> Artificial Sequence

<220>
<223> Description of Artificial Sequence:capture

<400> 842
cgggccctgg tgga                                                    14

<210> 843
<211> 17
<212> DNA
<213> Artificial Sequence

<220>
<223> Description of Artificial Sequence:capture

<400> 843
cgaggtgggc acctact                                                 17

<210> 844
<211> 17
<212> DNA
<213> Artificial Sequence

<220>
<223> Description of Artificial Sequence:capture

<400> 844
ggtggacaac tactgca                                                 17

<210> 845
<211> 17
<212> DNA
<213> Artificial Sequence

<220>
<223> Description of Artificial Sequence:capture

<400> 845
tactgcaggc acaacta                                                 17

<210> 846
<211> 17
<212> DNA
<213> Artificial Sequence

<220>
<223> Description of Artificial Sequence:capture

<400> 846
actgcagata caactac                                                 17

<210> 847
<211> 16
<212> DNA
<213> Artificial Sequence

<220>
<223> Description of Artificial Sequence:capture

<400> 847
aactacggag ttggtg                                                    16

<210> 848
<211> 17
<212> DNA
<213> Artificial Sequence

<220>
<223> Description of Artificial Sequence:capture

<400> 848
ctacggggct gtggaga                                                   17

<210> 849
<211> 17
<212> DNA
<213> Artificial Sequence

<220>
<223> Description of Artificial Sequence:capture

<400> 849
attgcagata caactac                                                   17

<210> 850
<211> 18
<212> DNA
<213> Artificial Sequence

<220>
<223> Description of Artificial Sequence:capture

<400> 850
cggagttggt gagagctt                                                  18

<210> 851
<211> 18
<212> DNA
<213> Artificial Sequence

<220>
<223> Description of Artificial Sequence:capture

<400> 851
cggcgttgtg gagagctt                                                  18

<210> 852
<211> 17
<212> DNA
<213> Artificial Sequence

<220>
<223> Description of Artificial Sequence:capture

<400> 852
acgtttctcg gagctgc                                                   17

<210> 853
<211> 17
<212> DNA
<213> Artificial Sequence

<220>
<223> Description of Artificial Sequence:capture

<400> 853
tggagctgtg taagtct                                                    17

<210> 854
<211> 17
<212> DNA
<213> Artificial Sequence

<220>
<223> Description of Artificial Sequence:capture

<400> 854
gtgagtgtca tatcttc                                                    17

<210> 855
<211> 17
<212> DNA
<213> Artificial Sequence

<220>
<223> Description of Artificial Sequence:capture

<400> 855
ctgagtgtca tttcttc                                                    17

<210> 856
<211> 17
<212> DNA
<213> Artificial Sequence

<220>
<223> Description of Artificial Sequence:capture

<400> 856
acgagtgcta tttctgc                                                    17

<210> 857
<211> 17
<212> DNA
<213> Artificial Sequence

<220>
<223> Description of Artificial Sequence:capture

<400> 857
gtgcagtacc tgaacag                                                    17

<210> 858
<211> 17
<212> DNA

<213> Artificial Sequence

<220>
<223> Description of Artificial Sequence:capture

<400> 858
gagcggtacc tgtaaag                                                    17

<210> 859
<211> 17
<212> DNA
<213> Artificial Sequence

<220>
<223> Description of Artificial Sequence:capture

<400> 859
atgcggtttg tgcagag                                                    17

<210> 860
<211> 16
<212> DNA
<213> Artificial Sequence

<220>
<223> Description of Artificial Sequence:capture

<400> 860
tacctgtaaa gatact                                                     16

<210> 861
<211> 16
<212> DNA
<213> Artificial Sequence

<220>
<223> Description of Artificial Sequence:capture

<400> 861
tacctgtaaa gatatt                                                     16

<210> 862
<211> 17
<212> DNA
<213> Artificial Sequence

<220>
<223> Description of Artificial Sequence:capture

<400> 862
gtacctgaac agataca                                                    17

<210> 863
<211> 17
<212> DNA
<213> Artificial Sequence

<220>

<223> Description of Artificial Sequence:capture

<400> 863
gtttgtgcag agactcg                                                    17

<210> 864
<211> 17
<212> DNA
<213> Artificial Sequence

<220>
<223> Description of Artificial Sequence:capture

<400> 864
taaagatact tctataa                                                    17

<210> 865
<211> 17
<212> DNA
<213> Artificial Sequence

<220>
<223> Description of Artificial Sequence:capture

<400> 865
taaagatatt tctataa                                                    17

<210> 866
<211> 25
<212> DNA
<213> Artificial Sequence

<220>
<223> Description of Artificial Sequence:capture

<400> 866
gatacatcca taaacgggag gagaa                                           25

<210> 867
<211> 25
<212> DNA
<213> Artificial Sequence

<220>
<223> Description of Artificial Sequence:capture

<400> 867
gatacttcta taaccaagag gagta                                           25

<210> 868
<211> 25
<212> DNA
<213> Artificial Sequence

<220>
<223> Description of Artificial Sequence:capture

<400> 868

gatatttcta taaccaagag gagta                                                25

<210> 869
<211> 24
<212> DNA
<213> Artificial Sequence

<220>
<223> Description of Artificial Sequence:capture

<400> 869
gactcgtcca caccggaagg agta                                                 24

<210> 870
<211> 16
<212> DNA
<213> Artificial Sequence

<220>
<223> Description of Artificial Sequence:capture

<400> 870
ggaggagaac ctgccc                                                          16

<210> 871
<211> 17
<212> DNA
<213> Artificial Sequence

<220>
<223> Description of Artificial Sequence:capture

<400> 871
gaaggagtat gcgcgct                                                         17

<210> 872
<211> 17
<212> DNA
<213> Artificial Sequence

<220>
<223> Description of Artificial Sequence:capture

<400> 872
gaacctgccc ttcgaca                                                         17

<210> 873
<211> 17
<212> DNA
<213> Artificial Sequence

<220>
<223> Description of Artificial Sequence:capture

<400> 873
ggaggagttc caggcag                                                         17

<210> 874

<211> 17
<212> DNA
<213> Artificial Sequence

<220>
<223> Description of Artificial Sequence:capture

<400> 874
cagaaagttc cgggcgg                                                    17

<210> 875
<211> 20
<212> DNA
<213> Artificial Sequence

<220>
<223> Description of Artificial Sequence:capture

<400> 875
gcctgtcgca gagaactgga                                                 20

<210> 876
<211> 20
<212> DNA
<213> Artificial Sequence

<220>
<223> Description of Artificial Sequence:capture

<400> 876
agaagagtcc aggaatgcaa                                                 20

<210> 877
<211> 18
<212> DNA
<213> Artificial Sequence

<220>
<223> Description of Artificial Sequence:capture

<400> 877
cagaagaacc tcttgggc                                                   18

<210> 878
<211> 18
<212> DNA
<213> Artificial Sequence

<220>
<223> Description of Artificial Sequence:capture

<400> 878
cagaagggaa tcctggag                                                   18

<210> 879
<211> 18
<212> DNA
<213> Artificial Sequence

<220>
<223> Description of Artificial Sequence:capture

<400> 879
cagaagggca tcccggag                                                    18

<210> 880
<211> 19
<212> DNA
<213> Artificial Sequence

<220>
<223> Description of Artificial Sequence:capture

<400> 880
cctggaggag aatcgggac                                                   19

<210> 881
<211> 19
<212> DNA
<213> Artificial Sequence

<220>
<223> Description of Artificial Sequence:capture

<400> 881
cccggaggag aagcgggac                                                   19

<210> 882
<211> 19
<212> DNA
<213> Artificial Sequence

<220>
<223> Description of Artificial Sequence:capture

<400> 882
cttgggctgc ttgcggggt                                                   19

<210> 883
<211> 17
<212> DNA
<213> Artificial Sequence

<220>
<223> Description of Artificial Sequence:capture

<400> 883
gagaatcggg acaaggt                                                     17

<210> 884
<211> 17
<212> DNA
<213> Artificial Sequence

<220>
<223> Description of Artificial Sequence:capture

<400> 884
gagaagcggg acaagat                                                    17

<210> 885
<211> 17
<212> DNA
<213> Artificial Sequence

<220>
<223> Description of Artificial Sequence:capture

<400> 885
cagaagcggg ccgaagt                                                    17

<210> 886
<211> 17
<212> DNA
<213> Artificial Sequence

<220>
<223> Description of Artificial Sequence:capture

<400> 886
tgcttgcggg gtctgtt                                                    17

<210> 887
<211> 15
<212> DNA
<213> Artificial Sequence

<220>
<223> Description of Artificial Sequence:capture

<400> 887
cgggacaagg tggac                                                      15

<210> 888
<211> 15
<212> DNA
<213> Artificial Sequence

<220>
<223> Description of Artificial Sequence:capture

<400> 888
cgggacaaga tggac                                                      15

<210> 889
<211> 15
<212> DNA
<213> Artificial Sequence

<220>
<223> Description of Artificial Sequence:capture

<400> 889
cgggccgaag tggac                                                      15

<210> 890
<211> 15
<212> DNA
<213> Artificial Sequence

<220>
<223> Description of Artificial Sequence:capture

<400> 890
cggggtctgt tggac                                                    15

<210> 891
<211> 17
<212> DNA
<213> Artificial Sequence

<220>
<223> Description of Artificial Sequence:capture

<400> 891
gacacctact gcagata                                                  17

<210> 892
<211> 17
<212> DNA
<213> Artificial Sequence

<220>
<223> Description of Artificial Sequence:capture

<400> 892
gacgactact gcagata                                                  17

<210> 893
<211> 17
<212> DNA
<213> Artificial Sequence

<220>
<223> Description of Artificial Sequence:capture

<400> 893
gacaactaat gcagaca                                                  17

<210> 894
<211> 17
<212> DNA
<213> Artificial Sequence

<220>
<223> Description of Artificial Sequence:capture

<400> 894
acaattacag ggttttt                                                  17

<210> 895
<211> 16

<212> DNA
<213> Artificial Sequence

<220>
<223> Description of Artificial Sequence:capture

<400> 895
ggggttttg agagct                                                        16

<210> 896
<211> 16
<212> DNA
<213> Artificial Sequence

<220>
<223> Description of Artificial Sequence:capture

<400> 896
   ggggtttttt gagagc                                                        16

<210> 897
<211> 16
<212> DNA
<213> Artificial Sequence

<220>
<223> Description of Artificial Sequence:capture

<400> 897
tacagggttt tttgag                                                       16

<210> 898
<211> 16
<212> DNA
<213> Artificial Sequence

<220>
<223> Description of Artificial Sequence:capture

<400> 898
ggggttgtgg agagat                                                       16

<210> 899
<211> 21
<212> DNA
<213> Artificial Sequence

<220>
<223> Description of Artificial Sequence:primer

<400> 899
cagccactcc tcgtccccag g                                                 21

<210> 900
<211> 21
<212> DNA
<213> Artificial Sequence

<220>
<223> Description of Artificial Sequence:primer

<400> 900
cagccactcc tcgcccccag g                                                21

<210> 901
<211> 21
<212> DNA
<213> Artificial Sequence

<220>
<223> Description of Artificial Sequence:primer

<400> 901
gggatgaggg gtcgtgacct g                                                21

<210> 902
<211> 21
<212> DNA
<213> Artificial Sequence

<220>
<223> Description of Artificial Sequence:primer

<400> 902
gggatgagag gtcgtgacct g                                                21

<210> 903
<211> 20
<212> DNA
<213> Artificial Sequence

<220>
<223> Description of Artificial Sequence:primer

<400> 903
ggtcggggcc aggttctcac                                                  20

<210> 904
<211> 20
<212> DNA
<213> Artificial Sequence

<220>
<223> Description of Artificial Sequence:primer

<400> 904
ggtccgggcc aggttctcac                                                  20

<210> 905
<211> 20
<212> DNA
<213> Artificial Sequence

<220>
<223> Description of Artificial Sequence:primer

<400> 905
ggtcgaggcc aggttctcac                                                    20

<210> 906
<211> 20
<212> DNA
<213> Artificial Sequence

<220>
<223> Description of Artificial Sequence:primer

<400> 906
ccgtggcccc tggtacccgt                                                    20

<210> 907
<211> 20
<212> DNA
<213> Artificial Sequence

<220>
<223> Description of Artificial Sequence:primer

<400> 907
ccgtggcccc tggtacccgc                                                    20

<210> 908
<211> 17
<212> DNA
<213> Artificial Sequence

<220>
<223> Description of Artificial Sequence:capture

<400> 908
ccaggctctc actccat                                                       17

<210> 909
<211> 17
<212> DNA
<213> Artificial Sequence

<220>
<223> Description of Artificial Sequence:capture

<400> 909
ggctctcagt ccatgag                                                       17

<210> 910
<211> 16
<212> DNA
<213> Artificial Sequence

<220>
<223> Description of Artificial Sequence:capture

<400> 910
gctcccaatc catgag                                                        16

<210> 911
<211> 17
<212> DNA
<213> Artificial Sequence

<220>
<223> Description of Artificial Sequence:capture

<400> 911
   catgaggtgt ttctcca                                                    17

<210> 912
<211> 18
<212> DNA
<213> Artificial Sequence

<220>
<223> Description of Artificial Sequence:capture

<400> 912
tatttcttca catccgtg                                                  18

<210> 913
<211> 17
<212> DNA
<213> Artificial Sequence

<220>
<223> Description of Artificial Sequence:capture

<400> 913
atttctacac ctccgtg                                                   17

<210> 914
<211> 17
<212> DNA
<213> Artificial Sequence

<220>
<223> Description of Artificial Sequence:capture

<400> 914
gtatttctcc acatccg                                                   17

<210> 915
<211> 16
<212> DNA
<213> Artificial Sequence

<220>
<223> Description of Artificial Sequence:capture

<400> 915
gtatttcacc acatcc                                                    16

<210> 916
<211> 17
<212> DNA

<213> Artificial Sequence

<220>
<223> Description of Artificial Sequence:capture

<400> 916
atttctacac ttccgtg                                                    17

<210> 917
<211> 17
<212> DNA
<213> Artificial Sequence

<220>
<223> Description of Artificial Sequence:capture

<400> 917
atttctacac ctccatg                                                    17

<210> 918
<211> 15
<212> DNA
<213> Artificial Sequence

<220>
<223> Description of Artificial Sequence:capture

<400> 918
ggttctccac atccg                                                      15

<210> 919
<211> 17
<212> DNA
<213> Artificial Sequence

<220>
<223> Description of Artificial Sequence:capture

<400> 919
ggcagtggag agccccg                                                    17

<210> 920
<211> 16
<212> DNA
<213> Artificial Sequence

<220>
<223> Description of Artificial Sequence:capture

<400> 920
gcagtgggaa gccccg                                                     16

<210> 921
<211> 17
<212> DNA
<213> Artificial Sequence

<220>

<223> Description of Artificial Sequence:capture

<400> 921
cttcatcgcc gtgggct                                                    17

<210> 922
<211> 17
<212> DNA
<213> Artificial Sequence

<220>
<223> Description of Artificial Sequence:capture

<400> 922
ttcatcgcag tgggcta                                                    17

<210> 923
<211> 17
<212> DNA
<213> Artificial Sequence

<220>
<223> Description of Artificial Sequence:capture

<400> 923
cttcatctca gtgggct                                                    17

<210> 924
<211> 17
<212> DNA
<213> Artificial Sequence

<220>
<223> Description of Artificial Sequence:capture

<400> 924
acgtggacaa cacgcag                                                    17

<210> 925
<211> 17
<212> DNA
<213> Artificial Sequence

<220>
<223> Description of Artificial Sequence:capture

<400> 925
gtgcggttgg acagcga                                                    17

<210> 926
<211> 17
<212> DNA
<213> Artificial Sequence

<220>
<223> Description of Artificial Sequence:capture

<400> 926

gtgcggtttg acagcga                                                    17

<210> 927
<211> 17
<212> DNA
<213> Artificial Sequence

<220>
<223> Description of Artificial Sequence:capture

<400> 927
cgacgccggg agccaga                                                    17

<210> 928
<211> 17
<212> DNA
<213> Artificial Sequence

<220>
<223> Description of Artificial Sequence:capture

<400> 928
gcgagccaga agatgga                                                    17

<210> 929
<211> 17
<212> DNA
<213> Artificial Sequence

<220>
<223> Description of Artificial Sequence:capture

<400> 929
gcgagccaga ggatgga                                                    17

<210> 930
<211> 17
<212> DNA
<213> Artificial Sequence

<220>
<223> Description of Artificial Sequence:capture

<400> 930
gcgagccgga ggatgga                                                    17

<210> 931
<211> 17
<212> DNA
<213> Artificial Sequence

<220>
<223> Description of Artificial Sequence:capture

<400> 931
gggagccaga ggatgga                                                    17

<210> 932

<211> 17
<212> DNA
<213> Artificial Sequence

<220>
<223> Description of Artificial Sequence:capture

<400> 932
ccgtggatgg agcagga                                                    17

<210> 933
<211> 17
<212> DNA
<213> Artificial Sequence

<220>
<223> Description of Artificial Sequence:capture

<400> 933
gatagagcgg gaggggc                                                    17

<210> 934
<211> 17
<212> DNA
<213> Artificial Sequence

<220>
<223> Description of Artificial Sequence:capture

<400> 934
gcaggagggg ccggagt                                                    17

<210> 935
<211> 17
<212> DNA
<213> Artificial Sequence

<220>
<223> Description of Artificial Sequence:capture

<400> 935
agcaggagag gcctgag                                                    17

<210> 936
<211> 17
<212> DNA
<213> Artificial Sequence

<220>
<223> Description of Artificial Sequence:capture

<400> 936
caggagggtc cggagta                                                    17

<210> 937
<211> 17
<212> DNA
<213> Artificial Sequence

<220>
<223> Description of Artificial Sequence:capture

<400> 937
gcaggagagt ccggagt                                                    17

<210> 938
<211> 17
<212> DNA
<213> Artificial Sequence

<220>
<223> Description of Artificial Sequence:capture

<400> 938
gcaggaggag ccggagt                                                    17

<210> 939
<211> 16
<212> DNA
<213> Artificial Sequence

<220>
<223> Description of Artificial Sequence:capture

<400> 939
cgggaggggc cggagt                                                     16

<210> 940
<211> 17
<212> DNA
<213> Artificial Sequence

<220>
<223> Description of Artificial Sequence:capture

<400> 940
gagaggcctg agtattg                                                    17

<210> 941
<211> 17
<212> DNA
<213> Artificial Sequence

<220>
<223> Description of Artificial Sequence:capture

<400> 941
ttgggaccag gagacac                                                    17

<210> 942
<211> 16
<212> DNA
<213> Artificial Sequence

<220>
<223> Description of Artificial Sequence:capture

<400> 942
ttgggacggg gagaca                                                    16

<210> 943
<211> 18
<212> DNA
<213> Artificial Sequence

<220>
<223> Description of Artificial Sequence:capture

<400> 943
tattgggacg aggagaca                                                  18

<210> 944
<211> 17
<212> DNA
<213> Artificial Sequence

<220>
<223> Description of Artificial Sequence:capture

<400> 944
ttgggaccgg aacacac                                                   17

<210> 945
<211> 17
<212> DNA
<213> Artificial Sequence

<220>
<223> Description of Artificial Sequence:capture

<400> 945
ttgggacctg cagacac                                                   17

<210> 946
<211> 16
<212> DNA
<213> Artificial Sequence

<220>
<223> Description of Artificial Sequence:capture

<400> 946
gggacgggca gacagg                                                    16

<210> 947
<211> 19
<212> DNA
<213> Artificial Sequence

<220>
<223> Description of Artificial Sequence:capture

<400> 947
ttgggacggg aagacacgg                                                 19

<210> 948
<211> 17
<212> DNA
<213> Artificial Sequence

<220>
<223> Description of Artificial Sequence:capture

<400> 948
gggacgggca gacaggg                                                17

<210> 949
<211> 17
<212> DNA
<213> Artificial Sequence

<220>
<223> Description of Artificial Sequence:capture

<400> 949
gaccggaaca cacggaa                                                17

<210> 950
<211> 15
<212> DNA
<213> Artificial Sequence

<220>
<223> Description of Artificial Sequence:capture

<400> 950
aggagacagg gaaag                                                  15

<210> 951
<211> 18
<212> DNA
<213> Artificial Sequence

<220>
<223> Description of Artificial Sequence:capture

<400> 951
gggcagacag ggaaagtg                                               18

<210> 952
<211> 16
<212> DNA
<213> Artificial Sequence

<220>
<223> Description of Artificial Sequence:capture

<400> 952
cacggaatat gaaggc                                                 16

<210> 953
<211> 17

<212> DNA
<213> Artificial Sequence

<220>
<223> Description of Artificial Sequence:capture

<400> 953
cacggaatgt gaaggcc                                                    17

<210> 954
<211> 16
<212> DNA
<213> Artificial Sequence

<220>
<223> Description of Artificial Sequence:capture

<400> 954
cacggaaagt gaaggc                                                     16

<210> 955
<211> 16
<212> DNA
<213> Artificial Sequence

<220>
<223> Description of Artificial Sequence:capture

<400> 955
cacggaacgt gaaggc                                                     16

<210> 956
<211> 18
<212> DNA
<213> Artificial Sequence

<220>
<223> Description of Artificial Sequence:capture

<400> 956
gacacggcaa gtgaaggc                                                   18

<210> 957
<211> 18
<212> DNA
<213> Artificial Sequence

<220>
<223> Description of Artificial Sequence:capture

<400> 957
gacacggcaa gtgaaggc                                                   18

<210> 958
<211> 16
<212> DNA
<213> Artificial Sequence

<220>
<223> Description of Artificial Sequence:capture

<400> 958
cagggaaagt gaaggc                                                    16

<210> 959
<211> 12
<212> DNA
<213> Artificial Sequence

<220>
<223> Description of Artificial Sequence:capture

<400> 959
gcccagtcac ag                                                        12

<210> 960
<211> 17
<212> DNA
<213> Artificial Sequence

<220>
<223> Description of Artificial Sequence:capture

<400> 960
agtcacaggc tgaccga                                                   17

<210> 961
<211> 17
<212> DNA
<213> Artificial Sequence

<220>
<223> Description of Artificial Sequence:capture

<400> 961
ctcacagatt caccgag                                                   17

<210> 962
<211> 17
<212> DNA
<213> Artificial Sequence

<220>
<223> Description of Artificial Sequence:capture

<400> 962
ctcacagagt caccgag                                                   17

<210> 963
<211> 17
<212> DNA
<213> Artificial Sequence

<220>
<223> Description of Artificial Sequence:capture

<400> 963
acagactgac cgagcga                                                    17

<210> 964
<211> 17
<212> DNA
<213> Artificial Sequence

<220>
<223> Description of Artificial Sequence:capture

<400> 964
cacagactca ccgagtg                                                    17

<210> 965
<211> 17
<212> DNA
<213> Artificial Sequence

<220>
<223> Description of Artificial Sequence:capture

<400> 965
acagactcat cgagtgg                                                    17

<210> 966
<211> 17
<212> DNA
<213> Artificial Sequence

<220>
<223> Description of Artificial Sequence:capture

<400> 966
cacagactaa ccgagcg                                                    17

<210> 967
<211> 17
<212> DNA
<213> Artificial Sequence

<220>
<223> Description of Artificial Sequence:capture

<400> 967
acagactgac cgagaga                                                    17

<210> 968
<211> 17
<212> DNA
<213> Artificial Sequence

<220>
<223> Description of Artificial Sequence:capture

<400> 968
cacagagtca ccgagtg                                                    17

<210> 969
<211> 17
<212> DNA
<213> Artificial Sequence

<220>
<223> Description of Artificial Sequence:capture

<400> 969
acagattgac cgagtgg                                                    17

<210> 970
<211> 17
<212> DNA
<213> Artificial Sequence

<220>
<223> Description of Artificial Sequence:capture

<400> 970
cacagactca ccgagag                                                    17

<210> 971
<211> 17
<212> DNA
<213> Artificial Sequence

<220>
<223> Description of Artificial Sequence:capture

<400> 971
cacaggctga ccgagtg                                                    17

<210> 972
<211> 18
<212> DNA
<213> Artificial Sequence

<220>
<223> Description of Artificial Sequence:capture

<400> 972
gaccgagcga acctgggg                                                   18

<210> 973
<211> 17
<212> DNA
<213> Artificial Sequence

<220>
<223> Description of Artificial Sequence:capture

<400> 973
tgaccgagag aacctgg                                                    17

<210> 974
<211> 17
<212> DNA

<213> Artificial Sequence

<220>
<223> Description of Artificial Sequence:capture

<400> 974
gaccgagtgg acctggg                                                    17

<210> 975
<211> 16
<212> DNA
<213> Artificial Sequence

<220>
<223> Description of Artificial Sequence:capture

<400> 975
gaccgagaga gcctgc                                                     16

<210> 976
<211> 18
<212> DNA
<213> Artificial Sequence

<220>
<223> Description of Artificial Sequence:capture

<400> 976
aaccgagaga acctgggg                                                   18

<210> 977
<211> 17
<212> DNA
<213> Artificial Sequence

<220>
<223> Description of Artificial Sequence:capture

<400> 977
tcaccgagag aacctgc                                                    17

<210> 978
<211> 17
<212> DNA
<213> Artificial Sequence

<220>
<223> Description of Artificial Sequence:capture

<400> 978
catcgagtgg acctggg                                                    17

<210> 979
<211> 17
<212> DNA
<213> Artificial Sequence

<220>

<223> Description of Artificial Sequence:capture

<400> 979
tgaccgagag aacctgc                                                    17

<210> 980
<211> 17
<212> DNA
<213> Artificial Sequence

<220>
<223> Description of Artificial Sequence:capture

<400> 980
caccgagtgg acctggg                                                    17

<210> 981
<211> 17
<212> DNA
<213> Artificial Sequence

<220>
<223> Description of Artificial Sequence:capture

<400> 981
gaccgagtgg acctggc                                                    17

<210> 982
<211> 18
<212> DNA
<213> Artificial Sequence

<220>
<223> Description of Artificial Sequence:capture

<400> 982
ggacctggcg accctgcg                                                   18

<210> 983
<211> 17
<212> DNA
<213> Artificial Sequence

<220>
<223> Description of Artificial Sequence:capture

<400> 983
actacaacga gagcgag                                                    17

<210> 984
<211> 17
<212> DNA
<213> Artificial Sequence

<220>
<223> Description of Artificial Sequence:capture

<400> 984

gagcgaggcc ggtgagt                                                    17

<210> 985
<211> 17
<212> DNA
<213> Artificial Sequence

<220>
<223> Description of Artificial Sequence:capture

<400> 985
ggccaggtac tcacacc                                                    17

<210> 986
<211> 17
<212> DNA
<213> Artificial Sequence

<220>
<223> Description of Artificial Sequence:capture

<400> 986
tcacaccatc cagataa                                                    17

<210> 987
<211> 17
<212> DNA
<213> Artificial Sequence

<220>
<223> Description of Artificial Sequence:capture

<400> 987
tcacaccgtc cagagga                                                    17

<210> 988
<211> 17
<212> DNA
<213> Artificial Sequence

<220>
<223> Description of Artificial Sequence:capture

<400> 988
tcacaccctc cagagga                                                    17

<210> 989
<211> 17
<212> DNA
<213> Artificial Sequence

<220>
<223> Description of Artificial Sequence:capture

<400> 989
tcacaccatc cagatga                                                    17

<210> 990

<211> 16
<212> DNA
<213> Artificial Sequence

<220>
<223> Description of Artificial Sequence:capture

<400> 990
tcacaccctc cagatg                                                    16

<210> 991
<211> 16
<212> DNA
<213> Artificial Sequence

<220>
<223> Description of Artificial Sequence:capture

<400> 991
tcacaccatc cagagg                                                    16

<210> 992
<211> 17
<212> DNA
<213> Artificial Sequence

<220>
<223> Description of Artificial Sequence:capture

<400> 992
catccagata atgtatg                                                   17

<210> 993
<211> 17
<212> DNA
<213> Artificial Sequence

<220>
<223> Description of Artificial Sequence:capture

<400> 993
atccagatga tgtatgg                                                   17

<210> 994
<211> 18
<212> DNA
<213> Artificial Sequence

<220>
<223> Description of Artificial Sequence:capture

<400> 994
catccagagg atgtatgg                                                  18

<210> 995
<211> 18
<212> DNA
<213> Artificial Sequence

<220>
<223> Description of Artificial Sequence:capture

<400> 995
cgtccagatg atgtatgg                                                    18

<210> 996
<211> 18
<212> DNA
<213> Artificial Sequence

<220>
<223> Description of Artificial Sequence:capture

<400> 996
cgtccagagg atgtatgg                                                    18

<210> 997
<211> 17
<212> DNA
<213> Artificial Sequence

<220>
<223> Description of Artificial Sequence:capture

<400> 997
ctccagatga tgtttgg                                                     17

<210> 998
<211> 18
<212> DNA
<213> Artificial Sequence

<220>
<223> Description of Artificial Sequence:capture

<400> 998
cctccagagg atgtatgg                                                    18

<210> 999
<211> 17
<212> DNA
<213> Artificial Sequence

<220>
<223> Description of Artificial Sequence:capture

<400> 999
ctccagatga tgtttgg                                                     17

<210> 1000
<211> 18
<212> DNA
<213> Artificial Sequence

<220>
<223> Description of Artificial Sequence:capture

<400> 1000
cgtccagagg atgtttgg                                                    18

<210> 1001
<211> 18
<212> DNA
<213> Artificial Sequence

<220>
<223> Description of Artificial Sequence:capture

<400> 1001
cgtccagagg atgtctgg                                                    18

<210> 1002
<211> 18
<212> DNA
<213> Artificial Sequence

<220>
<223> Description of Artificial Sequence:capture

<400> 1002
cgtccagagg atgtgtgg                                                    18

<210> 1003
<211> 17
<212> DNA
<213> Artificial Sequence

<220>
<223> Description of Artificial Sequence:capture

<400> 1003
gaggatgtgt ggctgcg                                                     17

<210> 1004
<211> 17
<212> DNA
<213> Artificial Sequence

<220>
<223> Description of Artificial Sequence:capture

<400> 1004
gaggatgttt ggctgcg                                                     17

<210> 1005
<211> 17
<212> DNA
<213> Artificial Sequence

<220>
<223> Description of Artificial Sequence:capture

<400> 1005
gaggatgtct ggctgcg                                                     17

<210> 1006
<211> 17
<212> DNA
<213> Artificial Sequence

<220>
<223> Description of Artificial Sequence:capture

<400> 1006
atggctgcca cgtgggg                                                17

<210> 1007
<211> 17
<212> DNA
<213> Artificial Sequence

<220>
<223> Description of Artificial Sequence:capture

<400> 1007
acgtggggtc ggacggg                                                17

<210> 1008
<211> 17
<212> DNA
<213> Artificial Sequence

<220>
<223> Description of Artificial Sequence:capture

<400> 1008
acgtggggtc ggactgg                                                17

<210> 1009
<211> 17
<212> DNA
<213> Artificial Sequence

<220>
<223> Description of Artificial Sequence:capture

<400> 1009
gggcgcttac tccgcgg                                                17

<210> 1010
<211> 17
<212> DNA
<213> Artificial Sequence

<220>
<223> Description of Artificial Sequence:capture

<400> 1010
cttcctccac gggtacc                                                17

<210> 1011
<211> 17

<212> DNA
<213> Artificial Sequence

<220>
<223> Description of Artificial Sequence:capture

<400> 1011
cgcgggtatg aacagca                                                    17

<210> 1012
<211> 18
<212> DNA
<213> Artificial Sequence

<220>
<223> Description of Artificial Sequence:capture

<400> 1012
cgggtaccac cagtacgc                                                   18

<210> 1013
<211> 18
<212> DNA
<213> Artificial Sequence

<220>
<223> Description of Artificial Sequence:capture

<400> 1013
cgggtaccac cagtacgc                                                   18

<210> 1014
<211> 17
<212> DNA
<213> Artificial Sequence

<220>
<223> Description of Artificial Sequence:capture

<400> 1014
cgggtaccag caggacg                                                    17

<210> 1015
<211> 18
<212> DNA
<213> Artificial Sequence

<220>
<223> Description of Artificial Sequence:capture

<400> 1015
cgggtaccag cagaacgc                                                   18

<210> 1016
<211> 17
<212> DNA
<213> Artificial Sequence

<220>
<223> Description of Artificial Sequence:capture

<400> 1016
ggtaccagcg ggacgct                                                    17

<210> 1017
<211> 17
<212> DNA
<213> Artificial Sequence

<220>
<223> Description of Artificial Sequence:capture

<400> 1017
cgggtatgaa cagcacg                                                    17

<210> 1018
<211> 17
<212> DNA
<213> Artificial Sequence

<220>
<223> Description of Artificial Sequence:capture

<400> 1018
ggtaccggca ggacgcc                                                    17

<210> 1019
<211> 17
<212> DNA
<213> Artificial Sequence

<220>
<223> Description of Artificial Sequence:capture

<400> 1019
accggcagca cgcctac                                                    17

<210> 1020
<211> 17
<212> DNA
<213> Artificial Sequence

<220>
<223> Description of Artificial Sequence:capture

<400> 1020
accaccagta cgcctac                                                    17

<210> 1021
<211> 17
<212> DNA
<213> Artificial Sequence

<220>
<223> Description of Artificial Sequence:capture

<400> 1021
accagcagta cgcctac                                                    17

<210> 1022
<211> 17
<212> DNA
<213> Artificial Sequence

<220>
<223> Description of Artificial Sequence:capture

<400> 1022
accagcagaa cgcttac                                                    17

<210> 1023
<211> 17
<212> DNA
<213> Artificial Sequence

<220>
<223> Description of Artificial Sequence:capture

<400> 1023
atgaacagca cgcctac                                                    17

<210> 1024
<211> 17
<212> DNA
<213> Artificial Sequence

<220>
<223> Description of Artificial Sequence:capture

<400> 1024
ggtaccggca ggacgct                                                    17

<210> 1025
<211> 17
<212> DNA
<213> Artificial Sequence

<220>
<223> Description of Artificial Sequence:capture

<400> 1025
ccggcaggtc gcctacg                                                    17

<210> 1026
<211> 17
<212> DNA
<213> Artificial Sequence

<220>
<223> Description of Artificial Sequence:capture

<400> 1026
acatcgcctt gaacgag                                                    17

<210> 1027
<211> 17
<212> DNA
<213> Artificial Sequence

<220>
<223> Description of Artificial Sequence:capture

<400> 1027
gccctgaaag aggacct                                                    17

<210> 1028
<211> 17
<212> DNA
<213> Artificial Sequence

<220>
<223> Description of Artificial Sequence:capture

<400> 1028
aggacctgag ctcctgg                                                    17

<210> 1029
<211> 17
<212> DNA
<213> Artificial Sequence

<220>
<223> Description of Artificial Sequence:capture

<400> 1029
accgcggcag acatggc                                                    17

<210> 1030
<211> 17
<212> DNA
<213> Artificial Sequence

<220>
<223> Description of Artificial Sequence:capture

<400> 1030
ggcggacaag gcagctc                                                    17

<210> 1031
<211> 17
<212> DNA
<213> Artificial Sequence

<220>
<223> Description of Artificial Sequence:capture

<400> 1031
gacatggcgg ctcagat                                                    17

<210> 1032
<211> 18
<212> DNA

<213> Artificial Sequence

<220>
<223> Description of Artificial Sequence:capture

<400> 1032
agctcagacc accaagca                                                    18

<210> 1033
<211> 17
<212> DNA
<213> Artificial Sequence

<220>
<223> Description of Artificial Sequence:capture

<400> 1033
agatcaccca gcgcaag                                                     17

<210> 1034
<211> 17
<212> DNA
<213> Artificial Sequence

<220>
<223> Description of Artificial Sequence:capture

<400> 1034
agatcaccga gcgcaag                                                     17

<210> 1035
<211> 17
<212> DNA
<213> Artificial Sequence

<220>
<223> Description of Artificial Sequence:capture

<400> 1035
agtgggagac ggcccat                                                     17

<210> 1036
<211> 17
<212> DNA
<213> Artificial Sequence

<220>
<223> Description of Artificial Sequence:capture

<400> 1036
ggcggcccgt gtggcgg                                                     17

<210> 1037
<211> 17
<212> DNA
<213> Artificial Sequence

<220>

<223> Description of Artificial Sequence:capture

<400> 1037
ggcggccctt gtggcgg                                                    17

<210> 1038
<211> 17
<212> DNA
<213> Artificial Sequence

<220>
<223> Description of Artificial Sequence:capture

<400> 1038
ggcccatgtg gcggagc                                                    17

<210> 1039
<211> 17
<212> DNA
<213> Artificial Sequence

<220>
<223> Description of Artificial Sequence:capture

<400> 1039
ggcccatgag gcggagc                                                    17

<210> 1040
<211> 17
<212> DNA
<213> Artificial Sequence

<220>
<223> Description of Artificial Sequence:capture

<400> 1040
ggcccgttgg gcggagc                                                    17

<210> 1041
<211> 17
<212> DNA
<213> Artificial Sequence

<220>
<223> Description of Artificial Sequence:capture

<400> 1041
ggcggacaag gcggctc                                                    17

<210> 1042
<211> 17
<212> DNA
<213> Artificial Sequence

<220>
<223> Description of Artificial Sequence:capture

<400> 1042

ggcggcccgt gaggcgg                                                    17

<210> 1043
<211> 17
<212> DNA
<213> Artificial Sequence

<220>
<223> Description of Artificial Sequence:capture

<400> 1043
ggcccgtgtg gcggagc                                                    17

<210> 1044
<211> 17
<212> DNA
<213> Artificial Sequence

<220>
<223> Description of Artificial Sequence:capture

<400> 1044
ggcccgtgag gcggagc                                                    17

<210> 1045
<211> 17
<212> DNA
<213> Artificial Sequence

<220>
<223> Description of Artificial Sequence:capture

<400> 1045
ggcccgtcgg gcggagc                                                    17

<210> 1046
<211> 17
<212> DNA
<213> Artificial Sequence

<220>
<223> Description of Artificial Sequence:capture

<400> 1046
ggcggcccgt cgggcgg                                                    17

<210> 1047
<211> 17
<212> DNA
<213> Artificial Sequence

<220>
<223> Description of Artificial Sequence:capture

<400> 1047
   ggcccttgtg gcggagc                                                    17

<210> 1048

<211> 17
<212> DNA
<213> Artificial Sequence

<220>
<223> Description of Artificial Sequence:capture

<400> 1048
ggcggcccgt tgggcgg                                                    17

<210> 1049
<211> 17
<212> DNA
<213> Artificial Sequence

<220>
<223> Description of Artificial Sequence:capture

<400> 1049
ggagcagcgg agagtct                                                    17

<210> 1050
<211> 17
<212> DNA
<213> Artificial Sequence

<220>
<223> Description of Artificial Sequence:capture

<400> 1050
ggagcagttg agagcct                                                    17

<210> 1051
<211> 18
<212> DNA
<213> Artificial Sequence

<220>
<223> Description of Artificial Sequence:capture

<400> 1051
cggagcagtg gagagcct                                                   18

<210> 1052
<211> 17
<212> DNA
<213> Artificial Sequence

<220>
<223> Description of Artificial Sequence:capture

<400> 1052
ggagcagcag agagcct                                                    17

<210> 1053
<211> 17
<212> DNA
<213> Artificial Sequence

<220>
<223> Description of Artificial Sequence:capture

<400> 1053
ggagcagctg agagcct                                                    17

<210> 1054
<211> 17
<212> DNA
<213> Artificial Sequence

<220>
<223> Description of Artificial Sequence:capture

<400> 1054
ggagcagcgg agagcct                                                    17

<210> 1055
<211> 17
<212> DNA
<213> Artificial Sequence

<220>
<223> Description of Artificial Sequence:capture

<400> 1055
cctacctgca gggccgg                                                    17

<210> 1056
<211> 17
<212> DNA
<213> Artificial Sequence

<220>
<223> Description of Artificial Sequence:capture

<400> 1056
tacctggatg gcacgtg                                                    17

<210> 1057
<211> 17
<212> DNA
<213> Artificial Sequence

<220>
<223> Description of Artificial Sequence:capture

<400> 1057
cctggatgcc acgtgcg                                                    17

<210> 1058
<211> 17
<212> DNA
<213> Artificial Sequence

<220>
<223> Description of Artificial Sequence:capture

<400> 1058
ggagggccgg tgcgtgg                                                    17

<210> 1059
<211> 17
<212> DNA
<213> Artificial Sequence

<220>
<223> Description of Artificial Sequence:capture

<400> 1059
tggagggctg gtgcgtg                                                    17

<210> 1060
<211> 18
<212> DNA
<213> Artificial Sequence

<220>
<223> Description of Artificial Sequence:capture

<400> 1060
tggagggcac gtgcgtgg                                                   18

<210> 1061
<211> 18
<212> DNA
<213> Artificial Sequence

<220>
<223> Description of Artificial Sequence:capture

<400> 1061
tggagggcga gtgcgtgg                                                   18

<210> 1062
<211> 17
<212> DNA
<213> Artificial Sequence

<220>
<223> Description of Artificial Sequence:capture

<400> 1062
ggagggcctg tgcgtgg                                                    17

<210> 1063
<211> 17
<212> DNA
<213> Artificial Sequence

<220>
<223> Description of Artificial Sequence:capture

<400> 1063
gcagggccgg tgcgtgg                                                    17

<210> 1064
<211> 18
<212> DNA
<213> Artificial Sequence

<220>
<223> Description of Artificial Sequence:capture

<400> 1064
tggatggcac gtgcgtgg                                                    18

<210> 1065
<211> 18
<212> DNA
<213> Artificial Sequence

<220>
<223> Description of Artificial Sequence:capture

<400> 1065
tggatgccac gtgcgtgg                                                    18

<210> 1066
<211> 16
<212> DNA
<213> Artificial Sequence

<220>
<223> Description of Artificial Sequence:capture

<400> 1066
gcgtggagtg gctccg                                                      16

<210> 1067
<211> 17
<212> DNA
<213> Artificial Sequence

<220>
<223> Description of Artificial Sequence:capture

<400> 1067
gcgtggagtg gctccgc                                                     17

<210> 1068
<211> 17
<212> DNA
<213> Artificial Sequence

<220>
<223> Description of Artificial Sequence:capture

<400> 1068
tccgcagaca cctggag                                                     17

<210> 1069
<211> 14

<212> DNA
<213> Artificial Sequence

<220>
<223> Description of Artificial Sequence:capture

<400> 1069
tggagaaccg gaag                                                    14

<210> 1070
<211> 17
<212> DNA
<213> Artificial Sequence

<220>
<223> Description of Artificial Sequence:capture

<400> 1070
cagcgcactg gtgagtg                                                 17

<210> 1071
<211> 17
<212> DNA
<213> Artificial Sequence

<220>
<223> Description of Artificial Sequence:capture

<400> 1071
gcgtggactg gctccgc                                                 17

<210> 1072
<211> 19
<212> DNA
<213> Artificial Sequence

<220>
<223> Description of Artificial Sequence:capture

<400> 1072
cctggagaag cggcggccc                                               19

<210> 1073
<211> 19
<212> DNA
<213> Artificial Sequence

<220>
<223> Description of Artificial Sequence:primer

<400> 1073
gcccctcctc gcccccagg                                               19

<210> 1074
<211> 18
<212> DNA
<213> Artificial Sequence

<220>
<223> Description of Artificial Sequence:primer

<400> 1074
ggccggggtc actcaccg                                                    18

<210> 1075
<211> 18
<212> DNA
<213> Artificial Sequence

<220>
<223> Description of Artificial Sequence:primer

<400> 1075
ggccggggcc agggtctc                                                    18

<210> 1076
<211> 18
<212> DNA
<213> Artificial Sequence

<220>
<223> Description of Artificial Sequence:primer

<400> 1076
gggcggggcc agggtctc                                                    18

<210> 1077
<211> 18
<212> DNA
<213> Artificial Sequence

<220>
<223> Description of Artificial Sequence:primer

<400> 1077
ggcctgggcc agggtctc                                                    18

<210> 1078
<211> 20
<212> DNA
<213> Artificial Sequence

<220>
<223> Description of Artificial Sequence:primer

<400> 1078
ccccactgcc cctggtacca                                                  20

<210> 1079
<211> 20
<212> DNA
<213> Artificial Sequence

<220>
<223> Description of Artificial Sequence:primer

<400> 1079
ccccactgcc cctggtaccc                                                    20

<210> 1080
<211> 17
<212> DNA
<213> Artificial Sequence

<220>
<223> Description of Artificial Sequence:capture

<400> 1080
acacctccgt gtcccgg                                                       17

<210> 1081
<211> 17
<212> DNA
<213> Artificial Sequence

<220>
<223> Description of Artificial Sequence:capture

<400> 1081
acaccgccat gtcccgg                                                       17

<210> 1082
<211> 17
<212> DNA
<213> Artificial Sequence

<220>
<223> Description of Artificial Sequence:capture

<400> 1082
tctacaccgc cgtgtcc                                                       17

<210> 1083
<211> 20
<212> DNA
<213> Artificial Sequence

<220>
<223> Description of Artificial Sequence:capture

<400> 1083
ctacaccgct atgtcccggc                                                    20

<210> 1084
<211> 18
<212> DNA
<213> Artificial Sequence

<220>
<223> Description of Artificial Sequence:capture

<400> 1084
gcttcatctc agtgggct                                                      18

<210> 1085
<211> 17
<212> DNA
<213> Artificial Sequence

<220>
<223> Description of Artificial Sequence:capture

<400> 1085
cttcatcacc gtgggct                                                    17

<210> 1086
<211> 17
<212> DNA
<213> Artificial Sequence

<220>
<223> Description of Artificial Sequence:capture

<400> 1086
gcttcatcgc agtgggc                                                    17

<210> 1087
<211> 16
<212> DNA
<213> Artificial Sequence

<220>
<223> Description of Artificial Sequence:capture

<400> 1087
gcttcattgc agtggg                                                     16

<210> 1088
<211> 17
<212> DNA
<213> Artificial Sequence

<220>
<223> Description of Artificial Sequence:capture

<400> 1088
ttcatctccg tgggcta                                                    17

<210> 1089
<211> 18
<212> DNA
<213> Artificial Sequence

<220>
<223> Description of Artificial Sequence:capture

<400> 1089
gtccgagaga ggagccgc                                                   18

<210> 1090
<211> 17
<212> DNA

<213> Artificial Sequence

<220>
<223> Description of Artificial Sequence:capture

<400> 1090
tccgagaggg gagccgc                                                    17

<210> 1091
<211> 17
<212> DNA
<213> Artificial Sequence

<220>
<223> Description of Artificial Sequence:capture

<400> 1091
gtccgaggat ggcgccc                                                    17

<210> 1092
<211> 17
<212> DNA
<213> Artificial Sequence

<220>
<223> Description of Artificial Sequence:capture

<400> 1092
gtccgaggac ggagccc                                                    17

<210> 1093
<211> 17
<212> DNA
<213> Artificial Sequence

<220>
<223> Description of Artificial Sequence:capture

<400> 1093
gtccgaggaa ggagccg                                                    17

<210> 1094
<211> 18
<212> DNA
<213> Artificial Sequence

<220>
<223> Description of Artificial Sequence:capture

<400> 1094
gtccgagaga ggagcccc                                                   18

<210> 1095
<211> 17
<212> DNA
<213> Artificial Sequence

<220>

<223> Description of Artificial Sequence:capture

<400> 1095
gtccgaggat ggctccc                                                    17

<210> 1096
<211> 17
<212> DNA
<213> Artificial Sequence

<220>
<223> Description of Artificial Sequence:capture

<400> 1096
gtccgaggac ggagccc                                                    17

<210> 1097
<211> 17
<212> DNA
<213> Artificial Sequence

<220>
<223> Description of Artificial Sequence:capture

<400> 1097
acagatctac aaggccc                                                    17

<210> 1098
<211> 17
<212> DNA
<213> Artificial Sequence

<220>
<223> Description of Artificial Sequence:capture

<400> 1098
acagatcttc aagacca                                                    17

<210> 1099
<211> 17
<212> DNA
<213> Artificial Sequence

<220>
<223> Description of Artificial Sequence:capture

<400> 1099
acagatctcc aagacca                                                    17

<210> 1100
<211> 17
<212> DNA
<213> Artificial Sequence

<220>
<223> Description of Artificial Sequence:capture

<400> 1100

acagatctgc aaggccc                                                    17

<210> 1101
<211> 17
<212> DNA
<213> Artificial Sequence

<220>
<223> Description of Artificial Sequence:capture

<400> 1101
acggaacatg aaggcct                                                    17

<210> 1102
<211> 17
<212> DNA
<213> Artificial Sequence

<220>
<223> Description of Artificial Sequence:capture

<400> 1102
acagatctcg aagacca                                                    17

<210> 1103
<211> 17
<212> DNA
<213> Artificial Sequence

<220>
<223> Description of Artificial Sequence:capture

<400> 1103
acagatctac aagacca                                                    17

<210> 1104
<211> 17
<212> DNA
<213> Artificial Sequence

<220>
<223> Description of Artificial Sequence:capture

<400> 1104
acagaccttc aagacca                                                    17

<210> 1105
<211> 16
<212> DNA
<213> Artificial Sequence

<220>
<223> Description of Artificial Sequence:capture

<400> 1105
acagatctgc aagacc                                                     16

<210> 1106

<211> 17
<212> DNA
<213> Artificial Sequence

<220>
<223> Description of Artificial Sequence:capture

<400> 1106
acagaagtac aagcgcc                                                    17

<210> 1107
<211> 17
<212> DNA
<213> Artificial Sequence

<220>
<223> Description of Artificial Sequence:capture

<400> 1107
acagaactgc aagacca                                                    17

<210> 1108
<211> 17
<212> DNA
<213> Artificial Sequence

<220>
<223> Description of Artificial Sequence:capture

<400> 1108
acagatctgc aaggcca                                                    17

<210> 1109
<211> 17
<212> DNA
<213> Artificial Sequence

<220>
<223> Description of Artificial Sequence:capture

<400> 1109
ccgagagagc ctgcgga                                                    17

<210> 1110
<211> 17
<212> DNA
<213> Artificial Sequence

<220>
<223> Description of Artificial Sequence:capture

<400> 1110
ccgagagaac ctgcgga                                                    17

<210> 1111
<211> 17
<212> DNA
<213> Artificial Sequence

<220>
<223> Description of Artificial Sequence:capture

<400> 1111
ccgagaggac ctgcgga                                                    17

<210> 1112
<211> 16
<212> DNA
<213> Artificial Sequence

<220>
<223> Description of Artificial Sequence:capture

<400> 1112
ccgagtgggc ctgcgg                                                     16

<210> 1113
<211> 17
<212> DNA
<213> Artificial Sequence

<220>
<223> Description of Artificial Sequence:capture

<400> 1113
ccgagtgagc ctgcgga                                                    17

<210> 1114
<211> 17
<212> DNA
<213> Artificial Sequence

<220>
<223> Description of Artificial Sequence:capture

<400> 1114
ccgagaggac ctgcgca                                                    17

<210> 1115
<211> 17
<212> DNA
<213> Artificial Sequence

<220>
<223> Description of Artificial Sequence:capture

<400> 1115
ccgagagagc ctgcgca                                                    17

<210> 1116
<211> 17
<212> DNA
<213> Artificial Sequence

<220>
<223> Description of Artificial Sequence:capture

<400> 1116
cctgcggaac ctgcgcg                                                    17

<210> 1117
<211> 19
<212> DNA
<213> Artificial Sequence

<220>
<223> Description of Artificial Sequence:capture

<400> 1117
gacctgcgga ccctgctcc                                                  19

<210> 1118
<211> 17
<212> DNA
<213> Artificial Sequence

<220>
<223> Description of Artificial Sequence:capture

<400> 1118
cctgcggatc gcgctcc                                                    17

<210> 1119
<211> 17
<212> DNA
<213> Artificial Sequence

<220>
<223> Description of Artificial Sequence:capture

<400> 1119
ctgcggaagc tgcgcgg                                                    17

<210> 1120
<211> 17
<212> DNA
<213> Artificial Sequence

<220>
<223> Description of Artificial Sequence:capture

<400> 1120
cctgcggaac ctgctcc                                                    17

<210> 1121
<211> 17
<212> DNA
<213> Artificial Sequence

<220>
<223> Description of Artificial Sequence:capture

<400> 1121
cctgcgcacc gcgctcc                                                    17

<210> 1122
<211> 17
<212> DNA
<213> Artificial Sequence

<220>
<223> Description of Artificial Sequence:capture

<400> 1122
cctgcgcacc ccgctcc                                                    17

<210> 1123
<211> 17
<212> DNA
<213> Artificial Sequence

<220>
<223> Description of Artificial Sequence:capture

<400> 1123
cctccagagc atgtacg                                                    17

<210> 1124
<211> 17
<212> DNA
<213> Artificial Sequence

<220>
<223> Description of Artificial Sequence:capture

<400> 1124
ctccagagga tgtacgg                                                    17

<210> 1125
<211> 17
<212> DNA
<213> Artificial Sequence

<220>
<223> Description of Artificial Sequence:capture

<400> 1125
ctccagacga tgtacgg                                                    17

<210> 1126
<211> 17
<212> DNA
<213> Artificial Sequence

<220>
<223> Description of Artificial Sequence:capture

<400> 1126
cctccagtgg atgtatg                                                    17

<210> 1127
<211> 17

<212> DNA
<213> Artificial Sequence

<220>
<223> Description of Artificial Sequence:capture

<400> 1127
ctccagaata tgtacgg                                                    17

<210> 1128
<211> 17
<212> DNA
<213> Artificial Sequence

<220>
<223> Description of Artificial Sequence:capture

<400> 1128
catccaggtg atgtatg                                                    17

<210> 1129
<211> 18
<212> DNA
<213> Artificial Sequence

<220>
<223> Description of Artificial Sequence:capture

<400> 1129
catccagagc atgtacgg                                                   18

<210> 1130
<211> 17
<212> DNA
<213> Artificial Sequence

<220>
<223> Description of Artificial Sequence:capture

<400> 1130
atccagagga tgtatgg                                                    17

<210> 1131
<211> 16
<212> DNA
<213> Artificial Sequence

<220>
<223> Description of Artificial Sequence:capture

<400> 1131
tggcagacga tgtatg                                                     16

<210> 1132
<211> 17
<212> DNA
<213> Artificial Sequence

<220>
<223> Description of Artificial Sequencc:capture

<400> 1132
ctccagaata tgtatgg                                                    17

<210> 1133
<211> 17
<212> DNA
<213> Artificial Sequence

<220>
<223> Description of Artificial Sequence:capture

<400> 1133
cctccagagc acgtacg                                                    17

<210> 1134
<211> 17
<212> DNA
<213> Artificial Sequence

<220>
<223> Description of Artificial Sequence:capture

<400> 1134
atccagagga tgtacgg                                                    17

<210> 1135
<211> 17
<212> DNA
<213> Artificial Sequence

<220>
<223> Description of Artificial Sequence:capture

<400> 1135
ctccagagga tgtatgg                                                    17

<210> 1136
<211> 17
<212> DNA
<213> Artificial Sequence

<220>
<223> Description of Artificial Sequence:capture

<400> 1136
ctccagagga tgtttgg                                                    17

<210> 1137
<211> 17
<212> DNA
<213> Artificial Sequence

<220>
<223> Description of Artificial Sequence:capture

<400> 1137
tggcagagga tgtatgg                                                    17

<210> 1138
<211> 17
<212> DNA
<213> Artificial Sequence

<220>
<223> Description of Artificial Sequence:capture

<400> 1138
ctccagagga tgtctgg                                                    17

<210> 1139
<211> 17
<212> DNA
<213> Artificial Sequence

<220>
<223> Description of Artificial Sequence:capture

<400> 1139
ctccagagga tgtgtgg                                                    17

<210> 1140
<211> 17
<212> DNA
<213> Artificial Sequence

<220>
<223> Description of Artificial Sequence:capture

<400> 1140
atccagagga tgtctgg                                                    17

<210> 1141
<211> 17
<212> DNA
<213> Artificial Sequence

<220>
<223> Description of Artificial Sequence:capture

<400> 1141
ctccacagga tgtacgg                                                    17

<210> 1142
<211> 17
<212> DNA
<213> Artificial Sequence

<220>
<223> Description of Artificial Sequence:capture

<400> 1142
tgaccagtac gcctacg                                                    17

<210> 1143
<211> 16
<212> DNA
<213> Artificial Sequence

<220>
<223> Description of Artificial Sequence:capture

<400> 1143
aaccagttag cctacg                                                    16

<210> 1144
<211> 17
<212> DNA
<213> Artificial Sequence

<220>
<223> Description of Artificial Sequence:capture

<400> 1144
accaccagga cgcctac                                                   17

<210> 1145
<211> 17
<212> DNA
<213> Artificial Sequence

<220>
<223> Description of Artificial Sequence:capture

<400> 1145
accaccagca cgcctac                                                   17

<210> 1146
<211> 17
<212> DNA
<213> Artificial Sequence

<220>
<223> Description of Artificial Sequence:capture

<400> 1146
tgaccagtcc gcctacg                                                   17

<210> 1147
<211> 17
<212> DNA
<213> Artificial Sequence

<220>
<223> Description of Artificial Sequence:capture

<400> 1147
ataaccagaa cgcctac                                                   17

<210> 1148
<211> 17
<212> DNA

<213> Artificial Sequence

<220>
<223> Description of Artificial Sequence:capture

<400> 1148
taaccagttc gcctacg                                                    17

<210> 1149
<211> 17
<212> DNA
<213> Artificial Sequence

<220>
<223> Description of Artificial Sequence:capture

<400> 1149
taaccagtac gcctacg                                                    17

<210> 1150
<211> 17
<212> DNA
<213> Artificial Sequence

<220>
<223> Description of Artificial Sequence:capture

<400> 1150
taaccagtta gcctacg                                                    17

<210> 1151
<211> 17
<212> DNA
<213> Artificial Sequence

<220>
<223> Description of Artificial Sequence:capture

<400> 1151
atgaccagga cgcctac                                                    17

<210> 1152
<211> 17
<212> DNA
<213> Artificial Sequence

<220>
<223> Description of Artificial Sequence:capture

<400> 1152
tgaccagttc gcctacg                                                    17

<210> 1153
<211> 17
<212> DNA
<213> Artificial Sequence

<220>

<223> Description of Artificial Sequence:capture

<400> 1153
taaacagtac gcctacg                                                    17

<210> 1154
<211> 16
<212> DNA
<213> Artificial Sequence

<220>
<223> Description of Artificial Sequence:capture

<400> 1154
aaccggttag cctacg                                                     16

<210> 1155
<211> 17
<212> DNA
<213> Artificial Sequence

<220>
<223> Description of Artificial Sequence:capture

<400> 1155
taaccagttc gcctacg                                                    17

<210> 1156
<211> 16
<212> DNA
<213> Artificial Sequence

<220>
<223> Description of Artificial Sequence:capture

<400> 1156
aaccagtacg cctacg                                                     16

<210> 1157
<211> 16
<212> DNA
<213> Artificial Sequence

<220>
<223> Description of Artificial Sequence:capture

<400> 1157
aaccagtacg cctacg                                                     16

<210> 1158
<211> 17
<212> DNA
<213> Artificial Sequence

<220>
<223> Description of Artificial Sequence:capture

<400> 1158

ggagcagcgg agagcct                                                    17

<210> 1159
<211> 17
<212> DNA
<213> Artificial Sequence

<220>
<223> Description of Artificial Sequence:capture

<400> 1159
ggagcaggac agagcct                                                    17

<210> 1160
<211> 17
<212> DNA
<213> Artificial Sequence

<220>
<223> Description of Artificial Sequence:capture

<400> 1160
ggagcagctg agagcct                                                    17

<210> 1161
<211> 17
<212> DNA
<213> Artificial Sequence

<220>
<223> Description of Artificial Sequence:capture

<400> 1161
cggagcagtg gagagcc                                                    17

<210> 1162
<211> 17
<212> DNA
<213> Artificial Sequence

<220>
<223> Description of Artificial Sequence:capture

<400> 1162
ggagcagcgg agaacct                                                    17

<210> 1163
<211> 17
<212> DNA
<213> Artificial Sequence

<220>
<223> Description of Artificial Sequence:capture

<400> 1163
ggagcagctg agaacct                                                    17

<210> 1164

<211> 17
<212> DNA
<213> Artificial Sequence

<220>
<223> Description of Artificial Sequence:capture

<400> 1164
ggagcagctg agagctt                                                17

<210> 1165
<211> 17
<212> DNA
<213> Artificial Sequence

<220>
<223> Description of Artificial Sequence:capture

<400> 1165
ggagggcgag tgcgtgg                                                17

<210> 1166
<211> 17
<212> DNA
<213> Artificial Sequence

<220>
<223> Description of Artificial Sequence:capture

<400> 1166
ggagggcacg tgcgtgg                                                17

<210> 1167
<211> 17
<212> DNA
<213> Artificial Sequence

<220>
<223> Description of Artificial Sequence:capture

<400> 1167
ggagggcctg tgcgtgg                                                17

<210> 1168
<211> 17
<212> DNA
<213> Artificial Sequence

<220>
<223> Description of Artificial Sequence:capture

<400> 1168
ggagggcgcg tgcgtgg                                                17

<210> 1169
<211> 17
<212> DNA
<213> Artificial Sequence

<220>
<223> Description of Artificial Sequence:capture

<400> 1169
ggaggacctg tgcgtgg                                                    17

<210> 1170
<211> 17
<212> DNA
<213> Artificial Sequence

<220>
<223> Description of Artificial Sequence:capture

<400> 1170
ggtatttcca cacctcc                                                    17

<210> 1171
<211> 17
<212> DNA
<213> Artificial Sequence

<220>
<223> Description of Artificial Sequence:capture

<400> 1171
ggtatttcga caccgcc                                                    17

<210> 1172
<211> 17
<212> DNA
<213> Artificial Sequence

<220>
<223> Description of Artificial Sequence:capture

<400> 1172
ccggcccgtc cgcgggg                                                    17

<210> 1173
<211> 17
<212> DNA
<213> Artificial Sequence

<220>
<223> Description of Artificial Sequence:capture

<400> 1173
ggccgcggag agccccg                                                    17

<210> 1174
<211> 17
<212> DNA
<213> Artificial Sequence

<220>
<223> Description of Artificial Sequence:capture

<400> 1174
cggggagctc cgcttca                                                    17

<210> 1175
<211> 16
<212> DNA
<213> Artificial Sequence

<220>
<223> Description of Artificial Sequence:capture

<400> 1175
acgacacgca gttcgt                                                     16

<210> 1176
<211> 17
<212> DNA
<213> Artificial Sequence

<220>
<223> Description of Artificial Sequence:capture

<400> 1176
agttcgtgcg gttcgac                                                    17

<210> 1177
<211> 17
<212> DNA
<213> Artificial Sequence

<220>
<223> Description of Artificial Sequence:capture

<400> 1177
tgaggttcaa cagcgac                                                    17

<210> 1178
<211> 17
<212> DNA
<213> Artificial Sequence

<220>
<223> Description of Artificial Sequence:capture

<400> 1178
gcgacgccac gagtccg                                                    17

<210> 1179
<211> 17
<212> DNA
<213> Artificial Sequence

<220>
<223> Description of Artificial Sequence:capture

<400> 1179
ggtatttcca caccgcc                                                    17

<210> 1180
<211> 16
<212> DNA
<213> Artificial Sequence

<220>
<223> Description of Artificial Sequence:capture

<400> 1180
acgacacgct gttcgt                                                    16

<210> 1181
<211> 18
<212> DNA
<213> Artificial Sequence

<220>
<223> Description of Artificial Sequence:capture

<400> 1181
aggatggcgc cccgggcg                                                  18

<210> 1182
<211> 18
<212> DNA
<213> Artificial Sequence

<220>
<223> Description of Artificial Sequence:capture

<400> 1182
aggatggctc cccgggcg                                                  18

<210> 1183
<211> 17
<212> DNA
<213> Artificial Sequence

<220>
<223> Description of Artificial Sequence:capture

<400> 1183
atggctcccc gggcgcc                                                   17

<210> 1184
<211> 17
<212> DNA
<213> Artificial Sequence

<220>
<223> Description of Artificial Sequence:capture

<400> 1184
cgggcgccat ggataga                                                   17

<210> 1185
<211> 17

<212> DNA
<213> Artificial Sequence

<220>
<223> Description of Artificial Sequence:capture

<400> 1185
gccgtgggtg gagcagg                                                    17

<210> 1186
<211> 17
<212> DNA
<213> Artificial Sequence

<220>
<223> Description of Artificial Sequence:capture

<400> 1186
gaggggccag aatattg                                                    17

<210> 1187
<211> 17
<212> DNA
<213> Artificial Sequence

<220>
<223> Description of Artificial Sequence:capture

<400> 1187
ggggccggcg tattggg                                                    17

<210> 1188
<211> 17
<212> DNA
<213> Artificial Sequence

<220>
<223> Description of Artificial Sequence:capture

<400> 1188
gggccgggat attggga                                                    17

<210> 1189
<211> 16
<212> DNA
<213> Artificial Sequence

<220>
<223> Description of Artificial Sequence:capture

<400> 1189
ggccggaata ttggga                                                     16

<210> 1190
<211> 17
<212> DNA
<213> Artificial Sequence

<220>
<223> Description of Artificial Sequence:capture

<400> 1190
tattgggagc gggagac                                                17

<210> 1191
<211> 17
<212> DNA
<213> Artificial Sequence

<220>
<223> Description of Artificial Sequence:capture

<400> 1191
attgggacgg ggagaca                                                17

<210> 1192
<211> 17
<212> DNA
<213> Artificial Sequence

<220>
<223> Description of Artificial Sequence:capture

<400> 1192
attgggacga ggagaca                                                17

<210> 1193
<211> 17
<212> DNA
<213> Artificial Sequence

<220>
<223> Description of Artificial Sequence:capture

<400> 1193
ggagccggag acacaga                                                17

<210> 1194
<211> 17
<212> DNA
<213> Artificial Sequence

<220>
<223> Description of Artificial Sequence:capture

<400> 1194
gggaccggga cacacag                                                17

<210> 1195
<211> 17
<212> DNA
<213> Artificial Sequence

<220>
<223> Description of Artificial Sequence:capture

<400> 1195
ggaccgggag acacaga                                                    17

<210> 1196
<211> 17
<212> DNA
<213> Artificial Sequence

<220>
<223> Description of Artificial Sequence:capture

<400> 1196
ccgggagata cagatct                                                    17

<210> 1197
<211> 17
<212> DNA
<213> Artificial Sequence

<220>
<223> Description of Artificial Sequence:capture

<400> 1197
ggagacacgg aacatga                                                    17

<210> 1198
<211> 17
<212> DNA
<213> Artificial Sequence

<220>
<223> Description of Artificial Sequence:capture

<400> 1198
cacacagacc ttcaaga                                                    17

<210> 1199
<211> 17
<212> DNA
<213> Artificial Sequence

<220>
<223> Description of Artificial Sequence:capture

<400> 1199
cacacagaac tgcaaga                                                    17

<210> 1200
<211> 17
<212> DNA
<213> Artificial Sequence

<220>
<223> Description of Artificial Sequence:capture

<400> 1200
gacacggaac atgaagg                                                    17

<210> 1201
<211> 17
<212> DNA
<213> Artificial Sequence

<220>
<223> Description of Artificial Sequence:capture

<400> 1201
tctacaagac caacaca                                                    17

<210> 1202
<211> 18
<212> DNA
<213> Artificial Sequence

<220>
<223> Description of Artificial Sequence:capture

<400> 1202
aagtacaagc gccaggca                                                   18

<210> 1203
<211> 17
<212> DNA
<213> Artificial Sequence

<220>
<223> Description of Artificial Sequence:capture

<400> 1203
gcaaggccaa ggcacag                                                    17

<210> 1204
<211> 18
<212> DNA
<213> Artificial Sequence

<220>
<223> Description of Artificial Sequence:capture

<400> 1204
aaggcctccg cgcagact                                                   18

<210> 1205
<211> 18
<212> DNA
<213> Artificial Sequence

<220>
<223> Description of Artificial Sequence:capture

<400> 1205
caagaccaac acacagac                                                   18

<210> 1206
<211> 17
<212> DNA

<213> Artificial Sequence

<220>
<223> Description of Artificial Sequence:capture

<400> 1206
caacacacgg acttacc                                                    17

<210> 1207
<211> 17
<212> DNA
<213> Artificial Sequence

<220>
<223> Description of Artificial Sequence:capture

<400> 1207
aggcacaggc tgaccga                                                    17

<210> 1208
<211> 17
<212> DNA
<213> Artificial Sequence

<220>
<223> Description of Artificial Sequence:capture

<400> 1208
cacagactta ccgagag                                                    17

<210> 1209
<211> 17
<212> DNA
<213> Artificial Sequence

<220>
<223> Description of Artificial Sequence:capture

<400> 1209
acagactggc cgagtga                                                    17

<210> 1210
<211> 17
<212> DNA
<213> Artificial Sequence

<220>
<223> Description of Artificial Sequence:capture

<400> 1210
tgaccgagtg agcctgc                                                    17

<210> 1211
<211> 17
<212> DNA
<213> Artificial Sequence

<220>

<223> Description of Artificial Sequence:capture

<400> 1211
atggcgcccc gggcgcc                                                    17

<210> 1212
<211> 17
<212> DNA
<213> Artificial Sequence

<220>
<223> Description of Artificial Sequence:capture

<400> 1212
ggaccgggag acacgga                                                    17

<210> 1213
<211> 17
<212> DNA
<213> Artificial Sequence

<220>
<223> Description of Artificial Sequence:capture

<400> 1213
gacacggaac atgaagg                                                    17

<210> 1214
<211> 18
<212> DNA
<213> Artificial Sequence

<220>
<223> Description of Artificial Sequence:capture

<400> 1214
caagaccaac acacggac                                                   18

<210> 1215
<211> 17
<212> DNA
<213> Artificial Sequence

<220>
<223> Description of Artificial Sequence:capture

<400> 1215
cgcagactta ccgagag                                                    17

<210> 1216
<211> 17
<212> DNA
<213> Artificial Sequence

<220>
<223> Description of Artificial Sequence:capture

<400> 1216

ttaccgagtg aacctgc                                                    17

<210> 1217
<211> 17
<212> DNA
<213> Artificial Sequence

<220>
<223> Description of Artificial Sequence:capture

<400> 1217
acggagcccc gggcgcc                                                    17

<210> 1218
<211> 17
<212> DNA
<213> Artificial Sequence

<220>
<223> Description of Artificial Sequence:capture

<400> 1218
ggaccgggag atacaga                                                    17

<210> 1219
<211> 17
<212> DNA
<213> Artificial Sequence

<220>
<223> Description of Artificial Sequence:capture

<400> 1219
cacggactta ccgagag                                                    17

<210> 1220
<211> 17
<212> DNA
<213> Artificial Sequence

<220>
<223> Description of Artificial Sequence:capture

<400> 1220
ttaccgagtg agcctgc                                                    17

<210> 1221
<211> 17
<212> DNA
<213> Artificial Sequence

<220>
<223> Description of Artificial Sequence:capture

<400> 1221
gaggagcccc gggcgcc                                                    17

<210> 1222

<211> 17
<212> DNA
<213> Artificial Sequence

<220>
<223> Description of Artificial Sequence:capture

<400> 1222
ggagccggag acacaga                                                    17

<210> 1223
<211> 17
<212> DNA
<213> Artificial Sequence

<220>
<223> Description of Artificial Sequence:capture

<400> 1223
cacagactta ccgagtg                                                    17

<210> 1224
<211> 17
<212> DNA
<213> Artificial Sequence

<220>
<223> Description of Artificial Sequence:capture

<400> 1224
tgaccgagtg ggcctgc                                                    17

<210> 1225
<211> 17
<212> DNA
<213> Artificial Sequence

<220>
<223> Description of Artificial Sequence:capture

<400> 1225
ggagcgggag acacaga                                                    17

<210> 1226
<211> 18
<212> DNA
<213> Artificial Sequence

<220>
<223> Description of Artificial Sequence:capture

<400> 1226
ctgcggatcg cgctccgc                                                   18

<210> 1227
<211> 17
<212> DNA
<213> Artificial Sequence

<220>
<223> Description of Artificial Sequence:capture

<400> 1227
gcgcaccccg ctccgct                                                    17

<210> 1228
<211> 17
<212> DNA
<213> Artificial Sequence

<220>
<223> Description of Artificial Sequence:capture

<400> 1228
accctgctcc gctacta                                                    17

<210> 1229
<211> 18
<212> DNA
<213> Artificial Sequence

<220>
<223> Description of Artificial Sequence:capture

<400> 1229
gcgctccgcg actacaac                                                   18

<210> 1230
<211> 17
<212> DNA
<213> Artificial Sequence

<220>
<223> Description of Artificial Sequence:capture

<400> 1230
tgcgcggcca ctacaac                                                    17

<210> 1231
<211> 17
<212> DNA
<213> Artificial Sequence

<220>
<223> Description of Artificial Sequence:capture

<400> 1231
tctcacatca tccagag                                                    17

<210> 1232
<211> 18
<212> DNA
<213> Artificial Sequence

<220>
<223> Description of Artificial Sequence:capture

<400> 1232
ggtctcacat catccaga                                                    18

<210> 1233
<211> 17
<212> DNA
<213> Artificial Sequence

<220>
<223> Description of Artificial Sequence:capture

<400> 1233
gtctcacact tggcaga                                                     17

<210> 1234
<211> 16
<212> DNA
<213> Artificial Sequence

<220>
<223> Description of Artificial Sequence:capture

<400> 1234
ctcacatcat ccagag                                                      16

<210> 1235
<211> 17
<212> DNA
<213> Artificial Sequence

<220>
<223> Description of Artificial Sequence:capture

<400> 1235
accctccaca ggatgta                                                     17

<210> 1236
<211> 17
<212> DNA
<213> Artificial Sequence

<220>
<223> Description of Artificial Sequence:capture

<400> 1236
ccagagcacg tacggct                                                     17

<210> 1237
<211> 17
<212> DNA
<213> Artificial Sequence

<220>
<223> Description of Artificial Sequence:capture

<400> 1237
gaggatgtgt ggctgcg                                                     17

<210> 1238
<211> 17
<212> DNA
<213> Artificial Sequence

<220>
<223> Description of Artificial Sequence:capture

<400> 1238
aggatgtatg gctgcga                                                    17

<210> 1239
<211> 17
<212> DNA
<213> Artificial Sequence

<220>
<223> Description of Artificial Sequence:capture

<400> 1239
aggatgtttg gctgcga                                                    17

<210> 1240
<211> 17
<212> DNA
<213> Artificial Sequence

<220>
<223> Description of Artificial Sequence:capture

<400> 1240
aggatgtctg gctgcga                                                    17

<210> 1241
<211> 16
<212> DNA
<213> Artificial Sequence

<220>
<223> Description of Artificial Sequence:capture

<400> 1241
gctgcgacct ggggcc                                                     16

<210> 1242
<211> 16
<212> DNA
<213> Artificial Sequence

<220>
<223> Description of Artificial Sequence:capture

<400> 1242
tggggcccga cgggcg                                                     16

<210> 1243
<211> 17

<212> DNA
<213> Artificial Sequence

<220>
<223> Description of Artificial Sequence:capture

<400> 1243
gacgggcgtc tcctccg                                                    17

<210> 1244
<211> 15
<212> DNA
<213> Artificial Sequence

<220>
<223> Description of Artificial Sequence:capture

<400> 1244
acgggcgctt cctcc                                                      15

<210> 1245
<211> 18
<212> DNA
<213> Artificial Sequence

<220>
<223> Description of Artificial Sequence:capture

<400> 1245
ctgcgcaccg cgctccgc                                                   18

<210> 1246
<211> 17
<212> DNA
<213> Artificial Sequence

<220>
<223> Description of Artificial Sequence:capture

<400> 1246
accgcgctcc gctacta                                                    17

<210> 1247
<211> 17
<212> DNA
<213> Artificial Sequence

<220>
<223> Description of Artificial Sequence:capture

<400> 1247
tctcacatca tccaggt                                                    17

<210> 1248
<211> 17
<212> DNA
<213> Artificial Sequence

<220>
<223> Description of Artificial Sequence:capture

<400> 1248
acgatgtatg gctgcga                                                    17

<210> 1249
<211> 17
<212> DNA
<213> Artificial Sequence

<220>
<223> Description of Artificial Sequence:capture

<400> 1249
gctgcgacct ggggccg                                                    17

<210> 1250
<211> 17
<212> DNA
<213> Artificial Sequence

<220>
<223> Description of Artificial Sequence:capture

<400> 1250
atcgcgctcc gctacta                                                    17

<210> 1251
<211> 17
<212> DNA
<213> Artificial Sequence

<220>
<223> Description of Artificial Sequence:capture

<400> 1251
tggatgtatg gctgcga                                                    17

<210> 1252
<211> 17
<212> DNA
<213> Artificial Sequence

<220>
<223> Description of Artificial Sequence:capture

<400> 1252
accccgctcc gctacta                                                    17

<210> 1253
<211> 17
<212> DNA
<213> Artificial Sequence

<220>
<223> Description of Artificial Sequence:capture

<400> 1253
aatatgtatg gctgcga                                                    17

<210> 1254
<211> 17
<212> DNA
<213> Artificial Sequence

<220>
<223> Description of Artificial Sequence:capture

<400> 1254
aacctgctcc gctacta                                                    17

<210> 1255
<211> 17
<212> DNA
<213> Artificial Sequence

<220>
<223> Description of Artificial Sequence:capture

<400> 1255
atcgcgctcc gcgacta                                                    17

<210> 1256
<211> 16
<212> DNA
<213> Artificial Sequence

<220>
<223> Description of Artificial Sequence:capture

<400> 1256
cctcctcccc gggcat                                                     16

<210> 1257
<211> 17
<212> DNA
<213> Artificial Sequence

<220>
<223> Description of Artificial Sequence:capture

<400> 1257
ctccgcggtt ataacca                                                    17

<210> 1258
<211> 17
<212> DNA
<213> Artificial Sequence

<220>
<223> Description of Artificial Sequence:capture

<400> 1258
tccgcgggta tgaccag                                                    17

<210> 1259
<211> 17
<212> DNA
<213> Artificial Sequence

<220>
<223> Description of Artificial Sequence:capture

<400> 1259
tccgcgggta ccaccag                                                    17

<210> 1260
<211> 17
<212> DNA
<213> Artificial Sequence

<220>
<223> Description of Artificial Sequence:capture

<400> 1260
gcgggcataa ccagtac                                                    17

<210> 1261
<211> 17
<212> DNA
<213> Artificial Sequence

<220>
<223> Description of Artificial Sequence:capture

<400> 1261
cgggcataaa cagtacg                                                    17

<210> 1262
<211> 17
<212> DNA
<213> Artificial Sequence

<220>
<223> Description of Artificial Sequence:capture

<400> 1262
gtataaccgg ttagcct                                                    17

<210> 1263
<211> 17
<212> DNA
<213> Artificial Sequence

<220>
<223> Description of Artificial Sequence:capture

<400> 1263
gacggcaaag attacat                                                    17

<210> 1264
<211> 17
<212> DNA

<213> Artificial Sequence

<220>
<223> Description of Artificial Sequence:capture

<400> 1264
gacggcaaag attacat                                                    17

<210> 1265
<211> 16
<212> DNA
<213> Artificial Sequence

<220>
<223> Description of Artificial Sequence:capture

<400> 1265
ccctgaagga ggacct                                                     16

<210> 1266
<211> 17
<212> DNA
<213> Artificial Sequence

<220>
<223> Description of Artificial Sequence:capture

<400> 1266
gccctgaatg aggacct                                                    17

<210> 1267
<211> 17
<212> DNA
<213> Artificial Sequence

<220>
<223> Description of Artificial Sequence:capture

<400> 1267
aggacctgag ctcctgg                                                    17

<210> 1268
<211> 17
<212> DNA
<213> Artificial Sequence

<220>
<223> Description of Artificial Sequence:capture

<400> 1268
tggaccgcgg cggacac                                                    17

<210> 1269
<211> 17
<212> DNA
<213> Artificial Sequence

<220>

<223> Description of Artificial Sequence:capture

<400> 1269
ccgccgcgaa cacggcg                                                    17

<210> 1270
<211> 17
<212> DNA
<213> Artificial Sequence

<220>
<223> Description of Artificial Sequence:capture

<400> 1270
cgcggacaag gcggctc                                                    17

<210> 1271
<211> 17
<212> DNA
<213> Artificial Sequence

<220>
<223> Description of Artificial Sequence:capture

<400> 1271
gcggacacag cggctca                                                    17

<210> 1272
<211> 17
<212> DNA
<213> Artificial Sequence

<220>
<223> Description of Artificial Sequence:capture

<400> 1272
gcggacaccg cggctca                                                    17

<210> 1273
<211> 17
<212> DNA
<213> Artificial Sequence

<220>
<223> Description of Artificial Sequence:capture

<400> 1273
tccgcgggta taaccag                                                    17

<210> 1274
<211> 17
<212> DNA
<213> Artificial Sequence

<220>
<223> Description of Artificial Sequence:capture

<400> 1274

gcgggcataa ccagtta 17

<210> 1275
<211> 17
<212> DNA
<213> Artificial Sequence

<220>
<223> Description of Artificial Sequence:capture

<400> 1275
tggacagcgg cggacac 17

<210> 1276
<211> 17
<212> DNA
<213> Artificial Sequence

<220>
<223> Description of Artificial Sequence:capture

<400> 1276
tccgcgggta taaccgg 17

<210> 1277
<211> 17
<212> DNA
<213> Artificial Sequence

<220>
<223> Description of Artificial Sequence:capture

<400> 1277
cgcgggtata accagtt 17

<210> 1278
<211> 17
<212> DNA
<213> Artificial Sequence

<220>
<223> Description of Artificial Sequence:capture

<400> 1278
gcgggcataa ccagttc 17

<210> 1279
<211> 17
<212> DNA
<213> Artificial Sequence

<220>
<223> Description of Artificial Sequence:capture

<400> 1279
gcgggcataa ccaggac 17

<210> 1280

<211> 17
<212> DNA
<213> Artificial Sequence

<220>
<223> Description of Artificial Sequence:capture

<400> 1280
gcgggtataa ccggtta                                                    17

<210> 1281
<211> 17
<212> DNA
<213> Artificial Sequence

<220>
<223> Description of Artificial Sequence:capture

<400> 1281
gcggttataa ccagtac                                                    17

<210> 1282
<211> 17
<212> DNA
<213> Artificial Sequence

<220>
<223> Description of Artificial Sequence:capture

<400> 1282
ccgggcataa ccagtac                                                    17

<210> 1283
<211> 17
<212> DNA
<213> Artificial Sequence

<220>
<223> Description of Artificial Sequence:capture

<400> 1283
gcgggtataa ccagttc                                                    17

<210> 1284
<211> 17
<212> DNA
<213> Artificial Sequence

<220>
<223> Description of Artificial Sequence:capture

<400> 1284
gatcacccgg cgcaagt                                                    17

<210> 1285
<211> 17
<212> DNA
<213> Artificial Sequence

<220>
<223> Description of Artificial Sequence:capture

<400> 1285
ggcccgtgtg gcggagc                                                    17

<210> 1286
<211> 17
<212> DNA
<213> Artificial Sequence

<220>
<223> Description of Artificial Sequence:capture

<400> 1286
ggcccgtgcg gcggagc                                                    17

<210> 1287
<211> 17
<212> DNA
<213> Artificial Sequence

<220>
<223> Description of Artificial Sequence:capture

<400> 1287
agctgagaac ctacctg                                                    17

<210> 1288
<211> 17
<212> DNA
<213> Artificial Sequence

<220>
<223> Description of Artificial Sequence:capture

<400> 1288
ctgagagctt acctgga                                                    17

<210> 1289
<211> 17
<212> DNA
<213> Artificial Sequence

<220>
<223> Description of Artificial Sequence:capture

<400> 1289
tgcgtggatt ggctccg                                                    17

<210> 1290
<211> 17
<212> DNA
<213> Artificial Sequence

<220>
<223> Description of Artificial Sequence:capture

<400> 1290
tgcgtggacg ggctccg                                                    17

<210> 1291
<211> 17
<212> DNA
<213> Artificial Sequence

<220>
<223> Description of Artificial Sequence:capture

<400> 1291
gcgtggaggg gctccgc                                                    17

<210> 1292
<211> 17
<212> DNA
<213> Artificial Sequence

<220>
<223> Description of Artificial Sequence:capture

<400> 1292
cgtggagtcg ctccgca                                                    17

<210> 1293
<211> 17
<212> DNA
<213> Artificial Sequence

<220>
<223> Description of Artificial Sequence:capture

<400> 1293
tccgcagaca cctggag                                                    17

<210> 1294
<211> 17
<212> DNA
<213> Artificial Sequence

<220>
<223> Description of Artificial Sequence:capture

<400> 1294
agcggagaac ctacctg                                                    17

<210> 1295
<211> 17
<212> DNA
<213> Artificial Sequencc

<220>
<223> Description of Artificial Sequence:capture

<400> 1295
ggaaggagac gctggag                                                    17

<210> 1296
<211> 17
<212> DNA
<213> Artificial Sequence

<220>
<223> Description of Artificial Sequence:capture

<400> 1296
ggaaggacac gctggag                                                    17

<210> 1297
<211> 16
<212> DNA
<213> Artificial Sequence

<220>
<223> Description of Artificial Sequence:capture

<400> 1297
agacgctgca gcgcgc                                                     16

<210> 1298
<211> 17
<212> DNA
<213> Artificial Sequence

<220>
<223> Description of Artificial Sequence:capture

<400> 1298
ggaaggagac gctgcag                                                    17

## Description

### Technical Field

The present invention relates to a typing kit and typing method for determining HLA genotypes. HLA typing is carried out to judge possibility of transplantation between immunogenetically different individuals. Specifically, its purpose is to provide genetic information to determine transplantation compatibility for transplantation of an organ or tissue such as bone marrow, kidney, liver, pancreas, islets of Langerhans or cornea. The kit of the present invention can also be applied to susceptibility test for a specific disease that is caused by a hereditary factor, or individual identification.

The present invention further relates to PCR primers used for the aforementioned kit and method and a method for producing the same.

### Background Art

### 1. Human leukocyte antigen (HLA) types and importance of histocompatibility

In autogenous transplantation or transplantation between immunogenetically homologous individuals such as identical twins of a tissue or cells, a graft is taken without inducing rejection reactions. On the other hand, in transplantation between immunogenetically different individuals such as other family members or unrelated individuals (allogenic transplantation) or transplantation between different species such as human being and other primates (interspecific transplantation), transplantation immunity is established and a rejection reaction is induced against a graft. There are antigens inducing particularly intense transplantation immunity as targets in this reaction, which are called major histocompatibility antigens. These antigens are controlled by a group of genes called MHC (Major Histcompatibility Complex). MHC is classified into classes I, II and III. class I and class II genes code for HLA (Human Leukocytic Antigen), which are transplantation antigens. HLA antigens are proteins responsible for discriminating self and non-self in the immune mechanism. Since a large number of alleles exist on their gene loci, the genes coding for these antigens are known to be highly polymorphic.

Determination of genotypes (typing) of HLA provides information for determination of the possibility of transplantation between immunogenetically different individuals. Specifically, this is employed for transplantation of an organ or tissue such as bone marrow, kidney, liver, pancreas, islets of Langerhans or cornea. The HLA genotypes are considered to be associated with certain refractory diseases and can also be utilized as supplementary means for diagnosing chronic rheumatoid arthritis, IDDM, insulin autoimmune syndrome and so forth. Besides, HLA typing is applied in a paternity test or individual identification because of the genetic polymorphism of HLA antigens.

### 2. Genes involved in HLA typing and polymorphism

Human MHC is an HLA gene complex located on the short arm of the 6th chromosome. HLA antigens encoded by these genes include class I antigens controlled by HLA-A, -B, -C loci and class II antigens controlled by the HLA-D region (DR, DQ and DP).

class I antigens have a double-stranded structure composed of L-chain called â2 microglobulin having a molecular weight of 12 kDa and H-chain having a molecular weight of 45 kDa. These antigens are involved in cellular immunity mediated by T cells, determine antigen specificity, and become target antigens in transplantation immunity. class II antigens have a double-stranded structure of two chains having molecular weights of 34 kDa and 29 kDa, respectively, determine antigen recognition and are involved in antigen presentation in humoral immunity. DP, DQ and DR in the HLA-D region are further subtyped according to polymorphism based on the amino acid or nucleotide sequence. DR is classified into about 20 subtypes, DQ is classified into about 10 subtypes, and DP is classified into a few subtypes. This large number of subtypes is attributed to the facts that the HLA antigens are composed of the á-chain and the â-chain each having a different molecular weight and that only the â-chain serves as an alloantigenic determinant except for the DQ antigens. Moreover, the subtypes are further subdivided based on the amino acid sequences or the corresponding nucleotide sequences of the antigenic determinants.

### 3. Means for HLA typing

Serological test utilizing antiserum reactions and cytological methods utilizing blast transformation of lymphocytes have been generally used. However, not only time and labor are required for examination and operation is complicated, but also accuracy of obtainable results are not so high. Therefore, DNA-based typing methods utilizing PCR are being developed to overcome these drawbacks and put into practical use. Examples thereof include the PCR-SSP method (2), PCR-RFLP method (3), PCR-SSOP method (4), PCR-SSCP method (5) and so forth.

In the PCR-SSP method, a gene sequence is confirmed by amplifying the hypervariable region of target HLA antigen to determine the HLA antigen type. In this method, a large number of specific primers must be prepared to enable high accuracy typing and an enormous number of times of PCR also must be performed. Thus, this method is not necessarily practical.

In the PCR-RFLP (PCR-restriction fragment length polymorphism) method, a nucleotide sequence is amplified by PCR so as to contain a region involved in HLA antigen typing and the obtained amplification product is digested with restriction enzymes. The nucleotide sequence of the amplification product is digested by using restriction enzymes cleaving different sites depending on the HLA type and separated by electrophoresis. Then, the type is determined based on the migration pattern. This method is characterized in that results can be rapidly obtained. However, the cleavage sites of the restriction enzymes may not necessarily exist in the sequence. Further, the amplification product must be digested with multiple kinds of restriction enzymes for one typing. Thus, this method is not practical to process a large amount of specimen.

In the PCR-SSOP (PCR-sequence specific oligonucleotide probe) method, a nylon membrane on which DNA amplified by primers specific to HLA gene is immobilized is prepared and respective HLA type specific oligonucleotide probes are hybridized for typing. In a modified method of PCR-SSOP, a membrane on which respective HLA type specific oligonucleotides are immobilized is prepared and DNA amplified by using HLA gene specific primers are hybridized. In the method of immobilizing amplified DNA, a membrane must be prepared for a probe of each type for hybridization and therefore much labor is required for multiple specimen typing. Since the substrate on which amplified DNA or oligonucleotides are immobilized is a membrane, a membrane having a large area must be prepared to perform high accuracy typing with one membrane. Therefore, the typing pattern may be complicated.

In the PCR-SSCP (PCR-single strand conformation polymorphism) method, DNA amplified by PCR is denatured to obtain single strands and changes in the high-order structure due to the difference in nucleotide sequences are detected by electrophoresis. In this method, all DNA of known types must be electrophoresed on the same gel. Thus, practical multiple specimen processing is almost impossible.

### 4. Importance of high accuracy HLA typing

To achieve ideal transplantation, it is important that HLA types of donor and recipient are compatible with high accuracy. Therefore, a tissue or individual information typed in high accuracy must be clarified in advance. In this respect, HLA antigens of the donor and the recipient must be compared with all the types currently discovered for determination before transplantation. Even if no donor having completely compatible HLA types is found, a high success rate can be expected by performing transplantation between individuals having similar types of which high accuracy HLA types are determined. Further, it is also expected that load of recipient can also be relieved in immune responses such as a rejection reaction occurring after transplantation.

When high accuracy typing is carried out in personal identification, one completely compatible individual is found out of tens or hundreds of thousands of individuals, but such an individual can be identified in high accuracy. Furthermore, if high accuracy typing is performed for each gene locus of HLA antigens, the accuracy of the personal identification can be further improved.

In a medical scene requiring HLA type information or a situation requiring a personal identification, provision of highly reliable HLA type information is being required. It is important that typing of a large number of specimens can be easily and conveniently performed by HLA typing methods therefor. Currently, various methods are being developed and improved, but there remain many drawbacks that operation is complicated, multiple specimen processing is not possible and so forth.

### Disclosure of the Invention

To solve these problems, an object of the present invention is to provide a kit and method that are suitable for processing of a large number of specimens and enable a large number of high accuracy typing tests by one test for one specimen.

The present invention provides a typing kit for determining HLA genotype of a test specimen by hybridization between a nucleic acid sequence derived from the test specimen and oligonucleotides, which comprises a substrate on which the oligonucleotides are immobilized through covalent bonds, wherein the oligonucleotides are of 10-24 nucleotide length and are derived from sequences of a group of genes belonging to HLA class I or class II antigen on a human genome and each of the oligonucleotides includes polymorphism of each gene as alloantigen in the sequence.

The present invention also provides a method for determining HLA genotype of a test specimen, which comprises allowing hybridization of the oligonucleotides on the substrate of the aforementioned typing kit with a nucleic acid sequence derived from the specimen and detecting occurrence of hybridization of the oligonucleotides and the nucleic acid sequence derived from the specimen.

The present invention also provides the aforementioned typing kit, wherein class I antigen is an antigen controlled by a gene locus coding for any of HLA-A, HLA-B, HLA-C, HLA-D, HLA-E, HLA-F and HLA-G or a null gene and class II antigen is an antigen controlled by a gene locus coding for any of HLA-DQ, HLA-DR or HLA-DP or a pseudogene;

The aforementioned typing kit, wherein HLA-DQ is an antigen derived from any of DQA1, DQA2, DQB1 and DQB2 gene loci or a pseudogene;
the aforementioned typing kit, wherein HLA-DR is an antigen derived from any of DRA, DRB1, DRB3, DRB4 and DRB5 gene loci or a pseudogene; and
the aforementioned typing kit, wherein HLA-DP is an antigen derived from any of DPA1, DPA2, DPB1 and DPB2 gene loci or a pseudogene.

The present invention also provides an oligonucleotide, which comprises a nucleotide sequence based on any of the aforementioned oligonucleotides of 10-24 nucleotide length comprising a nucleotide sequence specific to each type of HLA gene sequences on the human genome and obtained by extending or shortening one of the oligonucleotides at 5' or 3' end or at both ends, and in which the sequence specific to each HLA type is not substituted.

The present invention also provides the aforementioned typing kit, wherein at least one of the aforementioned oligonucleotides is replaced with an oligonucleotide of 8-24 nucleotide length obtained by extending or shortening any of the nucleic acid sequences of SEQ ID NOS: 1-397, 456-503, 507-589, 594-898, 908-1072 or 1080-1298 for a gene sequence on the genome at 5' or 3' end or at both ends without eliminating or changing nucleotides associated with the gene polymorphism to have optimized binding affinity for the aforementioned hybridization.

The present invention further provides the aforementioned typing kit, wherein at least one of the oligonucleotides is an oligonucleotide whose binding affinity for the hybridization is reduced by replacing an arbitrary nucleotide not involved in the gene polymorphism with a spacer compound, for example, a spacer compound having a nucleic acid frame that does not form a hydrogen bond with any nucleotide.

Furthermore, peptide nucleic acids (PNA) can be used instead of these oligonucleotides.

The present invention further provides a method for determining HLA genotype of a specimen, which comprises a step of carrying out low accuracy typing of HLA genotype of the specimen by performing first PCR amplification using primers for low accuracy genotyping and a nucleic acid sequence derived from the specimen as a template, allowing hybridization of the amplification product with oligonucleotides contained in the aforementioned typing kit, and detecting occurrence of hybridization of the nucleic acid sequence derived from the specimen and each of the oligonucleotides; and
a step of carrying out high accuracy HLA genotyping of the specimen by performing second PCR amplification based on the above determination result using primers appropriately selected from primers for high accuracy genotyping and a nucleic acid sequence derived from the specimen as a template, allowing hybridization of the amplification product with the oligonucleotides contained in the aforementioned typing kit, and detecting occurrence of hybridization of the nucleic acid sequence derived from the specimen and each of the oligonucleotides.

The present invention provides primers for low accuracy typing used for preparation of a probe for low accuracy typing, which consist of any oligonucleotide pair or an arbitrary combination of oligonucleotide pairs selected from a pair of oligonucleotides having nucleotide sequences of SEQ ID NOS: 398 and 400 for amplifying DQB1;
a pair of oligonucleotides having nucleotide sequences of SEQ ID NOS: 399 and 400 for amplifying DQB1;
a pair of oligonucleotides having nucleotide sequences of SEQ ID NOS: 401 and 403 for amplifying DRB1; and
a pair of oligonucleotides having nucleotide sequences of SEQ ID NOS: 402 and 403 for amplifying DRB1.

The present invention further provides primers for high accuracy typing used for preparation of a probe for high accuracy typing based on result of low accuracy typing, which consist of any oligonucleotides pair or an arbitrary combination of oligonucleotides pairs selected from:
a pair of oligonucleotides having nucleotide sequences of SEQ ID NOS: 404 and 405 for amplifying DQB1;
a pair of oligonucleotides having nucleotide sequences of SEQ ID NOS: 404 and 406 for amplifying DQB1;
a pair of oligonucleotides having nucleotide sequences of SEQ ID NOS: 407 and 409 for amplifying DQB1;
a pair of oligonucleotides having nucleotide sequences of SEQ ID NOS: 408 and 409 for amplifying DQB1;
a pair of oligonucleotides having nucleotide sequences of SEQ ID NOS: 410 and 412 for amplifying DQB1;
a pair of oligonucleotides having nucleotide sequences of SEQ ID NOS: 411 and 412 for amplifying DQB1;
a pair of oligonucleotides having nucleotide sequences of SEQ ID NOS: 413 and 417 for amplifying DRB1;
a pair of oligonucleotides having nucleotide sequences of SEQ ID NOS: 414 and 417 for amplifying DRB1;
a pair of oligonucleotides having nucleotide sequences of SEQ ID NOS: 415 and 417 for amplifying DRB1; and
a pair of oligonucleotides having nucleotide sequences of SEQ ID NOS: 416 and 417 for amplifying DRB1.

The present invention further provides primers for high accuracy typing used for preparation of a probe for high accuracy typing, which consist of any oligonucleotide pair or an arbitrary combination of oligonucleotides pair selected from:
a pair of oligonucleotides having nucleotide sequences of SEQ ID NOS: 418 and 420 for amplifying DQA1;
a pair of oligonucleotides having nucleotide sequences of SEQ ID NOS: 419 and 420 for amplifying DQA1;
a pair of oligonucleotides having nucleotide sequences of SEQ ID NOS: 421 and 422 for amplifying DQB1;
a pair of oligonucleotides having nucleotide sequences of SEQ ID NOS: 423 and 424 for amplifying DRA1;
a pair of oligonucleotides having nucleotide sequences of SEQ ID NOS: 425 and 428 for amplifying DRB1;
a pair of oligonucleotides having nucleotide sequences of SEQ ID NOS: 426 and 428 for amplifying DRB1;
a pair of oligonucleotides having nucleotide sequences of SEQ ID NOS: 427 and 428 for amplifying DRB1;
a pair of oligonucleotides having nucleotide sequences of SEQ ID NOS: 429 and 431 for amplifying DRB3;
a pair of oligonucleotides having nucleotide sequences of SEQ ID NOS: 430 and 431 for amplifying DRB3;
a pair of oligonucleotides having nucleotide sequences of SEQ ID NOS: 432 and 433 for amplifying DRB4;
a pair of oligonucleotides having nucleotide sequences of SEQ ID NOS: 434 and 435 for amplifying DRB4;
a pair of oligonucleotides having nucleotide sequences of SEQ ID NOS: 436 and 437 for amplifying DRB5;
a pair of oligonucleotides having nucleotide sequences of SEQ ID NOS: 438 and 439 for amplifying DRB6;
a pair of oligonucleotides having nucleotide sequences of SEQ ID NOS: 438 and 440 for amplifying DRB6;
a pair of oligonucleotides having nucleotide sequences of SEQ ID NOS: 441 and 442 for amplifying DRB7;
a pair of oligonucleotides having nucleotide sequences of SEQ ID NOS: 443 and 444 for amplifying DPA1;
a pair of oligonucleotides having nucleotide sequences of SEQ ID NOS: 445 and 446 for amplifying DPB1; and
a pair of oligonucleotides having nucleotide sequences of SEQ ID NOS: 445 and 447 for amplifying DPB1.

The term "class" used for HLA classes I and II in the present specification means classification based on the roles played in an organism. That is, the present specification is also according to this classification. class I antigen is a generic designation of HLA-A, HLA-B, HLA-C, HLA-D, HLA-E, HLA-F and HLA-G antigens. class II antigen is a generic designation of HLA-DQ, HLA-DR and HLA-DP antigens. Alloantigens are antigens characterizing individuals that belong to the same animal species but have genetic differences. Genetic differences mean differences in nucleotide sequences. Further, each of alloantigens classified based on genetic differences is designated as an alloantigen type.

In the present specification, the term "polymorphism" means a characteristic of having the same function but a different primary structure in a protein or DNA coding therefor.

In the present specification, the term "gene locus" means a position occupied by each HLA gene having polymorphism on a chromosome. As for gene loci of DR, DQ and DP antigens, gene loci of the DR antigens include DRA, DRB1, DRB3, DRB4 and DRB5, gene loci of the DQ antigens include DQA1, DQA2, DQB1 and DQB2, and gene loci of the DP antigens include DPA1, DPA2, DPB1 and DPB2.

In the present specification, the term "antigen derived from a gene locus" means an antigen translated from a gene present on the gene locus.

In the present specification, the term "nucleotides involved in gene polymorphism" means nucleotides having different primary sequences in genes coding for each alloantigen.

In the present specification, the term "test specimen" means a sample to be typed. In the present specification, it means chromosome DNA or RNA extracted from a tissue or somatic cell or a reactant enzymatically produced by using any of these as a template.

In the present specification, the term "homopolymer of thymidine residue" means a polymer obtained by repeatedly synthesizing deoxythymidine at 5' or 3' end of nucleotide sequence portion involved in typing. This synthesis of oligonucleotide can be performed by a commercially available DNA synthesizer.

Hereafter, a constitution of the kit of the present invention and a method of manufacturing the same, HLA typing method using the kit of the present invention, oligonucleotides for use in these kit and methods, a nucleic acid target derived from a test specimen human genome, which is a sample for use in the HLA typing using the kit of the present invention, and a method of manufacturing the same and primers for preparing the nucleic acid target will be explained.

### <1> Synthetic oligonucleotide for use in the present invention

The synthetic oligonucleotides for use in the present invention are derived from human genomic genes involved in HLA typing and synthesized based on nucleotide sequences obtained from these genes. Hereafter, these oligonucleotides are also referred to as capture oligos.

Capture oligos are designed so that they should contain a type-specific nucleotide sequence region present in HLA gene of each type. The essential characteristic of the present invention is that the HLA typing is performed by identifying whether a test human genome has these regions.

Typing of a type-specific nucleotide sequence in genes involved in HLA typing can be carried out by allowing hybridization of each type-specific capture oligo with a nucleic acid fragment target (also referred to as "nucleic acid target") having a part of nucleotide sequence of the genes involved in HLA typing of human chromosome DNA and determining with which capture oligo the fragment hybridizes.

The capture oligos can be prepared as synthetic oligonucleotides having a nucleotide sequence consisting of 10-24 nucleotides containing a nucleotide sequence specific to each type in a nucleotide sequence of gene involved in HLA typing. It is generally preferred that the capture oligos are designed so that the type-specific nucleotide sequence should be positioned in the center portions of the capture oligos. If the capture oligos are too short, it becomes difficult to detect hybridization. On the other hand, if the capture oligo is too long, inhibition of hybridization by a type-specific sequence does not occur. Therefore, the range of 10-24 nucleotides is preferred. However, this optimization of the capture oligo length primarily depends on characteristics of the sequence (content of specific nucleotide and repetition of the same nucleotide), and it has been confirmed by experiments according to the present invention that even a short chain can be used so long as it shows good binding property. In the process of hybrid formation of a capture oligo and a target, if there is an obstruction in the secondary structure negatively affecting the hybridization, a spacer or a nucleotide forming no hydrogen bond with any nucleotide can be introduced into the oligonucleotide sequence to obviate the obstruction. Further, the obstruction in the secondary structures of the target and capture oligo can also be obviated by extending or shortening the gene sequence on genome corresponding to the capture oligo at 5' or 3' end or at both ends of the capture oligo so that a type-specific sequence portion should be included.

DNA is usually used as the capture oligos, but a peptide nucleic acid (PNA) may also be used. The hybrids of a nucleic acid derived from a test human genome with a peptide nucleic acid have a higher Tm (melting temperature) compared with those of oligonucleotides and therefore they are expected to provide stable hybridization signals. The peptide nucleic acids can be readily synthesized by using a common peptide synthesizer.

Examples of capture oligo nucleotide sequences are shown as SEQ ID NOS: 1-397, 456-503, 507-589, 594-898, 908-1078 and 1080-1298. These capture oligos are designed based on nucleotide sequence data obtained from publications and the home page of the Japanese Society for Histocompatibility and Immunogenetics (http://square.umin.ac.jp/JSHI/frame.html) and cover the whole HLA types.

Typing of a specific HLA type can be carried out by appropriately selecting a capture oligo from them and using it in a test.

The capture oligo length is, in general, in a range of 14-22 nucleotides. From the viewpoint of hybridization, 16-17 nucleotides is a typical size. Besides the nucleotide sequences of SEQ ID NOS: 1-397, oligonucleotides having a nucleotide sequence obtained by extending or shortening any of the HLA type gene sequences at 5' or 3' end or at both ends of the nucleotide sequences can be used as a capture oligo.

The nucleotide sequences of SEQ ID NOS: 1-397, 456-503, 507-589, 594-898, 908-1078 and 1080-1298 may also be extended at either 5' or 3' end and shortened at the other end, but the length of a capture oligo is in a range of 10-24 nucleotides in any case.

In the present invention, the designed capture oligos well reflect the results of studies and researches before the present application. However, when additional information about nucleotide sequnences of novel HLA types is obtained thereafter, novel capture oligos will be designed based on the method described in the present specification and they will fall within the claimed scope of the present application.

Synthesis of oligonucleotides, preparation of chromosome DNA, hybridization and PCR can be performed by usual methods known to those skilled in the art (see Maniatis, T. et al., "Molecular Cloning, A Laboratory Manual, second edition", Cold Spring Harbor Laboratory Press (1989)). The oligonucleotides can also be synthesized by using a commercially available DNA synthesizer.

### <2> Preparation of substrate for use in present invention

Material of a substrate on which oligonucleotides are immobilized is not particularly limited so long as the oligonucleotides can be stably immobilized. For example, there can be mentioned glass, synthetic resins such as polycarbonates and plastics. The form of the substrate is not particularly limited, but there can be mentioned a form of plate or film. A uniform and flat surface is suitable as a surface of the substrate.

The oligonucleotides can be immobilized on a substrate by using a method employed in usual hybridization such as physical absorption, electric bond and molecular covalent bond. In the examples of the present specification, substrates coated with carbodiimide groups or isocyanate groups on the surface thereof (Japanese Patent Laid-Open (Kokai) No. 08-023975) were used. To prepare the substrates coated with a material having carbodiimide groups or isocyanate groups on the surface thereof, employed was a method in which the substrate surfaces were coated with a polymer compound containing carbodiimide groups or isocyanate groups and irradiated with ultraviolet ray so that oligonucleotides can be covalently bonded and immobilized thereon. This is because marked improvement of immobilization efficiency was successfully attained by irradiating a substrate having carbodiimide groups or isocyanate groups on the surface thereof with ultraviolet ray. As a linker for binding carbodiimide group or isocyanate group and an oligonucleotide, a compound having an amino group or imino group highly reactive to the carbodiimide group or isocyanate group is used. In case of the imino group, carbodiimide group or isocyanate group can be bound by polymerizing thymine at either end of the capture oligo.

When oligonucleotides are spotted, if the amount of spotted oligonucleotides is too small, sufficient reactivity between the oligonucleotides and the nucleic acid target may not be ensured and therefore typing may become difficult. Further, high-density spotting invites technical problems as well as high cost. In addition, a more precise and expensive detecting device (typically, a scanner) is required for determination of occurrence of hybridization utilizing fluorescent labeling of the nucleic acid target, chemical color development or the like. Therefore, the oligonucleotides are preferably immobilized on the substrate surface in a diameter of 10-1,000 µm. The oligonucleotides can be immobilized by spotting an oligonucleotide solution on a substrate by using, for example, a spotting machine. It is usually preferable that the oligonucleotide solution is spotted substantially in a circular shape.

Each oligonucleotide is spotted at a plurality of sites on one single substrate, and the spots are preferably arranged in a grid pattern. When the spot size is 1000 µm in diameter, the total number of the spots is preferably 1600 or less per cm², and when such spots are spotted in a square shape, the square dimension is preferably 40 x 40 or less. When the spot size is 10 µm in diameter, the total number of spots is preferably 400 or less per cm², and when such spots are spotted in a square shape, the square dimension is preferably 20 x 20 or less. If the longitudinal and transverse sizes are different, the numbers along longitudinal and transverse directions can be adjusted depending on the shape.

### <3> Arrangement of oligonucleotides on substrate

To facilitate typing of each HLA type, oligonucleotides are preferably arranged on a substrate so that oligonucleotides to be used for determining each HLA type should be placed in one partition, in a line, or the like. Oligonucleotides suitable for low accuracy genotyping and those suitable for high accuracy genotyping may be immobilized on the same substrate to simultaneously carry out low accuracy genotyping and high accuracy genotyping. To perform low accuracy genotyping and high accuracy genotyping stepwise, a substrate on which oligonucleotides suitable for low accuracy genotyping are immobilized and a substrate on which oligonucleotides suitable for high accuracy genotyping are immobilized may be separately prepared.

Alternatively, the typing may be performed by preparing alignment of gene sequence of each HLA, setting a nucleotide sequence in which at least two nucleotide polymorphisms or sequence polymorphisms involving the HLA typing are observed in a nucleotide sequence consisting of 1-10 nucleotides as a patchwork segment, and finding the patchworks in gene sequences of all of the HLA genes and, performing typing by combination of the patchworks. To perform advanced typing, the typing may be performed by setting a nucleotide sequence in which at least one nucleotide polymorphism or sequence polymorphism are observed in a nucleotide sequence consisting of 1-10 nucleotides based on the alignment of the nucleotide sequences of HLA genes as a satellite segment, and finding the satellites in gene sequences of all of the HLA genes and, performing typing by combination of the satellites, whereby a type which cannot be discriminated by the patchworks may be discriminated. Typing using patchworks and satellites may make it possible to judge whether each of HLA typing of the test specimen is homozygote or heterozygote and determine the types of HLA simultaneously. The oligonucleotides may be prepared to be allocated so that the HLA types are determined by developing progressively the patchoworks and satellites in a test specimen.

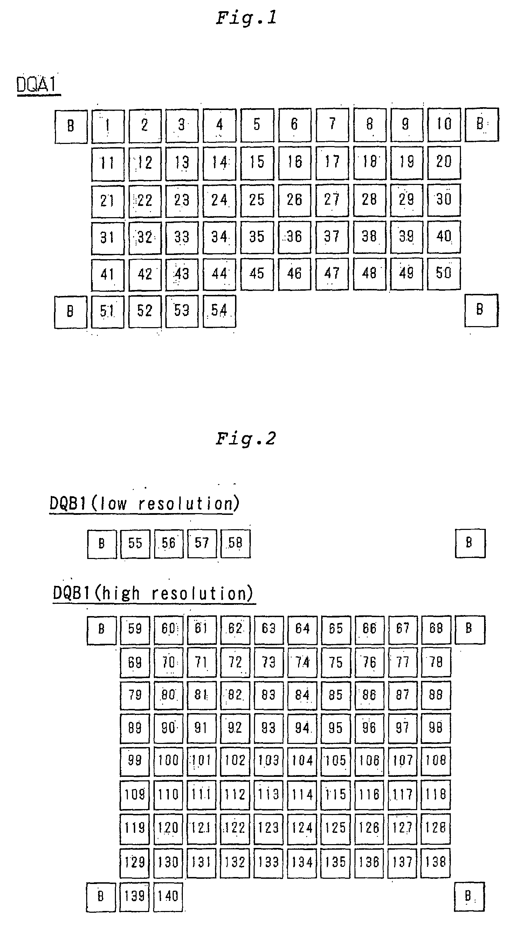

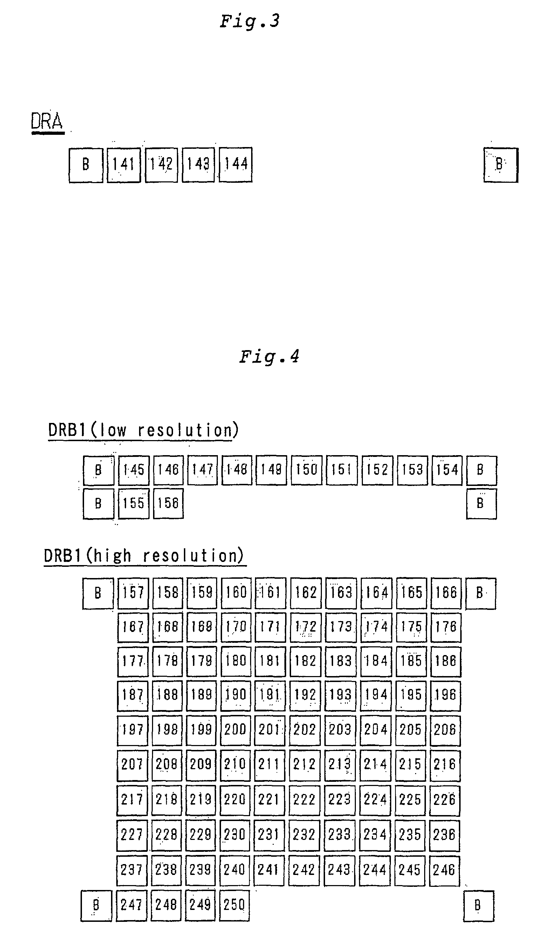

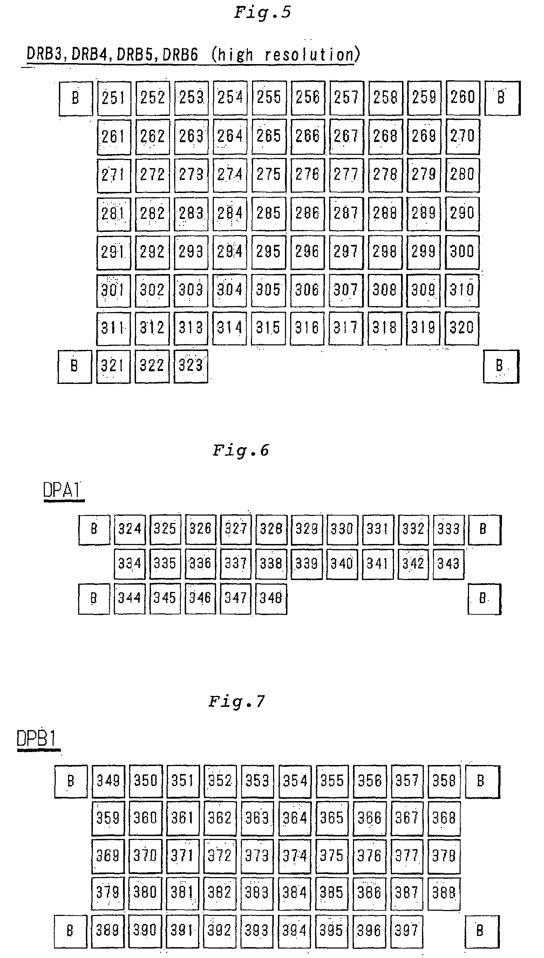

Spots on the substrate are usually arranged within an area of 1 cm². Figs. 1-7 and 11-17 show examples of preferred positions of oligonucleotides immobilized on the substrate. One square in the figure represents a spot of each oligonucleotide. The indicated numbers represent sequence numbers of the oligonucleotides.

### <4> Preparation of nucleic acid from test sample and preparation of nucleic acid probe

Nucleic acid can be prepared from a test sample by the same way as a usual method of preparing nucleic acids from animal cells. For example, DNA can be prepared by a method described in Maniatis, T. et al., "Molecular Cloning, A Laboratory Manual, second edition", Cold Spring Harbor Laboratory Press (1989). DNA can also be extracted from cells obtained by culture in a similar manner. This method is a standard test method, but there are many alternative methods and any of these methods may be employed.

A nucleic acid target for use in HLA typing is prepared by using the obtained DNA. The nucleic acid target can be prepared by amplifying a nucleic acid by using primers designed so as to correspond to a nucleotide sequence of capture oligo. While DNA is usually used as the nucleic acid target, RNA may also be used. As a nucleic acid amplification method, there can be mentioned, for example, a method of amplifying the nucleic acid as DNA by PCR (polymerize chain reaction) or as RNA by in vitro transcription.

Primers used for PCR are designed so that a nucleic acid target should include a complementary nucleotide sequence of a capture oligo except for a sequence region specific to each HLA type. A nucleic acid target may be longer or shorter than a capture oligo, so long as it is capable of hybridization. To increase specificity of amplification reactions, initial amplification may be carried out by using preliminary primers for amplifying a region larger than the target nucleic acid probe and then nucleic acid amplification may be carried out by using the amplified DNA as a template and primers for obtaining the target nucleic acid probe.

There may be a plurality of type-specific nucleotide sequence regions involved in typing depending on HLA types. In such a case, nucleic acid targets corresponding to each specific region can be prepared.

Furthermore, typing of DQB and DRB is carried out by selecting primers for high accuracy typing based on results obtained by using a nucleic acid sequence amplified by primers for low accuracy typing and then carrying out high accuracy typing, or allowing hybridization of nucleic acid sequences amplified by using all of the low accuracy primers for DQB and DRB and nucleic acid sequences amplified by using all of the high accuracy primers for DQB and DRB in separate blocks on which different captures are immobilized and determining low accuracy or high accuracy type from the obtained respective results. For typing of DQA, DRA, DPA and DPB, high accuracy typing is performed by using nucleic acid sequences amplified by using primers for high accuracy typing.

If a primer for use in final nucleic acid amplification is labeled in advance, a labeled nucleic acid target can be obtained. The nucleic acid target may be labeled during or after nucleic acid amplification. As the label, those labeling substances similar to those for a probe for use in usual hybridization, such as fluorescent substances or haptens, can be used. Specifically, as the fluorescent substances, there can be mentioned, for example, fluoresceine (FITC), rhodamine, phycoerythrin (PE), Texas Red, cyanine fluorescent dyes and so forth. As the haptens, there can be mentioned biotin, digoxigenin (Dig), dinitrophenyl (DNP) and so forth.

Primers for preparing a nucleic acid target can be included in an HLA typing kit together with a substrate on which oligonucleotides are immobilized.

### <5> Hybridization of oligonucleotides on substrate and nucleic acid target

Hybridization can be performed in the same way as usual nucleic acid hybridization. A specific procedure will be exemplified below.

A nucleic acid target is added to a fusion solution comprising a salt solution such as standard saline citrate (SSC), a blocking solution such as sodium dodecyl sulfate (SDS) or bovine serum albumin (BSA) and an additive for promoting fusion reactions. When the target is double-stranded, denaturation is performed by heating or the like. After a few µL of a nucleic acid target solution is added onto a substrate, heat treatment is performed for a few hours (usually at 37-70°C) so that a hybrid should be formed between an oligonucleotide immobilized on the substrate and the nucleic acid target.

5 x SSC or 3 M tetramethylammonium chloride is added onto the substrate and heated (usually at 37-50°C). Then, nonspecific nucleic acids that do not form hybrids are removed from the substrate and only specific hybrids are selectively maintained on the substrate.

To detect a hybrid, a fluorescent substance or hapten introduced into the nucleic acid target is used. When a hapten is used, a solution containing a conjugate of a protein recognizing the hapten or a protein that binds to the hapten and alkaline phosphatase, horseradish peroxidase or the like (enzyme conjugate) is added onto the substrate and allowed to react at room temperature for several tens of minutes. A nonspecific adsorption reaction of the enzyme conjugate and the substrate can be prevented by completely coating regions on the substrate other than the regions on which oligonucleotides are immobilized with a protein such as casein before this binding reaction of the hapten and the enzyme conjugate is performed. This treatment can be carried out by, after the oligonucleotides are immobilized, adding a solution of a protein such as casein onto the substrate and leaving it at room temperature for a several tens of minutes.

After the binding reaction of the enzyme conjugate and the hapten of the nucleic acid target is completed, the substrate is washed with an appropriate buffer containing a surfactant to remove enzyme conjugates that have not bound to the hapten. Thus, only enzyme conjugates bound to the hapten in the nucleic acid target should remain on the substrate.

To visualize the hybrid, a compound that becomes insoluble only in the presence of a hapten and an enzyme conjugate is added. The generation of such an insoluble compound is amplified by enzymatic reaction, and hence the hybrid is visualized. As the compound used for this purpose, when the enzyme in the enzyme conjugate is alkaline phosphatase, there can be used nitroblue tetrazolium chloride (NBT) and 5-bromo-4-chloro-3-indolylphosphate p-toluidine salt (BCIP). When the enzyme is horseradish peroxidase, 3,3',5,5'-tetramethylbenzidine (TMB) or the like can be used.

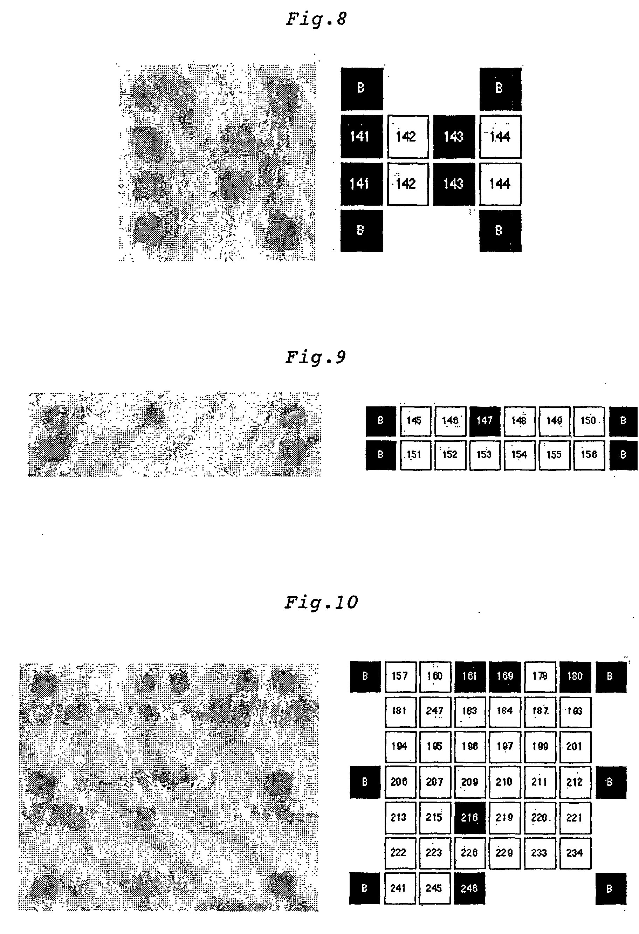

HLA typing based on the obtained results of hybridization is carried out by observing pigmentation or fluorescence at positions where capture oligos are immobilized. That is, the positions showing pigmentation or fluorescence indicate a corresponding gene. When all of oligonucleotides belonging to each type show color development, the type is determined. For example, in Figs. 8-10 referred to in the examples, hybridization signals were obtained only for oligonucleotides of SEQ ID NOS: 141 and 143 out of SEQ ID NOS: 141-144, and thus the test specimen is typed as DRA*0101. Correspondence of hybridization signals obtained at positions of capture oligos of SEQ ID NOS: 1-397 and HLA types are summarized in Tables 1-20.

**Table 1.**

| Correspondence of DQA1 types and high accuracy typing capture oligos | |
|---|---|
| Type | Corresponding capture oligos |
| DQA1*0101 | 1,4,7,10,12,14,16,18,22,24,27,31,35,39,42,47,51,53 |
| DQA1*01021 | 1,4,7,10,12,14,16,22,24,27,31,35,39,42,47,51,53 |
| DQA1*01022 | 1,4,7,10,12,14,16,20,22,24,2731,35,39,42,47,51,53 |
| DQA1*0103 | 2,4,7,10,15,20,22,26,27,31,35,39,42,47,51,53 |
| DQA1*0104 | 1,4,7,10,12,14,16,18,22,24,27,31,35,39,42,47,51,53 |
| DQA1*0105 | 1,4,7,10,12,14,16,18,22,24,27,31,35,39,42,47,51,53 |
| DQA1*0201 | 1,5,8,10,15,19,23,24,28,32,37,39,42,47,52,53 |
| DQA1*03011 | 2,5,9,13,17,19,23,24,29,33,37,41,43,48,49,52,53 |
| DQA1*0302 | 2,5,9,13,17,19,23,24,29,33,36,41,43,49,52,53 |
| DQA1*0303 | 1,5,9,12,17,19,23,24,29,33,36,41,43,49,52,53 |
| DQA1*0401 | 2,6,8,10,12,14,16,21,22,25,30,34,38,41,44,48,52,54 |
| DQA1*05011 | 3,6,8,10,12,14,16,21,22,25,30,34,38,41,45,50,52,53 |
| DQA1*05012 | 1,6,8,10,12,14,16,20,22,25,30,34,38,41,45,50,52,53 |
| DQA1*05013 | 3,6,8,10,12,14,16,20,22,25,30,34,38,41,45,50,52,53 |
| DQA1*0502 | 8,10,12,14,16,20,22,25,30,34,38,40,45,50,52,53 |
| DQA1*0503 | 3,6,8,10,12,14,16,20,22,25,30,34,38,41,45,50,52,53 |
| DQA1*0504 | 3,6,8,11,14,16,20,22,25,30,34,38,41,45,50,52,53 |
| DQA1*06011 | 2,6,8,10,15,21,22,25,30,34,38,41,46,48,52,53 |
| DQA1*06012 | 8,10,15,21,22,25,30,34,38,41,46,48,52,53 |

**Table 2.**

| Correspondence of DQB types and low accuracy typing capture oligos | |
|---|---|
| Type | Corresponding capture oligos |
| DQB1*0501 | 56 |
| DQB1*0502 | 56 |
| DQB1*05031 | 56 |
| DQB1*06011 | 55 |
| DQB1*06013 | 55 |
| DQB1*0602 | 55 |
| DQB1*0603 | 55 |
| DQB1*0604 | 56 |
| DQB1*06051 | 56 |
| DQB1*0609 | 56 |
| DQB1*0610 | 55 |
| DQB1*0611 | 55 |
| DQB1*0612 | 56 |
| DQB1*0614 | 55 |
| DQB1*0201 | 57, 58 |
| DQB1*0202 | 57, 58 |
| DQB1*0203 | 57, 58 |
| DQB1*03011 | 57 |
| DQB1*03012 | 57 |
| DQB1*0302 | 57 |
| DQB1*03032 | 57 |
| DQB1*0304 | 57 |
| DQB1*0305 | 57 |
| DQB1*0306 | 57 |
| DQB1*0307 | 57 |
| DQB1*0308 | 57 |
| DQB1*0401 | 57 |

**Table 3.**

| Correspondence of DQB types and high accuracy typing capture oligos | |
|---|---|
| Type | Corresponding capture oligos |
| DQB1*0501 | 62,65,68,70,74,79,82,86,89,92,97,99,103,114,116,118,126,131,137,138 |
| DQB1*0502 | 62,65,68,70,74,79,82,86,89,93,97,99,104,114,116,118,126,130,137,138 |
| DQB1*05031 | 62,65,68,70,74,79,82,86,94,97,99,106,114,116,118,126,130,137,138 |
| DQB1*05032 | 74,79,82,86,94,97,99,105,114,116 |
| DQB1*0504 | 65,68,70,74,80,83,86,89,93,97,99,104,114,117,119,126,131 |
| DQB1*06011 | 63,66,69,75,80,84,94,97,99,106,114,117,120,124,127,132,135,139 |
| DQB1*06012 | 66,68,70,75,80,84,94,98,99,106,114,117,120,124,127,132,135,139 |
| DQB1*06013 | 63,66,69,75,80,84,94,97,99,106,114,117,120,124,127,132,135,139 |
| DQB1*0602 | 64,67,68,70,76,80,87,89,95,97,99,105,114,116,121,125,127,132,135,139 |
| DQB1*0603 | 62,67,68,70,77,79,87,89,95,97,99,105,114,116,121,125,127,132,135,139 |
| DQB1*0604 | 62,67,68,70,77,79,87,89,93,97,99,103,114,116,122,125,127,132,134,138 |
| DQB1*06051 | 62,65,68,70,77,8087,89,93,97,99,103,114,116,122,125,127,132,134,138 |
| DQB1*06052 | 77,80,87,89,93,97,99107,114,116,122,125,127,132 |
| DQB1*0606 | 77,80,87,89,93,97,99,103,114,116,122,128,129,131 |
| DQB1*0607 | 67,68,70,77,79,87,89,95,97,99,105,114,116,122,125,127,132 |
| DQB1*0608 | 67,68,70,77,79,87,89,95,97,99,103,114,116,121,127,132 |
| DQB1*0609 | 62,67,68,7077,80,87,89,93,97,99,103,114,116,122,125,127,132,134,138 |
| DQB1*0610 | 64,67,68,70,76,80,87,89,95,97,99,104,114,116,121,125,127,132,135,139 |
| DQB1*0611 | 67,68,70,76,80,87,89,95,97,99, 105,114,116,121,125,127,132,135,139 |
| DQB1*0612 | 62,67,68,70,77,80,87,89,93,97,99,103,114,116,121,125,127,132,134,138 |
| DQB1*0613 | 64,67,68,70,76,80,87,89,95,97,99,103,114,116,121,125,127,132,135 |
| DQB1*0614 | 67,68,70,77,79,87,89,95,97,99, 105,114,116,121,125,127,132,135,139 |
| DQB1*0201 | 60,61,62,67,71,78,81,85,90,100,108,114,116,123,128,129,131,136,140 |
| DQB1*0202 | 59,60,61,62,67,71,78,81,85,90,100,108,114,117,123,128,129,131,136,140 |
| DQB1*0203 | 62,67,71,78,81,85,90,100,109,114,116,117,123,128,129,131,136,140 |
| DQB1*03011 | 62,66,68,70,75,80,88,91,93,101,110,114,116,122,125,127,132,136,140 |
| DQB1*03012 | 66,68,70,75,80,87,91,93,101,110,114,116,122,125,127,132,136,140 |
| DQB1*0302 | 62,67,68,70,76,80,88,94,101,111,114,116,122,125,127,132,136,140 |
| DQB1*03032 | 62,67,68,70,76,80,88,94,101,110,114,116,122,125,127,132,136,140 |
| DQB1*0304 | 62,66,68,70,75,80,88,91,93,101,111,114,116,122,125,127,132,136,140 |
| DQB1*0305 | 62,67,72,74,80,87,94,101,111,114,116,122,125,127,132,136,140 |
| DQB1*0306 | 62,67,68,70,76,80,88,94,101,110,115,117,119,126,133,136,140 |
| DQB1*0307 | 62,67,68,70,76,80,88,96,101,111,114,116,122,125,127,132,136,140 |
| DQB1*0308 | 62,67,68,70,76,80,88,94,101,111,114,116,121,125,127,132,136,140 |
| DQB1*0401 | 64,67,73,74,80,87,94,102,112,113,115,117,119,126,133,136,140 |
| DQB1*0402 | 64,67,72,74,80,87,94,102,112,113,115,117,119,126,133,136,140 |

**Table 4.**

| Correspondence of DRA types and high accuracy typing capture oligos | |
|---|---|
| Type | Corresponding capture oligos |
| DRA*0101 | 141,143 |
| DRA*0102 | 142,144 |

**Table 5.**

| Correspondence of DRB1 types and low accuracy and high accuracy typing capture oligos (1), alleles whose serological antigen type is DR1 | | |
|---|---|---|
| Type | Corresponding capture oligos | |
| | low accuracy | High accuracy |
| DRB1*0101 | 145 | 166, 173, 187, 213, 246, 250 |
| DRB1*01021 | 145 | 166, 173, 187, 213, 232, 236, 250 |
| DRB1*01022 | 145 | 166, 173, 187, 213, 228 ,232, 236, 250 |
| DRB1*0103 | 145 | 166, 173, 187, 211, 215, 246, 250 |
| DRB1*0104 | 145 | 173, 187, 213, 230, 250 |
| DRB1*0105 | 145 | 173, 187, 192, 213, 246, 250 |
| DRB1*0106 | 145 | |

**Table 6.**

| Correspondence of DRB1 types and low accuracy and high accuracy typing capture oligos (2), alleles whose serological antigen type is DR2 | | |
|---|---|---|
| Type | Corresponding capture oligos | |
| | low accuracy | high accuracy |
| DRB1*15011 | 149 | 157,187,194,201,211 ,212, 234, 247 |
| DRB1*15012 | 149 | 157,187,194,201,211,212,233,247 |
| DRB1*15021 | 149 | 157,18,7194,201,211 ,212, 246, 247 |
| DRB1*15022 | | 187, 194, 211 ,212, 247, 250 |
| DRB1*15023 | 149 | 157,181,187,194,201,211, 212 |
| DRB1*1503 | 149 | 157,178,187,194,201,211, 212, 234, 247 |
| DRB1*1504 | 149 | 157, 187, 194, 201, 210, 212, 234, 247 |
| DRB1*1505 | 149 | 157, 187, 194, 201, 212, 234, 247 |
| DRB1*1506 | 149 | 187, 194, 196 201, 211 ,212, 247 |
| DRB1*1507 | 149 | 157, 187, 201, 211, 212, 247 |
| DRB1*1508 | 149 | 157, 187, 194 201 212, 246, 247 |
| DRB1*1509 | 149 | |
| DRB1*1510 | 149 | |
| DRB1*16011 | 149 | 157, 187 201, 210 220, 246, 247 |
| DRB1*16012 | 149 | 157, 187, 20, 210, 246, 247 |
| DRB1*16021 | 149 | 157, 187, 201, 220, 246, 247 |
| DRB1*16022 | 149 | 187, 201, 247 |
| DRB1*1603 | 149 | 157, 187 201, 210 ,221, 246, 247 |
| DRB1*1604 | 149 | 187, 201, 210, 222, 247 |
| DRB1*1605 | 149 | 187, 201, 211, 220, 247 |
| DRB1*1607 | 149 | 157, 187, 201, 211, 220, 241, 246, 247 |
| DRB1*1608 | 149 | 157, 184 201, 210, 220, 246, 247 |

**Table 7.**

| Correspondence of DRB1 Types and low accuracy and high accuracy typing capture oligos (3), alleles whose serological antigen type is DR3 | | |
|---|---|---|
| Type | Corresponding capture oligos | |
| | low accuracy | high accuracy |
| DRB1*03011 | 146 | 171, 179, 184, 194, 216, 226, 229, 234, 229, 234, 248, 250 |
| DRB1*03012 | 146 | 171, 179, 184, 194, 216, 226 ,230, 234 ,230, 234, 248, 250 |
| DRB1*03021 | 146 | 174, 184 ,216 226, 229, 246, 248, 250 |
| DRB1*03022 | 146 | 174, 184, 216, 226, 230, 246, 248, 250 |
| DRB1*0303 | 146 | 174, 184, 216, 226, 229, 234, 248, 250 |
| DRB1*0304 | 146 | 171, 179 ,187 ,194 ,216, 226 ,229, 248, 250 |
| DRB1*0305 | 146 | 171, 179, 184, 194, 216, 226, 229, 246, 248, 250 |
| DRB1*0306 | 146 | 171, 179, 184, 216, 229, 234,248, 250 |
| DRB1*0307 | 146 | 179, 184, 194, 216, 226, 229, 234, 248, 250 |
| DRB1*0308 | 146 | 171, 179, 184, 194, 206 ,216, 226, 229, 234, 248 |
| DRB1*0309 | 146 | 171, 182, 184, 194, 216, 226. 229, 246, 250 |
| DRB1*0310 | 146 | 171, 179, 184, 194, 200, 216, 226, 229, 234, 244, 248 |
| DRB1*0311 | 146 | 171, 179, 184, 194, 216, 227, 229, 234, 248, 250 |
| DRB1*0312 | 146 | 171, 179, 184, 194, 199, 216, 226, 229, 248 |
| DRB1*0313 | 146 | |
| DRB1*0314 | 146 | |
| DRB1*0315 | 146 | |
| DRB1*0316 | 146 | |
| DRB1*0317 | 146 | |

**Table 8.**

| Correspondence of DRB1 types and low accuracy and high accuracy typing capture oligos (4), alleles whose serological antigen type is DR4 | | |
|---|---|---|
| Type | Corresponding capture oligos | |
| | low accuracy | high accuracy |
| DRB1*04011 | 147 | 160, 169, 180, 216, 246, 249, 250 |
| DRB1*04012 | 147 | 160, 169, 183, 216, 246, 249, 250 |
| DRB1*0402 | 147 | 160, 169, 180, 211, 215, 234, 249, 250 |
| DRB1*0403 | | 160, 169, 180, 213, 223, 234, 249, 250 |
| DRB1*04031 | 147 | |
| DRB1*04032 | 147 | 161, 169, 180, 201, 213, 223, 234, 249 |
| DRB1*0404 | 147 | 160, 169, 180, 213, 234, 249, 250 |
| DRB1*04051 | 147 | 160, 169, 180, 199, 213, 246, 249 |
| DRB1*04052 | 147 | 160, 169, 180, 193, 199, 213, 246 |
| DRB1*0406 | 147 | 160, 169, 180, 187, 213, 223, 234, 250 |
| DRB1*0407 | 147 | 160, 169, 180, 213, 223, 246, 249, 250 |
| DRB1*0408 | 147 | 160, 169, 180, 213, 246, 249, 250 |
| DRB1*0409 | | 169, 180, 199, 216, 249 |
| DRB1*0410 | 147 | 160, 169, 180, 199, 213, 234, 249 |
| DRB1*0411 | 147 | 160, 169, 180, 199, 213, 223, 234, 249 |
| DRB1*0412 | 147 | 160, 169, 199, 211, 222, 234, 249 |
| DRB1*0413 | | 169, 180, 216, 234, 249, 250 |
| DRB1*0414 | | 169, 180, 211, 215, 249, 250 |
| DRB1*0415 | 147 | 160, 169, 180, 206, 210, 249 |
| DRB1*0416 | | 169, 180, 207, 216, 249 |
| DRB1*0417 | | 169, 180, 199, 213, 223, 249 |
| DRB1*0418 | | 180, 211, 222, 234, 249, 250 |
| DRB1*0419 | | 169, 180, 187, 213, 246, 250 |
| DRB1*0420 | | 169, 180, 187, 213, 223, 250 |
| DRB1*0421 | 147 | 160, 169, 180, 187, 216, 246, 250 |
| DRB1*0422 | 147 | 160, 169, 180, 216, 226, 229, 234, 249, 250 |
| DRB1*0423 | 147 | 160, 169, 180, 213, 245, 249, 250 |
| DRB1*0424 | 147 | 160, 169, 180, 199, 214, 246, 249 |
| DRB1*0425 | 147 | 160, 169, 180, 210, 222, 249, 250 |
| DRB1*0426 | 147 | 160, 169, 180, 209, 216, 249 |
| DRB1*0427 | 147 | 160, 169, 118, 213, 223, 236, 249, 250 |
| DRB1*0428 | 147 | 160, 169, 180, 194, 199 ,213, 246, 249 |
| DRB1*0429 | 147 | 160, 169, 180, 197, 199 ,213, 246, 249 |
| DRB1*0430 | 147 | 160, 169, 180, 195, 213, 246, 249 |
| DRB1*0431 | 147 | 160, 169, 180, 213, 222, 246, 249, 250 |
| DRB1*0432 | 147 | 160, 169, 180, 219, 249,250 |
| DRB1*0433 | 147 | |
| DRB1*0434 | 147 | |

**Table 9.**

| Correspondence of DRB1 types and low accuracy and high accuracy typing capture oligos (5), alleles whose serological antigen type is DR5 and whose subtype is DR11 | | |
|---|---|---|
| Type | Corresponding capture oligos | |
| | low accuracy | high accuracy |
| DRB1*11011 | 146 | 194, 206, 210, 246, 249 |
| DRB1*11012 | 146 | 194, 206, 210, 239, 246, 249 |
| DRB1*11013 | 146 | 194, 206, 210, 220, 246, 249 |
| DRB1*1102 | 146 | 194, 206, 211, 215, 234, 249 |
| DRB1*1103 | 146 | 194, 206, 210, 220, 246, 249 |
| DRB1*11041 | 146 | 194, 206, 210, 234, 249 |
| DRB1*11042 | 146 | 194, 206, 210, 234, 239, 249 |
| DRB1*1105 | 146 | 194, 206, 210, 246, 249 |
| DRB1*1106 | 146 | 194, 206, 210, 236, 249 |
| DRB1*1107 | 146 | 194, 206, 216, 226, 229, 234, 249 |
| DRB1*11081 | | 194, 206, 246, 249 |
| DRB1*11082 | | 194, 206, 246, 249 |
| DRB1*1109 | | 179, 184, 194, 206, 210, 246, 248 |
| DRB1*1110 | | 179, 188, 194, 206, 210, 248 |
| DRB1*1111 | | 194, 206, 210, 215, 246, 249 |
| DRB1*1112 | | 188, 194, 206, 210 |
| DRB1*1113 | 146 | 179, 188, 194, 206, 214, 232, 234, 248 |
| DRB1*1114 | 146 | 194, 206, 211, 215, 246, 249 |
| DRB1*1115 | 146 | 186, 194, 206, 210, 246 |
| DRB1*1116 | 146 | 179, 184, 194, 206, 211, 215, 248 |
| DRB1*1117 | 146 | 179, 188, 206, 214, 223, 232, 234, 248 |
| DRB1*1118 | 146 | 194, 206, 211, 234, 249 |
| DRB1*1119 | 146 | 194, 206, 211, 246, 249 |
| DRB1*1120 | 146 | 179, 184, 194, 206, 211, 215, 246, 248 |
| DRB1*1121 | 146 | 194, 206, 211, 215, 249 |
| DRB1*1122 | 147 | 162, 194, 206 210, 246, 247, 249 |
| DRB1*1123 | 146 | 194, 206, 210, 222, 249 |
| DRB1*1124 | 146 | 185, 194, 206, 210 |
| DRB1*1125 | 146 | 194, 206, 210, 222, 234, 249 |
| DRB1*1126 | 146 | 194, 206, 213, 234, 249 |
| DRB1*1127 | | 194, 206, 210, 230, 249 |
| DRB1*11271 | 146 | |
| DRB1*11272 | 146 | |
| DRB1*1128 | 146 | 184, 194 206 |
| DRB1*1129 | 146 | 187, 194, 206, 210, 246 |
| DRB1*1130 | 154 | 165, 194, 206, 210, 246, 249 |
| DRB1*1131 | 146 | 194, 208, 211, 246, 249 |
| DRB1*1132 | 146 | 194, 206, 210, 224, 246, 249 |
| DRB1*1133 | 146 | 194, 210, 242, 246, 249, 250 |
| DRB1*1134 | 146 | 194, 206, 213, 234, 249 |
| DRB1*1135 | 146 | 194, 210, 234, 242, 249, 250 |
| DRB1*1136 | 146 | |
| DRB1*1137 | 146 | |

**Table 10.**

| Correspondence of DRB1 types and low accuracy and high accuracy typing capture oligos (6), alleles whose serological antigen type is DR5 and whose subtype is DR12 | | |
|---|---|---|
| Type | Corresponding capture oligos | |
| | low accuracy | high accuracy |
| DRB1*1201 | 146 | 158, 172, 191, 194, 202, 211, 220, 232, 236, 248 |
| DRB1*12021 | 146 | 158, 172, 191, 194, 202, 210, 220, 232, 246, 248 |
| DRB1*12022 | 146 | 158, 172, 191, 194, 202, 210, 220, 248 |
| DRB1*12032 | 146 | 158, 172, 191, 194, 202, 211, 220, 234, 248 |
| DRB1*1204 | 146 | 158, 172, 191, 194, 206, 211, 220, 232, 248 |
| DRB1*1205 | 146 | 158, 172, 190, 194, 202, 211, 220, 232, 236, 248 |
| DRB1*1206 | 146 | |

**Table 11.**

| Correspondence of DRB1 types and low accuracy and high accuracy typing capture oligos (7), alleles whose serological antigen type is DR6 and whose subtype is DR13 | | |
|---|---|---|
| Type | Corresponding capture oligos | |
| | low accuracy | high accuracy |
| DRB1*1301 | 146 | 179, 184, 194, 211, 215, 234, 248, 250 |
| DRB1*1302 | | 179, 184, 194 211, 215, 246, 248, 250 |
| DRB1*13021 | 146 | |
| DRB1*13022 | 146 | |
| DRB1*12031 | 146 | 199, 211 218, 239, 246, 249 |
| DRB1*13032 | | 199, 211 218, 239, 246, 249 |
| DRB1*13032 | 146 | |
| DRB1*1304 | 146 | 194, 199, 211, 215, 234, 249 |
| DRB1*1305 | 146 | 179, 184, 194, 210, 246, 248, 250 |
| DRB1*1306 | 146 | 179, 184, 194, 211, 234, 248, 250 |
| DRB1*13071 | 146 | 210, 246, 249, 250 |
| DRB1*13072 | 146 | 201, 210, 246, 249 |
| DRB1*1308 | 146 | 179, 188, 211, 215, 234, 248, 250 |
| DRB1*1309 | 146 | 179,184,194,211, 212, 234, 248, 250 |
| DRB1*1310 | 146 | 179, 184, 194, 211, 218, 234, 248,250 |
| DRB1*1311 | 146 | 194, 210, 249, 250 |
| DRB1*1312 | 146 | 199, 211, 246, 249 |
| DRB1*1313 | 146 | 199, 211, 222, 246, 249 |
| DRB1*1314 | | 194, 210, 246, 249, 250 |
| DRB1*13142 | 146 | |
| DRB1*1315 | 146 | 174, 184, 194, 211, 215, 234, 248, 250 |
| DRB1*1316 | 146 | 179, 184, 194, 211, 215, 235, 248, 250 |
| DRB1*1317 | 146 | 158, 194, 211, 215, 234, 249, 250 |
| DRB1*1318 | 146 | 179, 184, 194, 210, 222, 234, 248, 250 |
| DRB1*1319 | 146 | 174, 188, 211, 215, 222, 234, 248, 250 |
| DRB1*1320 | 146 | 179, 184, 194, 215, 234, 248, 250 |
| DRB1*1321 | 146 | 194, 199, 210, 246, 249 |
| DRB1*1322 | 146 | 194, 211, 215, 234, 249, 250 |
| DRB1*1323 | 146 | 194, 211, 215, 239, 246, 249, 250 |
| DRB1*1324 | 146 | 194, 210, 215, 234, 249, 250 |
| DRB1*1325 | 146 | 194, 249, 250 |
| DRB1*1326 | 146 | 174, 184, 201, 210, 220, 248 |
| DRB1*1327 | 146 | 171, 179, 184, 194, 211, 215, 220, 234, 248, 250 |
| DRB1*1328 | 146 | 179, 184, 194, 211 215, 237, 248, 250 |
| DRB1*1329 | 146 | 179, 184, 194, 215, 246, 248, 250 |
| DRB1*1330 | 146 | 194, 199 211, 246, 249 |
| DRB1*1331 | 146 | 179, 184 194 211 215, 246, 248, 250 |
| DRB1*1332 | 146 | 179, 184, 199, 211, 215, 234, 248 |
| DRB1*1333 | 146 | 199, 211, 218, 225, 229, 249 |
| DRB1*1334 | 146 | 179, 189, 194 ,211, 215, 240, 246, 248, 250 |
| DRB1*1335 | 146 | |
| DRB1*1336 | 146 | |
| DRB1*1337 | 146 | |
| DRB1*1338 | 146 | |
| DRB1*1339 | 146 | |
| DRB1*1340 | 146 | |
| DRB1*1341 | 146 | |

**Table 12.**

| Correspondence of DRB1 types and low accuracy and high accuracy typing capture oligos (8), alleles whose serological antigen type is DR6 and whose subtype is DR14 | | |
|---|---|---|
| Type | Corresponding capture oligos | |
| | low accuracy | high accuracy |
| DRB1*1401 | 146 | 179, 188, 200, 214, 223, 232, 234, 244, 248 |
| DRB1*1402 | 146 | 174, 184, 213, 246, 248, 250 |
| DRB1*1403 | 146 | 174, 184, 222, 246, 248, 250 |
| DRB1*1404 | 146 | 158, 179, 188, 200, 214, 223, 232, 234, 244, 248 |
| DRB1*1405 | 146 | 159, 179, 188, 205, 214, 223, 232, 234, 248 |
| DRB1*1406 | 146 | 174, 184, 213, 234, 248, 250 |
| DRB1*1407 | 146 | 179, 188, 200, 214, 223, 232, 244, 248 |
| DRB1*1408 | 146 | 179, 188, 203, 214, 223, 232, 234, 244, 248 |
| DRB1*1409 | 146 | 179, 184, 213, 246, 248, 250 |
| DRB1*1410 | 147 | 160, 179, 188, 200, 214, 223, 232, 234, 244, 248 |
| DRB1*1411 | 146 | 158, 179, 188, 206, 214, 223, 232, 248 |
| DRB1*1412 | | 174, 184, 222, 248, 250 |
| DRB1*1413 | 146 | 174, 184, 199, 213, 248 |
| DRB1*1414 | 146 | 179, 188, 214, 223, 232, 246, 248, 250 |
| DRB1*1415 | | 158, 179, 188, 210, 222, 234, 248, 250 |
| DRB1*1416 | 146 | 179, 188, 200, 211, 215, 234, 244, 248 |
| DRB1*1417 | 146 | 179, 184, 194, 213, 234, 248, 250 |
| DRB1*1418 | 146 | 174, 184, 205, 214, 223, 232, 234, 248 |
| DRB1*1419 | 146 | 174, 184, 216, 246, 248, 250 |
| DRB1*1420 | 146 | 174, 188, 213, 248, 250 |
| DRB1*1421 | 146 | 179, 184, 194, 216, 248, 250 |
| DRB1*1422 | 146 | 179, 188 200, 210, 244, 246, 248 |
| DRB1*1423 | 146 | 179, 188, 214, 223, 232, 234, 248, 250 |
| DRB1*1424 | 146 | 174, 184, 211 212, 246, 248, 250 |
| DRB1*1425 | 146 | 200, 210, 244, 249 |
| DRB1*1426 | 146 | 170, 179, 188, 200, 214, 223, 232, 234, 244, 248 |
| DRB1*1427 | 146 | 174, 184 210, 222, 246, 248, 250 |
| DRB1*1428 | 146 | 158, 179,188, 200, 214, 223, 232, 244, 248 |
| DRB1*1429 | 146 | 174, 184, 213, 236, 248, 250 |
| DRB1*1430 | 146 | 179, 184 194, 213, 246, 248, 250 |
| DRB1*1431 | 146 | 158, 179, 188, 200, 214, 232, 234, 244, 2480 |
| DRB1*1432 | 146 | |
| DRB1*1433 | 146 | 179, 184, 194, 213, 223, 234, 248, 250 |
| DRB1*1434 | 146 | |
| DRB1*1435 | 146 | |
| DRB1*1436 | 146 | |

**Table 13.**

| Correspondence of DRB1 types and low accuracy and high accuracy typing capture oligos (9), alleles whose serological antigen type is DR7, DR8, DR9 or DR10 | | |
|---|---|---|
| Type | Corresponding capture oligos | |
| | low accuracy | high accuracy |
| DRB1*0701 | | 164, 169, 170, 176, 188, 198, 202, 211, 217, 227, 231, 246, 247 |
| DRB1*07011 | 148 | |
| DRB1*07012 | 148 | |
| DRB1*0703 | 148 | 164, 169, 170, 177, 188, 198, 202, 211, 217, 227, 231, 247 |
| DRB1*0704 | 148 | 164, 169, 170, 176, 188, 198, 202, 211, 217, 227, 230, 247 |
| DRB1*0801 | 146 | 158, 199, 210, 222, 239, 246, 249 |
| DRB1*08021 | 146 | 158, 210, 222, 246, 249, 250 |
| DRB1*08022 | 146 | 158, 210, 222, 246, 249, 250 |
| DRB1*08032 | 146 | 158, 199, 211, 222, 246, 249 |
| DRB1*08041 | 146 | 158, 210, 222, 234, 249, 250 |
| DRB1*08042 | | 158, 210, 222, 238, 239, 249, 250 |
| DRB1*08043 | 146 | 158, 210, 222, 238, 239, 249, 250 |
| DRB1*0805 | 146 | 158, 199, 210, 249 |
| DRB1*0806 | 146 | 158, 199, 210, 222, 234, 249 |
| DRB1*0807 | 146 | 158, 210, 222, 239, 246, 249, 250 |
| DRB1*0808 | 146 | 158, 200, 210, 222, 244, 249 |
| DRB1*0809 | | 168, 179, 188, 210, 222, 239, 246, 248, 250 |
| DRB1*0810 | 146 | 158, 199, 211, 222, 234, 249 |
| DRB1*0811 | 146 | 158, 210, 222, 239, 246, 249, 250 |
| DRB1*0812 | 146 | 158, 199, 211, 222, 249 |
| DRB1*0813 | 146 | 158, 222, 249, 250 |
| DRB1*0814 | 146 | 167, 199, 211 222, 2467, 249 |
| DRB1*0815 | 146 | 158203211222, 244, 246, 249 |
| DRB1*0816 | 146 | 158,185,199,210,222 |
| DRB1*0817 | 146 | 158, 194, 199, 210, 222, 249 |
| DRB1*0818 | 146 | 158, 199, 211, 246, 249 |
| DRB1*0819 | 146 | 58, 211 222, 246, 249, 250 |
| DRB1*0820 | 146 | |
| DRB1*0821 | 146 | 168, 179, 188, 210, 222, 239, 246, 248, 250 |
| DRB1*09012 | 150 | 163, 169, 175, 184, 202, 210, 214, 223, 231, 243 |
| DRB1*10011 | 151 | |
| DRB1*10012 | 151 | |

**Table 14.**

| Correspondence of DRB3 types and low accuracy typing and high accuracy typing capture oligos | | |
|---|---|---|
| Type | Corresponding capture oligos | |
| | low accuracy | High accuracy |
| DRB3*01011 | 152 | 252, 255, 258, 260, 265, 267, 271, 273, 275, 277, 279 |
| DRB3*01012 | 152 | 252, 255, 258, 260, 265, 267, 271, 273, 275, 277, 279 |
| DRB3*01013 | 152 | 252, 255, 258, 260, 265, 268, 271, 273, 275, 277, 279 |
| DRB3*01014 | 152 | 252, 255, 258, 260, 265, 267, 271, 273, 275, 278, 279 |
| DRB3*0102 | 153 | 253, 255, 258, 260, 265, 267, 271, 273, 275, 277, 279 |
| DRB3*0103 | 152 | 252, 255, 258, 260, 265, 267, 271, 273, 275, 277, 279 |
| DRB3*0104 | 152 | 252, 255, 258, 260, 265, 267, 271, 273, 275, 277, 279 |
| DRB3*0105 | 152 | 252, 256, 258, 260, 265, 267, 271, 273, 275, 277, 279 |
| DRB3*0106 | 152 | 252, 255, 258, 261, 265, 267, 271, 273, 275, 277, 279 |
| DRB3*0107 | 152 | 252, 255, 258, 262, 266, 269, 272, 273, 275, 277, 279 |
| DRB3*0201 | 154 | 254, 257, 259, 262, 266, 269, 272, 273, 275, 277, 280 |
| DRB3*02021 | 154 | 254, 257, 259, 262, 266, 269, 272, 273, 275, 277, 279 |
| DRB3*02022 | 154 | 254, 257, 259, 262, 266, 269, 272, 274, 275, 277, 279 |
| DRB3*02023 | 154 | 254, 257, 259, 262, 266, 269, 272, 273, 276, 277, 279 |
| DRB3*0203 | 154 | 254, 257, 259, 263, 266, 269, 272, 273, 275, 277, 279 |
| DRB3*0204 | 154 | 254, 257, 259, 262, 266, 269, 271, 274, 275, 277, 280 |
| DRB3*0205 | 154 | 254, 257, 258, 262, 266, 269, 272, 273, 275, 277, 279 |
| DRB3*0206 | 154 | 254, 257, 259, 264, 266, 269, 272, 273, 275, 277, 279 |
| DRB3*0207 | 154 | 254, 257, 259, 262, 266, 267, 272, 273, 275, 277, 279 |
| DRB3*0208 | 154 | 254, 257, 259, 262, 266, 270, 272, 273, 275, 277, 279 |
| DRB3*0210 | 154 | 254, 257, 259, 262, 265, 269, 272, 273, 275, 277, 279 |
| DRB3*0211 | 154 | 254, 257, 259, 262, 266, 269, 272, 273, 275, 277, 279 |
| DRB3*0212 | 154 | 254, 257, 259, 262, 266, 269, 272, 273, 275, 277, 279 |
| DRB3*0301 | 154 | 254, 257, 258, 261, 265, 267, 272, 273, 275, 277, 280 |
| DRB3*0302 | 154 | 254, 257, 259, 261, 265, 267, 272, 273, 275, 277, 280 |
| DRB3*0303 | 154 | 254, 257, 258, 261, 265, 267, 271, 273, 275, 277, 279 |

**Table 15.**

| Correspondence of DRB4 types and low accuracy typing and high accuracy typing capture oligos | | |
|---|---|---|
| Type | Corresponding capture oligos | |
| | low accuracy | high accuracy |
| DRB4*01011 | 155 | 281, 284, 286, 288 |
| DRB4*0102 | | 282, 284, 287 |
| DRB4*0103101 | 155 | 281, 284, 287, 288 |
| DRB4*0103102 N | | 287 |
| DRB4*01032 | 155 | 281, 284, 287, 289 |
| DRB4*0104 | 155 | 283, 284 |
| DRB4*0105 | 155 | 281, 285 |
| DRB4*0201N | 155 | 281, 284, 286 |
| DRB4*0301N | | 286 |

**Table 16.**

| Correspondence of DRB5 types and low accuracy typing and high accuracy typing capture oligos | | |
|---|---|---|
| Type | Corresponding capture oligos | |
| | low accuracy | high accuracy |
| DRB5*01011 | 156 | 290, 292, 295, 297, 302, 304, 306 |
| DRB5*01012 | 156 | 290, 292, 295, 297, 300, 305, 306 |
| DRB5*0102 | 156 | 291, 293, 295, 297, 302, 304, 306 |
| DRB5*0103 | 156 | 291, 293, 295, 298, 304, 306 |
| DRB5*0104 | 156 | 290, 292, 295, 297, 303, 306 |
| DRB5*0105 | 156 | 290, 294, 295, 297, 302, 304, 306 |
| DRB5*0106 | 156 | 290, 292, 296, 299, 301, 305, 307 |
| DRB5*0107 | 156 | 290, 292, 296, 297, 302, 305, 307 |
| DRB5*0108 N | 156 | 291, 293, 295, 297, 302, 304, 306 |
| DRB5*0109 | 156 | 290, 292, 295, 297, 302, 304, 306 |
| DRB5*0110 N | 156 | 291, 293, 295, 297, 302, 304, 306 |
| DRB5*0202 | 156 | 291, 293, 296, 299, 301, 305, 306 |
| DRB5*0203 | 156 | 291, 293, 296, 299, 301, 305, 306 |
| DRB5*0204 | 156 | 291, 293, 295, 299, 301, 305, 307 |

**Table 17.**

| Correspondence of DRB2, DRB6 and DRB7 types and low accuracy typing and high accuracy typing capture oligos | | |
|---|---|---|
| Type | Corresponding capture oligos | |
| | low accuracy | high accuracy |
| DRB2*0101 | | 251 |
| DRB6*0101 | 155 | 308, 310, 312, 314, 316, 318 |
| DRB6*0201 | 155 | 309, 311, 313, 315, 317, 319 |
| DRB6*0202 | | 309, 311, 313, 315, 317, 320 |
| DRB7*01011 | | 321 |
| DRB7*01012 | | 322 |
| DRB7*01011 | 155 | 323 |

**Table 18.**

| Correspondence of DPA types and high accuracy typing capture oligos | |
|---|---|
| Type | Corresponding capture oligos |
| DPA1*0103 | 324, 326, 330, 331, 333, 336, 338, 341, 343, 345, 347 |
| DPA1*0104 | 324, 326, 330, 331, 334, 336, 338, 341, 343, 345, 347 |
| DPA1*0105 | 324, 326, 330, 331, 333, 336, 338, 341, 343, 345, 348 |
| DPA1*0106 | 324, 326, 330, 331, 333, 337, 339, 341, 343, 345, 347 |
| DPA1*02011 | 324, 327, 330, 331, 333, 337, 339, 342, 343, 345, 348 |
| DPA1*02012 | 324, 326, 330, 331, 333, 337, 339, 342, 343, 345, 348 |
| DPA1*02013 | 324, 326, 330, 331, 333, 337, 338, 342, 343, 345 |
| DPA1*02014 | 324, 330, 331, 333, 337, 339, 342, 343, 345, 348 |
| DPA1*02021 | 325, 327, 329, 331, 333, 337, 340, 342, 343, 345, 348 |
| DPA1*02022 | 325, 327, 329, 331, 333, 337, 338, 342, 343, 345, 348 |
| DPA1*0203 | 324, 327, 330, 331, 333, 336, 338, 342, 343, 345, 347 |
| DPA1*0301 | 325, 327, 330, 331, 333, 336, 338, 341, 344, 345, 347 |
| DPA1*0302 | 325, 327, 330, 331, 333, 336, 338, 341, 343, 345, 347 |
| DPA1*0401 | 324, 326, 328, 332, 335, 336, 338, 342, 343, 346, 348 |

**Table 19.**

| Correspondence of DPB high accuracy typing capture (1) | |
|---|---|
| Type | Corresponding capture oligos |
| DPB1*01011 | 349, 254, 358, 361, 366, 367, 376 |
| DPB1*01012 | 350, 355, 358, 364, 365, 368, 373, 376 |
| DPB1*02011 | 350, 354, 358, 364, 365, 368, 373, 376 |
| DPB1*02012 | 350, 354, 358, 364, 365, 369, 376 |
| DPB1*02013 | 350, 354, 358, 362, 365, 371, 376 |
| DPB1*0202 | 349, 356, 358, 364, 365, 370, 374, 377 |
| DPB1*0301 | 350, 354, 358, 363, 365, 367, 376 |
| DPB1*0401 | 350, 354, 358, 364, 365, 368, 373, 376 |
| DPB1*0402 | 350, 354, 358, 362, 365, 371, 376 |
| DPB1*0501 | 349, 356, 358, 364, 365, 370, 374, 377 |
| DPB1*0601 | 350, 354, 358, 364, 365, 368, 373, 376 |
| DPB1*0801 | 351, 356, 358, 364, 365, 370, 374, 376 |
| DPB1*0901 | 351, 356, 358, 364, 365, 368, 373, 376, 386, 388, 390, 393, 396 |
| DPB1*1001 | 349, 356, 360, 361, 366, 367, 377, 384, 385, 388, 391, 393, 396 |
| DPB1*11011 | 349, 356, 359, 361, 366, 367, 377, 384, 386, 388, 391, 393, 396 |
| DPB1*11012 | 349, 356, 358, 361, 365, 367, 376, 386, 388, 392, 393, 396 |
| DPB1*1301 | 351, 356, 358, 364, 365, 370, 374, 377, 382, 386, 388, 390, 393, 396 |
| DPB1*1401 | 349, 354, 360, 361, 366, 367, 377, 384, 386, 388, 391, 395, 396 |
| DPB1*1501 | 350, 354, 358, 364, 365, 368, 373, 376, 386, 388, 391, 393, 396 |
| DPB1*1601 | 351, 356, 358, 364, 365, 370, 374, 376, 386, 388, 391, 393, 396 |
| DPB1*1701 | 349, 354, 358, 364, 365, 368, 373, 376, 382, 386, 388, 391, 395, 396 |
| DPB1*1801 | 350, 354, 358, 364, 365, 371, 376, 386, 388, 392, 393, 396 |
| DPB1*1901 | 349, 356, 358, 364, 365, 370, 374, 377, 382, 386, 388, 391, 393, 396 |
| DPB1*20011 | 349, 356, 358, 364, 365, 370, 374, 377, 382, 386, 388, 391, 393, 396 |
| DPB1*20012 | 349, 356, 358, 362, 365, 371, 376, 386, 388, 391, 393, 396 |
| DPB1*2101 | 350, 354, 358, 362, 365, 371, 376, 386, 388, 391, 393, 396 |
| DPB1*2201 | 350, 354, 358, 364, 365, 367, 376, 382, 386, 388, 391, 394, 396 |
| DPB1*2301 | 350, 354, 358, 363, 365, 371, 376, 382, 386, 388, 391, 394, 396 |
| DPB1*2401 | 349, 356, 358, 364, 365, 368, 373, 377, 382, 386, 388, 390, 393, 396 |
| DPB1*2501 | 349, 356, 358, 361, 366, 367, 376, 382, 386, 388, 393, 396 |
| DPB1*26011 | 349, 356, 358, 361, 365, 367, 376, 382, 386, 388, 390, 393, 396 |
| DPB1*26012 | 349, 356, 358, 361, 365, 367, 376, 382, 386, 388, 391, 393, 396 |
| DPB1*2701 | 350, 354, 368, 363, 365, 368, 373, 377, 382, 386, 388, 391, 395, 396 |
| DPB1*2801 | 349, 356, 358, 364, 365, 370, 374, 377, 386, 388, 390, 393, 396 |
| DPB1*2901 | 351, 356, 358, 364, 365, 371, 376, 386, 388, 391, 393, 396 |
| DPB1*3001 | 350, 354, 358, 363, 365, 367, 377, 382, 387, 391, 393, 396 |
| DPB1*3101 | 350, 354, 358, 364, 365, 372, 375, 376, 386, 388, 391, 394, 396 |
| DPB1*3201 | 350, 354, 358, 363, 365, 367, 376, 386, 388, 391, 394, 396 |
| DPB1*3301 | 350, 354, 358, 362, 365, 367, 377, 382, 387, 391, 395, 396 |
| DPB1*3401 | 351, 356, 358, 364, 365, 370, 374, 376, 382, 386, 388, 390, 393, 396 |
| DPB1*3501 | 349, 356, 358, 362, 365, 371, 376, 382, 386, 388, 391, 393, 396 |
| DPB1*3601 | 349, 356, 358, 364, 365, 368, 373, 376, 386, 388, 390, 393, 396 |
| DPB1*3701 | 350, 354, 358, 362, 365, 371, 376, 382, 386, 388, 391, 393, 397 |
| DPB1*3801 | 350, 354, 358, 361, 365, 367, 376, 382, 386, 388, 391, 394, 396 |
| DPB1*3901 | 350, 354, 358, 361, 365, 367, 376, 382, 386, 388, 391, 395, 396 |
| DPB1*4001 | 350, 354, 358, 364, 365, 368, 373, 378, 386, 388, 391, 394, 396 |

**Table 20.**

| Correspondence of DPB types and high accuracy typing capture oligos (2) | |
|---|---|
| Type | Corresponding capture oligos |
| DPB1*4101 | 349, 356, 358, 362, 365, 370, 374, 377, 386, 388, 390, 393, 396 |
| DPB1*4401 | 351, 356, 358, 364, 365, 368, 373, 377, 382, 386, 388, 390, 393, 396 |
| DPB1*4501 | 350, 354, 358, 364, 365, 370, 374, 376, 386, 388, 391, 394, 396 |
| DPB1*4601 | 350, 354, 358, 364, 365, 371, 376, 386, 388, 391, 394, 396 |
| DPB1*4701 | 350, 354, 358, 362, 365, 368, 373, 376, 386, 388, 391, 394, 396 |
| DPB1*4801 | 350, 354, 358, 361, 365, 368, 373, 376, 382, 386, 388, 391, 394, 396 |
| DPB1*4901 | 349, 354, 358, 364, 365, 370, 374, 377, 382, 386, 388, 390, 393, 396 |
| DPB1*5001 | 350, 354, 358, 363, 365, 368, 373, 376, 382, 386, 388, 391, 394, 396 |
| DPB1*5101 | 349, 356, 358, 364, 365, 367, 377, 382, 386, 388, 390, 393, 396 |
| DPB1*5201 | 350, 354, 358, 361, 365, 368, 373, 376, 382, 386, 388, 391, 395, 396 |
| DPB1*5301 | 351, 356, 358, 364, 365, 371, 376, 386, 388, 390, 393, 396 |
| DPB1*5401 | 351, 356, 358, 364, 365, 367, 376, 386, 388, 391, 393, 396 |
| DPB1*5501 | 349, 356, 358, 363, 365, 367, 377, 382, 386, 388, 390, 393, 396 |
| DPB1*5601 | 350, 354, 358, 364, 365, 370, 374, 377, 382, 386, 388, 390, 393, 396 |
| DPB1*5701 | 351, 356, 358, 362, 365, 367, 376, 386, 388, 391, 393, 396 |
| DPB1*5801 | 350, 354, 358, 364, 365, 368, 373, 377, 382, 386, 388, 391, 394, 396 |
| DPB1*5901 | 350, 354, 358, 364, 365, 368, 373, 379, 382, 386, 388, 391, 394, 396 |
| DPB1*6001 | 349, 356, 358, 364, 365, 370, 374, 380, 381, 385, 386, 388, 390, 393 |
| DPB1*6101N | 350, 354, 358, 362, 365, 367, 376, 382, 386, 388, 391, 395, 396 |
| DPB1*6201 | 350, 354, 358, 362, 365, 367, 376, 382, 386, 388, 391, 393, 396 |
| DPB1*6301 | 352, 356, 358, 364, 365, 370, 374, 377, 386, 388, 391, 393, 396 |
| DPB1*6401N | 350, 354, 358, 361, 365, 367, 376, 382, 386, 388, 390, 393, 396 |
| DPB1*6501 | 351, 356, 358, 363, 365, 367, 376, 382, 386, 388, 391, 394, 396 |
| DPB1*6601 | 351, 356, 358, 364, 365, 367, 377, 382, 386, 388, 390, 393, 396 |
| DPB1*6701 | 350, 354, 358, 364, 365, 368, 373, 376, 382, 386, 388, 390, 393, 396 |
| DPB1*6801 | 349, 356, 358, 364, 365, 370, 374, 377, 384, 386, 388, 391, 393, 396 |
| DPB1*6901 | 353, 356, 358, 364, 365, 370, 374, 377, 382, 386, 388, 390, 393, 396 |
| DPB1*7001 | 350, 354, 358, 364, 365, 367, 376, 386, 388, 391, 394, 396 |
| DPB1*7101 | 350, 354, 358, 363, 365, 367, 377, 382, 386, 388, 391, 394, 396 |
| DPB1*7201 | 350, 354, 358, 364, 365, 368, 373, 377, 382, 386, 388, 390, 394, 396 |
| DPB1*7301 | 349, 356, 359, 364, 366, 367, 377, 386, 388, 391, 395, 396 |
| DPB1*7401 | 350, 354, 358, 364, 365, 368, 373, 376, 382, 386, 388, 390, 394, 396 |
| DPB1*7501 | 351, 356, 358, 363, 365, 368, 373, 377, 382, 386, 388, 390, 393, 396 |
| DPB1*7601 | 350, 357, 358, 361, 365, 368, 373, 376, 382, 386, 388, 391, 394, 396 |
| DPB1*7701 | 349, 356, 358, 364, 365, 370, 374, 377, 382, 389, 390, 393, 396 |
| DPB1*7801 | 349, 356, 358, 364, 365, 368, 373, 376, 382, 386, 388, 390, 393, 396 |
| DPB1*7901 | 349, 356, 358, 364, 365, 368, 373, 376, 382, 386, 388, 390, 393, 396 |

The kit of the present invention includes a substrate on which capture oligos are immobilized. The kit of the present invention can include primers for preparing a nucleic acid target or reagents for hybridization such as a labeled nucleic acid target, buffer and enzyme conjugate recognizing a hapten.

### Brief Description of the Drawings

Fig. 1 shows positions of capture oligos (for HLA-DQA1) immobilized on a substrate. Numbers in the figure represent sequence numbers (the same shall apply in Figs. 2-7).
Fig. 2 shows positions of capture oligos (for HLA-DQB1) immobilized on a substrate.
Fig. 3 shows positions of capture oligos (for HLA-DRA) immobilized on a substrate.
Fig. 4 shows positions of capture oligos (for HLA-DRB1) immobilized on a substrate.
Fig. 5 shows positions of capture oligos (for HLA-DRB3, HLA-DRB4, HLA-DRB5 and HLA-DRB6) immobilized on a substrate.
Fig. 6 shows positions of capture oligos (for HLA-DPA1) immobilized on a substrate.
Fig. 7 shows positions of capture oligos (for HLA-DPB1) immobilized on a substrate.
Fig. 8 shows a schematic view of a kit of the present invention and a detection result of DRA typing using the same (detection result typed as DRA*0101).

Numbers in the figure represent sequence numbers. B indicates that an oligonucleotide in which biotin is introduced at 5' end is immobilized, indicates that hybridization is not obtained. indicates that a hybridization signal is obtained (the same shall apply in Figs. 9 and 10).
Fig. 9 shows a schematic view of the kit of the present invention and a detection result of DRB low resolution typing using the same (detection result typed as DR4).
Fig. 10 shows a schematic view of the kit of the present invention and a detection result of DRB1 high resolution typing using the same (detection result typed as DRB*04011).

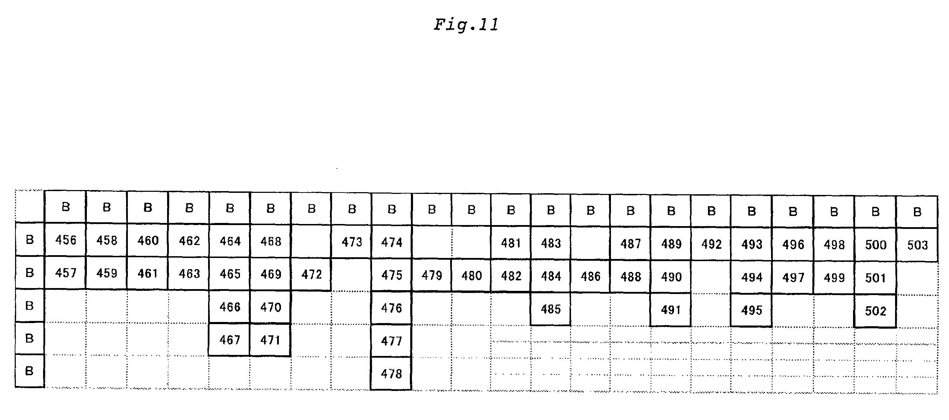

Fig. 11 shows positions of capture oligos (for HLA-DPB1) immobilized on a substrate.

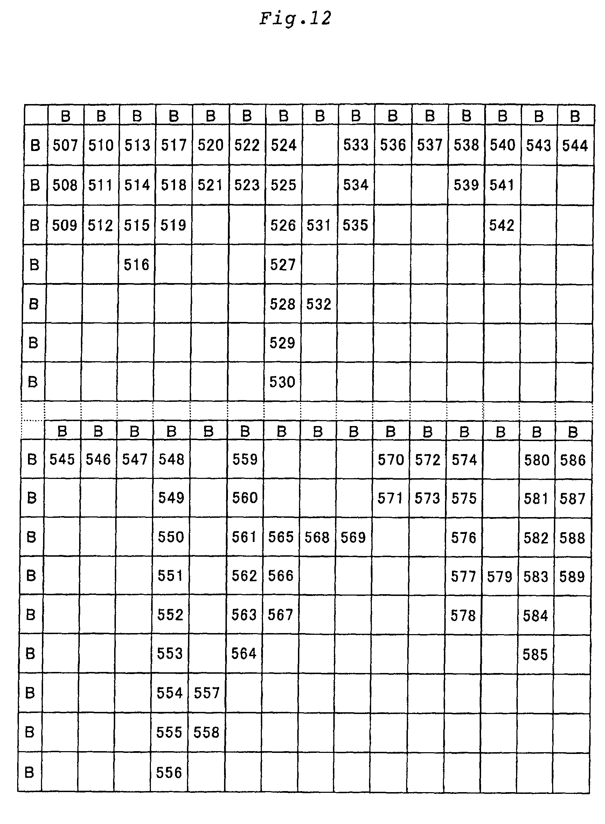

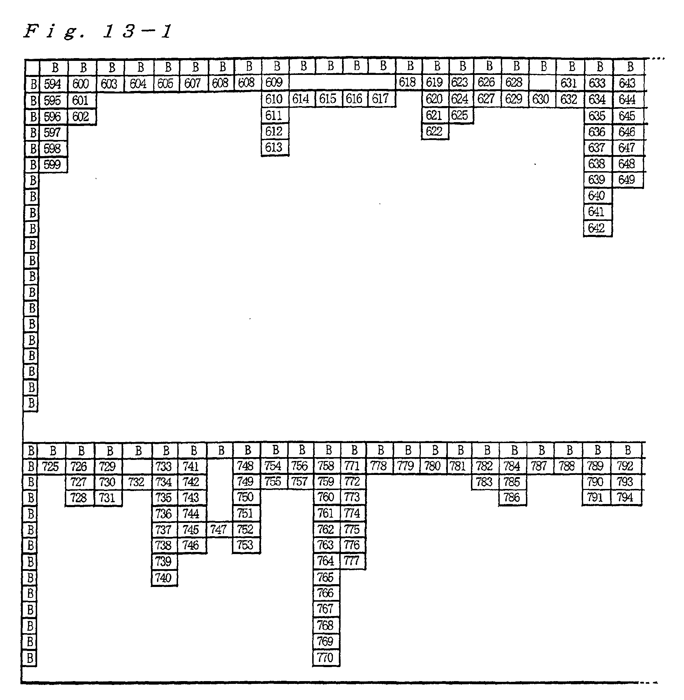

Fig. 12 shows positions of capture oligos (for HLA-DQB1) immobilized on a substrate.

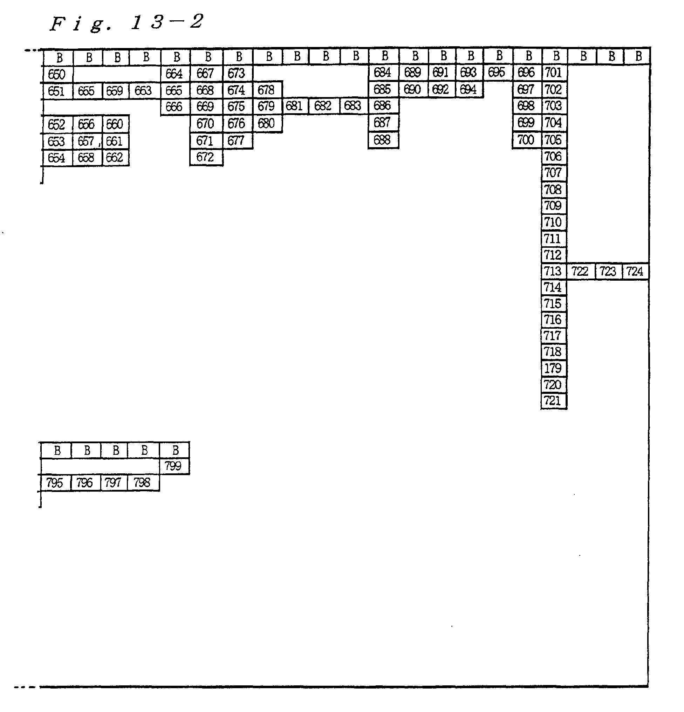

Fig. 13 shows positions of capture oligos (for HLA-DRB1) immobilized on a substrate.

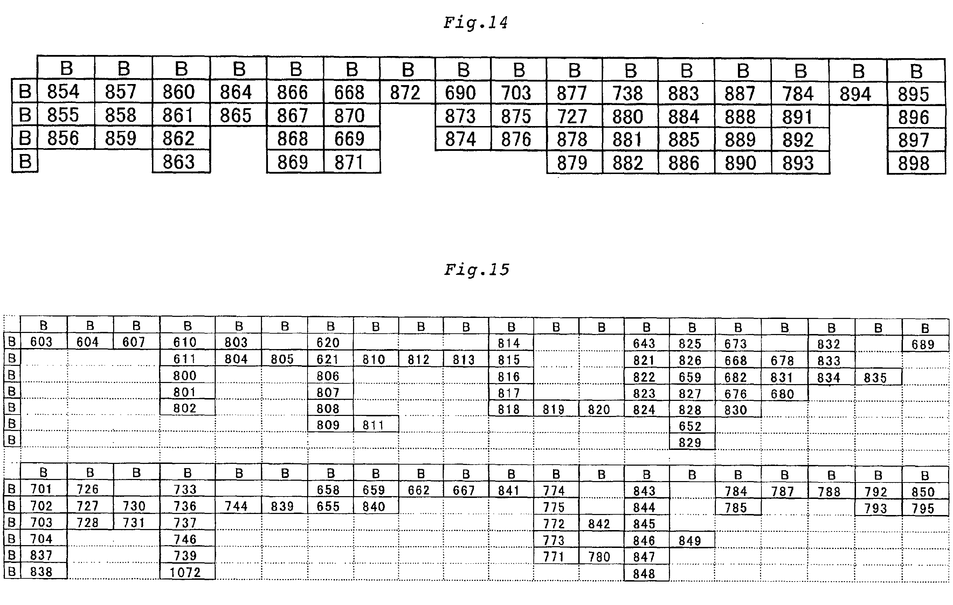

Fig. 14 shows positions of capture oligos (for HLA-DRB pseudogene) immobilized on a substrate.
Fig. 15 shows positions of capture oligos (for HLA-DPB3, 4, 5) immobilized on a substrate.

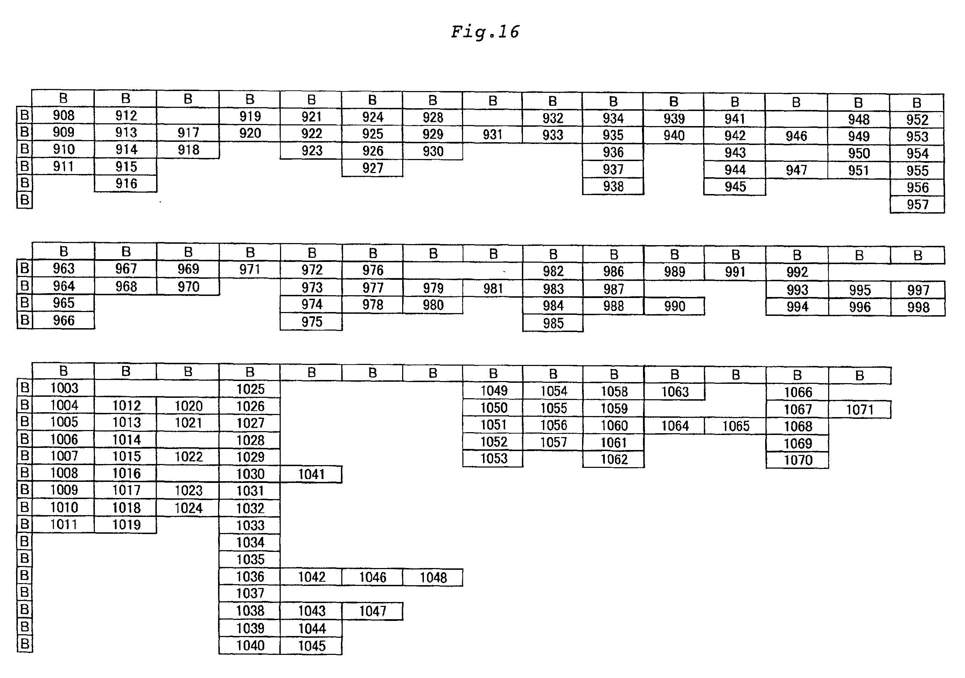

Fig. 16 shows positions of capture oligos (for HLA-A) immobilized on a substrate.

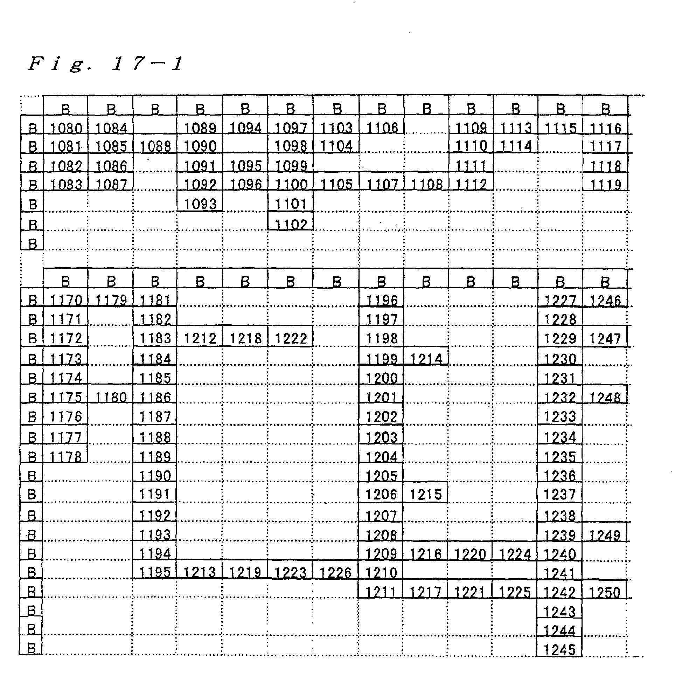

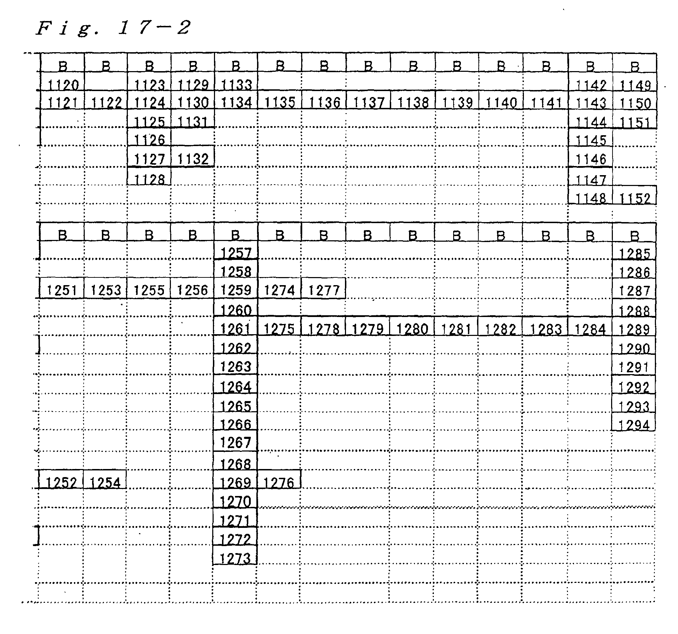

Fig. 17 shows positions of capture oligos (for HLA-B) immobilized on a substrate.

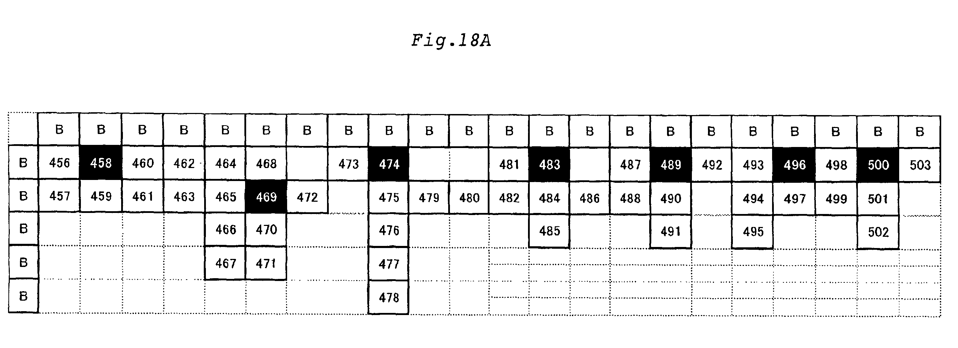

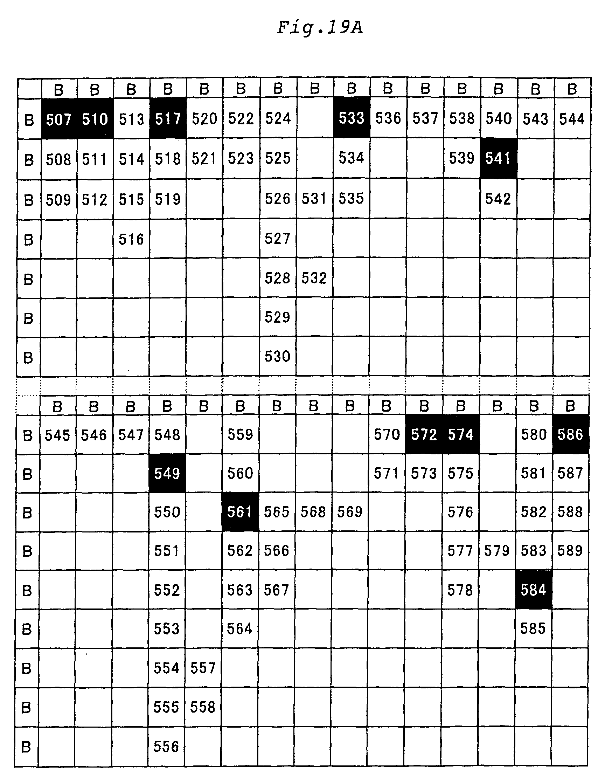

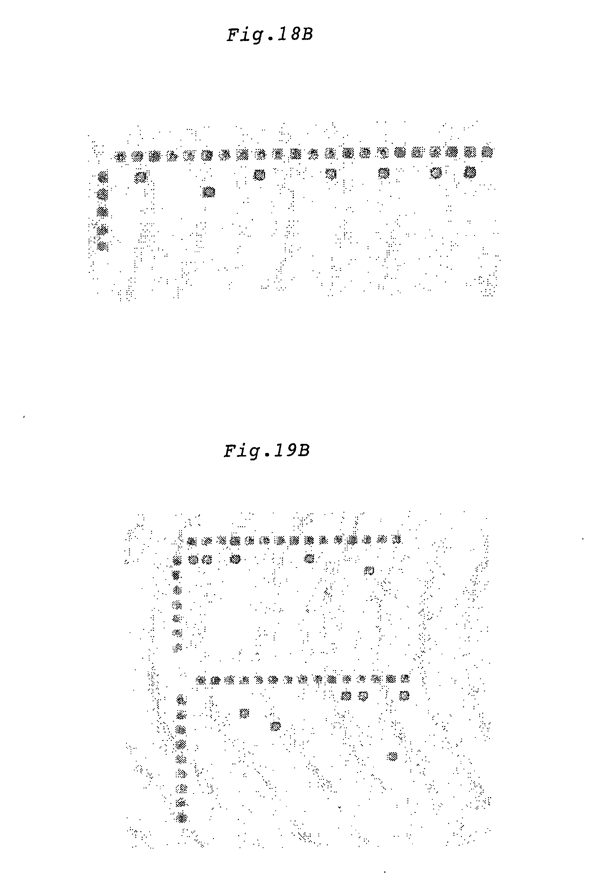

Fig. 18 shows a schematic view of the kit of the present invention (A) and a photograph showing detection result of DPB1 typing using the same (detection result typed as DPB1*01011) (B).
Fig. 19 shows a schematic view of the kit of the present invention (A) and a photograph showing detection result of DQB1 typing using the same (detection result typed as DQB1*05031) (B).

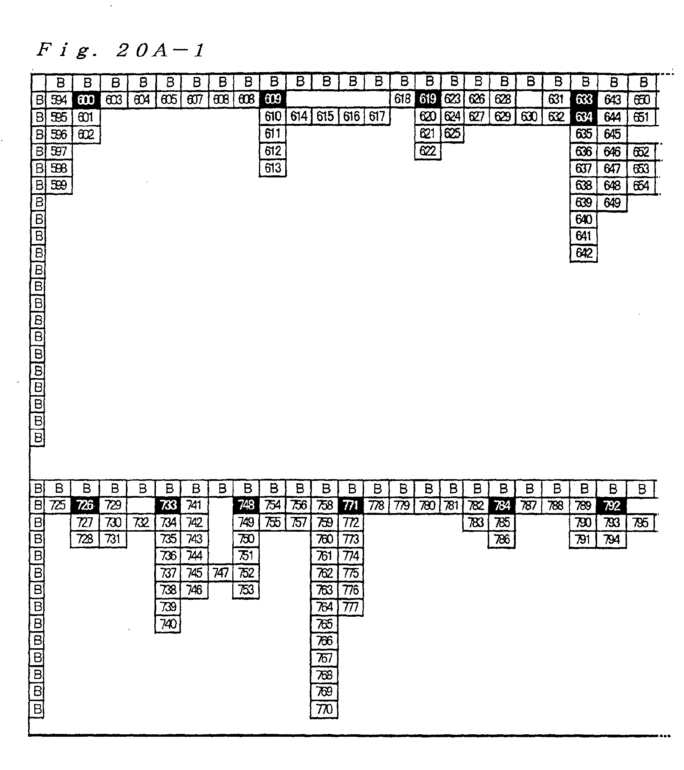

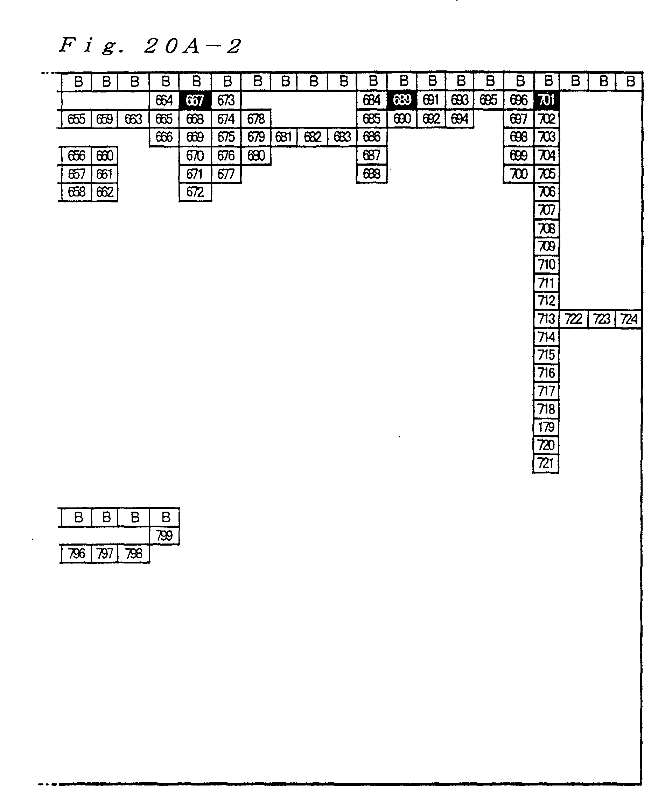

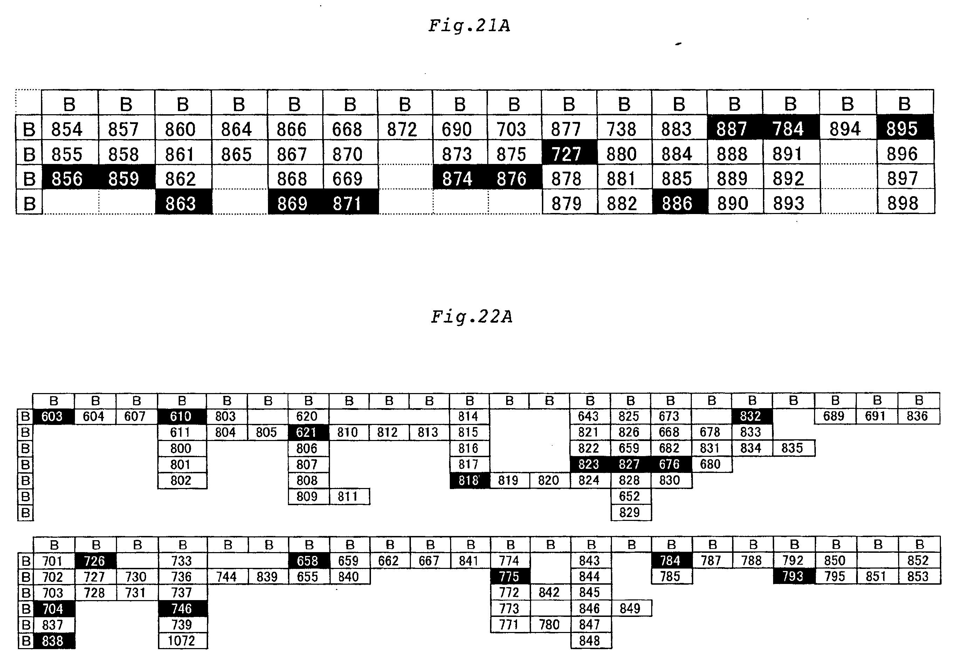

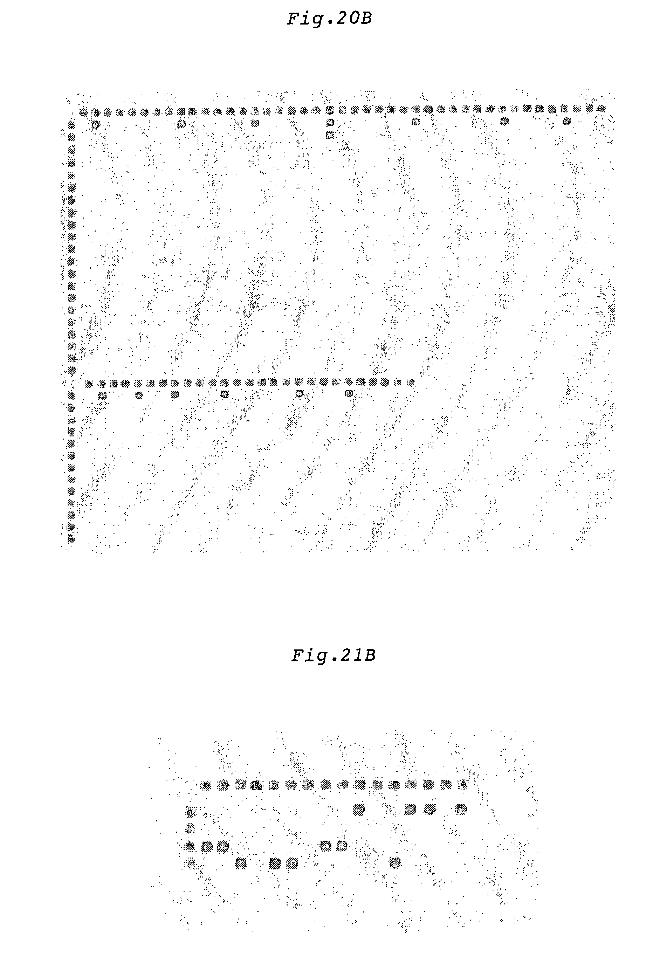

Fig. 20 shows a schematic view of the kit of the present invention (A) and a photograph showing detection result of DRB1 typing using the same (detection result typed as DRB1*0101) (B).
Fig. 21 shows a schematic view of the kit of the present invention (A) and a photograph showing detection result of DRB9 typing using the same (detection result typed as DRB9 0101) (B).

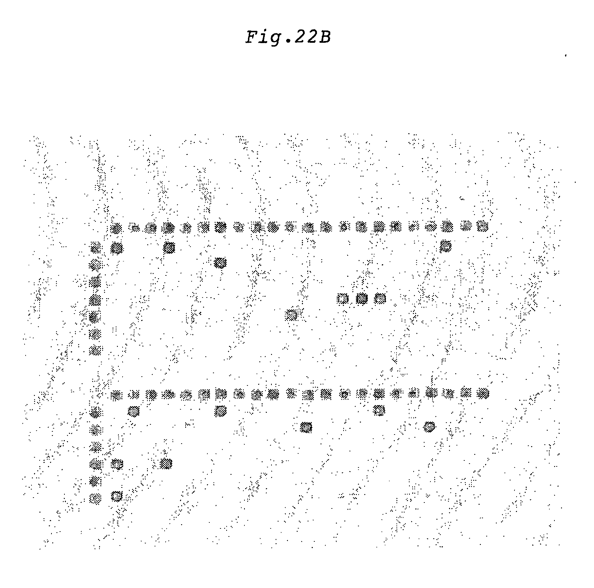

Fig. 22 shows a schematic view of the kit of the present invention (A) and a photograph showing detection result of DRB3 typing using the same (detection result typed as DRB3 0301) (B).

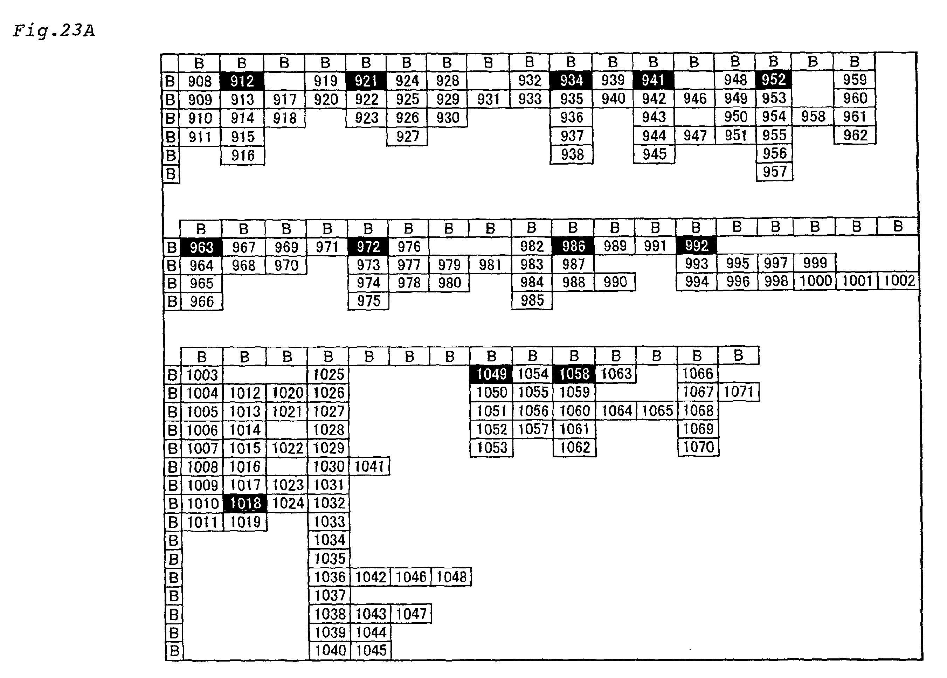

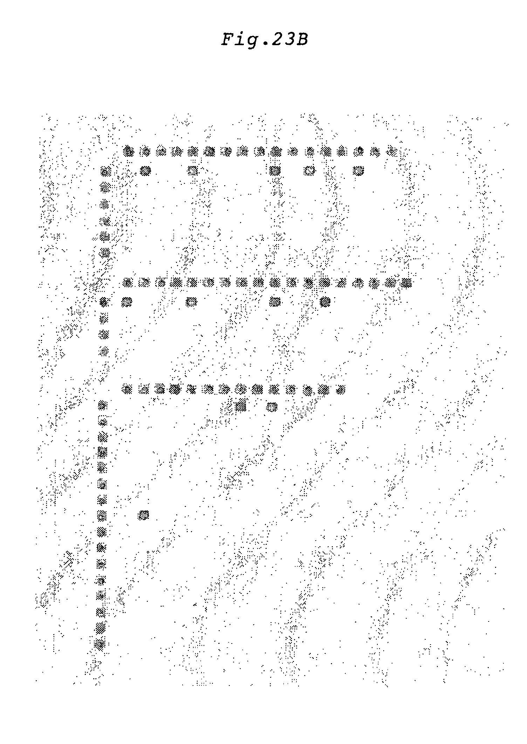

Fig. 23 shows a schematic view of the kit of the present invention (A) and a photograph showing detection result of A typing using the same (detection result typed as A 01011) (B).

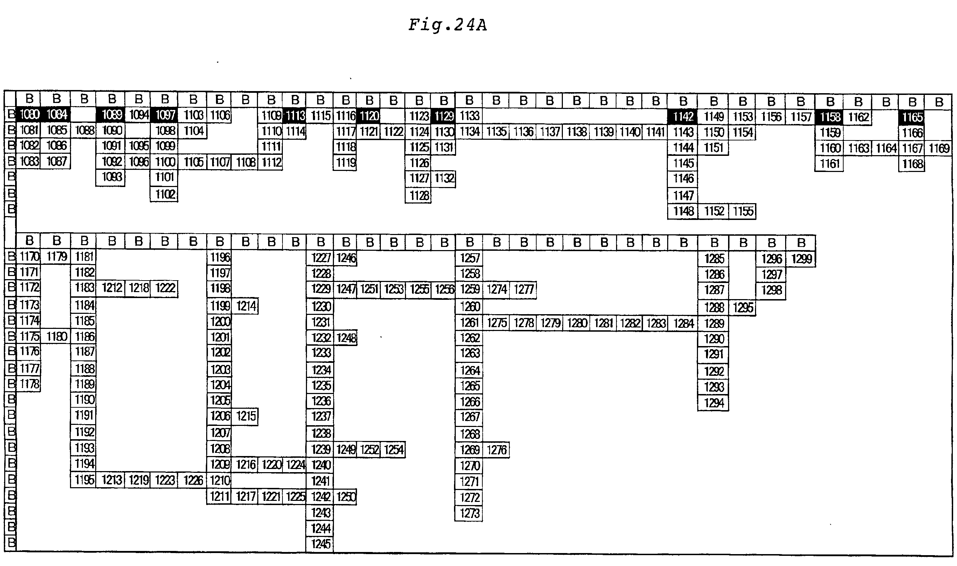

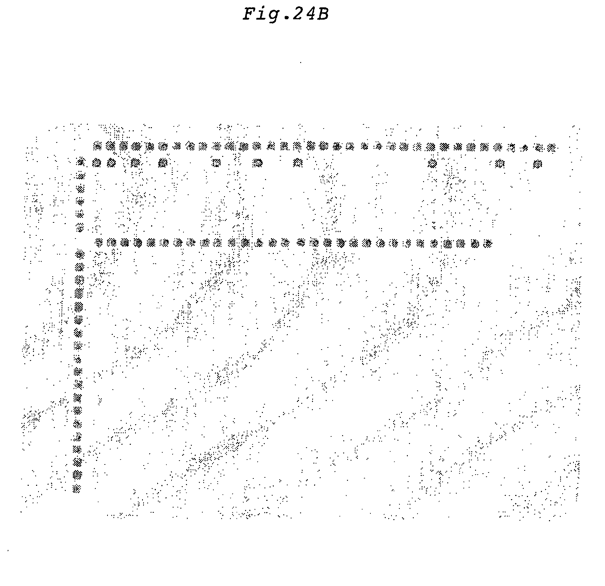

Fig. 24 shows a schematic view of the kit of the present invention (A) and a photograph showing detection result of B typing using the same (detection result typed as B 0724) (B).

### Best Mode for Carrying out the Invention

Hereafter, the present invention will be explained more specifically with reference to the following examples.

### Example 1. Synthesis of oligonucleotides

According to a usual method, oligonucleotides having an amino group or hydroxyl group at 5' end were synthesized by using an oligonucleotide synthesizer (Perkin-Elmer Applied Biosystems), deprotected and dried. These dried oligonucleotides were dissolved in a buffer of 10 mM Tris-HCl (pH 7.5) and 1 mM EDTA to prepare 100 pmol/µL solutions of oligonucleotides. This synthesis method can be employed both for oligonucleotides used as capture oligos and those used as primers. The nucleotide sequences of the synthesized oligonucleotides are listed in Sequence Listing as SEQ ID NOS: 1-447. Among these, SEQ ID NOS: 1-397 are capture oligos and SEQ ID NOS: 398-447 are primers.

### Example 2. Spotting of capture oligos on substrate (utilizing oligonucleotide having amino group at 5' end)

In an amount of 10 µL of a microspotting solution (TeleChem International Inc.) was mixed with 10 µL of a solution of oligonucleotide having an amino group at 5' end and added onto a microtiter plate (Greiner Laboratory Inc.). Silanized slide glass (Matsunami Glass Ind. Ltd.) was placed at a predetermined position in a spotting machine and then the spotting machine was operated. After the spotting was completed, the slide glass was exposed to vapor from hot water for a few seconds and then irradiated with ultraviolet ray of 30 mJ. After exposed to vapor again for a few seconds, the slide glass was placed on a hot plate to remove moisture. The slide glass was rinsed with 0.1% sodium dodecyl sulfate aqueous solution and then with distilled water. The slide glass was blocked by immersing it in 100 mM Tris-HCl (pH 7.5), 100 mM NaCl and 0.1% Triton X-100 containing 3% bovine serum albumin (BSA) at room temperature for 30 minutes. Then, after dried at room temperature, the slide glass was washed with a buffer of 10 mM Tris-HCl (pH7.5) and 1 mM EDTA. The slide glass was dried at room temperature again and maintained in a dry state in a dark cold place until use.

### Example 3. Spotting of capture oligos on substrate (utilizing oligonucleotide having hydroxyl group at 5' end)

In an amount of 10 µL of a microspotting solution (2 M sodium chloride aqueous solution) was mixed with 10 µL of a solution of oligonucleotide having a hydroxyl group at 5' at end and added onto a microtiter plate (Greiner Laboratory Inc.). Silanized slide glass (Matsunami Glass Ind. Ltd.) was placed at a predetermined position in a spotting machine and then the spotting machine was operated. After the spotting was completed, the slide glass was placed in a drier at 37°C for 30 minutes. The slide glass was blocked by immersing it in 100 mM Tris-HCl (pH 7.5), 100 mM NaCl and 0.1% Triton X-100 containing 3% bovine serum albumin (BSA) at room temperature for 30 minutes. Then, after dried at room temperature, the slide glass was washed with a buffer of 10 mM Tris-HCl (pH7.5) and 1 mM EDTA. The slide glass was dried at room temperature again and maintained in a dry state in a dark cold place until use.

### Example 4. Preparation of nucleic acid target

Nucleated cells were collected from peripheral blood, cultured cells or the like, placed in a microtube and washed 3 times with physiological saline. The cells were added and mixed with 800 µl of 0.1 M Tris-HCl (pH 8.0), 0.2 M NaCl, 0.04 M EDTA and 2% SDS, then added with 120 µg of proteinase K and incubated overnight at 50°C. The mixture was added with 800 µl of 1 M Tris-HCl saturated phenol was added and rotated on a rotator for 1 hour or longer, and extracted 3 times with phenol to remove proteins. The aqueous layer was added with 2 ml of 2-propanol and 60 µl of 5 M NaCl and gently mixed, and then the precipitated DNA was quickly sucked up with a micropipette. The DNA was washed 3 times by centrifugation with addition of 70% cold ethanol. The DNA was dried under reduced pressure and dissolved in 70 µl of sterilized water to prepare a PCR template solution.

The composition for PCR amplification consisted of 2 units of Taq polymerase, 25 pmol each of primers , 5 µl of reaction buffer, 10 nmol each of dNTP, 0.5 µl of template DNA solution and sterilized water giving a total volume of 50 µl. The solution placed in a tube was set on a thermal cycler. A program was operated such that, out of a cycle of (1) 95°C, 3 minutes, (2) 95°C, 30 seconds, (3) 71°C, 30 seconds and (4) 72°C, 3 minutes, (2) and (3) should be repeated 30 times.

In this example, agarose gel electrophoresis described below was performed as a confirmation test, but is not required in identification in an actual clinical case. In an amount of 1 µl of the PCR reaction mixture was taken and mixed with 1 µl of 6 x migration pigment (30% glycerol, 0.25% Bromophenol Blue, 0.25% xylene cyanol) and 4 µl of distilled water. After electrophoresis was performed on 2% agarose gel for 90 minutes at 100 V, the gel was immersed in distilled water containing 0.5 µg/ml of ethidium bromide for 30 minutes and photographed under ultraviolet ray irradiation by using a CCD camera.

### Example 5. Hybridization

In an amount of 2 µl of the nucleic acid target prepared in Example 4 was taken and mixed with 8 µl of ArrayIt Unihyb Hybridization Solution (TeleChem International Inc.), subjected to a heat treatment at 100°C for 10 minutes, and then immersed in ice for 5 minutes. In an amount of 5 µl of this nucleic acid target solution was taken and placed on a substrate on which the capture oligos prepared in Example 2 or 3 were immobilized and then cover glass was placed thereon. This substrate was placed in a moisture box, further placed in an incubator set at 42°C and left for 60 minutes. The substrate was taken out from the incubator and quickly immersed in 5 x SSC (0.083 M NaCl and 0.083 M sodium citrate) at 4°C to remove the cover glass. Immersion of the substrate in 5 x SSC at 4°C for 10 minutes was repeated twice and then the substrate was rinsed twice with 3 M tetramethylammonium chloride aqueous solution at room temperature. Immersion of the substrate in 3 M tetramethylammonium chloride aqueous solution at 45°C for 20 minutes was repeated twice. Finally, the substrate was immersed in 2 x SSC (0.033 M NaCl and 0.033 M sodium citrate).

### Example 6. Detection of chemical color development

After hybridization was completed in Example 5, the substrate was taken out from 2 x SSC. Block Ace (Dainippon Pharmaceutical Co., Ltd.) stock solution (4% aqueous solution) was placed on the substrate in an amount of 70 µl per cm² and left at room temperature for 20 minutes, and then the solution was removed. Subsequently, a mixed solution obtained by adding 2 drops each of avidin DH (Vector Laboratories, Inc.) and biotinylated horseradish peroxidase H (Vector Laboratories, Inc.) to 5 ml of TBST (0.05 M Tris-HCl (pH 7.6), 0.15 M NaCl, 0.05% Tween 20) and mixing them was placed on the substrate in an amount of 70 µl per cm² and left at room temperature for 30 minutes, and then the solution was removed. After immersion of the substrate in TBST at room temperature for 5 minutes was repeated twice, the substrate was taken out from the buffer and the moisture was sufficiently removed with paper towel. A TMB (3,3',5,5'-tetramethylbenzidine) color development substrate kit (Vector Laboratories, Inc.) was placed on the substrate in amount of 70 µl per cm² and left at room temperature for 10 minutes, and then the solution was removed. The substrate was immersed in deionized water to stop the enzymatic reaction.

The results of the above examples are shown in Figs. 8-10. It is evident that HLA typing can be carried out by the method of the present invention.

According to the present invention, there are provided a kit and method that enable multiple specimen processing and a highly precise HLA typing with one time of test as well as oligonucleotides and primers for use in the same.

### Example 7. Typing using Patchworks

Oligonucleotides used for preparation of arrays were synthesized in a similar manner as Example 1. Arrays were prepared in a similar manner as Example 2 or 3.
Correspondence of the sequences of oligonucleotides used in arrays for typing HLA and HLA types are shown in Tables 21-32. For amplifying targets, combinations of primers shown in Table 33 were used. Typing was performed in a similar manner as Examples 4-6.

The results are shown in Figs. 18-24. Thus, HLA typing of the test specimen can be performed with the arrays which were prepared using oligonucleotides designed according to patchworks and satellites.

**Table 21**

| Correspondence of the patchwork regions and satellite regions for types of DPB1 | | | | | | | | | | | |
|---|---|---|---|---|---|---|---|---|---|---|---|
| | patchwork region | | | | | | | satellite region | | | |
| DPB1‡01011 | DPB1 | DPB4 | DPB7 | DPB12 | DPB15 | DPB18 | DPB21 | | | | |
| DPB1‡6501 | DPB1 | DPB4 | DPB7 | DPB12 | DPB15 | DPB18 | DPB21 | | | | |
| DPB1‡01012 | DPB1 | DPB4 | DPB7 | DPB12 | DPB15 | DPB18 | DPB21 | 473 | | | |
| DPB1‡8901 | DPB1 | DPB4 | DPB7 | DPB12 | DPB15 | DPB19 | DPB21 | | | | |
| DPB1‡1301 | DPB2 | DPB4 | DPB7 | DPB12 | DPB16 | DPB20 | DPB21 | | | | |
| DPB1‡26011 | DPB2 | DPB4 | DPB7 | DPB12 | DPB15 | DPB18 | DPB21 | 473 | 495 | | |
| DPB1‡26012 | DPB2 | DPB4 | DPB7 | DPB12 | DPB15 | DPB18 | DPB21 | | | | |
| DPB1‡2701 | DPB2 | DPB4 | DPB7 | DPB12 | DPB15 | DPB19 | DPB21 | | | | |
| DPB1‡8501 | DPB2 | DPB4 | DPB7 | DPB12 | DPB15 | DPB19 | DPB21 | | | | 456 |
| DPB1‡3901 | DPB1 | DPB4 | DPB7 | DPB12 | DPB15 | DPB19 | DPB22 | | | | |
| DPB1‡4001 | DPB1 | DPB4 | DPB7 | DPB12 | DPB15 | DPB19 | DPB23 | | | | |
| DPB1‡0401 | DPB1 | DPB3 | DPB7 | DPB12 | DPB15 | DPB19 | DPB22 | | | | |
| DPB1‡3301 | DPB1 | DPB3 | DPB7 | DPB12 | DPB16 | DPB19 | DPB22 | | | | |
| DPB1‡6601 | DPB2 | DPB3 | DPB7 | DPB12 | DPB15 | DPB19 | DPB22 | | | | 456 |
| DPB1‡2301 | DPB1 | DPB5 | DPB7 | DPB12 | DPB15 | DPB19 | DPB22 | | | | |
| DPB1‡7101 | DPB1 | DPB5 | DPB7 | DPB12 | DPB16 | DPB19 | DPB22 | | | | |
| DPB1‡5501 | DPB2 | DPB5 | DPB7 | DPB12 | DPB16 | DPB19 | DPB21 | | | | 456 |
| DPB1‡5801 | DPB2 | DPB6 | DPB7 | DPB12 | DPB16 | DPB19 | DPB21 | | | | 456 |
| DPB1‡6301 | DPB1 | DPB6 | DPB7 | DPB12 | DPB15 | DPB19 | DPB21 | | | | |
| DPB1‡6201 | DPB1 | DPB6 | DPB7 | DPB12 | DPB15 | DPB19 | DPB23 | | | | |
| DPB1‡1601 | DPB1 | DPB4 | DPB8 | DPB12 | DPB16 | DPB19 | DPB21 | | | | |
| DPB1‡4901 | DPB1 | DPB4 | DPB8 | DPB12 | DPB15 | DPB19 | DPB22 | | | | |
| DPB1‡5301 | DPB1 | DPB4 | DPB8 | DPB12 | DPB15 | DPB19 | DPB23 | | | | |
| DPB1‡0801 | DPB1 | DPB5 | DPB8 | DPB12 | DPB16 | DPB18 | DPB21 | | | | |
| DPB1‡6801 | DPB1 | DPB5 | DPB8 | DPB12 | DPB15 | DPB18 | DPB21 | | | | |
| DPB1‡7501 | DPB1 | DPB5 | DPB8 | DPB12 | DPB15 | DPB18 | DPB22 | | | | |
| DPB1‡02012 | DPB1 | DPB5 | DPB8 | DPB12 | DPB16 | DPB19 | DPB22 | | | | |
| DPB1‡3201 | DPB1 | DPB5 | DPB8 | DPB12 | DPB16 | DPB19 | DPB22 | 481 | | | |
| DPB1‡4601 | DPB1 | DPB5 | DPB8 | DPB12 | DPB16 | DPB19 | DPB22 | 482 | | | |
| DPB1‡6001 | DPB1 | DPB5 | DPB8 | DPB12 | DPB15 | DPB19 | DPB22 | 487 | | | |
| DPB1‡7701 | DPB1 | DPB5 | DPB8 | DPB12 | DPB15 | DPB19 | DPB22 | 464 | | | |
| DPB1‡8001 | DPB1 | DPB5 | DPB8 | DPB12 | DPB15 | DPB19 | DPB22 | | | | |
| DPB1‡8201 | DPB1 | DPB5 | DPB8 | DPB12 | DPB15 | DPB19 | DPB22 | | | | |
| DPB1‡0901 | DPB2 | DPB5 | DPB8 | DPB12 | DPB16 | DPB18 | DPB21 | 482 | | | 456 |
| DPB1‡1001 | DPB2 | DPB5 | DPB8 | DPB12 | DPB16 | DPB18 | DPB21 | | | | 456 |
| DPB1‡3701 | DPB2 | DPB5 | DPB8 | DPB12 | DPB16 | DPB18 | DPB21 | | | | |
| DPB1‡3501 | DPB2 | DPB5 | DPB8 | DPB12 | DPB15 | DPB18 | DPB21 | 482 | | | 456 |
| DPB1‡7901 | DPB2 | DPB5 | DPB8 | DPB12 | DPB15 | DPB18 | DPB21 | | | | |
| DPB1‡8801 | DPB2 | DPB5 | DPB8 | DPB12 | DPB16 | DPB18 | DPB21 | 482 | | | |
| DPB1‡1701 | DPB2 | DPB5 | DPB8 | DPB12 | DPB16 | DPB19 | DPB21 | 482 | | | 456 |
| DPB1‡0402 | DPB2 | DPB5 | DPB8 | DPB12 | DPB15 | DPB19 | DPB22 | | | | |
| DPB1‡1801 | DPB1 | DPB5 | DPB8 | DPB12 | DPB15 | DPB19 | DPB23 | | | | |
| DPB1‡4801 | DPB1 | DPB6 | DPB8 | DPB12 | DPB16 | DPB19 | DPB22 | | | | |
| DPB1‡5101 | DPB1 | DPB3 | DPB8 | DPB12 | DPB15 | DPB19 | DPB22 | | | | |
| DPB1‡8101 | DPB1 | DPB3 | DPB8 | DPB12 | DPB16 | DPB19 | DPB22 | | | | |
| DPB1‡02013 | DPB1 | DPB5 | DPB9 | DPB12 | DPB16 | DPB19 | DPB22 | | | | |
| DPB1‡4701 | DPB1 | DPB5 | DPB10 | DPB12 | DPB16 | DPB19 | DPB22 | | | | |
| DPB1‡1901 | DPB1 | DPB5 | DPB10 | DPB12 | DPB16 | DPB20 | DPB21 | | | | |
| DPB1‡3001 | DPB2 | DPB5 | DPB10 | DPB12 | DPB16 | DPB19 | DPB21 | | | | 456 |
| DPB1‡5401 | DPB2 | DPB5 | DPB10 | DPB12 | DPB16 | DPB18 | DPB21 | | | | 456 |
| DPB1‡0202 | DPB1 | DPB6 | DPB10 | DPB12 | DPB16 | DPB19 | DPB22 | | | | |
| DPB1‡2201 | DPB1 | DPB6 | DPB10 | DPB12 | DPB16 | DPB19 | DPB21 | | | | |
| DPB1‡0501 | DPB1 | DPB6 | DPB10 | DPB12 | DPB15 | DPB19 | DPB21 | | | | |
| DPB1‡3801 | DPB1 | DPB6 | DPB10 | DPB12 | DPB15 | DPB19 | DPB21 | 465 | | | |
| DPB1‡2101 | DPB2 | DPB6 | DPB10 | DPB12 | DPB16 | DPB19 | DPB21 | | | | |
| DPB1‡3601 | DPB2 | DPB6 | DPB10 | DPB12 | DPB15 | DPB19 | DPB21 | | | | |
| DPB1‡2401 | DPB1 | DPB3 | DPB10 | DPB12 | DPB15 | DPB19 | DPB22 | | | | |
| DPB1‡8401 | DPB1 | DPB5 | DPB11 | DPB12 | DPB15 | DPB18 | DPB22 | | | | |
| DPB1‡11011 | DPB2 | DPB4 | DPB7 | DPB13 | DPB17 | DPB19 | DPB21 | 467 | 473 | | |
| DPB1‡11012 | DPB2 | DPB4 | DPB7 | DPB13 | DPB17 | DPB19 | DPB21 | 466 | 467 | 473 | |
| DPB1‡1501 | DPB1 | DPB4 | DPB7 | DPB13 | DPB17 | DPB19 | DPB23 | 467 | 473 | | |
| DPB1‡7401 | DPB2 | DPB4 | DPB7 | DPB13 | DPB17 | DPB19 | DPB23 | 467 | 473 | | |
| DPB1‡3101 | DPB1 | DPB3 | DPB7 | DPB13 | DPB15 | DPB19 | DPB21 | | | | |
| DPB1‡5601 | DPB2 | DPB3 | DPB7 | DPB13 | DPB15 | DPB18 | DPB21 | | | | |
| DPB1‡7201 | DPB1 | DPB3 | DPB7 | DPB13 | DPB15 | DPB19 | DPB22 | | | | |
| DPB1‡3401 | DPB1 | DPB6 | DPB7 | DPB13 | DPB15 | DPB19 | DPB23 | 493 | | | |
| DPB1‡5201 | DPB2 | DPB5 | DPB7 | DPB13 | DPB15 | DPB18 | DPB21 | | | | |
| DPB1‡03012 | DPB2 | DPB5 | DPB8 | DPB13 | DPB15 | DPB18 | DPB21 | 482 | 503 | | |
| DPB1‡1401 | DPB2 | DPB5 | DPB8 | DPB13 | DPB15 | DPB18 | DPB21 | 482 | | | 456 |
| DPB1‡6101N | DPB2 | DPB5 | DPB8 | DPB13 | DPB15 | DPB18 | DPB2 | 482 | | | |
| DPB1‡7001 | DPB2 | DPB5 | DPB8 | DPB13 | DPB15 | DPB18 | DPB21 | 482 | | | 457 |
| DPB1‡2501 | DPB2 | DPB5 | DPB8 | DPB13 | DPB15 | DPB18 | DPB21 | | | | |
| DPB1‡4501 | DPB2 | DPB5 | DPB8 | DPB13 | DPB15 | DPB18 | DPB21 | | | | 456 |
| DPB1‡6701 | DPB2 | DPB5 | DPB7 | DPB13 | DPB15 | DPB18 | DPB21 | | | | 456 |
| DPB1‡7801 | DPB2 | DPB5 | DPB8 | DPB13 | DPB15 | DPB18 | DPB21 | 482 | | | |
| DPB1‡2901 | DPB2 | DPB5 | DPB8 | DPB13 | DPB16 | DPB18 | DPB21 | 482 | | | |
| DPB1‡0601 | DPB2 | DPB5 | DPB8 | DPB13 | DPB16 | DPB19 | DPB21 | 482 | | | |
| DPB1‡20011 | DPB2 | DPB5 | DPB8 | DPB13 | DPB15 | DPB19 | DPB21 | 482 | | | |
| DPB1‡20012 | DPB2 | DPB5 | DPB8 | DPB13 | DPB15 | DPB19 | DPB21 | 482 | | | |
| DPB1‡6401N | DPB2 | DPB5 | DPB8 | DPB13 | DPB16 | DPB19 | DPB21 | | | | |
| DPB1‡6901 | DPB2 | DPB5 | DPB8 | DPB13 | DPB17 | DPB19 | DPB21 | 482 | | | |
| DPB1*5001 | DPB1 | DPB5 | DPB8 | DPB13 | DPB15 | DPB18 | DPB21 | 482 | | | |
| DPB1‡5701 | DPB1 | DPB5 | DPB8 | DPB13 | DPB15 | DPB18 | DPB21 | 482 | | | |
| DPB1‡5901 | DPB1 | DPB5 | DPB8 | DPB13 | DPB15 | DPB19 | DPB22 | | | | |
| DPB1‡7301 | DPB1 | DPB5 | DPB8 | DPB13 | DPB15 | DPB18 | DPB22 | | | | |
| DPB1‡8701 | DPB2 | DPB5 | DPB7 | DPB13 | DPB15 | DPB19 | DPB22 | | | | |
| DPB1‡2801 | DPB1 | DPB3 | DPB8 | DPB13 | DPB15 | DPB19 | DPB23 | | | | |
| DPB1‡7601 | DPB2 | DPB3 | DPB8 | DPB13 | DPB15 | DPB18 | DPB21 | 482 | | | 456 |
| DPB1‡4401 | DPB2 | DPB6 | DPB8 | DPB13 | DPB16 | DPB18 | DPB21 | 482 | | | |
| DPB1‡03011 | DPB2 | DPB5 | DPB10 | DPB13 | DPB15 | DPB18 | DPB21 | 482 | | | |
| DPB1‡4101 | DPB1 | DPB5 | DPB8 | DPB14 | DPB16 | DPB19 | DPB22 | | | | |
| DPB1‡8301 | DPB1 | DPB5 | DPB8 | DPB14 | DPB15 | DPB19 | DPB22 | | | | |
| DPB1‡8601 | DPB2 | DPB5 | DPB8 | DPB9 | DPB16 | DPB19 | DPB22 | 482 | | | |

**Table 22.**

| Correspondence of Patchwork DPB1 and SEQ ID NOS. for DPB1 | | | |
|---|---|---|---|
| Patchwork | SEQ ID NOS. | | |
| F | 458 | 460 | 462 |
| DPB2 | 459 | 461 | 463 |
| DPB3 | 468 | | |
| DPB4 | 469 | 472 | |
| DPB5 | 470 | | |
| DPB6 | 471 | | |
| DPB7 | 474 | | |
| DPB8 | 475 | 479 | 480 |
| DPB9 | 476 | | |
| DPB10 | 477 | | |
| DPB11 | 478 | | |
| DPB12 | 483 | | |
| DPB13 | 484 | 486 | |
| DPB14 | 485 | | |
| DPB15 | 489 | 92 | |
| DPB16 | 490 | | |
| DPB17 | 491 | | |
| DPB18 | 496 | 498 | |
| DPB19 | 497 | | |
| DPB20 | 499 | | |
| DPB21 | 500 | | |
| DPB22 | 501 | | |
| DPB23 | 502 | | |

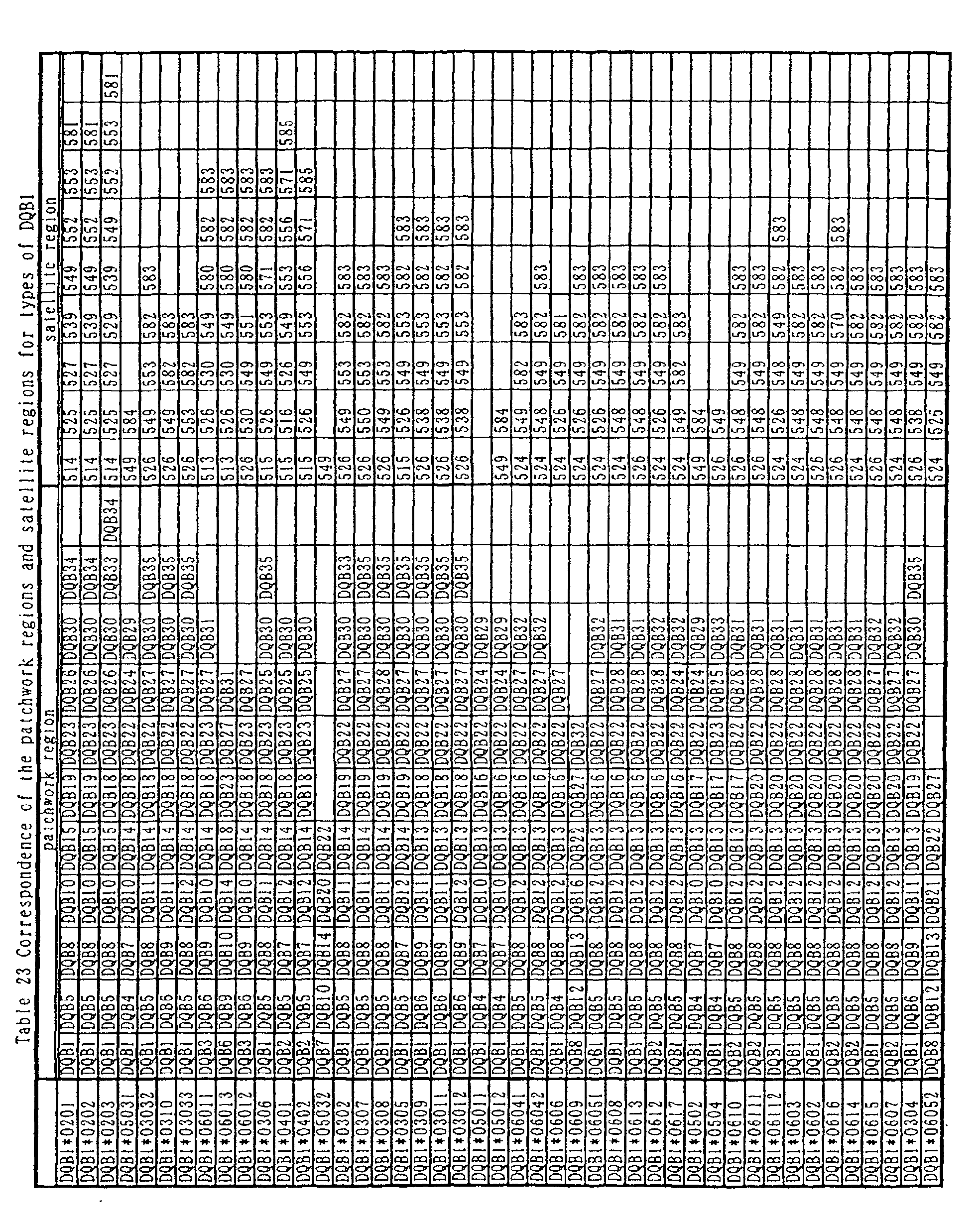

**Table 24**

| Correspondence of the patchworks and SEQ ID Nos for DQB1 | | | | | | |
|---|---|---|---|---|---|---|
| patchwork SEQ ID NO: | | | | | | |
| DQB1 | 507 | | | | | |
| DQB2 | 508 | | | | | |
| DQB3 | 509 | | | | | |
| DQB4 | 510 | | | | | |
| DQB5 | 511 | | | | | |
| DQB6 | 512 | | | | | |
| DQB7 | 517 | 520 | 522 | | | |
| DQB8 | 518 | 521 | 523 | | | |
| DQB9 | 519 | | | | | |
| DQB10 | 533 | 536 | 537 | | | |
| DQB11 | 534 | | | | | |
| DQB12 | 535 | | | | | |
| DQB13 | 540 | 543 | 544 | 545 | 546 | 547 |
| DQB14 | 541 | | | | | |
| DQB15 | 542 | | | | | |
| DQB16 | 559 | | | | | |
| DQB17 | 560 | | | | | |
| DQB18 | 561 | 565 | 568 | 569 | | |
| DQB19 | 562 | 566 | | | | |
| DQB20 | 563 | 567 | | | | |
| DQB21 | 564 | | | | | |
| DQB22 | 572 | | | | | |
| DQB23 | 573 | | | | | |
| DQB24 | 574 | | | | | |
| DQB25 | 575 | | | | | |
| DQB26 | 576 | | | | | |
| DQB27 | 577 | 579 | | | | |
| DQB28 | 578 | | | | | |
| DQB29 | 586 | | | | | |
| DQB30 | 587 | | | | | |
| DQB31 | 588 | | | | | |
| DQB32 | 589 | | | | | |
| DQB33 | 528 | 532 | | | | |
| DQB34 | 554 | 557 | | | | |
| DQB35 | 555 | 558 | | | | |

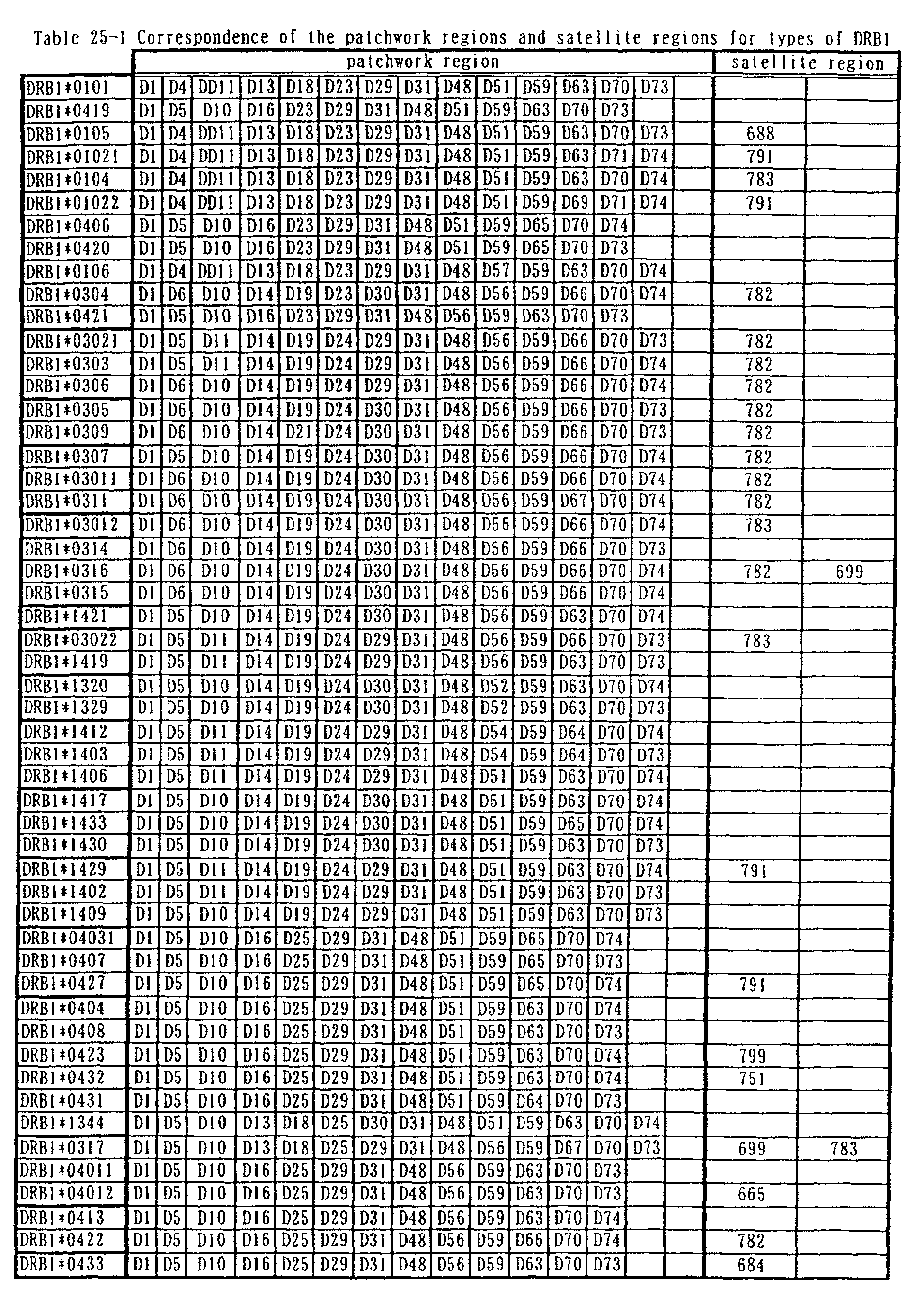

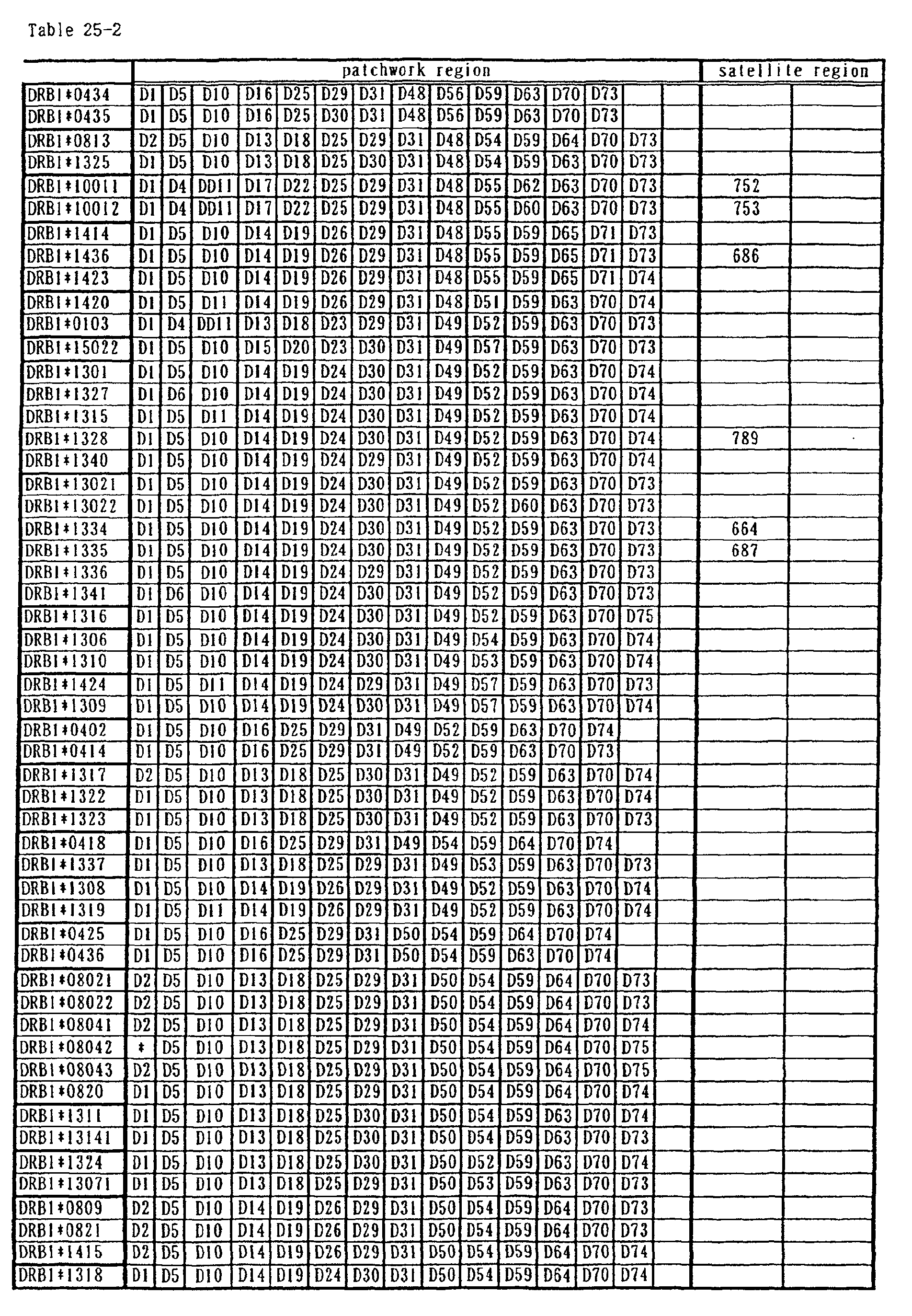

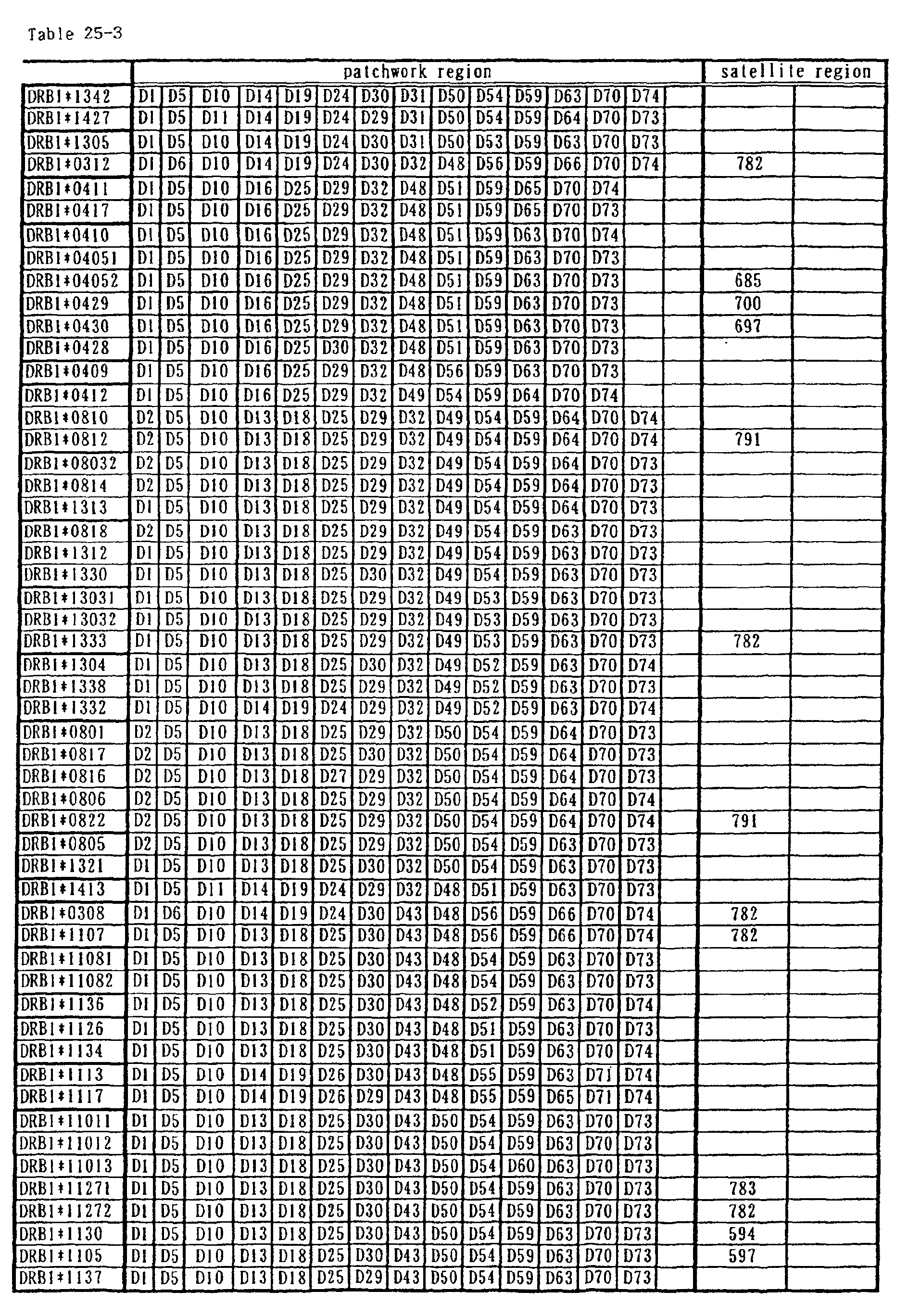

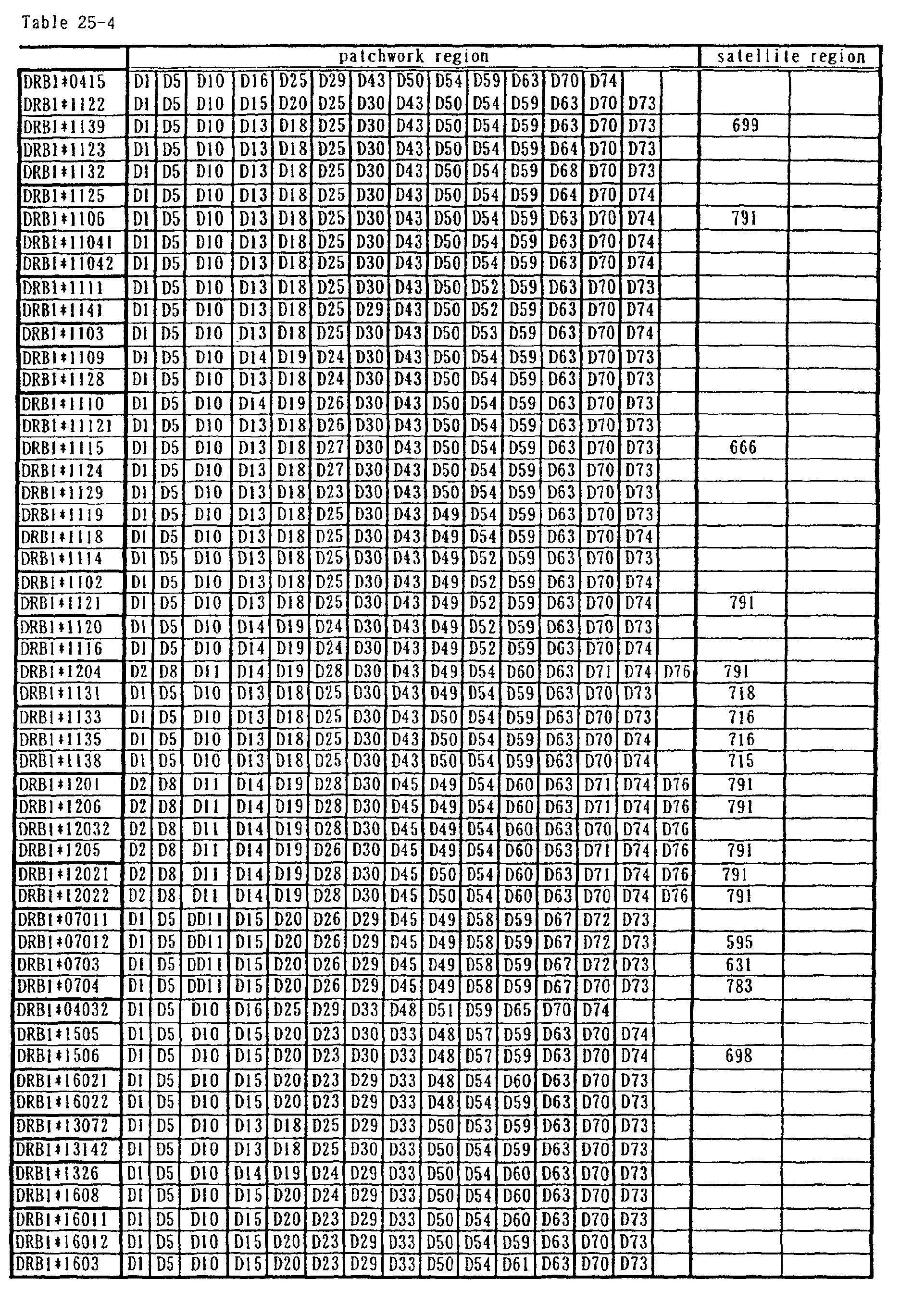

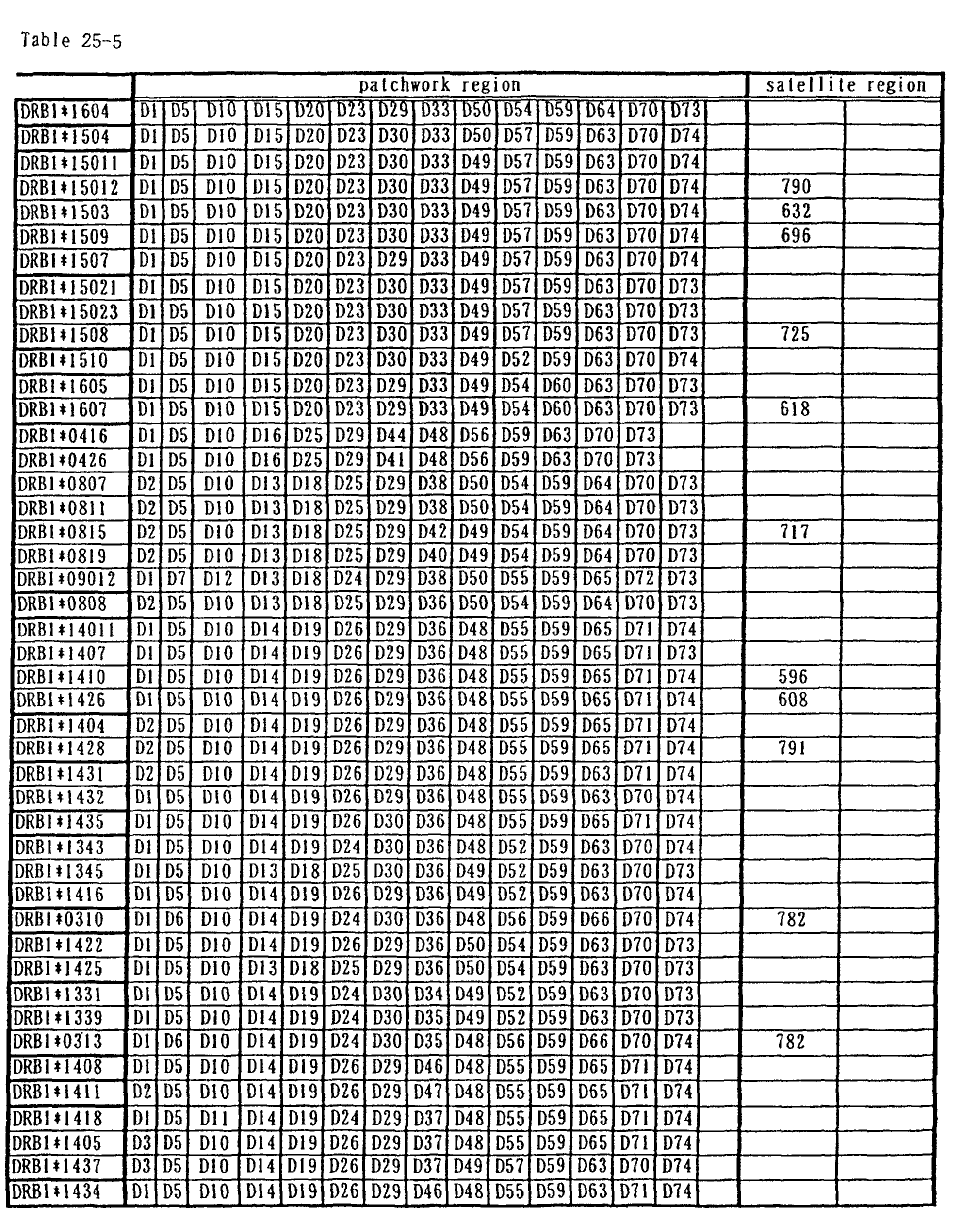

**Table 26**

| Correspondence of the patchworks and SEQ ID Nos for DRB1 | | | | | | | | | | | | | | | |
|---|---|---|---|---|---|---|---|---|---|---|---|---|---|---|---|
| patchwork | SEQ ID NO: | | | | | | | | | | | | | | |
| D1 | 600 | 603 | 604 | 605 | 606 | 607 | | | | | | | | | |
| D2 | 601 | | | | | | | | | | | | | | |
| D3 | 602 | | | | | | | | | | | | | | |
| D4 | 609 | | | | | | | | | | | | | | |
| D5 | 610 | 614 | 615 | 616 | 617 | | | | | | | | | | |
| D6 | 611 | | | | | | | | | | | | | | |
| D7 | 612 | | | | | | | | | | | | | | |
| D8 | 613 | | | | | | | | | | | | | | |
| D9 | 619 | 623 | 626 | 627 | | | | | | | | | | | |
| D10 | 620 | 624 | 627 | 629 | 630 | | | | | | | | | | |
| D11 | 621 | 625 | | | | | | | | | | | | | |
| D12 | 622 | | | | | | | | | | | | | | |
| D13 | 633 | 643 | 650 | | | | | | | | | | | | |
| D14 | 635 | 645 | | | | | | | | | | | | | |
| D15 | 637 | 647 | 653 | 657 | 661 | | | | | | | | | | |
| D16 | 639 | 649 | | | | | | | | | | | | | |
| D17 | 641 | | | | | | | | | | | | | | |
| D18 | 634 | 644 | 651 | 655 | 659 | 663 | | | | | | | | | |
| D19 | 636 | 646 | 652 | 656 | 660 | | | | | | | | | | |
| D20 | 638 | 648 | 654 | 658 | 662 | | | | | | | | | | |
| D21 | 640 | | | | | | | | | | | | | | |
| D22 | 642 | | | | | | | | | | | | | | |
| D23 | 667 | 673 | | | | | | | | | | | | | |
| D24 | 668 | 674 | 678 | | | | | | | | | | | | |
| D25 | 669 | 675 | 679 | 681 | 682 | 683 | | | | | | | | | |
| D26 | 670 | 676 | 680 | | | | | | | | | | | | |
| 027 | 671 | 677 | | | | | | | | | | | | | |
| D28 | 672 | | | | | | | | | | | | | | |
| D29 | 689 | 691 | 693 | 695 | | | | | | | | | | | |
| D30 | 690 | 692 | 694 | | | | | | | | | | | | |
| D31 | 701 | | | | | | | | | | | | | | |
| D32 | 702 | | | | | | | | | | | | | | |
| D33 | 703 | | | | | | | | | | | | | | |
| D34 | 704 | | | | | | | | | | | | | | |
| D35 | 705 | | | | | | | | | | | | | | |
| D36 | 706 | | | | | | | | | | | | | | |
| D37 | 707 | | | | | | | | | | | | | | |
| D38 | 708 | | | | | | | | | | | | | | |
| D39 | 709 | | | | | | | | | | | | | | |
| D40 | 710 | | | | | | | | | | | | | | |
| D41 | 711 | | | | | | | | | | | | | | |
| D42 | 712 | | | | | | | | | | | | | | |
| D43 | 713 | 722 | 723 | 724 | | | | | | | | | | | |
| D44 | 714 | | | | | | | | | | | | | | |
| D45 | 719 | | | | | | | | | | | | | | |
| D46 | 720 | | | | | | | | | | | | | | |
| D47 | 721 | | | | | | | | | | | | | | |
| D48 | 726 | 729 | | | | | | | | | | | | | |
| D49 | 727 | 730 | 732 | | | | | | | | | | | | |
| D50 | 728 | 731 | | | | | | | | | | | | | |
| D51 | 733 | 741 | | | | | | | | | | | | | |
| D52 | 734 | 742 | | | | | | | | | | | | | |
| D53 | 735 | 743 | | | | | | | | | | | | | |
| D54 | 736 | 744 | | | | | | | | | | | | | |
| D55 | 737 | 745 | 747 | | | | | | | | | | | | |
| D56 | 738 | 746 | | | | | | | | | | | | | |
| D57 | 739 | | | | | | | | | | | | | | |
| D58 | 740 | | | | | | | | | | | | | | |
| D59 | 748 | 754 | 756 | 758 | 759 | 760 | 761 | 762 | 763 | 764 | 765 | 767 | 768 | 769 | 770 |
| D60 | 749 | 755 | 757 | | | | | | | | | | | | |
| D61 | 750 | | | | | | | | | | | | | | |
| D62 | 752 | | | | | | | | | | | | | | |
| D63 | 771 | 778 | 779 | 780 | 781 | | | | | | | | | | |
| D64 | 772 | | | | | | | | | | | | | | |
| D65 | 773 | | | | | | | | | | | | | | |
| D66 | 774 | | | | | | | | | | | | | | |
| D67 | 775 | | | | | | | | | | | | | | |
| D68 | 776 | | | | | | | | | | | | | | |
| D69 | 777 | | | | | | | | | | | | | | |
| D70 | 784 | 787 | 788 | | | | | | | | | | | | |
| D71 | 785 | | | | | | | | | | | | | | |
| D72 | 786 | | | | | | | | | | | | | | |
| D73 | 792 | | | | | | | | | | | | | | |
| D74 | 793 | 795 | 796 | 797 | 798 | | | | | | | | | | |
| D75 | 794 | | | | | | | | | | | | | | |
| D76 | 597 | 598 | 599 | | | | | | | | | | | | |

**Table 27**

| Correspondence of the patchwork regions and satellite regions for types of DRB 3-5 | | | | | | | | | | | | | | | | | | | |
|---|---|---|---|---|---|---|---|---|---|---|---|---|---|---|---|---|---|---|---|
| | patchwork | | | | | | | | | | | | | | | | satellite | | |
| DRB3‡01011 | R1 | R3 | R7 | R17 | R19 | R21 | R25 | R27 | R31 | R32 | R34 | R40 | R43 | R45 | R50 | R52 | 832 | | |
| DRB3‡0101201 | R1 | R3 | R7 | R17 | R19 | R21 | R25 | R27 | R31 | R32 | R34 | R40 | R43 | R45 | R50 | R52 | 832 | | |
| DRB3‡0101202 | R1 | R3 | R7 | R17 | R19 | R21 | R25 | R27 | R31 | R32 | R34 | R40 | R43 | R45 | R50 | R52 | 832 | | |
| DRB3‡01014 | R1 | R3 | R7 | R17 | R19 | R21 | R25 | R27 | R31 | R32 | R34 | R40 | R43 | R45 | R50 | R52 | 832 | 847 | |
| DRB3‡0102 | R1 | R3 | R7 | R17 | R19 | R21 | R25 | R27 | R31 | R32 | R34 | R40 | R43 | R45 | R50 | R52 | 832 | 853 | |
| DRB3‡0103 | R1 | R3 | R8 | R17 | R19 | R21 | R25 | R27 | R31 | R32 | R34 | R40 | R43 | R45 | R50 | R52 | 832 | | |
| DRB3‡0104 | R1 | R3 | R7 | R17 | R19 | R21 | R25 | R27 | R31 | R32 | R34 | R40 | R43 | R45 | R50 | R52 | 832 | 852 | |
| DRB3‡0105 | R1 | R3 | R10 | R17 | R19 | R21 | R25 | R27 | R31 | R32 | R34 | R40 | R43 | R45 | R50 | R52 | 832 | | |
| DRB3‡0106 | R1 | R3 | R7 | R17 | R19 | R21 | R25 | R27 | R31 | R32 | R34 | R40 | R43 | R45 | R50 | R52 | | | |
| DRB3‡0209 | R1 | R2 | R8 | R16 | R19 | R21 | R24 | R27 | R31 | R32 | R34 | R40 | R43 | R46 | R50 | R52 | 832 | | |
| DRB3‡0301 | R1 | R2 | R8 | R17 | R19 | R21 | R25 | R27 | R31 | R32 | R34 | R40 | R43 | R46 | R50 | R53 | | | |
| DRB3‡03012 | R1 | R2 | R8 | R17 | R19 | R21 | R25 | R27 | R31 | R32 | R34 | R40 | R43 | R46 | R50 | R53 | 832 | | |
| DRB3‡0302 | R1 | R2 | R8 | R16 | R19 | R21 | R25 | R27 | R31 | R32 | R34 | R40 | R43 | R46 | R50 | R53 | | | |
| DRB3‡0303 | R1 | R2 | R8 | R17 | R19 | R21 | R25 | R27 | R31 | R32 | R34 | R40 | R43 | R45 | R50 | R52 | | | |
| DRB3‡01013 | R1 | R3 | R7 | R17 | R19 | R21 | R25 | R27 | R32 | R33 | R34 | R40 | R43 | R45 | R50 | R52 | 832 | | |
| DRB3‡0107 | R1 | R3 | R7 | R17 | R19 | R21 | R24 | R27 | R28 | R34 | R40 | R43 | R46 | R50 | R52 | | 834 | 847 | |
| DRB3‡02022 | R1 | R2 | R8 | R16 | R19 | R21 | R24 | R27 | R28 | R34 | R40 | R43 | R46 | R50 | R52 | | 834 | 847 | |
| DRB3‡02010 | R1 | R2 | R8 | R16 | R19 | R21 | R24 | R27 | R28 | R34 | R40 | R43 | R46 | R50 | R52 | | 832 | | |
| DRB3‡02012 | R1 | R2 | R8 | R16 | R19 | R21 | R24 | R27 | R28 | R34 | R40 | R43 | R46 | R50 | R52 | | 832 | 847 | |
| DRB3‡0205 | R1 | R2 | R8 | R17 | R19 | R21 | R24 | R27 | R28 | R34 | R40 | R43 | R46 | R50 | R52 | | 832 | 847 | |
| DRB3‡02021 | R1 | R2 | R8 | R16 | R19 | R21 | R24 | R27 | R28 | R34 | R40 | R43 | R46 | R50 | R52 | | 834 | 847 | |
| DRB3‡02011 | R1 | R2 | R8 | R16 | R19 | R21 | R24 | R27 | R28 | R35 | R40 | R43 | R46 | R50 | R52 | | 832 | 847 | |
| DRB3‡02023 | R1 | R2 | R8 | R16 | R19 | R21 | R24 | R27 | R28 | R34 | R40 | R43 | R46 | R50 | R52 | | 834 | 847 | 845 |
| DRB3‡0201 | R1 | R2 | R8 | R16 | R19 | R21 | R24 | R27 | R28 | R34 | R40 | R43 | R46 | R50 | R53 | | 834 | 847 | |
| DRB3‡0203 | R1 | R2 | R8 | R16 | R19 | R21 | R22 | R27 | R28 | R34 | R40 | R43 | R46 | R50 | R52 | | 847 | | |
| DRB3‡0206 | R1 | R2 | R8 | R16 | R19 | R21 | R23 | R27 | R28 | R34 | R40 | R43 | R46 | R50 | R52 | | 832 | 847 | |
| DRB3‡0204 | R1 | R2 | R8 | R16 | R19 | R21 | R24 | R27 | R28 | R34 | R40 | R43 | R45 | R50 | R52 | | 832 | 847 | |
| DRB3‡02013 | R1 | R4 | R8 | R16 | R19 | R21 | R24 | R27 | R28 | R34 | R40 | R43 | R46 | R50 | R52 | | 832 | 847 | |
| DRB3‡0207 | R1 | R2 | R8 | R16 | R19 | R21 | R24 | R27 | R31 | R34 | R40 | R43 | R46 | R50 | R52 | | 832 | 847 | |
| DRB3‡0208 | R1 | R2 | R8 | R16 | R19 | R21 | R24 | R27 | R29 | R34 | R40 | R43 | R46 | R50 | R52 | | 832 | 847 | |
| DRB4‡0103101 | R1 | R5 | R10 | R14 | R18 | R20 | R24 | R27 | R30 | R34 | R39 | R43 | R48 | R50 | R53 | | | | |
| DRB4‡01032 | R1 | R5 | R10 | R14 | R18 | R20 | R24 | R27 | R30 | R34 | R39 | R43 | R48 | R50 | R53 | | | | |
| DRB4‡01011 | R1 | R5 | R10 | R14 | R18 | R20 | R24 | R27 | R30 | R34 | R39 | R43 | R48 | R50 | R53 | | | | |
| DRB4‡01033 | R1 | R5 | R10 | R14 | R18 | R20 | R24 | R27 | R30 | R34 | R39 | R43 | R48 | R51 | R53 | | | | |
| DRB4‡0102 | | R5 | R10 | R14 | R18 | R20 | R24 | R27 | R30 | R34 | R39 | R43 | R48 | R50 | R53 | | 843 | | |
| DRB4‡0104 | R1 | R5 | R10 | R14 | R18 | R20 | R24 | R27 | R30 | R34 | R39 | R43 | R48 | R50 | R53 | | 844 | | |
| DRB4‡0105 | R1 | R5 | R10 | R14 | R18 | R20 | R24 | R27 | R30 | R34 | R39 | R43 | R48 | R50 | R53 | | | | |
| DRB4‡0201N | R1 | R6 | R11 | R14 | R18 | R20 | R24 | R27 | R30 | R34 | R39 | R43 | R48 | R50 | R53 | | | | |
| DRB5‡01011 | R1 | R2 | R12 | R15 | R18 | R20 | R26 | R27 | R30 | R36 | R38 | R44 | R49 | R50 | R52 | | 833 | | |
| DRB5‡01012 | R1 | R2 | R12 | R15 | R18 | R20 | R26 | R27 | R30 | R36 | R38 | R43 | R49 | R50 | R52 | | 833 | | |
| DRB5‡0105 | R1 | R2 | R12 | R15 | R18 | R20 | R26 | R27 | R30 | R36 | R38 | R44 | R49 | R50 | R52 | | | | |
| DRB5‡0104 | R1 | R2 | R12 | R15 | R18 | R20 | R26 | R27 | R30 | R36 | R38 | R43 | R47 | R50 | R52 | | | | |
| DRB5‡0106 | R1 | R2 | R12 | R15 | R18 | R20 | R26 | R27 | R30 | R35 | R41 | R43 | R49 | R50 | R53 | | | | |
| DRB5‡0107 | R1 | R2 | R12 | R15 | R18 | R20 | R26 | R27 | R30 | R35 | R38 | R44 | R49 | R50 | R52 | | | | |
| DRB5‡0109 | R1 | R2 | R12 | R15 | R18 | R20 | R26 | R27 | R30 | R36 | R42 | R44 | R49 | R50 | R52 | | | | |
| DRB5‡0108N | R1 | R2 | R12 | R13 | R18 | R20 | R23 | R27 | R30 | R36 | R38 | R44 | R49 | R50 | R52 | | | | |
| DRB5‡0102 | R1 | R2 | R12 | R13 | R18 | R20 | R23 | R27 | R30 | R36 | R38 | R44 | R49 | R50 | R52 | | | | |
| DRB5‡0110N | R1 | R2 | R12 | R13 | R18 | R20 | R23 | R27 | R30 | R36 | R38 | R44 | R49 | R50 | R52 | | | | |
| DRB5‡0103 | R1 | R2 | R12 | R13 | R18 | R20 | R23 | R27 | R30 | R36 | R31 | R44 | R49 | R50 | R52 | | | | |
| DRB5‡0202 | R1 | R2 | R12 | R13 | R18 | R20 | R23 | R27 | R30 | R35 | R41 | R43 | R49 | R50 | R53 | | | | |
| DRB5‡0203 | R1 | R2 | R12 | R13 | R18 | R20 | R23 | R27 | R30 | R35 | R41 | R43 | R49 | R50 | R52 | | | | |
| DRB5‡0204 | R1 | R2 | R12 | R13 | R18 | R20 | R23 | R27 | R30 | R36 | R41 | R43 | R49 | R50 | R53 | | 848 | | |
| DRB5‡0205 | R1 | R2 | R12 | R13 | R18 | R20 | R23 | R27 | R30 | R34 | R37 | R43 | R49 | R50 | R53 | | 848 | | |

**Table 28**

| Correspondence of the patchworks and SEQ ID NOs for DRB 3-5 | | | | | |
|---|---|---|---|---|---|
| Patchwork | SEQ ID NO: | | | | |
| R1 | 603 | 604 | 607 | | |
| R2 | 610 | 803 | | | |
| R3 | 611 | 804 | 805 | | |
| R4 | 800 | | | | |
| R5 | 801 | | | | |
| R6 | 802 | | | | |
| R7 | 620 | | | | |
| R8 | 621 | 810 | 812 | 813 | |
| R9 | 806 | | | | |
| R10 | 807 | | | | |
| R11 | 808 | | | | |
| R12 | 809 | 811 | | | |
| R13 | 814 | | | | |
| R14 | 815 | | | | |
| R15 | 816 | | | | |
| R16 | 817 | | | | |
| R17 | 818 | 819 | 820 | | |
| R18 | 643 | 821 | 822 | | |
| R19 | 823 | 824 | | | |
| R20 | 825 | 826 | 659 | | |
| R21 | 827 | 828 | 652 | 829 | |
| R22 | 673 | | | | |
| R23 | 668 | 678 | | | |
| R24 | 682 | 831 | | | |
| R25 | 676 | 680 | | | |
| R26 | 830 | | | | |
| R27 | 689 | 691 | | | |
| R28 | 701 | | | | |
| R29 | 702 | | | | |
| R30 | 703 | | | | |
| R31 | 704 | | | | |
| R32 | 837 | | | | |
| R33 | 838 | | | | |
| R34 | 726 | | | | |
| R35 | 727 | 730 | | | |
| R36 | 728 | 731 | | | |
| R37 | 733 | | | | |
| R38 | 736 | 744 | 839 | | |
| R39 | 737 | | | | |
| R40 | 746 | | | | |
| R41 | 739 | | | | |
| R42 | 1072 | | | | |
| R43 | 658 | 659 | 662 | 667 | 841 |
| R44 | 655 | 840 | | | |
| R45 | 774 | | | | |
| R46 | 775 | | | | |
| R47 | 772 | 842 | | | |
| R48 | 773 | | | | |
| R49 | 771 | 780 | | | |
| R50 | 784 | 787 | 788 | | |
| R51 | 785 | | | | |
| R52 | 792 | 850 | | | |
| R53 | 793 | 795 | 851 | | |

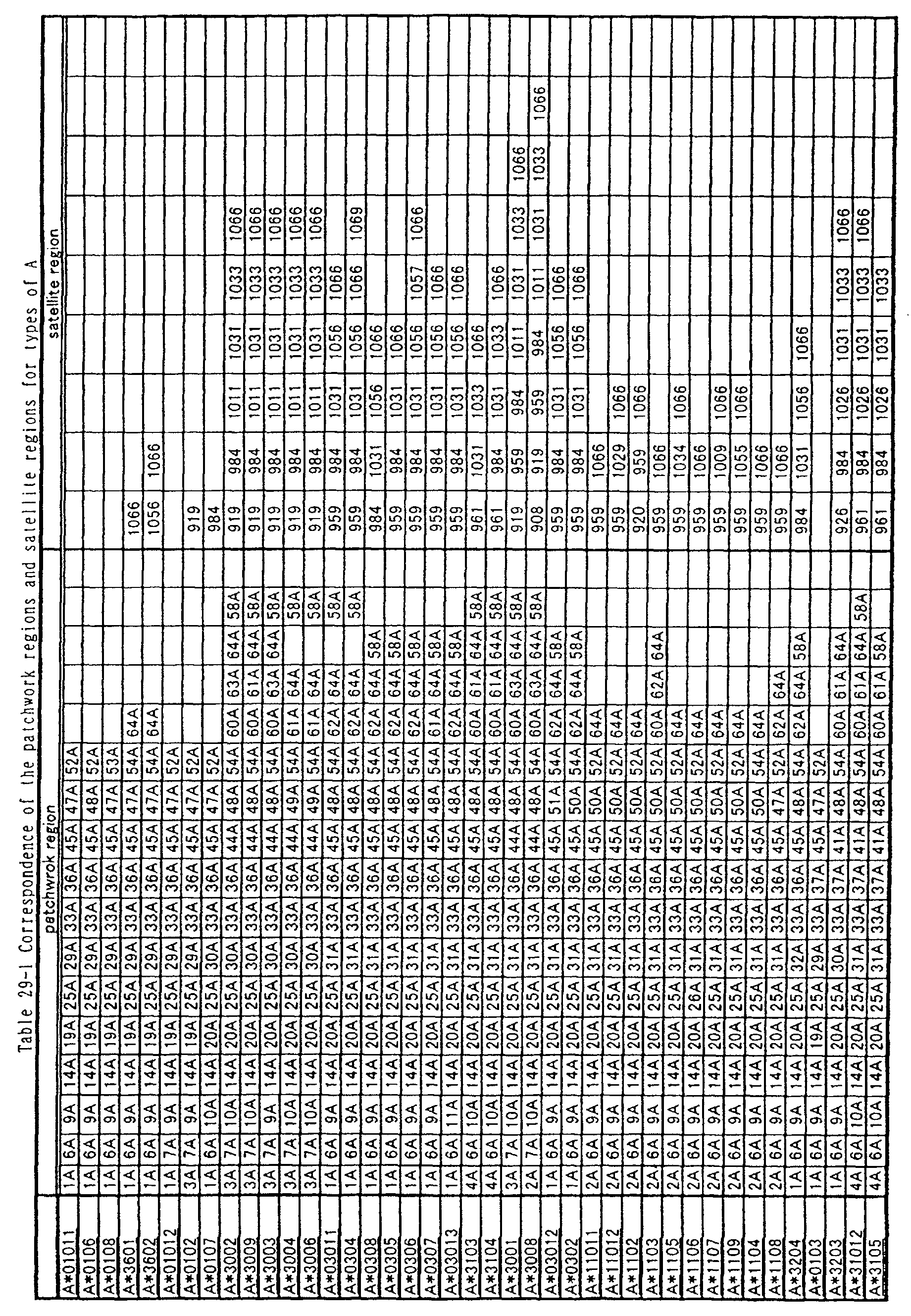

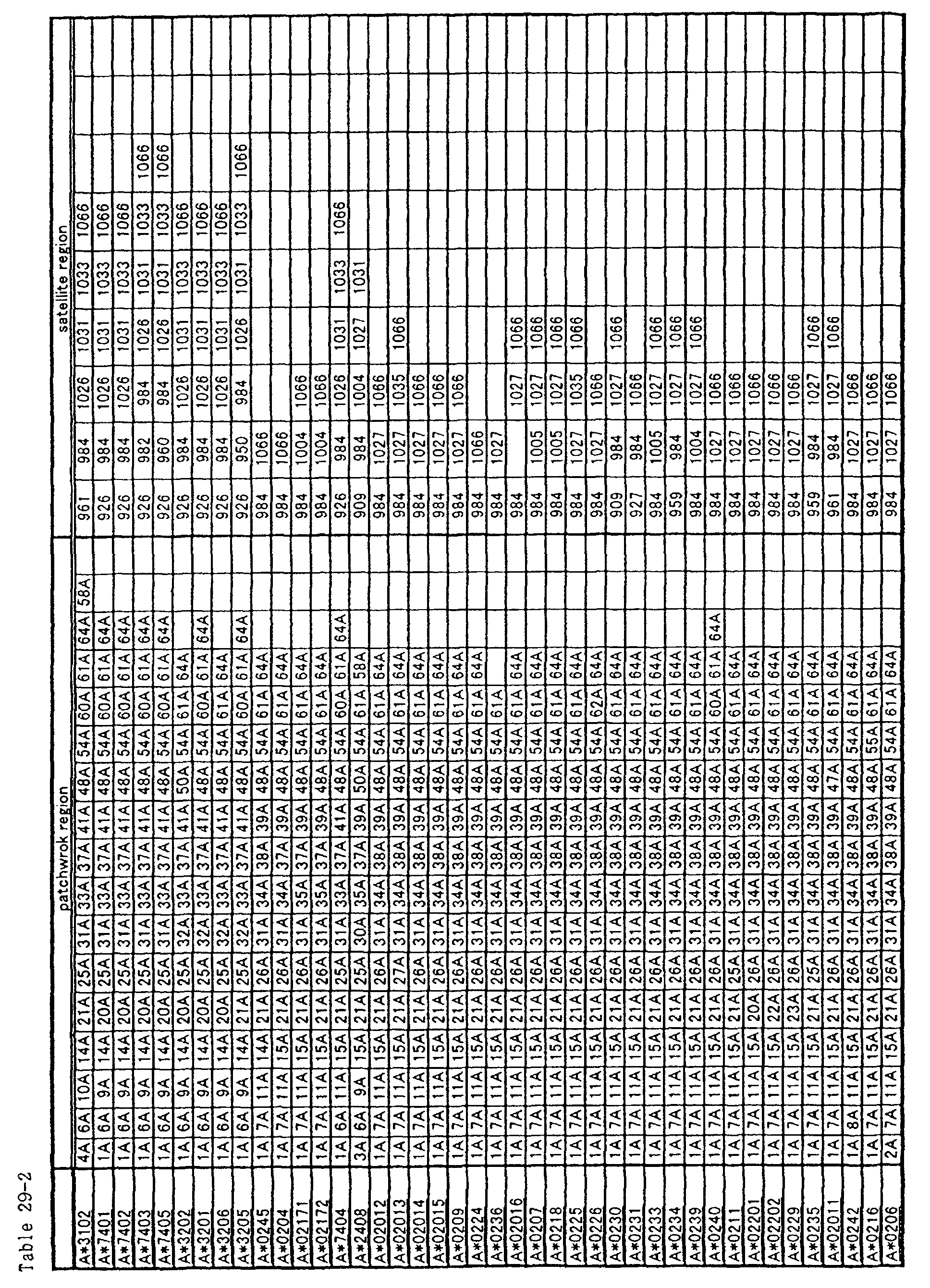

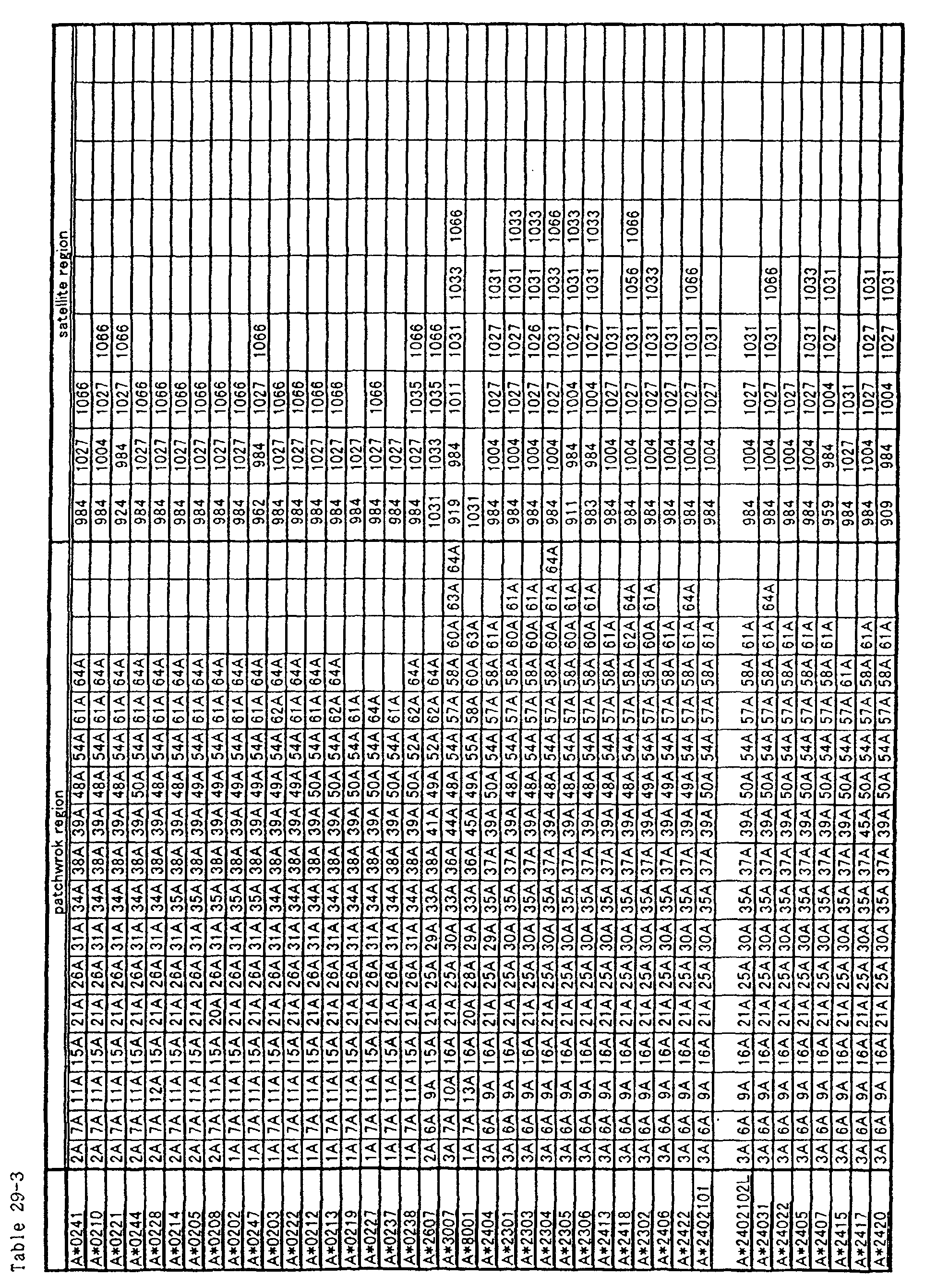

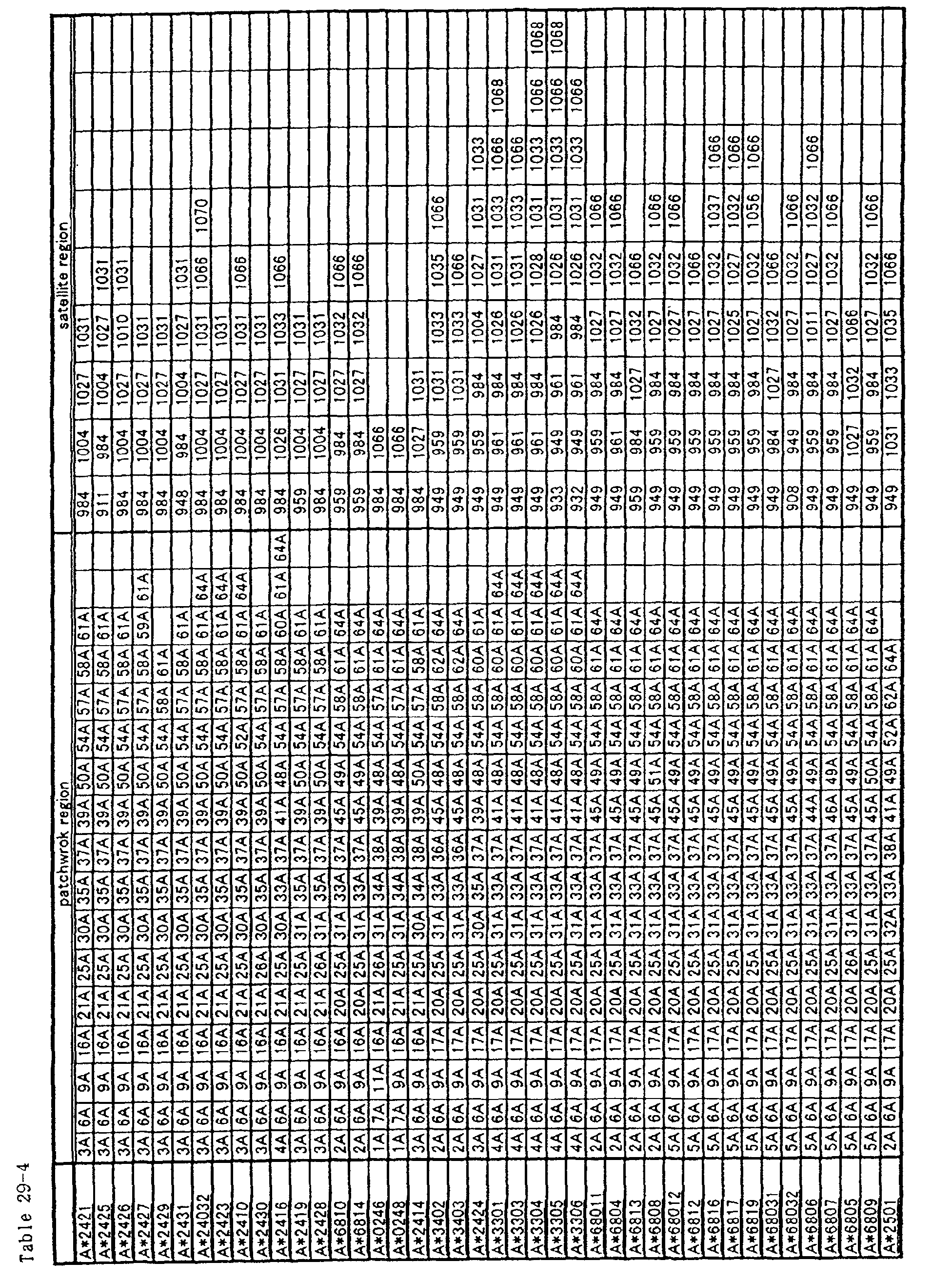

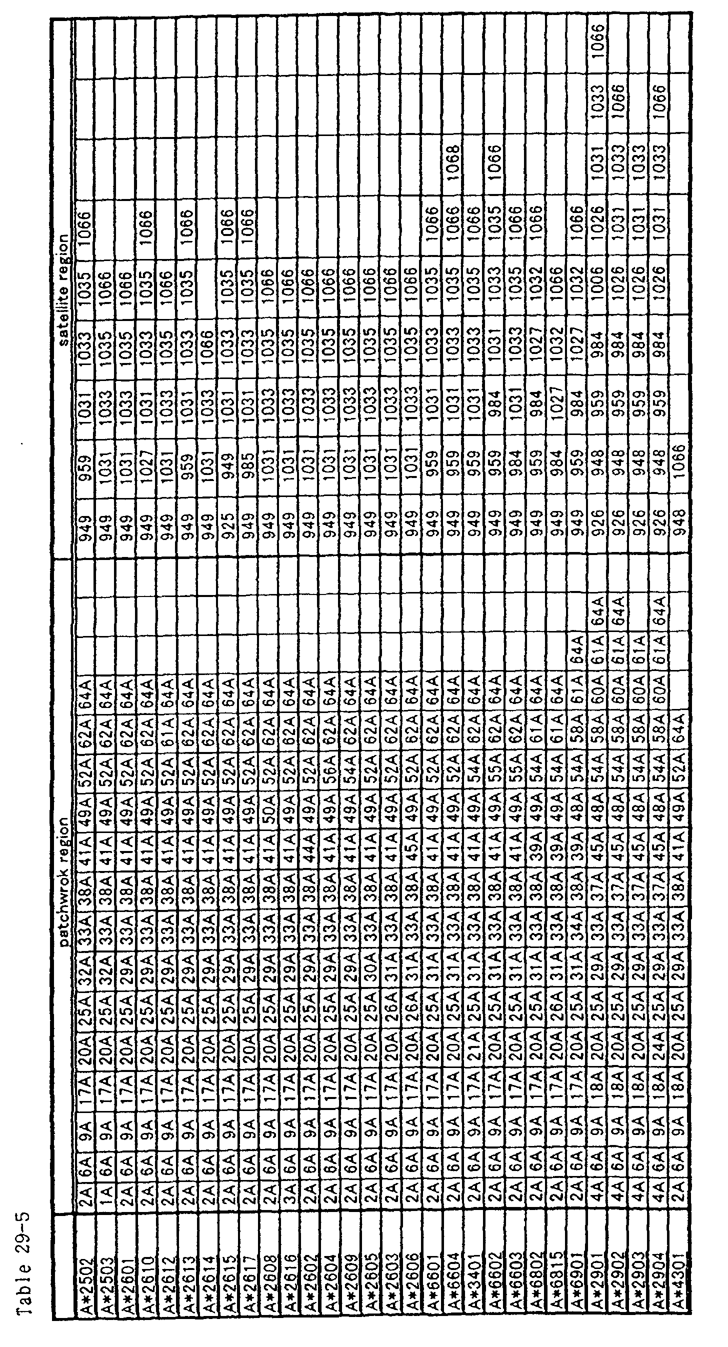

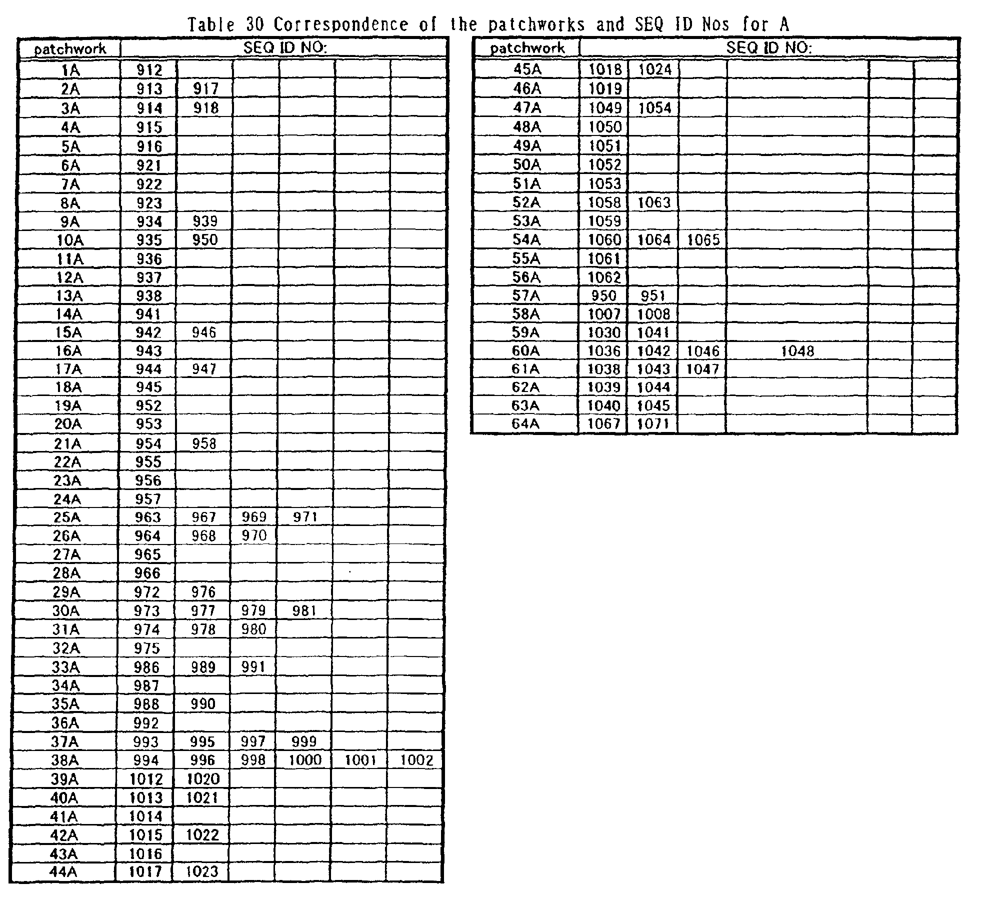

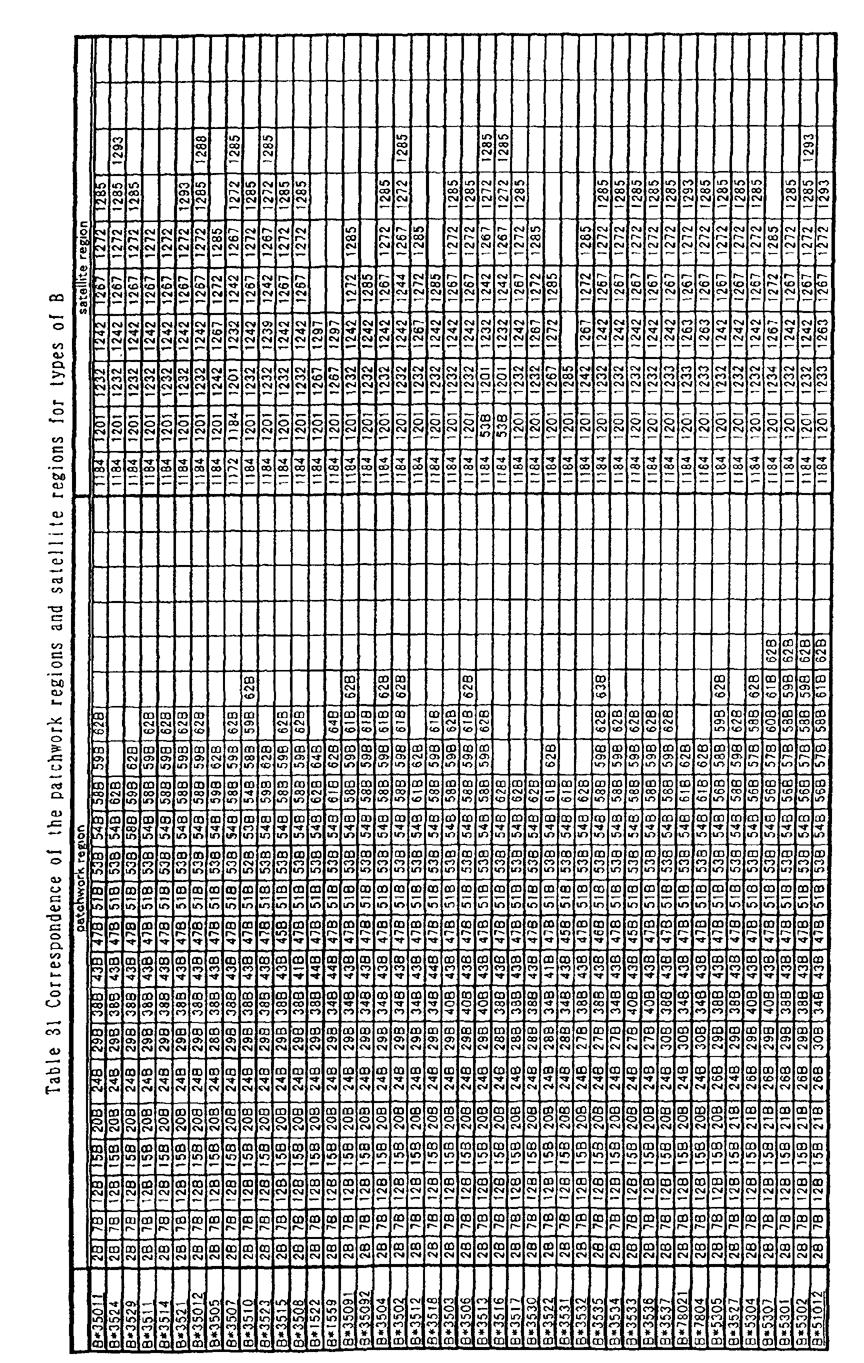

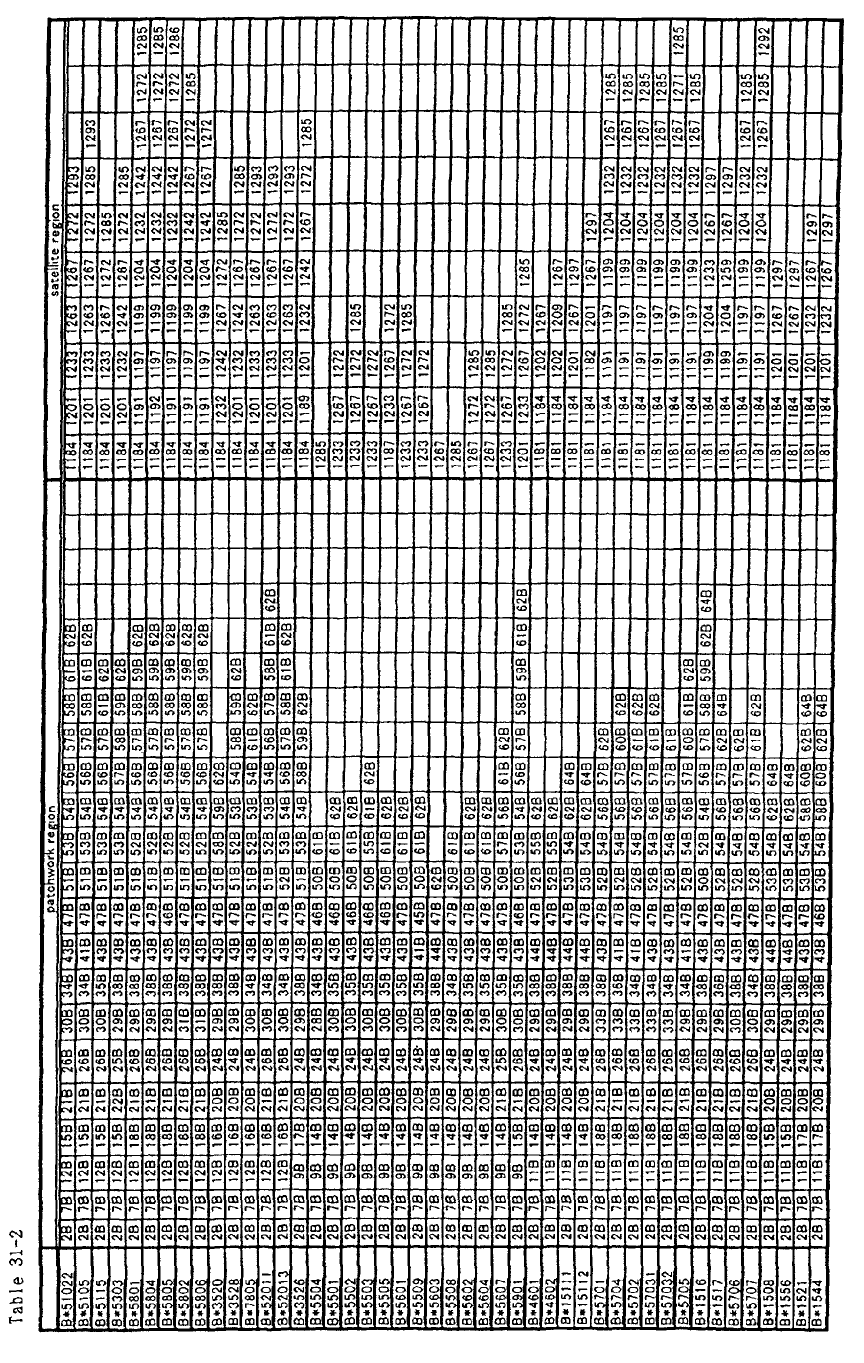

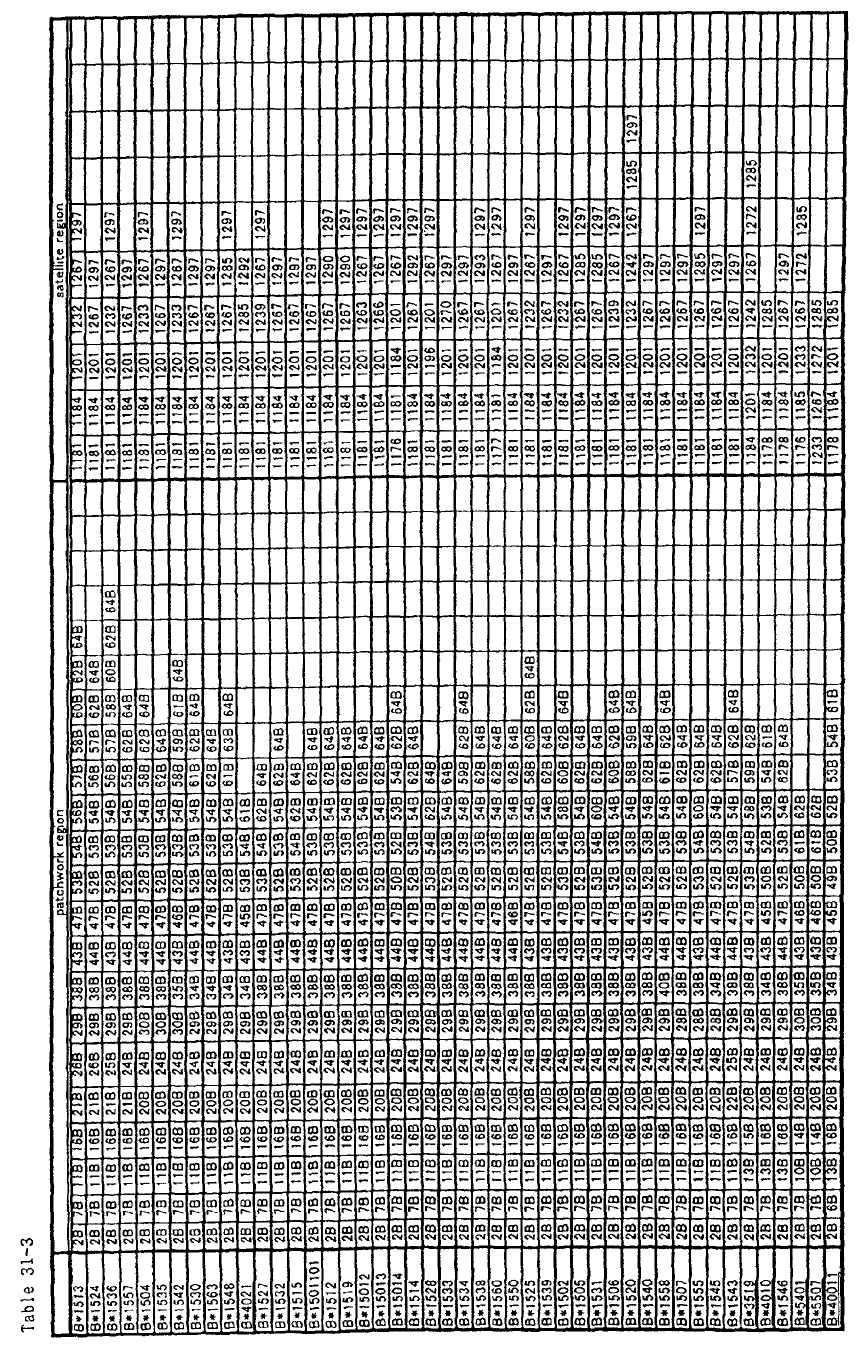

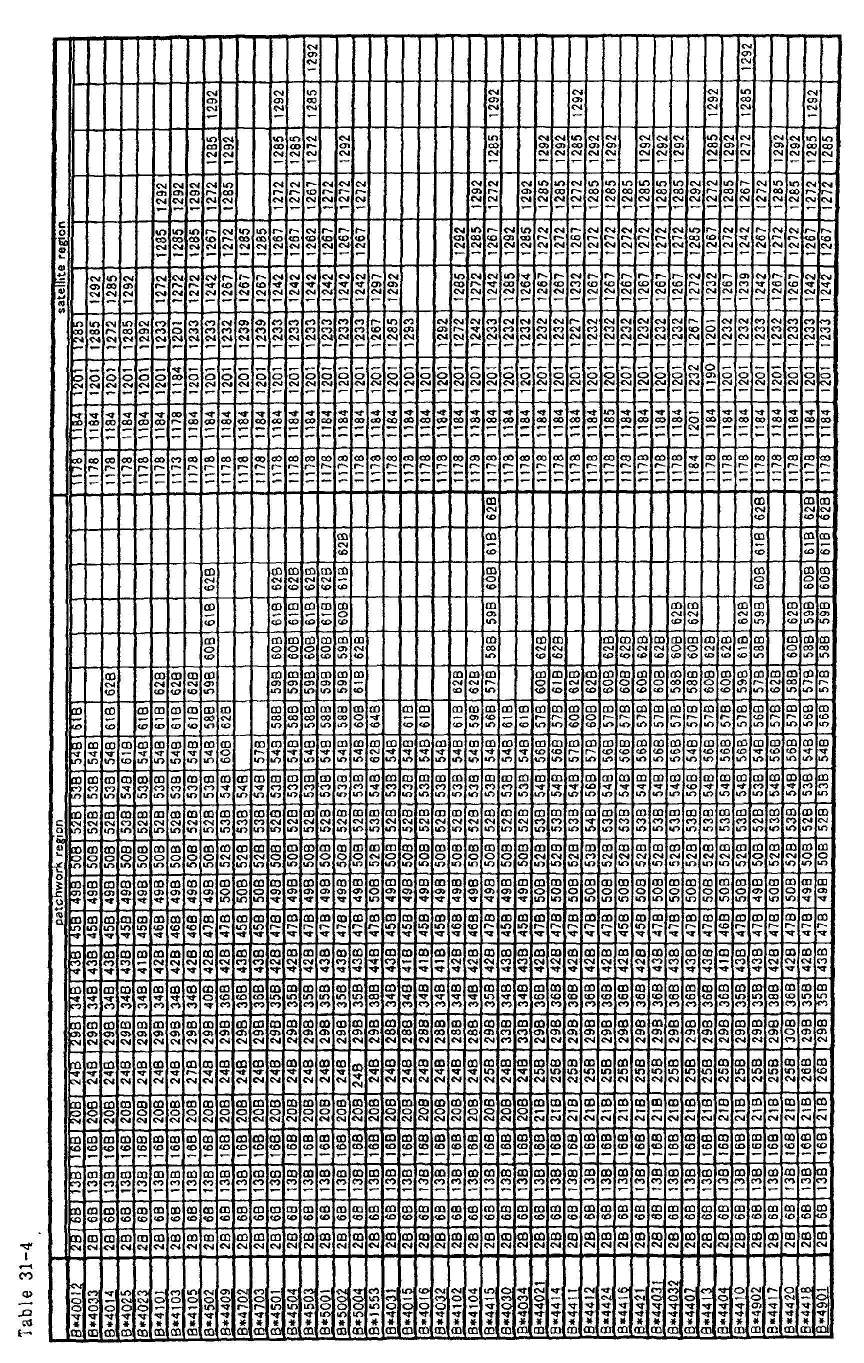

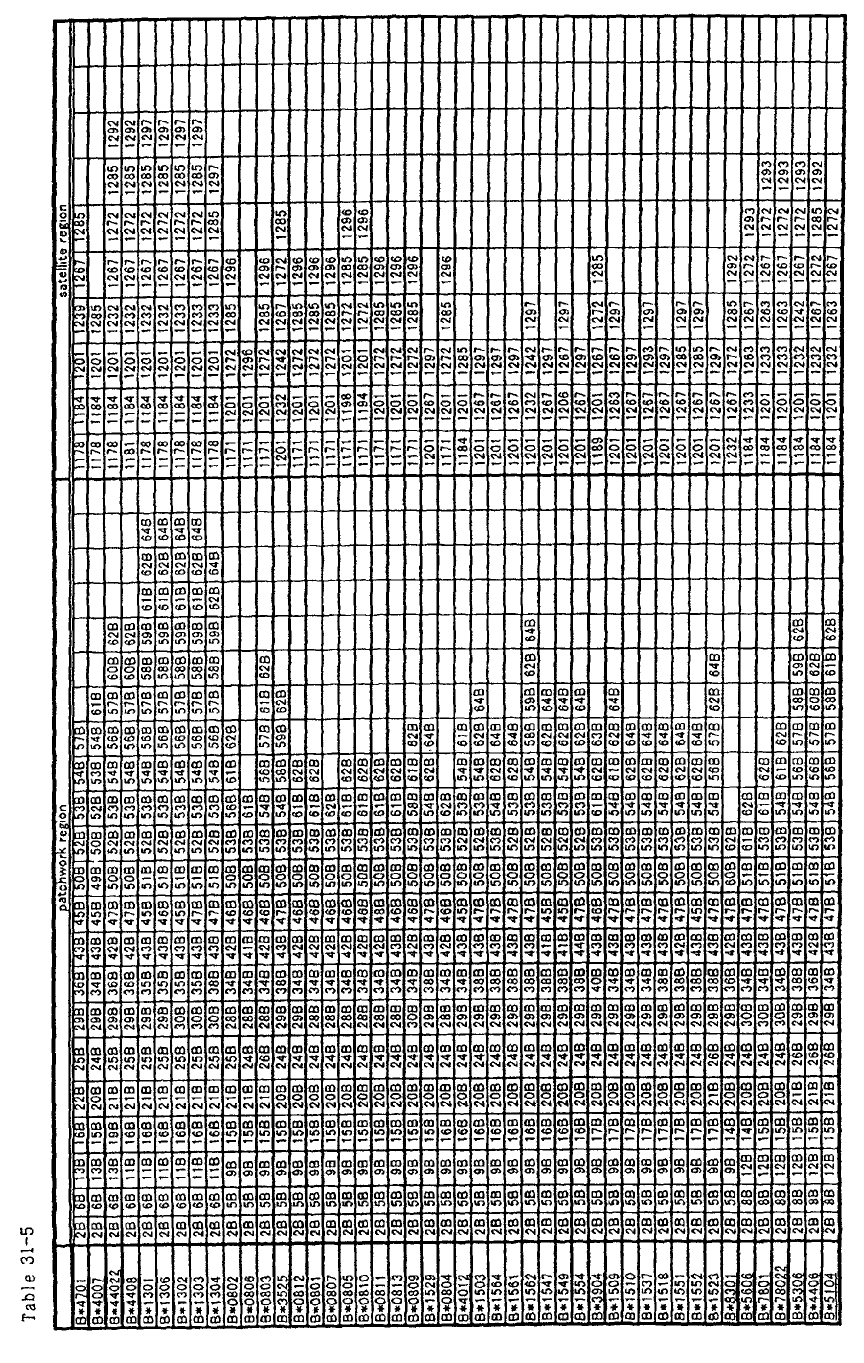

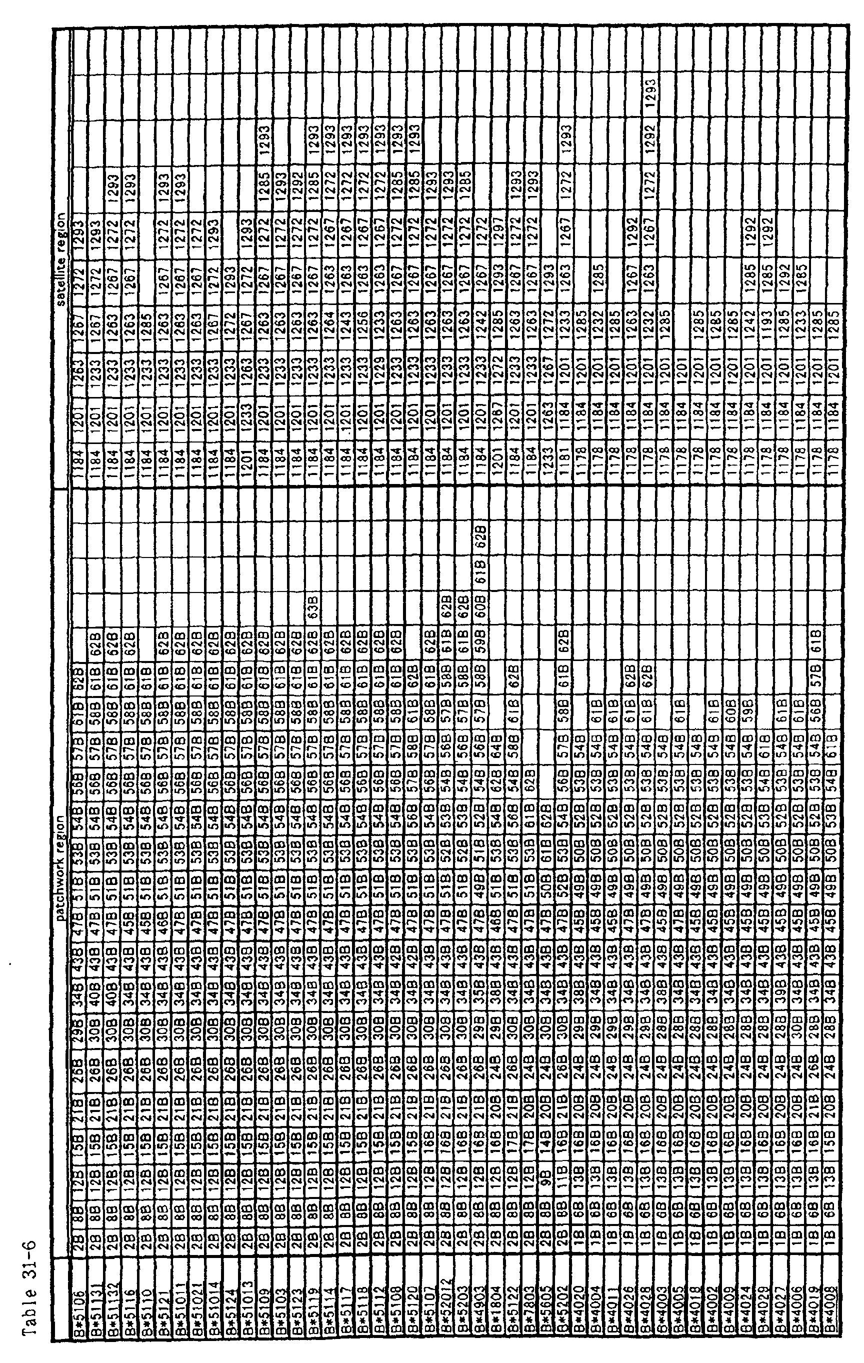

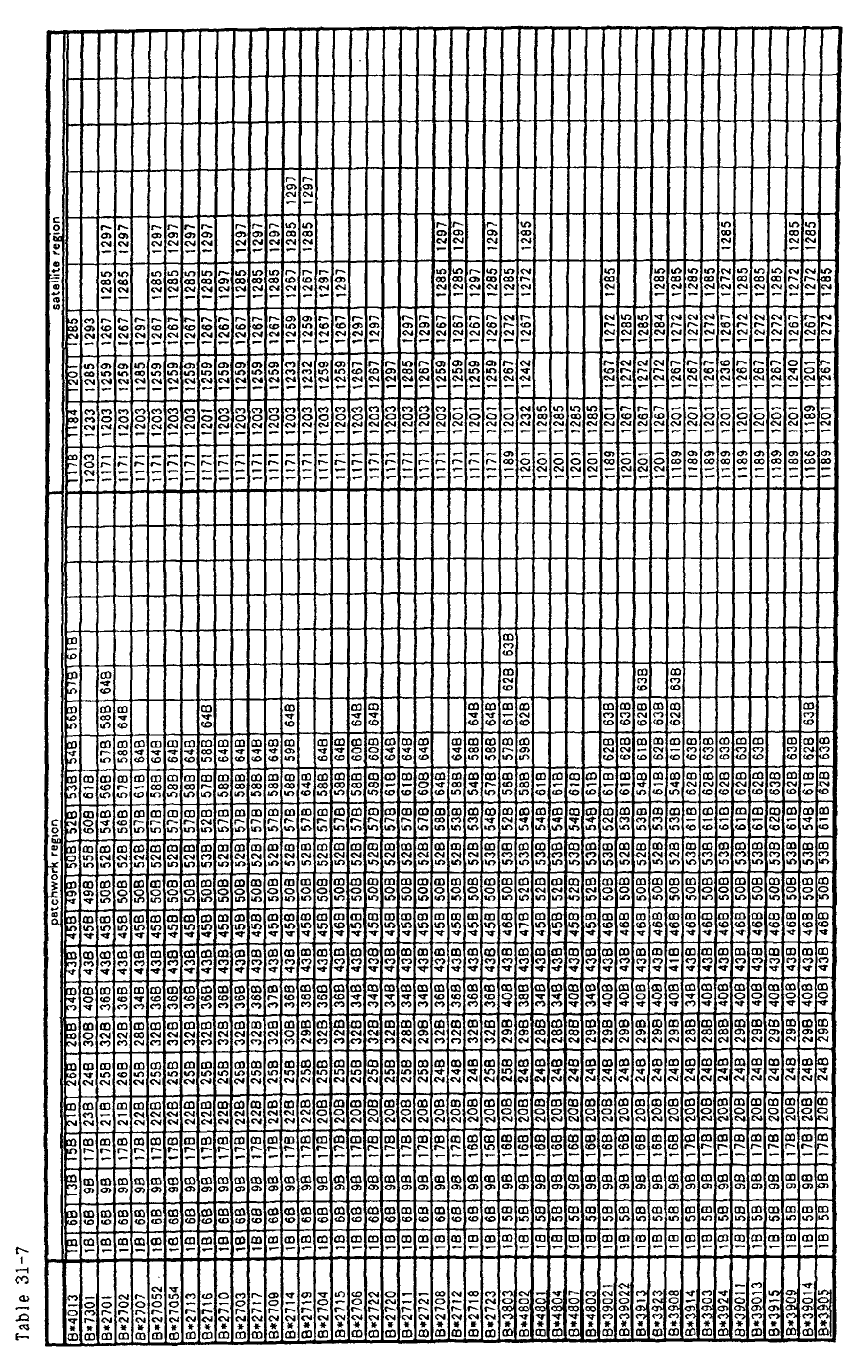

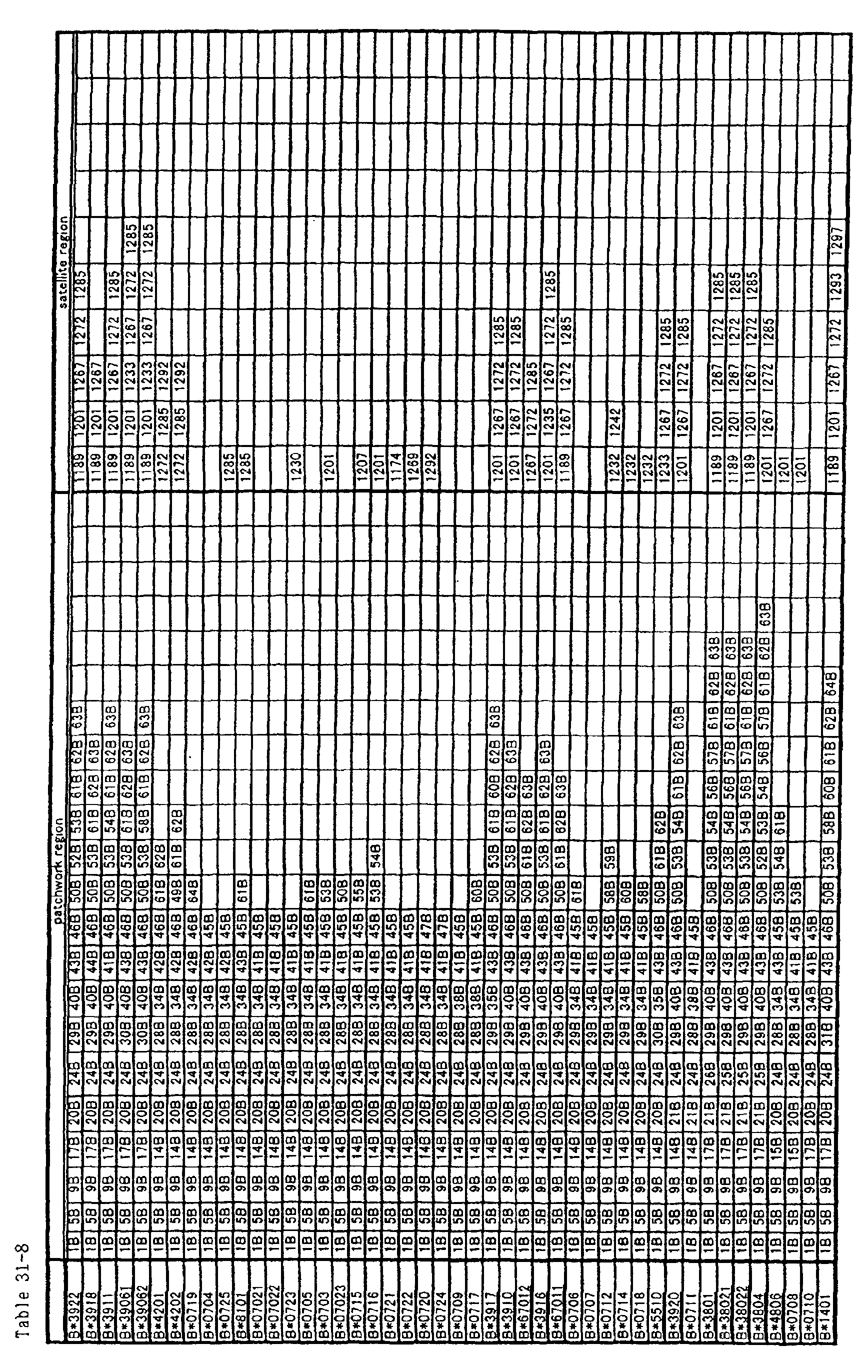

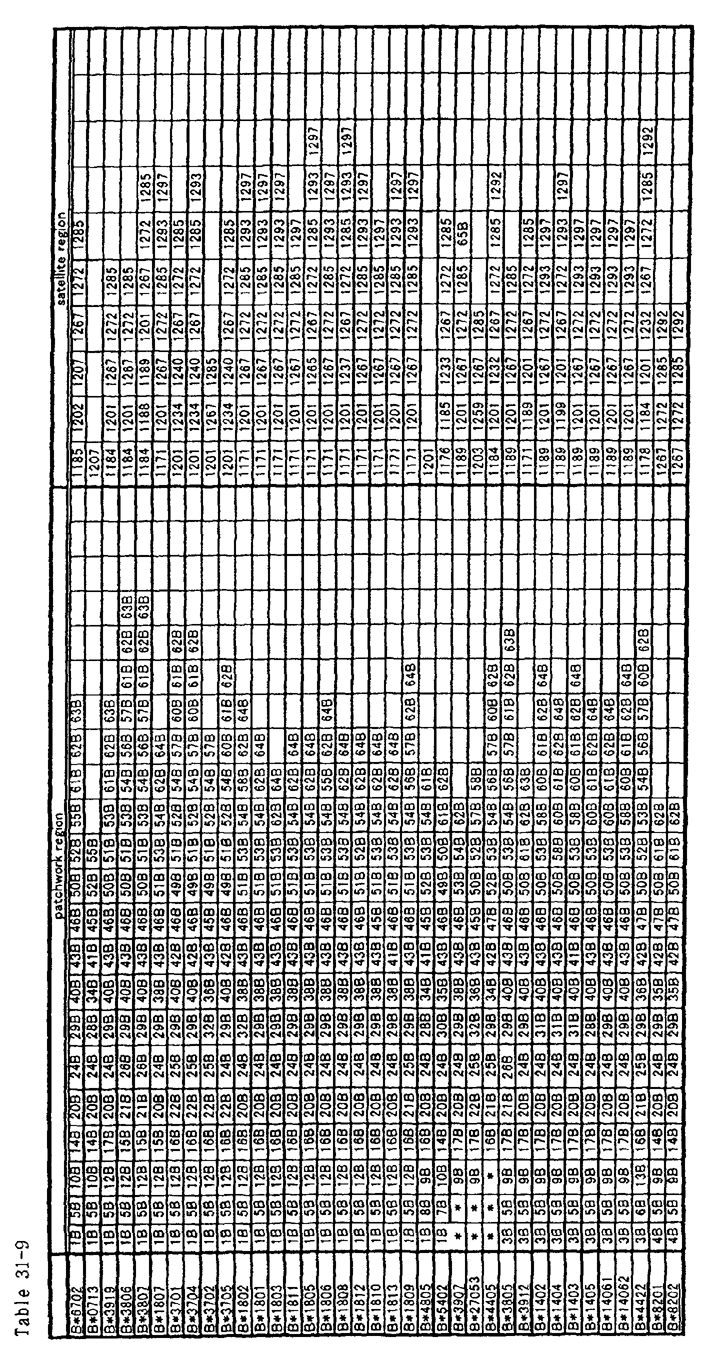

**Table 32**

| Correspondence of the patchworks and SEQ ID Nos for B | | | | | | | | | | |
|---|---|---|---|---|---|---|---|---|---|---|
| patchwork | SEQ ID NO: | | | | | | | | | |
| 1B | 1080 | | | | | | | | | |
| 2B | 1081 | | | | | | | | | |
| 3B | 1082 | | | | | | | | | |
| 4B | 1083 | | | | | | | | | |
| 5B | 1084 | | | | | | | | | |
| 6B | 1085 | 1088 | | | | | | | | |
| 7B | 1086 | | | | | | | | | |
| 8B | 1087 | | | | | | | | | |
| 9B | 1089 | 1094 | | | | | | | | |
| 10B | 1090 | | | | | | | | | |
| 11B | 1091 | 1095 | | | | | | | | |
| 12B | 1092 | 1096 | | | | | | | | |
| 13B | 1093 | | | | | | | | | |
| 14B | 1097 | 1103 | 1106 | | | | | | | |
| 15B | 1098 | 1104 | | | | | | | | |
| 16B | 1099 | | | | | | | | | |
| 17B | 1100 | 1105 | 1107 | 1108 | | | | | | |
| 18B | 1101 | | | | | | | | | |
| 19B | 1102 | | | | | | | | | |
| 20B | 1109 | 1113 | 1115 | | | | | | | |
| 21B | 1110 | 1114 | | | | | | | | |
| 22B | 1111 | | | | | | | | | |
| 23B | 1112 | | | | | | | | | |
| 24B | 1116 | 1120 | | | | | | | | |
| 25B | 1117 | 1121 | 1122 | | | | | | | |
| 26B | 1118 | | | | | | | | | |
| 27B | 1119 | | | | | | | | | |
| 28B | 1123 | 1129 | 1133 | | | | | | | |
| 29B | 1124 | 1130 | 1134 | 1135 | 1136 | 1137 | 1138 | 1139 | 1140 | 1141 |
| 30B | 1125 | 1131 | | | | | | | | |
| 31B | 1126 | | | | | | | | | |
| 32B | 1127 | 1132 | | | | | | | | |
| 33B | 1128 | | | | | | | | | |
| 34B | 1142 | 1149 | 1153 | 1156 | 1157 | | | | | |
| 35B | 1143 | 1150 | 1154 | | | | | | | |
| 36B | 1144 | 1151 | | | | | | | | |
| 37B | 1145 | | | | | | | | | |
| 38B | 1146 | | | | | | | | | |
| 39B | 1147 | | | | | | | | | |
| 40B | 1148 | 1152 | 1155 | | | | | | | |
| 41B | 1158 | 1162 | | | | | | | | |
| 42B | 1159 | | | | | | | | | |
| 43B | 1160 | 1163 | 1164 | | | | | | | |
| 44B | 1161 | | | | | | | | | |
| 45B | 1165 | | | | | | | | | |
| 46B | 1166 | | | | | | | | | |
| 47B | 1167 | 1169 | | | | | | | | |
| 48B | 1168 | | | | | | | | | |
| 49B | 1170 | 1179 | | | | | | | | |
| 50B | 1175 | 1180 | | | | | | | | |
| 51B | 1183 | 1212 | 1218 | 1222 | | | | | | |
| 528 | 1195 | 1213 | 1219 | 1223 | 1226 | | | | | |
| 53B | 1206 | 1215 | | | | | | | | |
| 54B | 1209 | 1216 | 1220 | 1224 | | | | | | |
| 55B | 1211 | 1217 | 1221 | 1225 | | | | | | |
| 56B | 1227 | 1246 | | | | | | | | |
| 57B | 1229 | 1247 | 1251 | 1253 | 1255 | 1256 | | | | |
| 58B | 1239 | 1249 | 1252 | 1254 | | | | | | |
| 59B | 1242 | 1250 | | | | | | | | |
| 60B | 1259 | 1274 | 1277 | | | | | | | |
| 61B | 1261 | 1275 | 1278 | 1279 | 1280 | 1281 | 1282 | 1283 | 1284 | |
| 62B | 1269 | 1276 | | | | | | | | |
| 63B | 1288 | 1295 | | | | | | | | |
| 64B | 1296 | 1299 | | | | | | | | |

**Table 33.**

| Correspondence of Primers | | |
|---|---|---|
| | SEQ ID NOS. | SEQ ID NOS. |
| Primers for DRB1 | 590, 591, 591 | 593 |
| Primers for DPB1 | 448, 449 | 450-455 |
| Primers for DQB1 | 504, 505 | 506 |
| Primers for A | 899, 900, 903-905 | 901, 902, 906, 907 |
| Primers for B | 1073, 1075-1077 | 1074, 1078, 1079 |

### [References]

(1) Kobayashi, T. et al.: The significance of HLA-DRB1 matching in clinical renal transplantation, Transplantation, 54: 238, 1992
(2) Olerup, O. et al.: HLA-DR typing by PCR amplification with sequence-specific primers (PCR-SSP) in 2 hours: An alternative to serological DR typing in clinical practice including donor-recipient matching in cadaveric transplantation, Tissue Antigen, 39: 225, 1992
(3) Inoko, H. et al.: PCR-RFLP, Handbook of HLA typing techniques (edited by Hui, K.M. & Bidwell, J.L.), CRC press, Florida, U.S.A., 1993, p.9
(4) Erlich, H. et al.: HLA-DR, -DQ and -DP typing using PCR amplification and immobilized probes, European Journal of Immunogenetics, 18: 33, 1991
(5) Carrington, M. et al.: Typing of HLA-DQA1 and DQB1 using DNA single-strand conformation polymorphism, Hum. Immunol., 33: 208, 1992

### Industrial Applicability

According to the present invention, there are provided a kit and method that are suitable for processing of a large number of specimens and enable a large number of high accuracy typing tests by one test for one specimen, and oligonucleotides and primers used therefor.

## Claims

1. A typing kit for determining HLA genotype of a test specimen by hybridization between a nucleic acid sequence derived from the test specimen and oligonucleotides, which comprises a substrate on which the oligonucleotides are immobilized through covalent bonds, wherein the oligonucleotides are 10-24 nucleotide length and are derived from sequences of a group of genes belonging to HLA class I or class II antigen on a human genome and each of the oligonucleotides includes polymorphism of each gene as alloantigen in the sequence.

2. The typing kit according to claim 1, wherein a surface of the substrate is coated with carbodiimlde groups or isocyanate groups and the covalent bonds are formed through reactions of the carbodiimide groups or isocyanate groups and linkers added to ends of the oligonucleotides.

3. The typing kit according to claim 1, wherein the linker is an amino group, or a compound having an amino group or a homopolymer of thymidine residues at an end of the compound.

4. The typing kit according to claim 1, wherein the oligonucleotides are immobilized in an area on a surface of the substrate having a size of 10-1,000 µm in diameter.

5. The typing kit according to claim 1, wherein the oligonucleotides consist ofDNA or a peptide nucleic acid.

6. The typing kit according to claim 1, wherein class I antigen is an antigen controlled by any of gene loci coding for HLA-A, HLA-B, HLA-C, HLA-D, HLA-E, HLA-F and HLA-G and class II antigen is an antigen controlled by any of gene loci coding for HLA-DQ, HLA-DR or HLA-DP.

7. The typing kit according to claim 1 or 6, wherein HLA-DQ is an antigen derived from any of DQA1, DQA2, DQB1 and DQB2 gene loci.

8. The typing kit according to claim 1 or 6, wherein HLA-DR is an antigen derived from any of DRA, DRB1, DRB3, DRB4 and DRB5 gene loci.

9. The typing kit according to claim 1 or 6, wherein HLA-DP is an antigen derived from any of DPA1, DPA2, DPB1 and DPB2 gene loci.

10. The typing kit according to claim 1 or 6, wherein the oligonucleotides contain at least one of the nucleic acid sequences of SEQ ID NOS: 1-397, 456-503, 507-589, 594-898, 908-1072 or 1080-1298.

11. The typing kit according to any one of claims 1, 6 and 7, which is for determining HLA-DQA1 genotype, and wherein the oligonucleotides contain at least one of the nucleic acid sequences of SEQ ID NOS: 1-54.

12. The typing kit according to any one of claims 1, 6 and 7, which is for determining HLA-DQB1 genotype, and wherein the oligonucleotides contain at least one of the nucleic acid sequences of SEQ ID NOS: 55-140 or 507-589.

13. The typing kit according to any one of claims 1, 6 and 8, which is for determining HLA-DRA genotype, and wherein the oligonucleotides contain at least one of the nucleic acid sequences of SEQ ID NOS: 141-144.

14. The typing kit according to any one of claims 1, 6 and 8, which is for determining genotypes of HLA-DRB1, HLA-DRB3, HLA-DRB4, HLA-DRB5, HLA-DRB6 or HLA-DRB7, and wherein the oligonucleotides contain at least one of the nucleic acid sequences of SEQ ID NOS: 145-323 or 594-853.

15. The typing kit according to any one of claims 1, 6 and 9, which is for determining HLA-DPA1 genotype, and wherein the oligonucleotides contain at least one of the nucleic acid sequences of SEQ ID NOS: 324-348.

16. The typing kit according to any one of claims 1, 6 and 9, which is for determining HLA-DPB1 genotype, and wherein the oligonucleotides contain at least one of the nucleic acid sequences of SEQ ID NOS: 349-397 or 456-503.

17. The typing kit according to claim 10, wherein at least one of the oligonucleotides is replaced with an oligonucleotide of 8-24 nucleotide length obtained by extending or shortening any of the nucleic acid sequences of SEQ ID NOS: 1-397, 456-503, 507-589, 594-898, 908-1072 or 1080-1298 for a gene sequence on the genome at 5' or 3' end or at both ends without eliminating or changing nucleotides associated with the gene polymorphism to have optimized binding affinity for the hybridization.

18. The typing kit according to any one of claims 1, 2, 4, 5 and 10-17, wherein at least one of the oligonucleotides is an oligonucleotide whose binding affinity for the hybridization is reduced by replacing an arbitrary nucleotide not involved in the gene polymorphism with a spacer compound.

19. The typing kit according to claim 18, wherein the spacer compound has a nucleic acid frame that does not have complementary binding property with any kind of nucleotides.

20. PCR primers for excising and amplifying a nucleic acid sequence associated with gene polymorphism of HLA class I antigen or class II antigen of a test specimen.

21. The PCR primers according to claim 20, which is for low accuracy genotyping, and wherein a combination of 5' primer and 3' primer corresponding to HLA-DQ consists of at least one of (SEQ ID NOS: 398 and 400) and (SEQ ID NOS: 399 and 400), and a combination of 5' primer and 3' primer corresponding to HLA-DR consists of at least one of (SEQ ID NOS: 401 and 403) and (SEQ ID NOS: 402 and 403).

22. The PCR primers according to claim 20, which is for high accuracy genotyping, and wherein a combination of 5' primer and 3' primer corresponding to HLA-DQB1 consists of at least one of (SEQ ID NOS: 404 and 406), (SEQ ID NOS: 405 and 406), (SEQ ID NOS: 407 and 409), (SEQ ID NOS: 408 and 409), (SEQ ID NOS: 410 and 412) and (SEQ ID NOS: 411 and 412), and a combination of 5' primer and 3' primer corresponding to HLA-DRB1 consists of at least one of (SEQ ID NOS: 413 and 417), (SEQ ID NOS: 414 and 417), (SEQ ID NOS: 415 and 417) and (SEQ ID NOS: 416 and 417).

23. The PCR primers according to claim 20, which is for high accuracy genotyping, and wherein a combination of 5' primer and 3' primer corresponding to HLA-DQA1 consists of at least one of (SEQ ID NOS: 418 and 420) and (SEQ ID NOS: 419 and 420), a combination of 5' primer and 3' primer corresponding to HLA-DQB1 is (SEQ ID NOS: 421 and 422), a combination of 5' primer and 3' primer corresponding to HLA-DRA is (SEQ ID NOS: 423 and 424), a combination of 5' primer and 3' primer corresponding to HLA-DRB2 consists of at least one of (SEQ ID NOS: 425 and 428), (SEQ ID NOS: 426 and 428) and (SEQ ID NOS: 427 and 428), a combination of 5' primer and 3' primer corresponding to HLA-DRB3 consists of at least one of (SEQ ID NOS: 429 and 431) and (SEQ ID NOS: 430 and 431), a combination of 5' primer and 3' primer corresponding to HLA-DRB4 consists of at least one of (SEQ ID NOS: 432 and 433) and (SEQ ID NOS: 434 and 435), a combination of 5' primer and 3' primer corresponding to HLA-DRB5 is (SEQ ID NOS: 436 and 437), a combination of 5' primer and 3' primer corresponding to HLA-DRB6 consists of at least one of (SEQ ID NOS: 438 and 439) and (SEQ ID NOS: 439 and 440), and
a combination of 5' primer and 3' primer corresponding to HLA-DRB7 is (SEQ ID NOS: 441 and 442), a combination of 5' primer and 3' primer corresponding to HLA-DPA1 is (SEQ ID NOS: 443 and 444) and a combination of 5' primer and 3' primer corresponding to HLA-DPB1 consists of at least one of (SEQ ID NOS: 445 and 446) and (SEQ ID NOS: 445 and 447).

24. A method for determining HLA genotype of a test specimen, which comprises allowing hybridization of the oligonucleotides on the substrate of the typing kit according to any one of claims 1-16 with a nucleic acid sequence derived from the specimen and detecting occurrence of hybridization of the oligonucleotides and the nucleic acid sequence derived from the specimen.

25. The method for determining HLA genotype of a test specimen according to claim 24, comprising the steps of:
carrying out low accuracy typing of HLA genotype of the specimen by performing first PCR amplification using the primers for low accuracy genotyping according to claim 21 and a nucleic acid sequence derived from the specimen as a template, allowing hybridization of the amplification product with the oligonucleotides contained in the typing kit according to any one of claims 1-16, and detecting occurrence of hybridization of the nucleic acid sequence derived from the specimen and each of the oligonucleotides; and
carrying out high accuracy HLA genotyping of the specimen by performing second PCR amplification based on the above determination result using primers appropriately selected from the primers for high accuracy genotyping according to claim 22 and a nucleic acid sequence derived from the specimen as a template, allowing hybridization of the amplification product with the oligonucleotides contained in the typing kit according to any one of claims 1-16, and detecting occurrence of hybridization of the nucleic acid sequence derived from the specimen and each of the oligonucleotides.

26. The method for determining HLA genotype of a test specimen according to claim 24, wherein PCR amplification is performed by using a nucleic acid sequence derived from the test specimen as a template and the primers for high accuracy genotyping according to claim 24 (22 or 23?) and the amplification product is hybridized with each of the oligonucleotides contained in the typing kit according to any one of claims 1-16.

27. The method for determining HLA genotype of a test specimen according to claim 24, which comprises preparing alignment of gene sequences of each HLA, and setting a nucleotide sequence in which at least two nucleotide polymorphisms or sequence polymorphisms involving the HLA typing are observed in a nucleotide sequence consisting of 1-10 nucleotides as a patchwork segment, and/or setting a nucleotide sequence in which at least one nucleotide polymorphism or sequence polymorphism is observed as a satellite segment, and
finding the patchworks and/or the satellites in gene sequences of all of the HLA genes, performing typing by combination of the patchworks and/or the satellites and judging whether each of HLA typing of the test specimen is homozygote or heterozygote together with determining the types of HLA.

28. The method for determining HLA genotype of a test specimen according to claim 27, wherein a pseudogene of DRB1 is included in the HLA types to be determined.

29. The typing kit according to claim 1 or 6, wherein the oligonucleotides comprises at least one of the nucleotide sequences of SEQ ID NOS: 908-1071, and the kit is for determining HLA-A genotype.

30. The typing kit according to claim 1 or 6, wherein the oligonucleotides comprises at least one of the nucleotide sequences of SEQ ID NOS: 1080-1298, and the kit is for determining HLA-DRA genotype.
